(19) Europäisches Patentamt
European Patent Office
Office européen des brevets

(11) EP 3 128 015 A2

(12) EUROPEAN PATENT APPLICATION

(43) Date of publication:
**08.02.2017 Bulletin 2017/06**

(51) Int Cl.:
***C12Q 1/68*** *(2006.01)* ***C12N 15/00*** *(2006.01)*
***A61K 39/21*** *(2006.01)*

(21) Application number: **16189709.5**

(22) Date of filing: **28.05.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **29.05.2007 US 932020 P**
**05.06.2007 US 933133 P**
**08.06.2007 US 933670 P**
**14.01.2008 US 6449 P**
**25.03.2008 US 64761 P**

(62) Document number(s) of the earlier application(s) in accordance with Art. 76 EPC:
**08769761.1 / 2 164 993**

(71) Applicant: **Reid, Christopher B.**
**Los Angeles, CA 90012 (US)**

(72) Inventor: **Reid, Christopher B.**
**Los Angeles, CA 90012 (US)**

(74) Representative: **Lapienis, Juozas et al**
**MSP Europe UAB**
**21-92 Seimyniskiu Str.**
**09236 Vilnius (LT)**

Remarks:
This application was filed on 20.09.2016 as a divisional application to the application mentioned under INID code 62.

# (54) A METHOD FOR PROVIDING A DESIRED CELL POPULATION CAPABLE OF FURTHER DIFFERENTIATION IN VIVO

(57) The claimed invention is directed towards the generation of pluripotent, multipotent, and/or self-renewing cells which are capable of beginning to differentiate in culture into a variety of cell types and capable of further differentiation in vivo. The claimed invention is also directed towards the generation of desirable, differentiating cell populations transplantable to patients, genetic modification of endogenous cells, and the treatment of patients suffering from diseases that may be ameliorated by these methods. This invention also provides methods for preventing, treating, or retarding disease related to immunodeficiency virus (e.g. HIV-I, HIV-2, SIV, FIV, etc.) infection.

EP 3 128 015 A2

## Description

## BACKGROUND OF THE INVENTION

The transcendent challenge for medicine in the 21st century will be replacing damaged, worn-out or genetically-compromised cells. Transcription factors binding specifically to DNA play a vital role in regulating gene expression. It is the particular complement of transcription factors within an individual cell, that determine which cellular programs are active and which are turned off. In this capacity transcription factors play a decisive role in determining and maintaining cellular identity, as well as determining cellular vulnerability.

## SUMMARY OF THE INVENTION

The ability to derive proliferating, self-renewing, multipotent and pluripotent cell population(s) from otherwise non-pluripotent, non-self renewing cells may have significant positive implications for all fields utilizing cellular therapies. These fields include bone marrow transplantation, transfusion medicine, and gene therapy and enable the production of patient-specific stem cells and other desired cell types. Likewise, the ability to initiate differentiation of cells into neural, muscle, and various other desirable cell populations is and will also be of significant value to medicine and commercial processes involving animals. Accordingly, the present invention provides methods for genetic production and uses of multipotent cell populations, pluripotent cell populations, neuronal cell populations, muscle cell populations, and other desired cell populations such as, for example, HIV resistant cell populations.

It is a proposition of the present invention that the efficient introduction or overexpression of specific transcription factors, alone or in combination with other cell fate determinants (such as notch, numb and numblike), enables the interconversion of what have been considered transitory (multipotent, pluripotent, and/or self-renewing) or fixed (differentiated or somatic) cellular phenotypes. The ability to reliably induce phenotypic conversion or cellular reprogramming allows the production of stem cells, replacement cells, tissues, and organs that match individual patients. In conjunction with gene therapy techniques and cell culture techniques, cell type interconversion also provides for the production of disease-resistant and genetically-repaired cells that are suitable for transplantation.

It is an object of this invention to provide various manners of generating proliferating, self-renewing, multipotent and/or pluripotent cell population(s), as well as other desirable cell populations, from either dividing or non-dividing cells without the use of oncogenes. Differentiating cell populations comprise cells expressing some, but not all markers associated with specific cell type categorization. It is disclosed herein that appropriate Numb isoform expression in combination with other transgenes (especially transcription factors) enables the production of dividing, pluripotent cell populations or differentiating cell populations. Moreover, the genetic vectors of the present invention may be used to produce genetic modification (e.g. expression of gene products deficient in the patient) and to transiently or permanently induce proliferation, self-renewal, or stem/progenitor cell behavior in endogenous cells in vivo, particularly those cells found in tissues which normally do not show or no longer show such behavior. Finally, other genetic vectors of the present invention may be used to produce genetic modification and/or to block proliferation, self-renewal, or stem/progenitor cell behavior in cells aberrantly displaying such behavior (e.g. cancer cells). It is also an object of the present invention to provide therapeutic vectors and cells capable of expressing synthetic oligonucleotide sequences predicted to attenuate disease processes. For example, the current invention discloses the use of synthetic oligonucleotides to reduce gene expression critical HIV and other immunodeficiency virus infection, propagation and spread.

The invention may be used with any suitable cells, including vertebrate cells, and including fish, mammalian, avian, amphibian, and reptilian cells.

## BRIEF DESCRIPTION OF THE DRAWING

Figure 1. A schematized vector map corresponding to the vector sequence of Example 13.

## DETAILED DESCRIPTION

All patents, patent applications, and publications cited in this application are hereby incorporated by reference herein in their entireties.

As discussed herein, "DNA" refers to deoxyribonucleic acid and "RNA" refers to ribonucleic acid. As discussed herein, "cDNA" refers to complementary DNA; "mRNA" refers to messenger RNA; "siRNA" refers to small interfering RNA; "shRNA" refers to small hairpin RNA; "miRNA" refers to microRNA, such as single-stranded RNA molecules, typically about 20-30 nucleotides in length, which may regulate gene expression; "decoy" and "decoy RNA" and "RNA decoy" refer to an RNA molecule that mimics the natural binding domain for a ligand.

As used herein, the meaning of the term "ameliorating" includes lessening an effect, or reducing damage, or minimizing the effect or impact of an action, activity, or function, and includes, for example, lessening the deleterious effects of a disease or condition.

As used herein, the meaning of the term "retarding" includes slowing or lessening the progress of an effect or action, and includes, for example, slowing the progress of a disease, slowing the rate of infection, or otherwise acting to slow or reduce the advance or progress of a disease or condition.

As used herein, an "inducing agent" is an agent that aids or is alone effective to promote an action. For example, an exogenous agent that affects a promoter, e.g., by initiating or enhancing its activity, and so affects expression of a gene under control of the promoter, may be termed an inducing agent. For example, tetracycline may be used as an inducing agent; and doxycycline may be used as an inducing agent.

A nucleic acid sequence (e.g., a nucleic acid seqeuence encoding a polypeptide) is termed "operably linked" to another nucleic acid sequence (e.g., a promoter) when the first nucleic acid sequence is placed in a functional relationship with the second nuceleic acid sequence. For example, a promoter is operably linked to a coding sequence if the promoter affects the transcription or expression of the coding sequence. As used herein, the term "driven by" refers to a gene or coding sequence that is operably linked to a promoter sequence, and that the promoter sequence affects the transcription or expression of the coding sequence.

As used herein, a "marker" is a molecule that is detectable, or codes for a detectable molecule, or acts on other molecules so that the presence of the marker is detectable. A "marker protein" or "marker polypeptide" is a protein or polypeptide that is detectable in a laboratory or clinical environment, and, in embodiments, may be detectable by eye. A "marker gene" encodes a marker protein or marker polypeptide.

As used herein, "HIV" refers to human immunodeficiency virus, and includes variants such as, e.g., HIV-1, HIV-2. Other immunodeficiency viruses include simian immunodeficiency virus (SIV) and feline immunodeficiency virus (FIV). Enzymes related to HIV may be termed "HIV enzymes" and include, for example,\integrase, protease, reverse transcriptase, and transactivating regulatory protein (TAT).

Infection by HIV is believed to involve receptors termed "HIV receptors." There may be multiple such receptors, some of which may be termed "HIV co-receptors." As discussed herein, HIV co-receptors include CXCR4 and CCR5.

A theoretical basis for the embodiments of the invention is described herein, however, this discussion is not in any way to be considered as binding or limiting on the present invention. Those of skill in the art will understand that the various embodiments of the invention may be practiced regardless of the model used to describe the theoretical underpinnings of the invention.

In a preferred embodiment, cells are "selected" from accessible, dividing or non-dividing cell populations for the purpose of generating the desired a) proliferating, multipotent or pluripotent cell population, differentiating b) populations of neuronal cells c) muscle cells, d) and/or any other desired cell population; moreover the desired cell population may be capable of further differentiation in vitro, in vivo, and/or tissue-appropriate and regionally-appropriate differentiation in vivo.

**Sources of cells selected for use in the invention:**

Selected cells may include any cell practicable in the present invention. Cells selected for use in the present invention (herein termed "selected cells") may originate as endogenous cells of the patient --including cells derived from other organ systems; or from exogenous sources (including those derived from cell lines, cryopreserved sources, banked sources, and donors). Cells may also be selected from cells genetically-modified with synthetic or natural nucleic acid sequences. The term "selected cells" as used herein does not include human embryonic stem cells.

In embodiments of the present invention, in order that they may be isolated without the involvement of invasive procedures, selected cells will preferably be easily accessible cells (e.g. peripheral blood leukocytes, circulating hematopoietic stem cells, epithelial cells (e.g. buccal cheek cells (e.g. Michalczyk et al., 2004)), adipose tissue (e.g. Gimble et al., 2007; Ma et al., 2007), umbilical cord blood cells (e.g. Zhao, et al., 2006; Tian et al., 2007), etc.). However, bone marrow stem cells, spermatogonia (e.g. Guan et al., 2006; Takahashi et al., 2007), primordial germ cells (PGCs), stem cells isolated from amniotic membranes (e.g. Hancheran et al., 2007), amniotic fluid (e.g. De Coppi et al., 2007), as well as cells isolated from the skin (e.g. Tumbar, 2006; Dunnwald et al., 2001; Szudal'tseva et al., 2007), etc., are also covered by the present invention. Such cells can be isolated from the tissues in which they reside by any means known to the art.

Spermatogonia cells can be isolated using a two-step enzymatic digestion followed by Percoll separation. Cells can then be resuspended in minimum essential medium (MEM) supplemented with bovine serum albumin to a final concentration of $10^6$/mL. In detail: Tubule fragments are accessed surgically and teased apart prior to treatment with 1 mg/ml trypsin, hyaluronidase, and collagenase, and then 1 mg/ml hyaluronidase and collagenase, in MEM containing 0.10% sodium bicarbonate, 4 mM L-glutamine, nonessential amino acids, 40 microgram/ml gentamycin, 100 IU to 100 microgram/ml penicillin-streptomycin, and 15 mM HEPES. Spermatogonia cells are further separated from tubule fragments by centrifugation at 30 times gravity. After filtration through nylon filters with 77- and/or 55-micron pore sizes, cells are collected and loaded onto a discontinuous Percoll density gradient. Fractions with a purity greater than 40% progenitor/stem/spermatogonia cells are washed and resuspended to a concentration of cells equivalent to $10^6$ progenitor/stem/spermatogonia cells per ml. Afterwards cells are cultured and/or stored by any cryopreservation technique known to the art.

The selected cells may be genetically-modified cells, especially cells that have been genetically modified by any means known to the art, to encode therapeutic or commercially useful deoxyribonucleic acid (DNA) or ribonucleic acid (RNA) sequences.

In accordance with an aspect of the present invention, there is provided a method of producing a desired cell population (e.g. pluripotent, neuronal, muscle, etc.) from the selected cells.

Achieving multipotent, pluripotent, and/or self renewing cell populations:

In order to achieve a) a population of proliferating, self renewing pluripotent cells, the selected cell(s) and/or their progeny are transfected with nucleotide sequence(s) including those encoding the "long" (PRR insert +) isoform(s) of the mammalian numb gene. At about the same time the selected cells may also be transfected with synthetic oligonucleotides targeting the short Numb isoforms and Numblike, then cultured under conditions which promote growth of the selected cells at an optimal growth rate. Selected cells are maintained under these conditions for the period of time sufficient to achieve the desired cell number.

The cells are grown at the (optimal) rate of growth achieved by incubation with LIF, steel factor, and/or equipotent concentrations of Il-6, hyper IL-6, IL-7, oncostatin-M and/or cardiotrophin-1; or that growth rate achieved in the presence of other growth enhancing cytokines (e.g. those conditions described for culturing pluripotent cells e.g. Guan et al., 2006). The growth rate is determined from the doubling times of the selected cells in said growth culture medium. Likewise, culture conditions such as those described in US Patents 6432711 and 5453357 may also be suitable for the propagation and expansion, at an optimal growth rate, of cells transfected with the long (PRR+) Numb isioform(s). Other appropriate protocols and reference cytokine concentrations have been taught by Koshimizu et al., 1996; Keller et al., 1996; Piquet-Pellorce, 1994; Rose et al., 1994; Park and Han, 2000; Guan et al., 2006; Dykstra et al., 2006; Zhang et al., 2007). However the practice of the present invention is not limited to the details of these teachings.

In a preferred embodiment, the selected cells are cultured in a standard growth medium (e.g. Minimal Essential Medium with or without supplements (e.g. glutamine, and beta.-mercaptoethanol). The medium may include basic fibroblast growth factor (bFGF), steel factor, leukemia inhibitory factor (LIF), and/or factors with LIF activity (e.g. LIF, LIF receptor (LIFR), ciliary Neurotrophic factor (CNTF), oncostatin M (OSM), OSM receptor (OSMR), cardiotrophin, interleukins (IL) such as IL-6, hyper IL-6, GP130, etc.) as well as horse serum. LIF, as well as other factors with LIF activity, prevents spontaneous differentiation of the cells. Under these conditions, selected cells transfected with the PRR+ Numb isoform(s) and their progeny are expected to achieve multipotency, pluripotency and/or self-renewal.

In a preferred embodiment, the selected cell(s) and/or their progeny are transfected with nucleotide sequence(s) encoding the "long" (PRR insert +) Numb isoform(s) as well as sequences encoding other transgenes. Many of those transgenes are listed below along with their corresponding identification numbers (accession numbers) in the NCBI sequence database.

In another preferred embodiment, the selected cell(s) and/or their progeny are transfected with nucleotide sequence(s) encoding a portion of the "long" (PRR insert +) Numb isoform(s) as well as sequences encoding other transgenes. Many of those transgenes are listed below along with their corresponding identification (accession) numbers (codes) in the NCBI sequence database.

In a preferred embodiment, the selected cells and/or their progeny are transfected with long (PRR+) Numb isoform encoding sequences as well as sequences encoding other transgenes, including LIF.

In a preferred embodiment, the selected cells and/or their progeny are transfected with long (PRR+) Numb isoform encoding sequences as well as sequences encoding other transgenes, including ones with LIF activity.

In a preferred embodiment, the selected cells and/or their progeny are transfected with long (PRR+) Numb isoform(s) encoding sequences as well as sequences encoding other transgenes, including the LIFR.

In a preferred embodiment, the selected cells and/or their progeny are transfected with long (PRR+) Numb isoform(s) encoding sequences as well as sequences encoding other transgenes, including oncostatin M (OSM).

In a preferred embodiment, the selected cells and/or their progeny are transfected with long (PRR+) Numb isoform(s) encoding sequences as well as sequences encoding other transgenes, including oncostatin M receptor (OSMR).

In a preferred embodiment, the selected cells and/or their progeny are transfected with long (PRR+) Numb isoform(s) encoding sequences as well as sequences encoding other transgenes, including cardiotrophin-1.

In a preferred embodiment, the selected cells and/or their progeny are transfected with long (PRR+) Numb isoform(s) encoding sequences as well as sequences encoding other transgenes, including CNTF.

In a preferred embodiment, the selected cells and/or their progeny are transfected with long (PRK+) Numb isoform(s) encoding sequences as well as sequences encoding other transgenes, including OCT3/4 and SOX2.

In a preferred embodiment, the selected cells and/or their progeny are transfected with long (PRR+) Numb isoform(s) encoding sequences as well as sequences encoding other transgenes, including NANOG, OCT3/4 and SOX2.

In a preferred embodiment, the selected cells and/or their progeny are transfected with long (PRR+) Numb isoform(s) encoding sequences as well as sequences encoding other transgenes, including OCT3/4 and SOX2 and a transgene with LIF activity.

In a preferred embodiment, the selected cells and/or their progeny are transfected sequences encoding other transgenes, including OCT3/4 and SOX2 and a transgene with LIF activity.

In a preferred embodiment, the selected cells and/or their progeny are transfected with long (PRR+) Numb isoform(s) encoding sequences as well as sequences encoding other transgenes, including Notch (e.g. Gaiano et al., 2000).

In a preferred embodiment, the selected cells and/or their progeny are transfected with long (PRR+) Numb isoform(s) encoding sequences as well as sequences encoding other transgenes, including OCT3/4, SOX2 and Notch (e.g. notch 1 and/or notch 2).

In a preferred embodiment, the selected cells and/or their progeny are transfected with long (PRR+) Numb isoform(s) encoding sequences as well as sequences encoding other transgenes, including OCT3/4, SOX2, NANOG, and Notch.

In a preferred embodiment, the selected cells and/or their progeny are transfected with long (PRR+) Numb isoform(s) encoding sequences as well as sequences encoding other transgenes, including OCT3/4, SOX2, NANOG, and a transgene with LIF activity.

In a preferred embodiment, the selected cells and/or their progeny are transfected with long (PRR+) Numb isoform(s) encoding sequences as well as sequences encoding other transgenes, including OCT3/4, SOX2, NANOG, and multiple transgenes with LIF activity.

In a preferred embodiment, the selected cells and/or their progeny are transfected with long (PRR+) Numb isoform(s) encoding sequences as well as sequences encoding other transgenes, including OCT3/4, Notch, HOXB4 and SOX2.

Over time, other gene combinations differing from those described herein may be described or discovered capable of causing cells to become multipotent, pluripotent, capable of self-renewal, or to begin differentiating. However this patent application covers such "genetic reprogramming" of any nucleated cell utilizing nucleic acid or protein electroporation (see Gagne et al., 1991; Saito et al., 2001; Yuan, 2008; Huang et al., 2007; Xia and Zhang, 2007; Cemazar and Sersa 2007; Isaka and Imai, 2007; Luxembourg et al., 2007; Van Tendeloos, 2007; Takahashi, 2007; etc.), liposomes, nanocapsules, nanovaults, etc. (see Goldberg et al., 2007; Li et al., 2007), and/or another approach avoiding viral integration or other random alteration of the cell's genome, as such means increase safety and efficiency.

Excluded, of course, from the category of random alteration are approaches involving gene-targeting and site-directed methods designed to introduce or remove DNA at specific locations in the genome.

Likewise, this patent application covers the genetic reprogramming of any nucleated cell utilizing nucleic acid or protein electroporation, liposomes, nanocapsules, nanovaults, etc., and/or another approach avoiding retroviral/lentiviral integration or other random alteration of the cell's genome, as such means increase safety and efficiency. Such approaches and methods include all known to the art and practicable in the present invention.

In a preferred embodiment, nucleic acid(s) or protein(s) corresponding to a single gene, or portion thereof, (particularly those named herein, discovered according to methods described herein, discovered according to other published methods; or known to be multipotency, pluripotency, or self-renewal inducing) are the only nucleic acid(s) or protein(s) overexpressed and/or introduced to produce multipotent, pluripotent, and/or self-renewing cells from the selected cells.

In a preferred embodiment, nucleic acid(s) or protein(s) corresponding to a single gene, or portion thereof, (particularly those named herein, discovered according to methods described herein, discovered according to other published methods; or known to be multipotency, pluripotency, or self-renewal inducing) are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) can be utilized in concert with the nucleic acid(s) or protein(s) corresponding to a single gene, or portion thereof, (particularly those named herein, discovered according to methods described herein, discovered according to other published methods; or known to be multipotency, pluripotency, or self-renewal inducing) so long as a population of multipotent. pluripotent, and/or self-renewing cells is produced from the selected cells.

In a separate preferred embodiment, other nucleic acid(s) or protein(s) can be utilized in concert with the nucleic acid(s) or protein(s) corresponding to a single gene, or portion thereof, (particularly those named herein, discovered according to methods described herein, discovered according to other published methods; or known to be multipotency, pluripotency, or self-renewal inducing) so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a preferred embodiment, nucleic acid(s) or protein(s) corresponding to Nanog are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) can be utilized in concert with the nucleic acid(s) or protein(s) corresponding to Nanog so long as a population of multipotent, pluripotent, and/or self-renewing cells is produced from the selected cells and the method utilized is electroporation, liposomes, nanocapsules, nanovaults, and/or another approach avoiding viral integration or other random alteration of the cell's genome.

In a separate preferred embodiment, nucleic acid(s) or protein(s) corresponding to Oct4 and Sox2 are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding viral integration or other random alteration of the cell's genome.

In a separate preferred embodiment, other nucleic acid(s) or protein(s) are utilized in concert with the nucleic acid(s) or protein(s) corresponding to Oct4/Sox2 so long as a population of multipotent, pluripotent, and/or self-renewing cells is produced from the selected cells and the method utilized is electroporation, liposomes, nanocapsules, nanovaults, and/or another approach avoiding viral integration or other random alteration of the cell's genome.

In a separate preferred embodiment, nucleic acid(s) or protein(s) corresponding to Long (PRR+) Numb isoforms are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding viral integration or other random alteration of the cell's genome.

In a separate preferred embodiment, other nucleic acid(s) or protein(s) are utilized in concert with the nucleic acid(s) or protein(s) corresponding to Long (PRR+) Numb isoforms so long as a population of multipotent, pluripotent, and/or self-renewing cells is produced from the selected cells and the method is electroporation, liposomes, nanocapsules, nanovaults, and/or another approach avoiding viral integration or other random alteration of the cell's genome.

In a preferred embodiment, nucleic acid(s) or protein(s) corresponding to Nanog are the only nucleic acid(s) or protein(s) overexpressed and/or introduced to produce multipotent, pluripotent, and/or self-renewing cells from the selected cells.

In a preferred embodiment, nucleic acid(s) or protein(s) corresponding to Nanog are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) can be utilized in concert with the nucleic acid(s) or protein(s) corresponding to Nanog so long as a population of multipotent, pluripotent, and/or self-renewing cells is produced from the selected cells.

In a separate preferred embodiment, other nucleic acid(s) or protein(s) can be utilized in concert with the nucleic acid(s) or protein(s) corresponding to Nanog so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a preferred embodiment, nucleic acid(s) or protein(s) corresponding to a gene with LIF activity are the only nucleic acid(s) or protein(s) overexpressed and/or introduced to produce multipotent, pluripotent, and/or self-renewing cells from the selected cells.

In a preferred embodiment, nucleic acid(s) or protein(s) corresponding to a gene with LIF activity are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) can be utilized in concert with the nucleic acid(s) or protein(s) corresponding to to a gene with LIF activity so long as a population of multipotent, pluripotent, and/or self-renewing cells is produced from the selected cells.

In a separate preferred embodiment, other nucleic acid(s) or protein(s) can be utilized in concert with the nucleic acid(s) or protein(s) corresponding to a gene with LIF activity so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a preferred embodiment, nucleic acid(s) or protein(s) corresponding to Oct4 are the only nucleic acid(s) or protein(s) overexpressed and/or introduced to produce multipotent, pluripotent, and/or self-renewing cells from the selected cells.

In a preferred embodiment, nucleic acid(s) or protein(s) corresponding to Oct4 are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) can be utilized in concert with the nucleic acid(s) or protein(s) corresponding to Oct4 so long as a population of multipotent, pluripotent, and/or self-renewing cells is produced from the selected cells.

In a separate preferred embodiment, other nucleic acid(s) or protein(s) can be utilized in concert with the nucleic acid(s) or protein(s) corresponding to Oct4 so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a preferred embodiment, nucleic acid(s) or protein(s) corresponding to Sox2 are the only nucleic acid(s) or protein(s) overexpressed and/or introduced to produce multipotent, pluripotent, and/or self-renewing cells from the selected cells.

In a preferred embodiment, nucleic acid(s) or protein(s) corresponding to Sox2 are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding rctroviral/lentiviral integration or other random alteration of the cell's genome.

In a separate preferred embodiment, other nucleic acid(s) or protein(s) can be utilized in concert with the nucleic acid(s) or protein(s) corresponding to Sox2 so long as a population of multipotent, pluripotent, and/or self-renewing cells is produced from the selected cells.

In a separate preferred embodiment, other nucleic acid(s) or protein(s) can be utilized in concert with the nucleic acid(s) or protein(s) corresponding to Sox2 so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a preferred embodiment, nucleic acid(s) or protein(s) corresponding to lin28 are the only nucleic acid(s) or protein(s) overexpressed and/or introduced to produce multipotent, pluripotent, and/or self-renewing cells from the selected cells.

In a preferred embodiment, nucleic acid(s) or protein(s) corresponding to lin28 are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) can be utilized in concert with the nucleic acid(s) or protein(s) corresponding to lin28 so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a preferred embodiment, nucleic acid(s) or protein(s) corresponding to c-myc are the only nucleic acid(s) or protein(s) overexpressed and/or introduced to produce multipotent, pluripotent, and/or self-renewing cells from the selected cells.

In a preferred embodiment, nucleic acid(s) or protein(s) corresponding to c-myc are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) can be utilized in concert with the nucleic acid(s) or protein(s) corresponding to c-myc so long as a population of multipotent, pluripotent, and/or self-renewing cells is produced from the selected cells.

In a separate preferred embodiment, other nucleic acid(s) or protein(s) can be utilized in concert with the nucleic acid(s) or protein(s) corresponding to c-myc so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a separate preferred embodiment, nucleic acid(s) or protein(s) corresponding Oct4 and Sox2 are the only nucleic acid(s) or protein(s) overexpressed and/or introduced to produce multipotent, pluripotent, and/or self-renewing cells from the selected cells.

In a separate preferred embodiment, nucleic acid(s) or protein(s) corresponding to Oct4 and Sox2 are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) are utilized in concert with the nucleic acid(s) or protein(s) corresponding to Oct4 and Sox2 so long as a population of multipotent, pluripotent, and/or self-renewing cells is produced from the selected cells.

In a separate preferred embodiment, other nucleic acid(s) or protein(s) are utilized in concert with the nucleic acid(s) or protein(s) corresponding to Oct4 and Sox2 so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a separate preferred embodiment, nucleic acid(s) or protein(s) corresponding to Long (PRR+) Numb isoforms are the only nucleic acid(s) or protein(s) overexpressed and/or introduced to produce multipotent, pluripotent, and/or self-renewing cells from the selected cells.

In a separate preferred embodiment, nucleic acid(s) or protein(s) corresponding to Long (PRR+) Numb isoforms are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) are utilized in concert with the nucleic acid(s) or protein(s) corresponding to Long (PRR+) Numb Isoforms so long as a population of multipotent, pluripotent, and/or self-renewing cells is produced from the selected cells.

In a separate preferred embodiment, other nucleic acid(s) or protein(s) are utilized in concert with the nucleic acid(s) or protein(s) corresponding to Long (PRR+) Numb Isoforms so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a separate preferred embodiment, nucleic acid(s) or protein(s) corresponding to Oct4, Sox2, and Nanog are the only nucleic acid(s) or protein(s) overexpressed and/or introduced to produce multipotent, pluripotent, and/or self-renewing cells from the selected cells.

In a separate preferred embodiment, nucleic acid(s) or protein(s) corresponding to Oct4, Sox2, and Nanog are the only nucleic acid(s) or protein(s) overexpressed and/or introduced to produce multipotent, pluripotent, and/or self-renewing cells from the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) are utilized in concert with the nucleic acid(s) or protein(s) corresponding to Oct4, Sox2, and Nanog so long as a population of multipotent, pluripotent, and/or self-renewing cells is produced from the selected cells.

In a separate preferred embodiment, other nucleic acid(s) or protein(s) are utilized in concert with the nucleic acid(s) or protein(s) corresponding to Oct4, Sox2, and Nanog so long as a population of multipotent, pluripotent, and/or self-renewing cells is produced from the selected cells and the method utilized is electroporation, liposomes, nanocapsules, nanovaults, and/or another approach avoiding retroviral/lentiviral integration or other random alteration of the cell's genome.

In a separate preferred embodiment, nucleic acid(s) or protein(s) corresponding to Long (PRR+) Numb isoforms are the only nucleic acid(s) or protein(s) overexpressed and/or introduced to produce multipotent, pluripotent, and/or self-renewing cells from the selected cells.

In a separate preferred embodiment, nucleic acid(s) or protein(s) corresponding to Long (PRR+) Numb isoforms are the only nucleic acid(s) or protein(s) overexpressed and/or introduced to produce multipotent, pluripotent, and/or self-renewing cells from the selected cells and the method is electroporation, liposomes, nanocapsules, nanovaults, and/or another approach avoiding retroviral/lentiviral integration or other random alteration of the cell's genome.

In a separate preferred embodiment, other nucleic acid(s) or protein(s) are utilized in concert with the nucleic acid(s) or protein(s) corresponding to Long (PRR+) Numb isoforms so long as a population ofmultipotent, pluripotent, and/or self-renewing cells is produced from the selected cells.

In a separate preferred embodiment, other nucleic acid(s) or protein(s) are utilized in concert with the nucleic acid(s) or protein(s) corresponding to Long (PRR+) Numb isoforms so long as a population of multipotent, pluripotent, and/or self-renewing cells is produced from the selected cells and the method is electroporation, liposomes, nanocapsules, nanovaults, and/or another approach avoiding retroviral/lentiviral integration or other random alteration of the cell's genome.

It is to be understood that any combination of nucleic acid or protein sequences described herein can be modified by excluding those corresponding to Numb and/or Numblike so long as the desired cell population or behavior is achieved.

Similarly, it should be understood that the methods described herein for initiating differentiation are applicable to any induced or non-induced multipotent, pluripotent, or self-renewing stem cells, other progenitor cells, or other selected cells, not only those obtained in the manner described herein.

It is to be understood that any combination of nucleic acid or protein sequences described herein can be modified by excluding nucleic acid sequences or proteins corresponding to Numb and/or Numblike so long as the desired cell population is achieved.

In another embodiment, the various nucleic acid or protein combinations described herein are employed with the exclusion of the nucleic acid or protein corresponding to the Numblike and/or Numb isoforms.

In a preferred embodiment, the selected cells and/or their progeny are cells that have been genetically-modified beforehand.

In a preferred embodiment, the transfection steps described herein represent transient transfection.

In a further preferred embodiment such transient transfection is accomplished using viral vectors that do not integrate into the host genome.

In another preferred embodiment, such transient transfection is accomplished using standard transfection techniques (electroporation, chemically mediated transfection, fusogenic or non-fusogenic liposomes, nanocapsules, nanovaults, etc.).

Over time, other gene combinations differing from those described herein may be described or discovered capable of causing cells to become multipotent, pluripotent, capable of self-renewal or to begin differentiating. However this patent application also covers the genetic reprogramming of any nucleated cell utilizing nucleic acid or protein electroporation (for example methods see Gagne et al., 1991; Saito et al., 2001; Yuan, 2008; Huang et al., 2007; Xia and Zhang, 2007; Cemazar and Sersa 2007; Isaka and Imai, 2007; Luxembourg ct al., 2007; Van Tendeloos, 2007; Takahashi, 2007; etc.) electroporation, liposomes, nanocapsules, nanovaults, and/or another approach avoiding viral integration or other random alteration of the cell's genome as such means increase safety and efficiency.

In another preferred embodiment, transfection with long (PRR+) numb isoform encoding sequences (and/or synthetic oligonucleotides targeting numblike and short numb isoforms) is accompanied or replaced by transient or permanent transfection with other sequences including ones selected from those encoding human LIF (e.g. Du and Shi, 1996) oncostatin-M, cardiotrophin-1, IL-11, IL-6, IL6R, hyper IL-6, LIFR, gp130, OCT3 (OCT4), Nanog, SOX2, and/or FGF-4.

Simultaneous transfection with any subset of these distinct transgene sequences can be accomplished by any means known to the art including the use of a single genetic vector, multiple genetic vectors, serial transfection and selection based on distinct marker proteins and/or antibiotic resistances.

In another preferred embodiment, cells transfected with long (PRR+) numb isoform(s) are cultured in a cell culture promoting an optimal growth rate, such as described above, and that includes EGF, bFGF, oncostatin, LIF (e.g. Du and Shi, 1996), steel factor, IL-11, cardiotrophin-1, IL-6, hyper-IL-6, CNTF, and/or soluble gp130.

## Assessment of Potency and Differentiation

Pluripotency and multipotency can be assessed by any means known to the art including 1) transplantation, 2) culture under conditions promoting embryoid body formation, 3) injection of cells into animal blastocyst stage embryos with subsequent development, and 4) RNA expression assays (e.g. RT-PCR and microarray based analyses) for gene expression associated with differentiation, multipotency, pluripotency, etc. (see Guan ct al., 2006), 5) colony-formation, as well as by ES-like morphology. One approach disclosed herein for detecting pluripotency in selected cells and/or their progeny involves transfection with a reporter construct comprising the Nanog promoter operably linked to a fluorescent protein gene. This allows identification and enrichment of Nanog expressing cells using Fluorescence Activated Cell Sorting (FACS), etc.

In a preferred embodiment, endogenous cells (e.g. cells surrounding a burn or injury site) are transfected in vivo with genetic vectors encoding the long (PRR+) numb isoform(s) alone or in conjuction with other transgenes named herein to transiently promote renewed or increased cell proliferation. This approach can also be utilized clinically in the setting of hypoplastic tissues, disorders where stem/progenitor cells are abnormally depleted, and other disorders where the approach can be shown to be beneficial.

## Achieving Differentiating Cell Populations

In order to achieve b) neural c) muscle d) and other cell populations capable of further environmentally-regulated differentiation in vivo, selected cell(s) and/or their progeny are optionally transfected with long (PRR+) Numb isoform sequence(s) and/or synthetic oligonucleotide sequences and expanded by growth for sufficient time to achieve the desirable number of cell progeny in vitro (as described above).

Following this optional step, the selected cells and/or their progeny are washed free of the cytokines and agents comprising the expansion/optimal growth media, and are optionally transfected with the nucleotide sequence(s) encoding the Numblike gene and/or "short"(PRR-) Numb isoform(s) and/or synthetic oligonucleotides targeting the long (PRR+) isoforms, etc. (e.g. Zaehres et al., 2005), then cultured under conditions which promote differentiation of the selected cells into the desired cell type(s).

In most instances, the cells are then cultured in the presence of 5-10% fetal bovine serum and agents(s) promoting differentiation of the selected cells and/or their progeny into a desired cell population. The presence of the fetal bovine and of the agents(s) provides for growth or proliferation at a rate that is less than the optimal (or expansion) growth rate, and favors differentiation of the cells into a desired cell population. The agents and precise culture conditions are selected according to the desired cell population as described below.

Achieving Neuronal or Neural Cell Populations

When the desired cell population is a neural cell population, the successfully transfected cells are cultured under conditions that promote growth at a rate which is less than the optimal rate and in the presence of agent(s) promoting differentiation of the cells into neural cells. Conditions promoting differentiation into neurons have been described in numerous publications including (Benninger et al., 2003; Chung et al. 2005; Harkany et al., 2004; Ikeda et al., 2004; Ikeda et al., 2005; Wernig et al., 2002; and Wernig et al., 2004). Furthermore, combining retinoic acid exposure with the presence of additional cytokines favors specific neuronal cell type differentiation in vitro (e.g. Soundararajan et al., 2006; Soundararajan et al., 2007; U.S. Patent 6432711).

In a preferred embodiment, in vitro differentiation of neurons or neural cells occurs in the presence of 50 ng/mL nerve growth factor (NGF).

In a preferred embodiment, when a neuronal population is the desired cell population, transfection with sequences encoding short numb isoforms (and/or numblike) is accompanied or replaced by transient or permanent transfection with other sequences including ones selected from those encoding Nurr1, REN, Neurogenin1, Neurogenin2, Neurogenin3, Mash 1, Phox2b, Phox2a, dHand, Gata3, Shh, FGF8, Lmx1b, Nkx2.2, Pet1, Lbx1, and/or Rnx.

In another preferred embodiment, when dopaminergic neurons are the desired neuronal population, transfection with sequences encoding short numb isoforms (and/or numblike) is accompanied or replaced by transient or permanent transfection with other sequences including ones selected from those encoding Mash1, Ngn2, Nurr1, Lmx1b, and/or Ptx-3.

In another preferred embodiment, when serotonergic neurons are the desired neuronal population, transfection with sequences encoding short numb isoforms (and/or numblike) is accompanied or replaced by transient or permanent transfection with other sequences including ones selected from those encoding Mash 1, Phox2b, Lmx1b, Nkx2.2, Gata2, Gata3 and/or Pet1.

In another preferred embodiment, when cholinergic neurons are the desired neuronal population, transfection with sequences encoding short numb isoforms (and/or numblike) is accompanied or replaced by transient or permanent transfection with other sequences including ones selected from those encoding MASH1, Phox2a and/or REST4.

In another preferred embodiment, when GABAergic neurons are the desired neuronal population, transfection with sequences encoding short numb isoforms (and/or numblike) is accompanied or replaced by transient or permanent transfection with other sequences including ones selected from those encoding MASH1, Phox2a and/or REST4, followed, optionally, by culture in media supplemented with LIF, Neurotrophin 3 (NT3), and/or nerve growth factor (NGF).

In another preferred embodiment, when noradrenergic neurons are the desired neuronal population, transfection with sequences encoding short numb isoforms (and/or numblike) is accompanied or replaced by transient or permanent transfection with other sequences including ones selected from those encoding Mash1, dHand, Phox2a, Phox2b, Gata2 and/or Gata3.

In another preferred embodiment, when GABAergic neurons are the desired neuronal population, transfection with sequences encoding short numb isoforms (and/or numblike) is accompanied or replaced by transient or permanent transfection with other sequences including ones selected from those encoding PITX2, Dlx2, Dlx5, antisense Hes1 RNA and/or other HES1 targeting synthetic oligonucleotides..

In another preferred embodiment, when a neuronal or neural cell population is the desired population, cells transfected with short (PRR-) numb isoforms (and/or numblike) are cultured in a cell culture medium promoting differentiation, such as described above and that includes one or more of the following agents: retinoic acid, NT3, NGF, glial cell-line derived growth factor (GDNF), and interferon gamma (IFN-gamma).

Achieving Muscle Cell Populations

When the desired cell population is a muscle population, the successfully transfected cells are cultured in the presence of an agent promoting differentiation of the cells into muscle cells and growth at a rate less than the optimal rate. Conditions promoting differentiation into muscle cells have also been described previously (Nakamura et al., 2003; Pal and Khanna, 2005; Pipes et al., 2005; Albilez et al., 2006; Pal and Khanna, 2007; Behfar ct al., 2007; U.S. Patent 6432711). Furthermore, exposure of selected cells and/or their progeny to hexamethylene bis-acrylamide or dimethylsulfoxide in the presence of additional cytokines favors the initiation of muscle type differentiation in vitro.

In a preferred embodiment, when a cardiac muscle cell population is the desired population, cells transfected with short (PRR-) numb isoforms (and/or numblike) are cultured in a cell culture medium promoting differentiation into cardiomycytes (He et al., 2003; Guan et al., 2007; etc.), or that includes specific agents at concentrations promoting cardiac cell differentiation (e.g. 0.75%-1% dimethyl sulfoxide (DMSO), 20% normal bovine serum (NBS), 10(-7) mM retinoic acid (RA) and 20% cardiomyocytes conditioned medium (Hua et al., 2006).

In another preferred embodiment, when a cardiac muscle cell population is the desired population, the cells are also transfected with nucleotide sequences including ones selected from those sequences encoding Gata 4, Gata 5, and Gata 6.

In a preferred embodiment, when a muscle cell population is the desired cell population, transfection with sequences encoding short numb isoforms (and/or numblike) is accompanied or replaced by transient or permanent transfection with other sequences including ones selected from those encoding muscle type specific bHLH-encoding sequences, MyoD, Myogenin, Myf5, Myf6, Mef2, Myocardin, Ifrd1 and/or other muscle transcription factors.

In a preferred embodiment, when a smooth muscle cell population is the desired cell population, transfection with sequences encoding short numb isoforms (and/or numblike) is accompanied or replaced by transient or permanent transfection with other sequences including ones selected from those encoding the muscle type specific Myocardin nucleotide sequence.

In a preferred embodiment, when a skeletal muscle cell population is the desired cell population, transfection with sequences encoding short numb isoforms (and/or numblike) is accompanied or replaced by transient or permanent transfection with other sequences including ones selected from those encoding the muscle type specific MyoD and myogenin nucleotide sequences.

In a preferred embodiment, when an oligodensrocyte cell population is the desired cell population, transfection with sequences encoding short numb isoforms (and/or numblike) is accompanied or replaced by transient or permanent transfection with other sequences including ones selected from those encoding the oligodendrocyte -specific OLIG1, OLIG2, and Zfp488 nucleotide sequences.

Simultaneous transfection with any subset of these distinct transgene sequences listed above can be accomplished by any means known to the art including the use of multiple genetic vectors, serial transfection as well as selection based on distinct marker proteins and/or antibiotic resistance.

When the desired cell population is a hematopoietic cell population, the differentiation medium includes specific agents at concentrations promoting differentiation into hematopoietic progenitor cells (e.g. vascular endothelial growth factor (VEGF), thrombopoietin, etc. (e.g. Ohmizono, 1997; Wang et al., 2005; Srivastava et al., 2007; Gupta et al., 2007) or differentiated hematopoietic cell types (according to methods known to the art for providing differentiated hematopoietic cell types from undifferentiated or pluripotent cells).

When the desired cell population is a germ cell population, the differentiation medium includes specific agents at concentrations promoting differentiation into germ cells (e.g. Nayernia et al. 2006a, 2006b).

When the desired cell population is an endoderm and pancreatic islet cell population, the differentiation media includes specific agents at concentrations promoting differentiation into endoderm and pancreatic islet cells (e.g. Xu et al., 2006; Denner et al., 2007; Shim et al., 2007; Jiang et al., 2007).

In a preferred embodiment, differentiation of selected cells and/or their progeny may occur in the differentiation medium in the absence of transfection with numblike, short Numb idsoforms or other transgencs, although the differentiation medium may be unchanged.

In embodiments, a single vector will be utilized which controls the expression of nucleotide sequence(s) encoding the "long" (PRR+) isoform(s) of the mammalian numb gene (and/or synthetic oligonucleotides targeting numblike or the short numb isoforms) under one regulable promoter (e.g. a tetracycline-regulated promoter), while the Numblike and short Numb isoforms (and/or synthetic oligonucleotides targeting the long (PRR+) isoforms) are expressed under the control of another, distinct, but also regulable promoter. Thus, the long (PRR+) numb isoform(s) can be expressed (and/or short isoforms repressed) when expansion of the selected cells is desired and an inducing agent (e.g. tetracycline) is added to the growth medium; later numblike and the short isoforms can be expressed (and/or long (PRR+) numb isoform(s) repressed) when differentiation is desired.

Alternatively, proteins and peptides corresponding to Numb isoforms, Notch, OCT3/4, SOX2, and other DNA sequences listed herein may be applied in analogous fashion to selected cells and/or their progeny via electroporation (e.g. Koken et al., 1994; Ritchie and Gilroy, 1998), using nano particles, cationic lipids, fusogenic liposomes (e.g. Yoshikawa et al., 2005; 2007), etc. in lieu of, or in combination with genetic transfection. Generally, electroporation allows for high transfection efficiency (and efficient production of the desired cells) without genomic integration of the transgene and is therefore associated with increased safety.

The DNA or RNA encoding protein(s) or polypeptide(s) promoting proliferation, multipotentiality, pluripotentiality or differentiation of the selected cells may be isolated in accordance with standard genetic engineering techniques (for example, by isolating such DNA from a cDNA library of the specific cell line) and placing it into an appropriate expression vector, which then is transfected into the selected cells.

In another preferred embodiment, endoderm and pancreatic islet cells are the desired population, and transfection with sequences encoding short numb isoforms (and/or numblike) is accompanied or replaced by transient or permanent transfection with other sequences including ones selected from those encoding Foxa2, Sox17, HLXB9 and/or Pyx1.

In another preferred embodiment, hepatocytes are the desired population, and transfection with sequences encoding short numb isoforms (and/or numblike) is accompanied or replaced by transient or permanent transfection with other sequences including ones selected from those encoding hepatic nuclear factor (HNF)-1, HNF-3, HNF-4, I INF-6 and creb-binding protein.

In another preferred embodiment, hematopoietic cells are the desired population, and transfection with sequences encoding short numb isoforms (and/or numblike) is accompanied or replaced by transient or permanent transfection with other sequences including ones selected from those encoding Runx1/AML1 and NOV(CCN3), and/or cell culture in the presence of colony stimulating factors specific for the desired cell populations. The Runx1/AML1a isoform is introduced when engraftment is desired and the b isoform when differentiation is desired (Creemers et al., 2006).

In another preferred embodiment, chondrocytes are the desired population, and transfection with sequences encoding short numb isoforms (and/or numblike) is accompanied or replaced by transient or permanent transfection of other sequences including ones encoding Sox9, CREB-binding protein, Gata6, and/or Runx2.

In another preferred embodiment, bone cells (especially osteoblasts) are the desired population, and transfection with sequences encoding short numb isoforms (and/or numblike) is accompanied or replaced by transient or permanent transfection of other sequences including Runx2.

In a preferred embodiment, the genetic vectors encoding the long Numb isoforms (such as those described herein) arc introduced transiently or under the control of a regulable promoter, into endogenous cells in vivo in order to cause those cells proliferate transiently.

In a preferred embodiment, endogenous cells (e.g. ependymal zone cells of the central nervous system) are transfected in vivo with genetic vectors encoding either the shortest numb isoform or the numblike protein(s) alone or in conjuction with other transgenes named herein, in order to transiently or permanently promote renewed or increased differentiation (especially neuronal differentiation) and migration of progenitor/ependymal cells in the central nervous system). This renewal or increase is measured in terms of the number of cells showing new-onset expression of markers associated with differentiation. This may be accomplished by introduction of the genetic vectors into the organ system using methods suitable for that purpose (see examples).

In a preferred embodiment, endogenous cells (e.g. ependymal zone cells of the central nervous system) are transfected in vivo with genetic vectors encoding the long numb isoform(s) and/or other transgenes named herein, in order to transiently promote renewed or increased stem cell proliferation (with subsequent differentiation of progeny cells). This renewal or increase is measured in terms of the number of cells showing new-onset expression of marlers associated with dividing progenitors. This may be accomplished by introduction of the genetic vectors into the organ system using methods suitable for that purpose (see examples).

Likewise this approach is also be suitable for inducing renewed or increased differentiation from other stem cell populations in other tissues (such as the skin, etc). This approach can be utilized, for example, clinically in the setting of central nervous system injury, disorders of other tissues where normal differentiation or migration are inadequate, dysplastic disorders and other disorders where the approach is beneficial.

In a preferred embodiment, nucleic acid(s) or protein(s) corresponding to a single gene, or portion thereof, (particularly those named herein, discovered according to methods described herein, discovered according to other published methods; and/or known to be capable of initiating the desired manner of differentiation) are the only nucleic acid(s) or protein(s) overexpressed and/or introduced to initiate differentiation in the selected cells.

In a preferred embodiment, nucleic acid(s) or protein(s) corresponding to a single gene, or portion thereof, (particularly those named herein, discovered according to methods described herein, discovered according to other published methods; and/or known to be capable of initiating the desired manner of differentiation) are the only nucleic acid(s) or protein(s) overexpressed and/or introduced to initiate differentiation in the selected cells and the method utilized is electroporation, liposomes, nanocapsules, nanovaults, and/or another approach avoiding retroviral/lentiviral integration or other random alteration of the cell's genome.

In a separate preferred embodiment, other nucleic acid(s) or protein(s) can be utilized in concert with the nucleic acid(s) or protein(s) corresponding to a single gene, or portion thereof, (particularly those named herein, discovered according to methods described herein, discovered according to other published methods; and/or known to be capable of initiating the desirable manner of differentiation) so long as a population of differentiating cells is produced from the selected cells.

In a separate preferred embodiment, other nucleic acid(s) or protein(s) can be utilized in concert with the nucleic acid(s) or protein(s) corresponding to a single gene, or portion thereof, (particularly those named herein, discovered according to methods described herein, discovered according to other published methods; and/or known to be capable of initiating the desirable manner of differentiation) so long as a population of differentiating cells is produced from the selected cells and the method utilized is electroporation, liposomes, nanocapsules, nanovaults, and/or another approach avoiding retroviral/lentiviral integration or other random alteration of the cell's genome.

It is to be understood that any combination of nucleic acid or protein sequences described herein can be modified by excluding those corresponding to Numb and/or Numblike so long as the desired cell population or behavior is achieved.

Similarly, it should be understood that the methods described herein (or elsewhere) for initiating differentiation are applicable to any induced or non-induced multipotent, pluripotent, or self-renewing stem cells, or other selected cells, not only those obtained in the manner described herein.

Sources of Selected cells

The population of selected cells may derive from various stem cells, progenitor cells and somatic cells. However somatic cells lacking nuclei (e.g. mature, human red blood cells) are specifically excluded. Selected stem cells may be derived from existing cell lines or isolated from stored, banked, or cryopreserved sources. Typical sources of stem cells include bone marrow, peripheral blood, placental blood, amniotic fluid (e.g. De Coppi et al., 2007), umbilical cord blood (e.g. Zhao, et al., 2006; Tian et al., 2007), adipose tissue (e.g. Gimble et al., 2007; Ma et al., 2007), non-human embryos, and others. Circulating leukocytes and other non-stem cells may likewise be selected and subjected to the same culture conditions as described above effective that they acquire multipotency, pluripotency and/or self-renewal as a result. Examples of other accessible somatic cells useful in this invention include lymphocytes and epithelial (e.g. buccal check) cells. Isolation and collection of cells selected for use within the present invention may be performed by any method known to the art.

In embodiments involving animals, stem cells isolated from prostate, testis, embryonic brain, and intestine are also disclosed as being preferred sources of selected cells.

In a preferred embodiment, the selected cells and/or their progeny are cultured in a three-dimensional format.

A further aim of the present invention is to provide cells for use in the production of patient-compatible and patient-specific tissues and organs for transplantation to patients deemed to be requiring such organs or tissues. It is disclosed herein that the pluripotent, multipotent, and/or differentiating cells provided by the methods described herein (or similar methods) be utilized in conjunction with techniques aimed at the production of such organs and/or tissues (e.g. Boland et al., 2006. Xu et al., 2006; Campbell and Weiss, 2007). Such utilization is specifically covered by the present invention.

For instance, pluripotent, multipotent, and/or differentiating cells produced or treated according to the methods desribed herein (or other published methods) may be grown in association with three-dimesnisonal or two-dimensional scaffoldings engineered to replicate normal tissue structure and/or organ structures (e.g.Yarlagada et al., 2005; Kim et al, 1998; WO/2003/070084; EP1482871; WO03070084; US Patents 2395698; 7297540; 6995013; 6800753; Isenberg et al., 2006).

Similarly, scaffoldings to be occupied by the pluripotent, multipotent, and/or differentiating cells may be derived from cadaveric organ(s) or tissue(s) after the cadaveric organs or tissues (e.g. bone, heart, kidney, liver, lung, etc.) may be treated in such away that the host immune cells resident in that tissue, and other undesirable or ancillary host cells, are eliminated (e.g. by ionizing radiation, sterilization (e.g. Mroz et al., 2006), and/or various methods of decellularization (United States Patents 6734018; 6962814; 6479064; 6376244; U.S. Pat. Nos. 5032508; 4902508; 4956178; 5281422, 5554389; 6099567; and 6206931; 4361552 and 6576618; 6753181; U.S. application Ser. No. 11/162,715; WO/2001/048153; WO/2002/024244; WO003002165; WO/2001/049210; WO/2007/025233; European Patents EP1482871; EP1246903; EP1244396; EP0987998; EP1244396; EP1333870; Rieder et al., 2004; Ott et al., 2008; Taylor et al., 1998)).

Likewise, it is anticipated that the pluripotent, multipotent, and/or differentiating cells of the present invention may be used in applications utilizing inkjet-style printing for tissue engineering (e.g. Boland et al., 2006. Xu et al., 2006; Campbell et al., 2007). Therefore such use of the cells produced or treated according to the methods described herein is covered.

In another preferred embodiment, the selected cells and/or their progeny are cultured in hanging drops.

In accordance with another aspect of the present invention, selected cells may be modified genetically beforehand.

In accordance with another aspect of the present invention, selected cells may be modified with DNA or RNA encoding protein(s) or polypeptide(s) promoting differentiation of the cell into a desired cell population.

Screening Cell Populations

In one embodiment, the methods of this invention comprise screening cells from cell lines, donor sources, umbilical cord blood, and autologous or donor bone marrow, blood, spermatogonia, primordial germ cells, buccal cheek cells, or any other cell source effective in the current invention. Selected cells can be screened to confirm successful transfection with beneficial sequence(s) or therapeutic vector(s) as well as successful initiation of differentiation by any method known to the art (Guan et al., 2006; US Patent 6,432,711). In some embodiments, the cells are screened using standard PCR and nucleic acid hybridization-based methods or using rapid typing methods. In preferred embodiments, the cells are screened according to expression of reporter genes. In some embodiments, cells are screened by expression of a marker gene encoded by the transgene expressing vector(s) such as an antibiotic resistance gene or a fluorescent protein (e.g. GFP) gene.

Screening for Therapeutic Vectors and Beneficial Sequences

Cells can be screened for the presence of beneficial sequence(s) and therapeutic vector(s) using any method(s) known to the art for detection of specific sequences. Each cell sample can be screened for a variety of sequences simultaneously. Alternatively, multiple samples can be screened simultaneously.

Cell differentiation may be monitored by several means: including (i) morphological assessment, (ii) utilizing reverse transcriptase polymerase chain reaction (RT-PCR), Northern blot, or microarray techniques to monitor changes in gene expression, (iii) assaying cellular expression of specific markers such as beta tubulin III (for neurons) etc. (Ozawa, et al., 1985). In some embodiments, the cells are screened for successful initiation of differentiation using FACS sorting based on cell type specific markers or transgenic marker expression (e.g. antibiotic resistance or fluorescent protein expression) under the control of cell type specific promoters such as the myosin promoter in muscle cells; the human cardiac $\alpha$-actin promoter in cardiomyocytes; the insulin promoter in insulin producing cells; the neuronal-specific enolase (NSE) promoter for neuronal differentiation, or neurotransmitter related promoters such as the tyrosine hydroxylase promoter in dopaminergic neurons; etc.).

In some embodiments, the cells are screened using standard PCR and nucleic acid hybridization-based methods. In a particularly preferred embodiment, the cells are screened using rapid typing methods.

Screening for human leukocyte antigen (HLA) type

In certain embodiments, the selected cells are selected with respect to compatible HLA typing. The HLA genotype can be determined by any means known to those of skill in the art.

The cells used for screening may consist of cells taken directly from a donor, or from cell lines established from donor cells, or other practicable cell sources. The cells can be screened for beneficial sequence(s), and/or therapeutic vector(s) and HLA type at once, or separately. Those cells successfully transfected with a beneficial sequence and showing an appropriate HLA genotype can be prepared for transplantation to a patient.

In certain embodiments, the transfected cells are transplanted without HLA typing. In other embodiments, the cells are HLA typed for compatibility.

Screening for agents promoting a cellular phenotype

The present invention also provides for a methods of screening proteins and agents for their ability to induce phenotypic changes or differentiation of the selected cells and/or their progeny into desired cell populations. Briefly, vectors encoding complementary DNAs (cDNAs) from appropriate cDNA libraries are transfected into the selected cells/and or their progeny. Once a specific cDNA that induces differentiation or other phenotypic change is identified, such cDNA then may be isolated and cloned into an appropriate expression vector for protein production in appropriate cells (e.g. COS cells) in vitro. Later the protein containing supernatant can be applied to the selected cell cultures to determine if any secreted proteins from such cells induce differentiation Alternatively, candidate agents can be applied to the selected cell cultures to determine if any secreted proteins from such cells induce differentiation (see US Patent 6432711).

The present invention also provides for methods of screening nucleic acids for their ability to induce multipotentiality, pluripotentiality, and/or self-renewal, or to initiate differentiation of selected cells and/or their progeny. In these methods, vectors encoding selected cDNAs (or cDNAs from appropriate cDNA libraries, or other sequences) are introduced into the selected cells/and or their progeny using electroporation, nanocapsules, nanovaults, liposomes, retroviruses, lentiviruses, and/or any other practicable means of transfection. Once a specific cDNA that induces a phenotypic change, multipotentiality, pluripotentiality, and/or self-renewal, is identified, such cDNA then may be isolated and cloned into an appropriate expression vector. Assays for determining such changes include those described elsewhere herein.

Likewise the protein corresponding to the identified cDNA may be produced in appropriate cells (e.g. COS cells) in vitro to determine whether the protein containing supernatant can be applied to the selected cell cultures and induce the desired changes.

Finally, proteins may be introduced into the selected cells/and or their progeny using electroporation, nanocapsules, nanovaults, liposomes, retroviruses, lentiviruses, and/or any other practicable means of transfection, and the resulting cells assessed as described herein for multipotentiality, pluripotentiality, self-renewal or the initiation of differentiation.

Transplantation of Cells into Patients

After screening, selected cells and/or their progeny may be cryopreserved, maintained as cell lines in culture, or may be administered to the patient. Selected cells can be cryopreserved or maintained in culture by any means known to the art and preserved for future transplantation procedures.

Preferably, the cells to be screened are obtained from accessible sources allowing easy collection.

With regard to producing HIV resistant cells: targeted somatic cells and stem cells of this invention can be of any type capable of differentiating into cells that can be infected by HIV, that can sustain the transcription and/or replication of HIV, that can alter the HIV immune response, or that can retard progression to AIDS. Such stem cells include, but are not limited to, pluripotent cells derived from spermatogonia, primordial germ cells, hematopoietic stem cells, peripheral blood cells, placental blood cells, amniotic fluid cells, umbilical cord blood cells, buccal cheek cells, adipose tissue cells (including stem cells derived from those tissues), reprogrammed cells, induced multipotent cells, induced pluripotent cells, etc., non-human embryos, and/or any other cell type that can form blood and immune cells, HIV target cells, and other cells.

Therapeutic vector(s) express "beneficial sequence(s)" intended to render transfected or infected cells less capable of sustaining HIV replication and transcription. The genetic vector expressing "beneficial sequence(s)" as well as any virus derived from such genetic vector, are herein termed "therapeutic vector".

After screening, cells transfected with the desired therapeutic vector(s) and expressing beneficial sequence (with or without compatible HLA genotype) may be expanded ex vivo (in vitro) using standard methods to culture dividing cells and maintained as stable cell lines (US Patents 6432711 and 5453357 herein incorporated by reference). Alternatively, these cells can be administered to the patient and expanded in vivo.

Selected cells can be cryopreserved by any means known to the art and preserved for future transplantation procedures.

Transplantation of desirable cell populations into Patients

In certain embodiments, cell populations are enriched for stem cells prior to transplantation. Various methods to select for stem cells are well known in the art. For example, cell samples can be enriched by fluorescently labeled monoclonal antibodies recognizing cell-surface markers of undifferentiated hematopoietic stem cells (e.g., CD34, CD59, Thyl, CD38 low, C-kit low, lin- minus) for sorting via fluorescence-activated cell sorting (FACS).

In other embodiments, a sample of the selected cells is transplanted, without enrichment.

In some embodiments, the endogenous stem cells of the bone marrow are eliminated or reduced prior to transplantation of the therapeutic stem cells. Therapeutic stem cells are defined as those stem cells containing beneficial sequence(s) or therapeutic vector(s).

In some embodiments, the transplantation process may involve the following phases: (1) conditioning, (2) stem cell infusion, (3) neutropenic phase, (4) engraftment phase, and (5) postengraftment period.

In some embodiments, the endogenous stem cells that normally produce the desired cells (e.g. bone marrow stem cells) are eliminated or reduced prior to transplantation. Chemotherapy, radiation, etc. and/or methods analogous to those described in U.S. Patent 6,217,867 may be used to condition the bone marrow for appropriate engraftment of the transplant. Finally, therapeutic stem cells may be transplanted into the patient using any method known to the art.

Design of Numb / Numblike and other transgene encoding vectors

In one embodiment transfection with nucleic acid sequence(s) encoding numblike/numb isoform(s) is accomplished via viral transfection. The term "Numb/Numblike encoding vector(s)" refers to the vectors incorporating the nucleic acid sequence(s) encoding numblike/numb isoform(s) and/or synthetic oligonucleotides targeting numblike or numb isoforms, as well as any additional transgene sequences, synthetic oligonucleoties, etc, and any associated viral supernatant incorporated in those vector sequences.

The Numb / Numblike encoding vector(s) may comprise an expression vector. Appropriate expression vectors are those that may be employed for transfecting DNA or RNA into eukaryotic cells. Such vectors include, but are not limited to, prokaryotic vectors such as, for example, bacterial vectors; eukaryotic vectors, such as, for example, yeast vectors and fungal vectors; and viral vectors, such as, but not limited to adenoviral vectors, adeno-associated viral vectors, and retroviral vectors. Examples of retroviral vectors which may be employed include, but are not limited to, those derived from Moloney Murine Leukemia Virus, Moloney Murine Sarcoma Virus, and Rous Sarcoma Virus, FIV, HIV, SIV and hybrid vectors.

It is disclosed that the Numb / Numblike encoding vector(s) may be used to transfect cells in vitro and/or in vivo. Transfection can be carried out by any means known to the art, especially through virus produced from viral packaging cells. Such virus may be encapsidated so as to be capable of infecting a variety of cell types. Nevertheless, any encapsidation technique allowing infection of selected cell types and/or their progeny is practicable within the context of the present invention.

Design of human immunodeficiency virus (HIV) Gene therapy Vector(s)

The "therapeutc vector(s)" may incorporate an expression vector. Appropriate expression vectors are those that may be employed for transfecting DNA or RNA into eukaryotic cells. Such vectors include, but are not limited to, prokaryotic vectors such as, for example, bacterial vectors; eukaryotic vectors, such as, for example, yeast vectors and fungal vectors; and viral vectors, such as, but not limited to adenoviral vectors, adeno-associated viral vectors, and retroviral vectors. Examples of retroviral vectors which may be employed include, but are not limited to, those derived from Moloney Murine Leukemia Virus, Moloney Murine Sarcoma Virus, and Rous Sarcoma Virus, feline immunodeficiency virus (FIV), HIV, simian immunodeficiency virus (SIV) and hybrid vectors.

It is disclosed herein that the therapeutic vector(s) may be used to transfect target cells in vitro and/or in vivo. Transfection can be carried out by any means known to the art, especially through virus produced from viral packaging cells. Such virus may be encapsidated so as to be capable of infecting CD34+ cells and/or CD4+ cells. However, in some instances, other cell types are transfected by means not involving the CD4 or CD34 proteins. Nevertheless, any encapsidation technique allowing infection of such cell types may therefore be included in the disclosure of the present invention.

Pseudotyping with different envelope proteins expands the range of host cells transduceable by viral vectors and therapeutic vectors, and allows the virus to be concentrated to high titers, especially when pseudotyped with the vesicular stomatitis virus envelope glycoprotein (VSV-G) (Li et al., 1998; Reiser et al., 2000).

Vector Construction

Viral vectors utilized in this invention may be of various types including hybrid vectors. Vectors may, for instance, be third-generation lentiviral vectors which include only a very small fraction of the native genome (Zufferey et al., 1998). Production of transgene encoding vector(s) may also involve self-inactivating transfer vectors (Zufferey et al., 1998; Miyoshi et al., 1998) eliminating the production of full-length vector RNA after infection of target cells.

Viral vectors may be utilized which are replication-incompetent due to failure to express certain viral proteins necessary for replication. However the possibility exists that helper virus may enable therapeutic virus replication. This likelihood can be reduced by the use of self-inactivating vectors.

In a preferred embodiment, transgene sequences are driven by a ubiquitin promoter, U6 promoter, EF1alpha promoter, CMV promoter, regulable promoters and/or desired cell type specific promoters.

Viral Tropism

In a preferred embodiment, virus derived from the Numb isoform / Numblike encoding vector(s), therapeutic vector(s) and/or other transgeneic vector(s) of this invention is pseudotyped with vesicular stomatitis virus envelope glycoprotein to enable concentration of the virus to high titers and to facilitate infection of CD34+ cells.

Sequence Selection

The use of any sequence with 70% or greater identity (or complementarity) to any sequence referred to as a NUMB or Numblike sequence (searchable using the Entrez-Pubmed database) is covered by the invention if utilized in the manner described in the present invention.

The current invention also relates in part to a genetic vector that includes sequences capable of markedly reducing the susceptibility of mammalian cells to infection by HIV 1 and HIV-2 viruses (both together referred to herein as HIV).

The current invention discloses the novel combination of synthetic oligonucleotides to reduce the expression of genes critical to the HIV/AIDS disease process.

The desirability of combining synthetic oligonucleotides to effect co-receptor "knock down" with expression of TAR and RRE decoy sequences arises from the proposition, expressed herein, that combining multiple gene therapy approaches simultaneously targeting 1) HIV infection, 2) HIV transcription, and 3) HIV replication in individual cells is likely to produce superior therapeutic benefits than any of these approaches in isolation.

Therapeutic vector(s) express "beneficial sequence(s)" intended to render transfected or infected cells less capable of sustaining HIV replication and transcription. The genetic vector expressing "beneficial sequence(s)" as well as any virus derived from such genetic vector, are herein termed "therapeutic vector".

The present invention is directed in part to the genetic modification of cells susceptible to infection by HIV or capable of propagating HIV. Such cells are herein termed "target cells".

The present invention provides a composition and method for using therapeutic viral vectors to reduce the susceptibility of mature or immature target cells, leukocytes, blood cells, any stem/progenitor cells, and/or their progeny to infection by HIV.

It follows that the present invention also provides a composition and method for using therapeutic viral vectors to reduce the susceptibility of reprogrammed cells, induced multipotent cells, induced pluripotent cells, and/or their progeny to infection by HIV.

It is a further objective of this invention to reduce the ability of mature or immature target cells, stem/progenitor cells, (including reprogrammed cells, induced multipotent cells, induced pluripotent cells) and/or their progeny to sustain immunodeficiency virus replication and transcription.

It is another objective of this invention to achieve efficient, long-term expression of the therapeutic sequences in mature or immature target cells, other quiescent cells, stem/progenitor cells, and/or their progeny.

In one aspect, this invention provides a method for preventing or treating HIV infection. The method involves transplanting stem cells transfected with therapeutic vector(s) or sequence(s), into patients with HIV infection.

Beneficial sequence(s) may be ones that reduce the ability of HIV to infect a cell, transcribe viral DNA, or replicate within an infected cell, or which enhances the ability of a cell to neutralize HIV infection.

In certain embodiments, the beneficial sequence(s) represent synthetic oligonucleotide(s) which interfere with HIV entry, including siRNA, shRNA, antisense RNA or miRNA directed against any of the HIV co-receptors (including, but not limited to, CXCR4, CCR5, CCR2b, CCR3, and CCR1).

In a preferred embodiment, the therapeutic vector(s) includes synthetic oligonucleotides targeting one or more HIV co-receptors including CXCR4, CCR5, CCR1, CCR2, CCR3, CXCR6 and/or BOB.

In another preferred embodiment the therapeutic vector(s) includes synthetic oligonucleotides targeting the major HIV co-receptors CXCR4 and CCR5

In a further preferred embodiment the therapeutic vector(s) includes synthetic oligonucleotides targeting one or more HIV enzymes such as HIV reverse transcriptase, integrase and protease.

Appropriate sequences for the synthetic oligonucleotides are those 1) predictable by computer algorithms to be effective in reducing targeted sequences, and 2) capable of successfully reduce the amount of targeted enzyme by > 70% in standard quantitative RNA assays and in assays of enzymatic activity or to a lesser but therapeutic degree.

The phrase "targeted sequence" indicates that a particular sequence has a nucleotide base sequence that has at least 70% identity to a viral genomic nucleotide sequence or its complement (e.g., is the same as or complementary to such viral genomic sequence), or is a corresponding RNA sequence. In particular embodiments of the present invention, the term indicates that the sequence is at least 70% identical to a viral genomic sequence of the particular virus against which the oligonucleotide is directed, or to its complementary sequence.

Any of the various types of synthetic oligonucleotides may be expressed via therapeutic vector transfection, and the current invention is directed to all possible combinations of such oligonucleotides.

In a preferred embodiment, the synthetic oligonucleotide sequences are driven by target cell, specific promoter(s).

In another preferred embodiment, the synthetic oligonucleotide sequences are driven by U6 promoter(s).

Synthetic oligonucleotides, by the same token, may be included in the same therapeutic vector(s) with decoy RNA.

## Decoy RNA

Decoy RNA are sequences of RNA that are effective at binding to certain proteins and inhibiting their function.

In a preferred embodiment, the therapeutic vector(s) comprise(s) multiple decoy RNA sequences.

In a further embodiment the decoy RNA sequences are flanked by sequences that provide for stability of the decoy sequence.

In another preferred embodiment the decoy RNA sequences are RRE and/or TAR decoy sequences.

In a preferred embodiment, the RRE and TAR decoy sequences are HIV-2 derived TAR and RRE sequences.

In another preferred embodiment the decoy sequences also include Psi element decoy sequences.

In a preferred embodiment, the decoy sequences are each driven by a U6 promoter.

In another preferred embodiment, the decoy sequences are driven by target-cell specific promoters.

In a preferred embodiment, the therapeutic vector targets multiple stages of the I IIV life cycle by encoding synthetic nucleotide sequence(s) in combination with HIV-2 TAR and/or RRE decoy sequences.

In another preferred embodiment, the vector includes miRNA oligonucleotide sequences.

In another preferred embodiment, the vector includes shRNA oligonucleotide sequences.

In another preferred embodiment, the vector includes siRNA oligonucleotide sequences.

In another preferred embodiment, the vector includes RNAi oligonucleotide sequences.

In another preferred embodiment, the vector includes ribozyme sequences.

In another preferred embodiment, the vector includes a combination of synthetic oligonucleotide classes.

In a further embodiment, the synthetic nucleotide sequences target HIV co-receptors such as CCR5, CXCR4, etc.

In a further embodiment, the synthetic nucleotide sequences target HIV enzymes such as integrase, protease, reverse transcriptase, TAT, etc.

In a further embodiment, the ribozyme sequences target HIV co-receptors such as CCR5, CXCR4, etc, or HIV enzymes such as integrase, protease, reverse transcriptase, TAT, etc.

In a preferred embodiment, virus is generated using the therapeuic vector(s) and the virus is pseudotyped.

In a preferred embodiment, virus is generated using the therapeuic vector(s) and the virus is not pseudotyped and the virus shows native HIV tropism.

In a preferred embodiment, the therapeutic vector(s) is a viral vector.

In a preferred embodiment, the therapeutic vector(s) is a lentiviral vector.

In a preferred embodiment, the therapeutic vector(s) is a third generation lentiviral vector.

In a preferred embodiment, the therapeutic vector(s) includes a combination of synthetic oligonucleotide classes.

In a preferred embodiment, synthetic nucleotide sequence expression is driven by the EF-1 alpha promoter or other target-cell appropriate promoters.

In a preferred embodiment, synthetic nucleotide sequence expression is driven by the U6 promoter or other target-cell appropriate promoters.

In a preferred embodiment, synthetic nucleotide sequence expression is driven by a combination of EF-1 alpha and U6, and/or other target-cell appropriate promoters.

In a preferred embodiment, EF-1 alpha drives miRNA expression while the U6 promoter drives RNA decoy expression.

In a preferred embodiment, EF-1 alpha drives siRNA sequence expression while the U6 promoter drives RNA decoy expression.

In a preferred embodiment, EF-1 alpha drives shRNA sequence expression while the U6 promoter drives RNA decoy expression.

In a preferred embodiment, the therapeutic vector(s) includes multiple miRNA sequences directed against CXCR4, multiple miRNA sequences directed against CCR5, an HIV-2 RRE decoy sequence and an HIV-2 TAR decoy sequence, and the vector is a viral vector.

In a preferred embodiment, treatment involving the therapeutic vector(s) is combined with other modes of antiretroviral therapy including pharmacological therapies. Antiretroviral therapies appropriate for combination with the therapeutic vector(s) are those that have additive or synergistic effects in combination with the therapeutic vector.

Cells targeted for gene therapy in HIV may include, but are not necessarily be limited to mature peripheral blood T lymphocytes, monocytes, tissue macrophages, T cell progenitors, macrophage-monocyte progenitor cells, and/or multipotent hematopoietic stem cells, such as those found in umbilical cord blood, peripheral blood, and occupying bone marrow spaces.

The present invention also relates to transfection of CD4+ T cells, macrophages, T cell progenitors, macrophage-monocyte progenitors, CD 34+ stem/progenitor cells and/or any other quiescent cell, dividing cell, stem cell or progenitor cell capable of differentiation in vitro or in vivo into HIV target cells, CD4+ T cells, macrophages, T cell progenitors, macrophage-monocyte progenitors, and/or CD 34+ stem/progenitor cells. Transfected cells, therefore, can be endogenous cells in situ, or exogenous cells derived from other body regions or even other individual donors. Cells selected for this purpose are herein termed "selected cells".

By the same token, self-renewing, multipotent and/or pluripotent stem cells (including reprogrammed and induced pluripotent cells) represent another logical target for HIV gene therapy, and their use is specifically covered by the present invention.

In one embodiment of this process, selected cells (e.g. hematopoietic stem cells, skin stem cells, umbilical cord cells, primordial germ cells (PGCs), spermatogonia, any accessible somatic cell, etc.) are 1) propagated in culture using one or more cytokines such as steel factor, leukemia inhibitory factor (LIF), cardiotropin-1, IL-11, IL-6, IL-6 R, GP-130, CNTF, IGF-I, bFGF, and/or oncostatin-M and 2) transfected with the therapeutic vector(s) or beneficial sequence(s) prior to differentiation using any methods known to the art, such as those described in US Patent 5677139 herein incorporated by reference, or by methods analogous to US Patent 5677139 with respect to other target cells.

In separate embodiments, it may be desirable to perform the various steps prior to transfection.

In separate embodiments, for the purpose of generating pluripotent stem cell populations, it may be desirable to perform only the incubation steps above.

Appropriate concentrations of LIF and steel factor for stem/progenitor cell propagation/proliferation as well as other cell culture conditions have been described previously (e.g. US Patents 6432711 and 5453357 herein incorporated by reference). Other appropriate protocols and reference cytokine concentrations have been taught by Koshimizu et al., 1996; Keller et al., 1996; Piquet-Pellorce, 1994; Rose et al., 1994; Park and Han, 2000; Guan et al., 2006; Dykstra et al., 2006).

The population of target cells may include somatic cells, stem cells and progenitor cells. Stem cells may be derived from existing cell lines or isolated from stored, banked, or cryopreserved sources. Typical sources of stem cells include marrow, peripheral blood, placental blood, amniotic fluid, umbilical cord blood, adipose tissue, non-human embryos, etc.

Somatic cells, especially circulating leukocytes and other non-progenitor/stem cells may likewise be subjected to the same culture conditions as described above for stem/progenitor cells effective that they acquire stem/progenitor cell properties as a result.

The invention also discloses the production (e.g. US Patent Application 20030099621) of target cells from stem/progenitor cells that may be made relatively resistant to HIV infection and/or HIV replication.

It is understood, however, that any method of differentiating previously propagated stem/progenitor/leukocyte cells into the desired target cells may be employed within the scope of the invention so long as functional target cells relatively resistant to HIV infection and/or HIV replication/ and/or HIV transcription are produced.

In a preferred embodiment, the therapeutic viral vector is packaged with one or more envelope proteins from native HIV viruses conferring upon the therapeutic virus the capacity to infect any cell that native HIV strains are capable of infecting.

Cells selected for use in this invention wll be in some instances accessible (e.g. umbilical cord stem cells, bone marrow stem cells, spermatogonia and primordial germ cells of the testis, stem cells isolated from amniotic fluid, stem cells isolated from the skin, etc.). Such cells can be isolated from the tissues in which they reside by any means known to the art.

Other selected cells may comprise reprogrammed cells, induced multipotent cells, induced pluripotent cells, etc.

In accordance with an aspect of the present invention, there is provided a method of producing a desired cell line, cell type, or cell class from the selected cells. Generally, the method comprises culturing the selected cells and/or their progeny under conditions which promote growth of the selected cells at an optimal growth rate. The resulting cell population is then cultured under conditions which promote cell growth at a rate which is typically less than the optimal rate, and in the presence of an agent promoting differentiation of the cells into the desired cell line, cell type, or cell class (e.g. CD4+ T cells).

The present invention also discloses the propagation of the selected cells and/or their progeny in culture, before or after transfection with the therapeutic vector, by any means known to the art (e.g. US Patent Application 20060099177). Such methods also include incubation with LIF, steel factor, Il-6, IL-7, oncostatin-M and/or cardiotropin-1 and other growth enhancing cytokines, etc.

The present invention further discloses the directed differentiation of cells transfected with the therapeutic vector(s) into desired cell types by further incubation in media containing the appropriate cytokines and growth factors such as colony stimulating factors such as M-CSF (CSF-1), GM-CSF, IL-7, any cytokine promoting CD4+ T cell differentiation, etc.

Transfection

Genetic modification of selected cells and target cells, whether they be exogenous cells or endogenous cells can be performed according to any published or unpublished method known to the art (e.g. U.S. Pat. Nos. 6,432,711, U.S. 05,593,875, U.S. 05,783,566, U.S. 5,928,944, U.S. 05,910,488, U.S. 05,824,547, etc.) or by other generally accepted means. Suitable methods for transforming host cells can be found in Sambrook et al. (Molecular Cloning: A Laboratory Manual, 2nd Edition, Cold Spring Harbor Laboratory press (1989)), and other laboratory textbooks.

Successfully transfected cells can be identified by selection protocols involving markers such as antibiotic resistance genes in addition to RNA expression assays and morphological analyses. Clones from successfully transfected cells, expressing the appropriate exogenous DNA at appropriate levels, can be preserved as cell lines by cryopreservation (utilizing any appropriate method of cryopreservation known to the art).

Selectable markers (e.g., antibiotics resistance genes) may include those which confer resistance to drugs, such as G418, hygromycin, ampicillin and blasticidin, etc. Cells containing the gene of interest can be identified by drug selection where cells that have incorporated the selectable marker gene survive, and others die.

A theoretical basis for the embodiments of the invention is described herein, however, this discussion is not in any way to be considered as binding or limiting on the present invention. Those of skill in the art will understand that the various embodiments of the invention may be practiced regardless of the model used to describe the theoretical underpinnings of the invention.

The invention will now be described and illustrated with respect to the following examples; however, the scope of the present invention is not intended to be limited thereby.

Example 1: Construction of the transgenic vectors suitable for use in the present invention.

Suitable EGFP-Numb and EGFP- Numblike, and EGFP-X lentiviral vectors (where X is any transgene described in the present invention) can be produced by cloning into an appropriate viral vector (e.g. the two-gene HIV-EGFP-HSA vector (Reiser et al., 2000)). Adapter primers can be selected for PCR amplification of Numblike and Numb isoform cDNAs and cloning into a genetic vector. In preparation for cloning, the gene vector is digested with enzymes. Subsequently, the cDNA for each transgene is inserted into the nef coding region previously occupied by the HSA cDNA. EGFP (enhanced green fluorescent protein) and a cell population-appropriate promoter (e.g. CMV ie or EF1 alpha) having been previously inserted into the viral coding region. Genetic constructs may include a vector backbone, and a transactivator which regulates a promoter operably linked to heterologous nucleic acid sequences.

Examples of retroviral vectors which may be employed include, but are not limited to, those derived from Moloney Murine Leukemia Virus, Moloney Murine Sarcoma Virus, and Rous Sarcoma Virus, FIV, and HIV. Appropriate expression vectors are those that may be employed for transfecting DNA or RNA into eukaryotic cells. Such vectors include, but are not limited to, prokaryotic vectors such as, for example, bacterial vectors; eukaryotic vectors, such as, for example, yeast vectors and fungal vectors; and viral vectors, such as, but not limited to, lentiviral vectors, adenoviral vectors, adeno-associated viral vectors, and retroviral vectors.

The replication incompetent pcDNA 6.2/EmGFP-Bsd/V5-DEST vector is an example of an appropriate expression vector (Invitrogen) and allows expression of synthetic oligonucleotides (e.g. miRNAs) transferred from the pcDNA 6.2 GW/miR vector that have the capacity to cleave targeted sequences. These vectors include flanking and loop sequences from endogenous miRNA to direct the excision of the engineered miRNA from a longer Pol II transcript (pre-miRNA).

Combining multiple miRNA sequences directed against specific endogenous RNA species increases the likelihood of success in reducing target sequence expression. miRNA sequences may be operably linked to regulable or tissue specific promoters.

By utilizing lentiviral vectors for gene expression, the resulting Numb / Numblike encoding vector(s) and/or other transgenic vector(s) of this invention, becomes capable of stably transducing both dividing and non-dividing cell types.

In a preferred embodiment, the resulting Numb / Numblike encoding vector(s), and/or other transgenic vector(s) of this invention contain multiple synthetic oligonucleotide sequences driven by one or more promoters so as to reduce expression of specific numb isoforms and/or numblike.

Example 2: Another example of a suitable vector is a retroviral vector. Retroviruses are RNA viruses that contain an RNA genome. The gag, pol, and env genes are flanked by long terminal repeat (LTR) sequences. The 5' and 3' LTR sequences promote transcription and polyadenylation of mRNA's.

The retroviral vector may provide a regulable transactivating element, an internal ribosome reentry site (IRES), a selection marker, and a target heterologous gene operated by a regulable promoter.

Alternatively, multiple sequences may be expressed under the control of multiple promoters. Finally, the retroviral vector may contain cis-acting sequences necessary for reverse transcription and integration. Upon infection, the RNA is reverse transcribed to DNA that integrates efficiently into the host genome. The recombinant retrovirus of this invention is genetically modified in such a way that some of the retroviral, infectious genes of the native virus have been removed and in certain instances replaced instead with a target nucleic acid sequence for genetic modification of the cell. The sequences may be exogenous DNA or RNA, in its natural or altered form.

Example 3: Example methods for generation of Numb / Numblike encoding vector(s), and/or other transgenic vector(s) of this invention

The methods for generation of the resulting Numb / Numblike encoding vector(s), and/or other transgenic vector(s) of this invention include those taught in Invitrogen's Viral Power Lentiviral Expression Systems Manual, 2007. Briefly, the EmGFP-bsd cassette is cloned as a PmlI-BlpI fragment into the pLenti6/R4R2/V5-DEST vector, while the miR-long (PRR+) numb isoform or miR-short numb isoform/numblike cassettes are simultaneously transferred by BP reaction into pDONR221. Then the regulable promoter(s) and miR-isoform cassettes are Multisite LR crossed into the modified pLenti6/EmGFP-bsd/R4R2-DESTvector.

Multiple vectors can be generated in this manner comprising different combinations of synthetic oligonucleotides and transgene cassettes.

pLenti6/R4R2/V5-DEST vector sequence:

aatgtagtcttatgcaatactcttgtagtcttgcaacatggtaacgatgagttagcaacatgccttacaaggagagaaaaagcaccgtgcatgccgattggtggaagt
aaggtggtacgatcgtgccttattaggaaggcaacagacgggtctgacatggattggacgaaccactgaattgccgcattgcagagatattgtatttaagtgcctag
ctcgatacataaacgggtctctctggttagaccagatctgagcctgggagctctctggctaactagggaacccactgcttaagcctcaataaagcttgccttgagtg
cttcaagtagtgtgtgcccgtctgttgtgtgactctggtaactagagatccctcagacccttttagtcagtgtggaaaatctctagcagtggcgcccgaacagggact
tgaaagcgaaagggaaaccagaggagctctctcgacgcaggactcggcttgctgaagcgcgcacggcaagaggcgaggggcggcgactggtgagtacgcc
aaaaattttgactagcggaggctagaaggagagagatgggtgcgagagcgtcagtattaagcgggggagaattagatcgcgatgggaaaaaattcggttaagg
ccagggggaaagaaaaaatataaattaaaacatatagtatgggcaagcagggagctagaacgattcgcagttaatcctggcctgttagaaacatcagaaggctgt
agacaaatactgggacagctacaaccatcccttcagacaggatcagaagaacttagatcattatataatacagtagcaaccctctattgtgtgcatcaaaggataga
gataaaagacaccaaggaagctttagacaagatagaggaagagcaaaacaaaagtaagaccaccgcacagcaagcggccgctgatcttcagacctggagga
ggagatatgagggacaattggagaagtgaattatataaatataaagtagtaaaaattgaaccattaggagtagcacccaccaaggcaaagagaagagtggtgca
gagagaaaaaagagcagtgggaataggagctttgttccttgggttcttgggagcagcaggaagcactatgggcgcagcgtcaatgacgctgacggtacaggcc
agacaattattgtctggtatagtgcagcagcagaacaatttgctgagggctattgaggcgcaacagcatctgttgcaactcacagtctggggcatcaagcagctcc
aggcaagaatcctggctgtggaaagatacctaaaggatcaacagctcctggggatttggggttgctctggaaaactcatttgcaccactgctgtgccttggaatgct
agttggagtaataaatctctggaacagatttggaatcacacgacctggatggagtgggacagagaaattaacaattacacaagcttaatacactccttaattgaaga
atcgcaaaaccagcaagaaaagaatgaacaagaattattggaattagataaatgggcaagtttgtggaattggtttaacataacaaattggctgtggtatataaaatt
attcataatgatagtaggaggcttggtaggtttaagaatagtttttgctgtactttctatagtgaatagagttaggcagggatattcaccattatcgtttcagacccacctc
ccaaccccgaggggacccgacaggcccgaaggaatagaagaagaaggtggagagagagacagagacagatccattcgattagtgaacggatctcgacggt
atcgatgtcgacgttaacgctagtgatatcaactttgtatagaaaagttgaacgagaaacgtaaaatgatataaatatcaatatattaaattagattttgcataaaaaaca
gactacataatactgtaaaacacaacatatccagtcactatggcggccgcattaggcaccccaggctttacactttatgcttccggctcgtataatgtgtggattttga
gttaggatccgtcgagattttcaggagctaaggaagctaaaatggagaaaaaaatcactggatataccaccgttgatatatcccaatggcatcgtaaagaacattttg
aggcatttcagtcagttgctcaatgtacctataaccagaccgttcagctggatattacggcctttttaaagaccgtaaagaaaaataagcacaagttttatccggccttt
attcacattcttgcccgcctgatgaatgctcatccggaattccgtatggcaatgaaagacggtgagctggtgatatgggatagtgttcacccttgttacaccgttttcca
tgagcaaactgaaacgttttcatcgctctggagtgaataccacgacgatttccggcagtttctacacatatattcgcaagatgtggcgtgttacggtgaaaacctggc
ctatttccctaaagggtttattgagaatatgtttttcgtctcagccaatccctgggtgagtttcaccagttttgatttaaacgtggccaatatggacaacttcttcgccccc
gttttcaccatgggcaaatattatacgcaaggcgacaaggtgctgatgccgctggcgattcaggttcatcatgccgtttgtgatggcttccatgtcggcagaatgctt
aatgaattacaacagtactgcgatgagtggcagggcggggcgtaaagatctggatccggcttactaaaagccagataacagtatgcgtatttgcgcgctgatttttg
cggtataagaatatatactgatatgtatacccgaagtatgtcaaaaagaggtatgctatgaagcagcgtattacagtgacagttgacagcgacagctatcagttgctc
aaggcatatatgatgtcaatatctccggtctggtaagcacaaccatgcagaatgaagcccgtcgtctgcgtgccgaacgctggaaagcggaaaatcaggaaggg
atggctgaggtcgcccggtttattgaaatgaacggctcttttgctgacgagaacagggactggtgaaatgcagtttaaggtttacacctataaaagagagagccgtt
atcgtctgtttgtggatgtacagagtgatattattgacacgcccgggcgacggatggtgatccccctggccagtgcacgtctgctgtcagataaagtctcccgtgaa
ctttacccggtggtgcatatcggggatgaaagctggcgcatgatgaccaccgatatggccagtgtgccggtctccgttatcggggaagaagtggctgatctcagc
caccgcgaaaatgacatcaaaaacgccattaacctgatgttctggggaatataaatgtcaggctccgttatacacagccagtctgcaggtcgaccatagtgactgg
atatgttgtgttttacagtattatgtagtctgtttttttatgcaaaatctaatttaatatattgatatttatatcattttacgtttctcgttcagctttcttgtacaaagtggttgatatc
cagcacagtggcggccgctcgagtctagagggcccgcggttcgaaggtaagcctatccctaaccctctcctcggtctcgattctacgcgtaccggttagtaatga
gtttggaattaattctgtggaatgtgtgtcagttagggtgtggaaagtccccaggctccccagcaggcagaagtatgcaaagcatgcatctcaattagtcagcaacc
aggtgtggaaagtccccaggctccccagcaggcagaagtatgcaaagcatgcatctcaattagtcagcaaccatagtcccgcccctaactccgcccatcccgcc
cctaactccgcccagttccgcccattctccgccccatggctgactaattttttttatttatgcagaggccgaggccgcctctgcctctgagctattccagaagtagtga
ggaggcttttttggaggcctaggcttttgcaaaaagctcccgggagcttgtatatccattttcggatctgatcagcacgtgttgacaattaatcatcggcatagtatatc

ggcatagtataatacgacaaggtgaggaactaaaccatggccaagcctttgtctcaagaagaatccaccctcattgaaagagcaacggctacaatcaacagcatc
cccatctctgaagactacagcgtcgccagcgcagctctctctagcgacggccgcatcttcactggtgtcaatgtatatcattttactgggggaccttgtgcagaactc
gtggtgctgggcactgctgctgctgcggcagctggcaacctgacttgtatcgtcgcgatcggaaatgagaacaggggcatcttgagcccctgcggacggtgccg
acaggtgcttctcgatctgcatcctgggatcaaagccatagtgaaggacagtgatggacagccgacggcagttgggattcgtgaattgctgccctctggttatgtgt
gggagggctaagcacaattcgagctcggtacctttaagaccaatgacttacaaggcagctgtagatcttagccactttttaaaagaaaaggggggactggaaggg
ctaattcactcccaacgaagacaagatctgctttttgcttgtactgggtctctctggttagaccagatctgagcctgggagctctctggctaactagggaacccactgc
ttaagcctcaataaagcttgccttgagtgcttcaagtagtgtgtgcccgtctgttgtgtgactctggtaactagagatccctcagacccttttagtcagtgtggaaaatct
ctagcagtagtagttcatgtcatcttattattcagtatttataacttgcaaagaaatgaatatcagagagtgagaggaacttgtttattgcagcttataatggttacaaata
aagcaatagcatcacaaatttcacaaataaagcatttttttcactgcattctagttgtggtttgtccaaactcatcaatgtatcttatcatgtctggctctagctatcccgcc
cctaactccgcccatcccgcccctaactccgcccagttccgcccattctccgccccatggctgactaattttttttatttatgcagaggccgaggccgcctcggcctct
gagctattccagaagtagtgaggaggcttttttggaggcctagggacgtacccaattcgccctatagtgagtcgtattacgcgcgctcactggccgtcgttttacaac
gtcgtgactgggaaaaccctggcgttacccaacttaatcgccttgcagcacatccccctttcgccagctggcgtaatagcgaagaggcccgcaccgatcgccctt
cccaacagttgcgcagcctgaatggcgaatgggacgcgccctgtagcggcgcattaagcgcggcgggtgtggtggttacgcgcagcgtgaccgctacacttgc
cagcgccctagcgcccgctcctttcgctttcttcccttcctttctcgccacgttcgccggctttccccgtcaagctctaaatcgggggctccctttagggttccgattta
gtgctttacggcacctcgaccccaaaaaacttgattagggtgatggttcacgtagtgggccatcgccctgatagacggtttttcgccctttgacgttggagtccacgt
tctttaatagtggactcttgttccaaactggaacaacactcaaccctatctcggtctattcttttgatttataagggattttgccgatttcggcctattggttaaaaaatgag
ctgatttaacaaaaatttaacgcgaattttaacaaaatattaacgcttacaatttaggtggcacttttcggggaaatgtgcgcggaacccctatttgtttatttttctaaata
cattcaaatatgtatccgctcatgagacaataaccctgataaatgcttcaataatattgaaaaaggaagagtatgagtattcaacatttccgtgtcgcccttattcccttttt
tgcggcattttgccttcctgtttttgctcacccagaaacgctggtgaaagtaaaagatgctgaagatcagttgggtgcacgagtgggttacatcgaactggatctca
acagcggtaagatccttgagagttttcgccccgaagaacgttttccaatgatgagcacttttaaagttctgctatgtggcgcggtattatcccgtattgacgccgggca
agagcaactcggtcgccgcatacactattctcagaatgacttggttgagtactcaccagtcacagaaaagcatcttacggatggcatgacagtaagagaattatgca
gtgctgccataaccatgagtgataacactgcggccaacttacttctgacaacgatcggaggaccgaaggagctaaccgcttttttgcacaacatgggggatcatgt
aactcgccttgatcgttgggaaccggagctgaatgaagccataccaaacgacgagcgtgacaccacgatgcctgtagcaatggcaacaacgttgcgcaaactat
taactggcgaactacttactctagcttcccggcaacaattaatagactggatggaggcggataaagttgcaggaccacttctgcgctcggcccttccggctggctg
gtttattgctgataaatctggagccggtgagcgtgggtctcgcggtatcattgcagcactggggccagatggtaagccctcccgtatcgtagttatctacacgacgg
ggagtcaggcaactatggatgaacgaaatagacagatcgctgagataggtgcctcactgattaagcattggtaactgtcagaccaagtttactcatatatactttaga
ttgatttaaaacttcatttttaatttaaaaggatctaggtgaagatcctttttgataatctcatgaccaaaatcccttaacgtgagttttcgttccactgagcgtcagacccc
gtagaaaagatcaaaggatcttcttgagatcctttttttctgcgcgtaatctgctgcttgcaaacaaaaaaaccaccgctaccagcggtggtttgtttgccggatcaag
agctaccaactctttttccgaaggtaactggcttcagcagagcgcagataccaaatactgttcttctagtgtagccgtagttaggccaccacttcaagaactctgtagc
accgcctacatacctcgctctgctaatcctgttaccagtggctgctgccagtggcgataagtcgtgtcttaccgggttggactcaagacgatagttaccggataagg
cgcagcggtcgggctgaacggggggttcgtgcacacagcccagcttggagcgaacgacctacaccgaactgagatacctacagcgtgagctatgagaaagc
gccacgcttcccgaagggagaaaggcggacaggtatccggtaagcggcagggtcggaacaggagagcgcacgagggagcttccagggggaaacgcctgg
tatctttatagtcctgtcgggtttcgccacctctgacttgagcgtcgatttttgtgatgctcgtcaggggggcggagcctatggaaaaacgccagcaacgcggccttttt
acggttcctggccttttgctggccttttgctcacatgttctttcctgcgttatcccctgattctgtggataaccgtattaccgcctttgagtgagctgataccgctcgccg
cagccgaacgaccgagcgcagcgagtcagtgagcgaggaagcggaagagcgcccaatacgcaaaccgcctctccccgcgcgttggccgattcattaatgca
gctggcacgacaggtttcccgactggaaagcgggcagtgagcgcaacgcaattaatgtgagttagctcactcattaggcaccccaggctttacactttatgcttcc
ggctcgtatgttgtgtggaattgtgagcggataacaatttcacacaggaaacagctatgaccatgattacgccaagcgcgcaattaaccctcactaaagggaacaa
aagctggagctgcaagctt

Example 4: Additional methods for generation of therapeutic vector(s).

"Packaging cell lines" derived from human and/or animal fibroblast cell lines result from transfecting or infecting normal cell lines with viral gag, pol, and env structural genes. On the other hand, packaging cell lines produce RNA devoid of the psi sequence, so that the viral particles produced from packaging cell do not contain the gag, pol, or env genes. Once the therapeutic vector's DNA containing the psi sequence (along with the therapeutic gene) is introduced into the packaging cell, by means of transfection or infection, the packaging cell may produce virions capable of transmitting the therapeutic RNA to the final target cell (e.g. a CD4+ cell).

The "infective range" of the therapeutic vector(s) is determined by the packaging cell line. A number of packaging cell lines are available for production of virus suitable for infecting a broad range of human cell types. These packaging cell lines are nevertheless generally capable of encapsidating viral vectors derived from viruses that in nature usually infect different animal species. For example, vectors derived from SIV or MMLV can be packaged by GP120 encapsidating cell lines.

An example protocol for producing a therapeutic viral supernatant is provided as follows:

1. Twenty micrograms of retrovirus vector are mixed with 2-3 micrograms of viral DNA containing the selectable marker gene (e.g. antibiotic resistance gene) by gentle tapping in 0.8-1 milliliter of Hepes buffered saline (pH=7.05) in a 1.5 ml plastic tube.

2. Seventy microliters of 2M $CaCl^2$ are added to the mixture by repeated gentle tapping.

3. When a blue precipitate first begins to appear within the tube, the product should be gently applied to a 30% confluent layer of packaging cells (from any number of commercial vendors). The DNA mixture should be applied only after first removing the medium from the packaging cells.

4. The packaging cells are set to incubate for 20-30 minutes at room temperature (25 degrees Celsius) before transferring them back to an incubator at 36-38 degrees Celsius for 3.5 hours.

5. Add 3.5-4 milliliters of Hepes buffered saline containing 15% glycerol for 3 minutes then wash cell with Dulbecco's Modified Eagle's Medium (DMEM)+10% FBS x2.

6. Add back DMEM +10% FBS, and incubate cells for 20 hours at 37 degrees Celsius.

7. Remove and filter medium containing therapeutic viral particles.

Excess viral supernatant is immediately stored or concentrated and stored at -80 degrees Celsius). Supernatant may stored with 5-8 micrograms of polybrene to increase the efficiency of target cell infection. Otherwise polybrene may be excluded or added just before infection.

8. Stable producer lines can be established by splitting packaging cell lines 1 to 20, or 1 to 40 and subsequently incubating these cells for up to 10 days (changing medium every three days) in medium containing selective drugs (e.g. certain antibiotics corresponding to transfected resistance genes).

9. After 10 days isolated colonies are picked, grown-up aliquoted and frozen for storage.

Assay of Retrovirus Infectivity/Titration is achieved by application of a defined volume of viral supernatant to a layer of confluent "test" cells such as NIH 3T3 cells plated at 20% confluence. After 2-3 cell division times (24-36 hours for NIH 3T3 cells) colonies of "test" cells incubated at 37 degrees in antibiotic-containing medium are counted. The supernatant's titer are estimated from these colony counts by the following formula:

Colony Forming Units/ml=colonies identified X 0.5(split factor)/volume of virus (ml)

The accuracy of this estimate is increased by testing large volumes of supernatant over many plates of "test" cells.

Application of the therapeutic viral supernatant to target cells may be accomplished by various means appropriate to the clinical situation.

Example 5: Growth Medium for selected cells

Selected cells can be expanded / grown in Dulbecco's modified Minimal Essential Medium (DMEM) supplemented with glutamine, beta.-mercaptoethanol, 10% (by volume) horse serum, and human recombinant Leukemia Inhibitory Factor (LIF). LIF replaces the need for maintaining selected cells on feeder layers of cells, (which may also be employed) and is essential for maintaining selected cells in an undifferentiated, multipotent, or pluripotent state, such cells can be maintained in Dulbecco's modified Minimal Essential Medium (DMEM) supplemented with glutamine, beta.-mercaptoethanol, 10% (by volume) horse serum, and human recombinant Leukemia Inhibitory Factor (LIF). The LIF replaces the need for maintaining cells on feeder layers of cells, (which may also be employed) and is essential for maintaining cells in an undifferentiated state (per US Patent 6432711).

In order to initiate the differentiation of the selected cells into neuronal cells, the cells are trypsinized and washed free of LIF, and placed in DMEM supplemented with 10% fetal bovine serum (FBS). After resuspension in DMEM and 10% FBS, $1X10^6$ cells are plated in 5 ml DMEM, 10% FBS, 0.5 microM retinoic acid in a 60 mm Fisher bacteriological grade Petri dishes, where the cells are expected to form small aggregates. Aggregation aids in proper cell differentiation. High efficiency transfection with appropriate neuronal transcription factors can occur before or after plating in DMEM, FBS, and retinoic acid. (See U.S. Patents 6432711 and 5453357 for additional details).

Example 6: HLA matching. Selected cells (e.g. umbilical cord blood or cells from any other suitable source and/or their progeny), can be screened, genetically-modified (optional), expanded, and induced to begin differentiating into the desired cell type(s) (optional). The cells are then transplanted according to standard stem cell transplantation protocols. In certain instances, cells may be transplanted into patients without HLA matching.

Example 7: In some rare instance, it may be appropriate to introduce transgene encoding vectors into patients in order to stimulate or inhibit cellular division or cellular differentiation, in vivo.

Example 8: Genetic Modification of Selected Cells.

In vitro genetic modification of exogenous cells or patient's endogenous cells can be performed according to any published or unpublished method known to the art (e.g. U.S. Pat. Nos. 6,432,711, U.S. 05,593,875, U.S. 05,783,566, U.S. 5,928,944, U.S. 05,910,488, U.S. 05,824,547, etc.) or by other generally accepted means. Suitable methods for transforming host cells can be found in Sambrook et al. (Molecular Cloning: A Laboratory Manual, 2nd Edition, Cold Spring Harbor Laboratory press (1989)), and other laboratory textbooks.

Successfully transfected cells are identified by selection protocols involving markers such as antibiotic resistance genes in addition to RNA expression assays and morphological analyses. Clones from successfully transfected cells, expressing the appropriate exogenous DNA at appropriate levels, can be preserved as cell lines by cryopreservation (utilizing any appropriate method of cryopreservation known to the art).

Selectable markers (e.g., antibiotics resistance genes) may include those conferring resistance to drugs, such as G418, hygromycin and methotrexate. Cells containing the gene of interest can be identified by drug selection where cells that have incorporated the selectable marker gene survive, and others die.

The current invention discloses the selection of genetically-modified cells as "selected cells" of the invention. The term genetic modification refers to alteration of the cellular genotype by introducing natural or synthetic nucleic acids into selected cells and/or their progeny or immortalized cell lines and/or their progeny by any means known to the art. Alternatively culture conditions that induce permanent changes in gene expression patterns are considered herein to represent genetic modification. Modification of stem cells, whether they be derived from the host brain, endogenous donor sources, exogenous donor sources, or cell lines, represents a feasible approach to the treatment of certain human diseases, especially those of the human nervous system.

Genetic modifications covered by this disclosure include, but are not limited to: genetic modifications performed in vivo; modifications that alter the activity or amount of metabolic enzymes expressed by endogenous or exogenous selected cells and/or their progeny; modifications which alter the activity, amount, or antigenicity of cellular proteins; modifications which alter the activity or amount of proteins involved in signal transduction pathways; modifications which alter HLA type; modifications which alter cellular differentiation; modifications which alter neoplastic potential; modifications which alter cellular differentiation; modifications which alter the amount or activity of structural proteins; modifications which after the amount or activity of membrane associated proteins (structural or enzymatic); modifications which alter the activity or amount of proteins involved in DNA repair and chromosome maintenance; modifications which alter the activity or amount of proteins involved in cellular transport; modifications which alter the activity or amount of enzymes; modifications which alter the activity or amount of proteins involved in synapse formation and maintenance; modifications which alter the activity or amount of proteins involved in neurite outgrowth or axon outgrowth and formation; modifications altering the amount or activity of antioxidant producing enzymes within the cell; modifications which lead to altered post-translational modification of cellular proteins; modifications which alter the activity or amount of proteins involved in other aspects of cellular repair, and alterations which increase the lifespan of the cell (such as production of telomerase). Such proteins as those mentioned above may be encoded for by DNA or RNA derived from the human genome or other animal, plant, viral, or bacterial genomes. This invention also covers sequences designed de novo.

In addition, this invention relates to the in situ, genetic modification of selected cells and/or their progeny cells for the treatment of disease. Endogenous stem cells may be modified in situ by direct injection or application of DNA or RNA vectors, including viruses, retroviruses, liposomes, etc, into the substance of the tissue or into the appropriate portion of the ventricular system of the brain. Since 1992, we have modified thousands of stem/progenitor cells and many thousand progeny cells in this manner. Our data shows that this manner of modifying progenitor cells results in a tremendous variety of modified cell types throughout the nervous system, and has never resulted in adverse effects.

Example 9: Introduction of Genetic Vectors into the Host

In a preferred embodiment, endogenous cells are transfected with vectors such as those described herein in vivo by introduction of the therapeutic vector(s) into the host blood, tissues, nervous system, bone marrow, etc. The greatest benefit may be achieved by modifying a large number of endogenous target cells. This may be accomplished by using an appropriately-sized, catheter-like device, or needle to inject the therapeutic vector(s) into the venous or arterial circulation, into a specific tissue, such as muscle tissue, or into the nervous system. In a preferred embodiment, the virus is pseudotyped with VSV-G envelope glycoprotein and native HIV-1 env proteins.

Example 10: Injection into the Nervous System

Transplantation of selected cells (from either the growth or differentiation media) into the fetal nervous system or genetic modification of endogenous fetal cells utilizing genetic vectors may be accomplished in the following manner: Under sterile conditions, the uterus and fetuses are visualized by ultrasound or other radiological guidance. Alternatively the uterus may be exposed surgically in order to facilitate direct identification of fetal skull landmarks. Selected cells can then be introduced by injection (using an appropriately-sized catheter or needle) into the ventricular system, germinal zone(s), or into the substance of the nervous system. Injections may be performed in certain instances, through the mother's abdominal wall. the uterine wall and fetal membranes into the fetus. The accuracy of the injection is monitored by direct observation, ultrasound, contrast, or radiological isotope based methods, or by any other means of radiological guidance known to the art.

Under appropriate sterile conditions, direct identification of fetal skull landmarks is accomplished visually as well as by physical inspection and palpation coupled with stereotaxic and radiologic guidance. Following cell culture, appropriate amounts of the selected or differentiating cells can then be introduced by injection or other means into the ventricular system, germinal zones, or into the substance of the nervous system. The accuracy of the injection may be monitored by direct observation, ultrasound, or other radiological guidance.

In certain, neurological diseases of the adult nervous system, such as Huntington's disease and Parkinson's disease, cells of a specific portion of the brain are selectively affected. In the case of Parkinson's disease, it is the dopaminergic cells of the substantia nigra. In such regionally-specific diseases affecting adults, localized transplantation of cells may be accomplished by radiologically-guided transplantation of differentiating cells under sterile conditions. Radiologic guidance may include the use of CT and/or MRI, and may take advantage contrast or isotope based techniques to monitor injected materials.

In certain neurologic diseases, such as some metabolic storage disorders, cells are affected across diverse regions of the nervous system, and the greatest benefit may be achieved by genetically-modifying endogenous cells or introducing selected cells of the present invention (either from the growth culture media or the differentiating medium) into the tissue in large numbers in a diffuse manner. In the nervous system, these diseases may be best approached by intraventricular injections (using an appropriately-sized, catheter-like device, or needle) (especially at early stages of development) which allows diffuse endogenous cell modification or diffuse engraftment of selected cells isolated from the growth and/or differentiation media. Nevertheless, injection of the cells into the circulatory system for the same purpose is also covered. However, with regard to any disorder affecting multiple organs or the body diffusely (e.g. lysosomal storage disorders, hemoglobinpathies, muscular dystrophy), the cells isolated from the growth and/or differentiation media may also be preferentially introduced directly into the circulation and/or visceral organs, such as the liver, kidney, gut, spleen, adrenal glands, pancreas, lungs, and thymus using endoscopic guidance and any appropriately-sized, catheter-like device, allowing diffuse engraftment of the cells throughout the body, as well as specific introduction and infiltration of the cells into the selected organs.

Example 11: Delivery of Cells by Injection in to the Circulatory Stream and Organs.

Diseases of one organ system may be treatable with genetically modified cells from a separate organ system. Also, in some instances, it may become apparent that the selected cells may integrate and differentiate on their own, in vivo, in sufficient numbers if they are injected into blood stream either arterial, venous or hepatic, after culturing in the growth and/or differentiation media. This approach is covered by the present invention. The treatment of diffuse muscle (e.g. muscular dystrophies), organ, tissue, or blood disorders (e.g. Hereditary Spherocytosis, Sickle cell anemia, other hemoglobinopathies, etc,) may, for instance, involve the injection of cells isolated from the growth media or differentiating media into the patient, especially the patient's circulation. This approach is also believed to ameliorate ischemic injuries such as myocardial infarction, stroke, etc., as well as traumatic injuries to brain and other tissues. Injection of such cells produced by the current invention, directly into the circulation, by needle or catheter, so that the cells are enabled to "home" to the bone marrow, muscle, kidneys, lungs, and/or any other other organ system, as well as injection directly into the bone marrow space is suitable for the practice of the present invention. Likewise injection of the cells directly into a lesion site with or without radiologic, ultrasonic or fluoroscopic guidance is also suitable for the practice of the present invention.

Methods of isolating selected cells useful in the present invention include those described by Zhao et al., 2006.

In a preferred embodiment, genetic vectors encoding numblike and/or numb isoforms comprise regulable promoters operably linked to the Numb or numblike transgenes.

In another preferred embodiment, the mode of transfection may be selected from those modes of transfection that provide for transient rather than permanent expression of the numblike and numb isoforms.

Example 12. Example Genetic Modifications

It is believed that hundreds of diseases and clinical conditions are able to be treated and/or ameliorated by the methods of the present invention including, but in no way limited to Canavan's disease (ASP); Tay-Sach's disease (HEXA); Lesch-Nyhan syndrome (HRPT); Huntington's disease(HTT); Sly syndrome; type A and type B Niemann Pick disease; Sandhoffs disease (HEXB); Fabry's disease (GLA); type C Niemann-Pick disease(NPC1); Gaucher's disease (GBA); Parkinson's disease(PARK2, etc.); Von Hippel Lindau's disease, Sickle cell anemia (HBB) and other thalassemias as well as similar diseases. These transgenes may represent the coding region or portions of the coding region of the normal genes.

It is to be understood, however, that the scope of the present invention is not to be limited to the specific embodiments and examples described above. The invention may be practiced other than as particularly described and still be within the scope of the accompanying claims.

Example 13: An example sequence for a vector capable of rendering cells pluripotent and expressing a long Numb isoform, Oct-4, Sox-2, and EmGFP nucleic acid sequences under the control of tetracycline-sensitive promoters is:

aatgtagtcttatgcaatactcttgtagtcttgcaacatggtaacgatgagttagcaacatgccttacaaggagagaaaaagcaccgtgcatgccgattggtggaagt
aaggtggtacgatcgtgccttattaggaaggcaacagacgggtctgacatggattggacgaaccactgaattgccgcattgcagagatattgtatttaagtgcctag
ctcgatacataaacgggtctctctggttagaccagatctgagcctgggagctctctggctaactagggaacccactgcttaagcctcaataaagcttgccttgagtg
cttcaagtagtgtgtgcccgtctgttgtgtgactctggtaactagagatccctcagacccttttagtcagtgtggaaaatctctagcagtggcgcccgaacagggact
tgaaagcgaaagggaaaccagaggagctctctcgacgcaggactcggcttgctgaagcgcgcacggcaagaggcgaggggcggcgactggtgagtacgcc
aaaaattttgactagcggaggctagaaggagagagatgggtgcgagagcgtcagtattaagcgggggagaattagatcgcgatgggaaaaaattcggttaagg
ccagggggaaagaaaaaatataaattaaaacatatagtatgggcaagcagggagctagaacgattcgcagttaatcctggcctgttagaaacatcagaaggctgt
agacaaatactgggacagctacaaccatcccttcagacaggatcagaagaacttagatcattatataatacagtagcaaccctctattgtgtgcatcaaaggataga
gataaaagacaccaaggaagctttagacaagatagaggaagagcaaaacaaaagtaagaccaccgcacagcaagcggccgctgatcttcagacctggagga
ggagatatgagggacaattggagaagtgaattatataaatataaagtagtaaaaattgaaccattaggagtagcacccaccaaggcaaagagaagagtggtgca
gagagaaaaaagagcagtgggaataggagctttgttccttgggttcttgggagcagcaggaagcactatgggcgcagcgtcaatgacgctgacggtacaggcc
agacaattattgtctggtatagtgcagcagcagaacaatttgctgagggctattgaggcgcaacagcatctgttgcaactcacagtctggggcatcaagcagctcc
aggcaagaatcctggctgtggaaagatacctaaaggatcaacagctcctggggatttggggttgctctggaaaactcatttgcaccactgctgtgccttggaatgct
agttggagtaataaatctctggaacagatttggaatcacacgacctggatggagtgggacagagaaattaacaattacacaagcttaatacactccttaattgaaga
atcgcaaaaccagcaagaaaagaatgaacaagaattattggaattagataaatgggcaagtttgtggaattggtttaacataacaaattggctgtggtatataaaatt
attcataatgatagtaggaggcttggtaggtttaagaatagtttttgctgtactttctatagtgaatagagttaggcagggatattcaccattatcgtttcagacccacctc
ccaaccccgaggggacccgacaggcccgaaggaatagaagaagaaggtggagagagagacagagacagatccattcgattagtgaacggatctcgacggt
atcgatgtcgacgttaacgctagtgatatcaactttgtatagaaaagttgaacgagaaacgtaaaatgatataaatatcaatatattaaattagattttgcataaaaaaca
gactacataatactgtaaaacacaacatatccagtcactatgggacggatcgggagatctcccgatcccctatggtgcactctcagtacaatctgctctgatgccgc

atagttaagccagtatctgctccctgcttgtgtgttggaggtcgctgagtagtgcgcgagcaaaatttaagctacaacaaggcaaggcttgaccgacaattgcatga
agaatctgcttagggttaggcgttttgcgctgcttcgcgatgtacgggccagatatacgcgttgacattgattattgactagttattaatagtaatcaattacggggtcat
tagttcatagcccatatatggagttccgcgttacataacttacggtaaatggcccgcctggctgaccgcccaacgacccccgcccattgacgtcaataatgacgtat
gttcccatagtaacgccaatagggactttccattgacgtcaatgggtggagtatttacggtaaactgcccacttggcagtacatcaagtgtatcatatgccaagtacg
ccccctattgacgtcaatgacggtaaatggcccgcctggcattatgcccagtacatgaccttatgggactttcctacttggcagtacatctacgtattagtcatcgctat
taccatggtgatgcggttttggcagtacatcaatgggcgtggatagcggtttgactcacggggatttccaagtctccaccccattgacgtcaatgggagtttgttttgg
aaccaaaatcaacgggactttccaaaatgtcgtaacaactccgccccattgacgcaaatgggcggtaggcgtgtacggtgggaggtctatataagcagagctctc
cctatcagtgatagagatctccctatcagtgatagagatcgtcgacgagctcgtttagtgaaccgtcagatcgcctggagacgccatccacgctgttttgacctccat
agaagacaccgggaccgatccagcctccggactctagcgtttaaacttaagcttaccatgccttcgcaagccctcatttcaccaggcccccggcttggggcgcctt
ccttccccatggcgggacacctggcttcggatttcgccttctcgcccccctccaggtggtggaggtgatgggccaggggggccggagccgggctgggttgatcct
cggacctggctaagcttccaaggccctcctggagggccaggaatcgggccgggggttgggccaggctctgaggtgtgggggattcccccatgccccccgccg
tatgagttctgtgggggggatggcgtactgtgggccccaggttggagtggggctagtgccccaaggcggcttggagacctctcagcctgagggcgaagcagga
gtcggggtggagagcaactccgatggggcctccccggagccctgcaccgtcacccctggtgccgtgaagctggagaaggagaagctggagcaaaacccgg
aggagtcccaggacatcaaagctctgcagaaagaactcgagcaatttgccaagctcctgaagcagaagaggatcaccctgggatatacacaggccgatgtggg
gctcaccctgggggttctatttgggaaggtattcagccaaacgaccatctgccgctttgaggctctgcagcttagcttcaagaacatgtgtaagctgcggcccttgct
gcagaagtgggtggaggaagctgacaacaatgaaaatcttcaggagatatgcaaagcagaaaccctcgtgcaggcccgaaagagaaagcgaaccagtatcga
gaaccgagtgagaggcaacctggagaatttgttcctgcagtgcccgaaacccacactgcagcagatcagccacatcgcccagcagcttgggctcgagaaggat
gtggtccgagtgtggttctgtaaccggcgccagaagggcaagcgatcaagcagcgactatgcacaacgagaggattttgaggctgctgggtctcctttctcaggg
ggaccagtgtcctttcctctggccccagggccccattttggtaccccaggctatgggagccctcacttcactgcactgtactcctcggtccctttccctgaggggga
agcctttccccctgtctccgtcaccactctgggctctcccatgcattcaaactgaggtgcctgcccttctaggaatgggggacagggggaggggaggagctagg
gaaagaaaacctggagtttgtgccagggtttttgggattaagttcttcattcactaaggaaggaattgggaacacaaagggtgggggcaggggagtttggggcaa
ctggttggagggaaggtgaagttcaatgatgctcttgattttaatcccacatcatgtatcacttttttcttaaataaagaagcctgggacacagtagatagacacactta
aaaaaaaaaacctcgactgtgccttctagttgccagccatctgttgtttgcccctcccccgtgccttccttgaccctggaaggtgccactcccactgtcctttcctaata
aaatgaggaaattgcatcgcattgtctgagtaggtgtcattctattctggggggtggggtggggcaggacagcaagggggaggattgggaagacaatagcagg
catgctggggatgcggtgggctctatgggacggatcgggagatctcccgatcccctatggtgcactctcagtacaatcttgctctgatgccgcatagttaagccagt
atctgctccctgcttgtgtgttggaggtcgctgagtagtgcgcgagcaaaatttaagctacaacaaggcaaggcttgaccgacaattgcatgaagaatctgcttagg
gttaggcgttttgcgctgcttcgcgatgtacgggccagatatacgcgttgacattgattattgactagttattaatagtaatcaattacggggtcattagttcatagccca
tatatggagttccgcgttacataacttacggtaaatggcccgcctggctgaccgcccaacgacccccgcccattgacgtcaataatgacgtatgttcccatagtaac
gccaatagggactttccattgacgtcaatgggtggagtatttacggtaaactgcccacttggcagtacatcaagtgtatcatatgccaagtacgccccctattgacgt
caatgacggtaaatggcccgcctggcattatgcccagtacatgaccttatgggactttcctacttggcagtacatctacgtattagtcatcgctattaccatggtgatg
cggttttggcagtacatcaatgggcgtggatagcggtttgactcacggggatttccaagtctccaccccattgacgtcaatgggagtttgttttggaaccaaaatcaa
cgggactttccaaaatgtcgtaacaactccgccccattgacgcaaatgggcggtaggcgtgtacggtgggaggtctatataagcagagctctccctatcagtgata
gagatctccctatcagtgatagagatcgtcgacgagctcgtttagtgaaccgtcagatcgcctggagacgccatccacgctgttttgacctccatagaagacaccg
ggaccgatccagcctccggactctagcgtttaaacttaagcttaccatgctattaacttgttcaaaaagtatcaggagttgtcaaggcagagaagagagtgtttgca
aaagggggaaagtagtttgctgcctctttaagactaggactgagagaaagaagaggagagagaaagaaagggagagaagtttgagccccaggcttaagccttt
ccaaaaaataataataacaatcatcggcggcggcaggatcggccagaggaggagggaagcgctttttttgatcctgattccagtttgcctctctcttttttcccccaa
attattcttcgcctgatttcctcgcggagccctgcgctcccgacacccccgcccgcctcccctcctcctctcccccgcccgcgggcccccaaagtcccggccg
ggccgagggtcggcggccgccggcgggccgggcccgcgcacagcgcccgcatgtacaacatgatggagacggagctgaagccgccgggcccgcagcaa
acttcgggggcggcggcggcaactccaccgcggcggcggccggcggcaaccagaaaaacagcccggaccgcgtcaagcggcccatgaatgccttcatg

gtgtggtcccgcgggcagcggcgcaagatggcccaggagaaccccaagatgcacaactcggagatcagcaagcgcctgggcgccgagtggaaacttttgtc
ggagacggagaagcggccgttcatcgacgaggctaagcggctgcgagcgctgcacatgaaggagcacccggattataaataccggccccggcggaaaacc
aagacgctcatgaagaaggataagtacacgctgcccggcgggctgctggcccccggcggcaatagcatggcgagcggggtcggggtgggcgccggcctgg
gcgcgggcgtgaaccagcgcatggacagttacgcgcacatgaacggctggagcaacggcagctacagcatgatgcaggaccagctgggctacccgcagca
cccgggcctcaatgcgcacggcgcagcgcagatgcagcccatgcaccgctacgacgtgagcgccctgcagtacaactccatgaccagctcgcagacctacat
gaacggctcgcccacctacagcatgtcctactcgcagcagggcacccctggcatggctcttggctccatgggttcggtggtcaagtccgaggccagctccagcc
cccctgtggttacctcttcctcccactccagggcgccctgccaggccggggacctccgggacatgatcagcatgtatctccccggcgccgaggtgccggaaccc
gccgcccccagcagacttcacatgtcccagcactaccagagcggcccggtgcccggcacggccattaacggcacactgcccctctcacacatgtgagggccg
gacagcgaactggagggggggagaaattttcaaagaaaaacgagggaaatgggaggggtgcaaaagaggagagtaagaaacagcatggagaaaacccggta
cgctcaaaaagaaaaaggaaaaaaaaaaatcccatcacccacagcaaatgacagctgcaaaagagaacaccaatcccatccacactcacgcaaaaaccgcga
tgccgacaagaaaacttttatgagagagatcctggacttctttttgggggactatttttgtacagagaaaacctggggagggtggggagggcgggggaatggacc
ttgtatagatctggaggaaagaaagctacgaaaaactttttaaaagttctagtggtacggtaggagctttgcaggaagtttgcaaaagtctttaccaataatatttagag
ctagtctccaagcgacgaaaaaaatgttttaatatttgcaagcaacttttgtacagtatttatcgagataaacatggcaatcaaaatgtccattgtttataagctgagaatt
tgccaatatttttcaaggagaggcttcttgctgaattttgattctgcagctgaaatttaggacagttgcaaacgtgaaaagaagaaaattattcaaatttggacattttaat
tgtttaaaaattgtacaaaaggaaaaaattagaataagtactggcgaaccatctctgtggtcttgtttaaaaagggcaaaagttttagactgtactaaattttataacttac
tgttaaaagcaaaaatggccatgcaggttgacaccgttggtaatttataatagcttttgttcgatcccaactttccattttgttcagataaaaaaaaccatgaaattactgt
gtttgaaatatttcttatggtttgtaatatttctgtaaatttattgtgatattttaaggttttcccccctttattttccgtagttgtattttaaaagattcggctctgtattatttgaat
cagtctgccgagaatccatgtatatatttgaactaatatcatccttataacaggtacattttcaacttaagtttttactccattatgcacagtttgagataaataaattttgaa
atatggacactgaaaaaaaaaaaaaaaaaacctcgactgtgccttctagttgccagccatctgttgtttgcccctcccccgtgccttccttgaccctggaaggtgcca
ctcccactgtcctttcctaataaaatgaggaaattgcatcgcattgtctgagtaggtgtcattctattctggggggtggggtggggcaggacagcaagggggagga
ttgggaagacaatagcaggcatgctggggatgcggtgggctctatgggacggatcgggagatctcccgatcccctatggtgcactctcagtacaatctgctctgat
gccgcatagttaagccagtatctgctccctgcttgtgtgttggaggtcgctgagtagtgcgcgagcaaaatttaagctacaacaaggcaaggcttgaccgacaatt
gcatgaagaatctgcttagggttaggcgttttgcgctgcttcgcgatgtacgggccagatatacgcgttgacattgattattgactagttattaatagtaatcaattacg
gggtcattagttcatagcccatatatggagttccgcgttacataacttacggtaaatggcccgcctggctgaccgcccaacgacccccgcccattgacgtcaataat
gacgtatgttcccatagtaacgccaatagggactttccattgacgtcaatgggtggagtatttacggtaaactgcccacttggcagtacatcaagtgtatcatatgcca
agtacgccccctattgacgtcaatgacggtaaatggcccgcctggcattatgcccagtacatgaccttatgggactttcctacttggcagtacatctacgtattagtca
tcgctattaccatggtgatgcggttttggcagtacatcaatgggcgtggatagcggtttgactcacggggatttccaagtctccaccccattgacgtcaatgggagttt
gttttggaaccaaaatcaacgggactttccaaaatgtcgtaacaactccgccccattgacgcaaatgggcggtaggcgtgtacggtgggaggtctatataagcag
agctctccctatcagtgatagagatctccctatcagtgatagagatcgtcgacgagctcgtttagtgaaccgtcagatcgcctggagacgccatccacgctgttttga
cctccatagaagacaccgggaccgatccagcctccggactctagcgtttaaacttaagcttaccatggttgtcatgggggaggtggtggcgcttggtggccactg
gcggccgaggtagaggcagtggcgcttgagttggtcgggggcagcggcagatttgaggcttaagcaacttcttccggggaagagtgccagtgcagccactgtt
acaattcaagatcttgatctatatccatagattggaatattggtgggccagcaatcctcagacgcctcacttaggacaaatgaggaaactgaggcttggtgaagttac
gaaacttgtccaaaatcacacaacttgtaaagggcacagccaagattcagagccaggctgtaaaaattaaaatgaacaaattacggcaaagttttaggagaaaga
aggatgtttatgttccagaggccagtcgtccacatcagtggcagacagatgaagaaggcgttcgcaccggaaaatgtagcttcccggttaagtaccttggccatgt
agaagttgatgaatcaagaggaatgcacatctgtgaagatgctgtaaaaagattgaaagctgaaaggaagttcttcaaaggcttctttggaaaaactggaaagaaa
gcagttaaagcagttctgtgggtctcagcagatggactcagagttgtggatgaaaaaactaaggacctcatagttgaccagacgatagagaaagtttctttctgtgc
cccagacaggaactttgatagagcctttttcttacatatgccgtgatggcaccactcgtcgctggatctgtcactgcttcatggctgtcaaggacacaggtgaaaggtt
gagccatgcagtaggctgtgcttttgcagcctgtttagagcgcaagcagaagcgggagaaggaatgtggagtgactgctacttttgatgctagtcggaccactttta
caagagaaggatcattccgtgtcacaacagccactgaacaagcagaaagagaggagatcatgaaacaaatgcaagatgccaagaaagctgaaacagataaga

tagtcgttggttcatcagttgcccctggcaacactgccccatccccatcctctcccacctctcctacttctgatgccacgacctctctggagatgaacaatcctcatgc
catcccacgccggcatgctccaattgaacagcttgctcgccaaggctctttccgaggttttcctgctcttagccagaagatgtcaccctttaaacgccaactatcccta
cgcatcaatgagttgccttccactatgcagaggaagactgatttccccattaaaaatgcagtgccagaagtagaaggggaggcagagagcatcagctccctgtgc
tcacagatcaccaatgccttcagcacacctgaggacccccttctcatctgctccgatgaccaaaccagtgacagtggtggcaccacaatctcctaccttccaagctaa
tggcactgactcagccttccatgtgcttgctaagccagcccatactgctctagcacccgtagcaatgcctgtgcgtgaaaccaacccttgggcccatgcccctgatg
ctgctaacaaggaaattgcagccacatgttcggggaccgagtggggtcaatcttctggtgctgcctctccaggtctcttccaggccggtcatagacgtactccctct
gaggccgaccgatggttagaagaggtgtctaagagcgtccgggctcagcagccccaggcctcagctgctcctctgcagccagttctccagcctcctccacccac
tgccatctcccagccagcatcacctttccaagggaatgcattcctcacctctcagcctgtgccagtgggtgtggtcccagccctgcaaccagcctttgtccctgccc
agtcctatcctgtggccaatggaatgccctatccagcccctaatgtgcctgtggtgggcatcactccctcccagatggtggccaacgtatttggcactgcaggcca
ccctcaggctgcccatccccatcagtcacccagcctggtcaggcagcagacattccctcactacgaggcaagcagtgctaccaccagtcccttctttaagcctcct
gctcagcacctcaacggttctgcagctttcaatggtgtagatgatggcaggttggcctcagcagacaggcatacagaggttcctacaggcacctgcccagtggat
cctttgaagcccagtgggctgcattagaaaataagtccaagcagcgtactaatccctcccctaccaaccctttctccagtgacttacagaagacgtttgaaattgaa
ctttaagcaatcattatggctatgtatcttgtccataccagacagggagcagggggtagcggtcaaaggagcaaaacagactttgtctcctgattagtactcttttcac
taatcccaaaggtcccaaggaacaagtccaggcccagagtactgtgaggggtgatttgaaagacatgggaaaaagcattcctagagaaaagctgccttgcaatt
aggctaaagaagtcaaggaaatgttgctttctgtactccctcttcccttaccccttacaaatctctggcaacagagaggcaaagtatctgaacaagaatctatattcc
aagcacatttactgaaatgtaaaacacaacaggaagcaaagcaatctccctttgtttttcaggccattcacctgcctcctgtcagtagtggcctgtattagagatcaag
aagagtggtttgtgctcaggctggggaacagagaggcacgctatgctgccagaattcccaggagggcatatcagcaactgcccagcagagctatatttggggg
agaagttgagcttccatttgagtaacagaataaatattatatatatcaaaagccaaaatctttatttttatgcatttagaatattttaaatagttctcagatattaagaagttg
tatgagttgtaagtaatcttgccaaaggtaaaggggctagttgtaagaaattgtacataagattgatttatcattgatgcctactgaaataaaaagaggaaaggctgga
agctgcagacaggatccctagcttgttttctgtcagtcattcattgtaagtagcacattgcaacaacaatcatgcttatgaccaatacagtcactaggttgtagtttttttta
aataaaggaaaagcagtattgtcctggttttaaacctatgatggaattctaatgtcattattttaatggaatcaatcgaaatatgctctatagagaatatatcttttatatatt
gctgcagtttccttatgttaatcctttaacactaaggtaacatgacataatcataccatagaagggaacacaggttaccatattggtttgtaatatgggtcttggtgggttt
tgttttatcctttaaatttgttcccatgagttttgtggggatggggattctggttttattagctttgtgtgtgtcctcttcccccaaacccccttttggtgagaacatccccttg
acagttgcagcctcttgacctcggataacaataagagagctcatctcatttttacttttgaacgttggccttacaatcaaatgtaagttatatatatttgtactgatgaaaat
ttataatctgctttaacaaaaataaatgttcatggtagaagcttttaaaaaaaaaaaaacctcgactgtgccttctagttgccagccatctgttgtttgcccctcccccgtg
ccttccttgaccctggaaggtgccactcccactgtcctttcctaataaaatgaggaaattgcatcgcattgtctgagtaggtgtcattctattctggggggtggggtgg
ggcaggacagcaagggggaggattgggaagacaatagcaggcatgctggggatgcggtgggctctatgggacggatcgggagatctcccgatcccctatggt
gcactctcagtacaatctgctctgatgccgcatagttaagccagtatctgctccctgcttgtgtgttggaggtcgctgagtagtgcgcgagcaaaatttaagctacaa
caaggcaaggcttgaccgacaattgcatgaagaatctgcttagggttaggcgttttgcgctgcttcgcgatgtacgggccagatatacgcgttgacattgattattga
ctagttattaatagtaatcaattacggggtcattagttcatagcccatatatggagttccgcgttacataacttacggtaaatggcccgcctggctgaccgcccaacga
cccccgcccattgacgtcaataatgacgtatgttcccatagtaacgccaatagggactttccattgacgtcaatgggtggagtatttacggtaaactgcccacttggc
agtacatcaagtgtatcatatgccaagtacgccccctattgacgtcaatgacggtaaatggcccgcctggcattatgcccagtacatgaccttatgggactttcctact
tggcagtacatctacgtattagtcatcgctattaccatggtgatgcggttttggcagtacatcaatgggcgtggatagcggtttgactcacggggatttccaagtctcc
accccattgacgtcaatgggagtttgttttggaaccaaaatcaacgggactttccaaaatgtcgtaacaactccgccccattgacgcaaatgggcggtaggcgtgta
cggtgggaggtctatataagcagagctctccctatcagtgatagagatctccctatcagtgatagagatcgtcgacgagctcgtttagtgaaccgtcagatcgcctg
gagacgccatccacgctgttttgacctccatagaagacaccgggaccgatccagcctccggactctagcgtttaaacttaagcttaccatggtgagcaagggcga
ggagctgttcaccggggtggtgcccatcctggtcgagctggacggcgacgtaaacggccacaagttcagcgtgtccggcgagggcgagggcgatgccaccta
cggcaagctgaccctgaagttcatctgcaccaccggcaagctgcccgtgccctggcccaccctcgtgaccaccttcacctacggcgtgcagtgcttcgcccgcta
ccccgaccacatgaagcagcacgacttcttcaagtccgccatgcccgaaggctacgtccaggagcgcaccatcttcttcaaggacgacggcaactacaagacc

cgcgccgaggtgaagttcgagggcgacaccctggtgaaccgcatcgagctgaagggcatcgacttcaaggaggacggcaacatcctggggcacaagctgga
gtacaactacaacagccacaaggtctatatcaccgccgacaagcagaagaacggcatcaaggtgaacttcaagacccgccacaacatcgaggacggcagcgt
gcagctcgccgaccactaccagcagaacacccccatcggcgacggccccgtgctgctgcccgacaaccactacctgagcacccagtccgccctgagcaaag
accccaacgagaagcgcgatcacatggtcctgctggagttcgtgaccgccgccgggatcactctcggcatggacgagctgtacaagtaacctcgactgtgcctt
ctagttgccagccatctgttgtttgcccctcccccgtgccttccttgaccctggaaggtgccactcccactgtcctttcctaataaaatgaggaaattgcatcgcattgt
ctgagtaggtgtcattctattctggggggtggggtggggcaggacagcaagggggaggattgggaagacaatagcaggcatgctggggatgcggtgggctct
atgggacggatcgggagatctcccgatcccctatggtgcactctcagtacaatctgctctgatgccgcatagttaagccagtatctgctccctgcttgtgtgttggag
gtcgctgagtagtgcgcgagcaaaatttaagctacaacaaggcaaggcttgaccgacaattgcatgaagaatctgcttagggttaggcgttttgcgctgcttcgcg
atgtacgggccagatatacgcgttgacattgattattgactagttattaatagtaatcaattacggggtcattagttcatagcccatatatggagttccgcgttacataac
ttacggtaaatggcccgcctggctgaccgcccaacgacccccgcccattgacgtcaataatgacgtatgttcccatagtaacgccaatagggactttccattgacgt
caatgggtggagtatttacggtaaactgcccacttggcagtacatcaagtgtatcatatgccaagtacgccccctattgacgtcaatgacggtaaatggcccgcctg
gcattatgcccagtacatgaccttatgggactttcctacttggcagtacatctacgtattagtcatcgctattaccatggtgatgcggttttggcagtacatcaatgggc
gtggatagcggtttgactcacggggatttccaagtctccaccccattgacgtcaatgggagtttgttttggaaccaaaatcaacgggactttccaaaatgtcgtaaca
actccgccccattgacgcaaatgggcggtaggcgtgtacggtgggaggtctatataagcagagctcgtgagtttggggacccttgattgttctttctttttcgctattg
taaaattcatgttatatggagggggcaaagttttcagggtgttgtttagaatgggaagatgtcccttgtatcaccatggaccctcatgataatttgtttctttcactttcta
ctctgttgacaaccattgtctcctcttattttcttttcattttctgtaactttttcgttaaactttagcttgcatttgtaacgaatttttaaattcacttttgtttatttgtcagattgtaa
gtactttctctaatcactttttttcaaggcaatcagggtatattatattgtacttcagcacagttttagagaacaattgttataattaaatgataaggtagaatatttctgcata
taaattctggctggcgtggaaatattcttattggtagaaacaactacatcctggtcatcatcctgcctttctctttatggttacaatgatatacactgtttgagatgaggata
aaatactctgagtccaaaccgggcccctctgctaaccatgttcatgccttcttctttttcctacagctcctgggcaacgtgctggttattgtgctgtctcatcattttggca
aagaattgtaatacgactcactatagggcgaattgatatgtctagattagataaaagtaaagtgattaacagcgcattagagctgcatgtctagattagataaaagtaa
agtgattaacagcgcattagagctgcttaatgaggtcggaatcgaaggtttaacaacccgtaaactcgcccagaagctaggtgtagagcagcctacattgtattgg
catgtaaaaaataagcgggctttgctcgacgccttagccattgagatgttagataggcaccatactcacttttgccctttagaaggggaaagctggcaagatttttac
gtaataacgctaaaagttttagatgtgctttactaagtcatcgcgatggagcaaaagtacatttaggtacacggcctacagaaaaacagtatgaaactctcgaaaatc
aattagcctttttatgccaacaaggtttttcactagagaatgcattatatgcactcagcgctgtggggcattttactttaggttgcgtattggaagatcaagagcatcaag
tcgctaaagaagaaagggaaacacctactactgatagtatgccgccattattacgacaagctatcgaattatttgatcaccaaggtgcagagccagccttcttattcg
gccttgaattgatcatatgcggattagaaaaacaacttaaatgtgaaagtgggtccgcgtacagcggatcccgggaattcagatcttattaaagcagaacttgtttatt
gcagcttataatggttacaaataaagcaatagcatcacaaatttcacaaataaagcatttttttcactgcattctagttgtggtttgtccaaactcatcaatgtatcttatcat
gtctggtcaatgtgtgtcagttagggtgtggaaagtccccaggctccccagcaggcagaagtatgcaaagcatgcatctcaattagtcagcaaccaggtgtggaa
agtccccaggctccccagcaggcagaagtatgcaaagcatgcatctcaattagtcagcaaccatagtcccgcccctaactccgcccatcccgcccctaactccgc
ccagttccgcccattctccgccccatggctgactaattttttttatttatgcagaggccgaggccgcctctgcctctgagctattccagaagtagtgaggaggcttttt
ggaggcctaggcttttgcaaaaagctccccatagtgactggatatgttgtgttttacagtattatgtagtctgtttttttatgcaaaatctaatttaatatattgatatttatatc
atttacgtttctcgttcagctttcttgtacaaagtggttgatatccagcacagtggcggccgctcgagtctagagggcccgcggttcgaaggtaagcctatccctaac
cctctcctcggtctcgattctacgcgtaccggttagtaatgagtttggaattaattctgtggaatgtgtgtcagttagggtgtggaaagtccccaggctccccagcagg
cagaagtatgcaaagcatgcatctcaattagtcagcaaccaggtgtggaaagtccccaggctccccagcaggcagaagtatgcaaagcatgcatctcaattagtc
agcaaccatagtcccgcccctaactccgcccatcccgcccctaactccgcccagttccgcccattctccgccccatggctgactaattttttttatttatgcagaggcc
gaggccgcctctgcctctgagctattccagaagtagtgaggaggcttttttggaggcctaggcttttgcaaaaagctcccgggagcttgtatatccattttcggatctg
atcagcacgtgttgacaattaatcatcggcatagtatatcggcatagtataatacgacaaggtgaggaactaaaccatggccaagcctttgtctcaagaagaatcca
ccctcattgaaagagcaacggctacaatcaacagcatccccatctctgaagactacagcgtcgccagcgcagctctctctagcgacggccgcatcttcactggtgt
caatgtatatcatttactgggggaccttgtgcagaactcgtggtgctgggcactgctgctgctgcggcagctggcaacctgacttgtatcgtcgcgatcggaaatg

agaacaggggcatcttgagcccctgcggacggtgccgacaggtgcttctcgatctgcatcctgggatcaaagccatagtgaaggacagtgatggacagccgac
ggcagttgggattcgtgaattgctgccctctggttatgtgtgggagggctaagcacaattcgagctcggtacctttaagaccaatgacttacaaggcagctgtagat
cttagccactttttaaaagaaaaggggggactggaagggctaattcactcccaacgaagacaagatctgctttttgcttgtactgggtctctctggttagaccagatct
gagcctgggagctctctggctaactagggaacccactgcttaagcctcaataaagcttgccttgagtgcttcaagtagtgtgtgcccgtctgttgtgtgactctggta
actagagatccctcagacccttttagtcagtgtggaaaatctctagcagtagtagttcatgtcatcttattattcagtatttataacttgcaaagaaatgaatatcagaga
gtgagaggaacttgtttattgcagcttataatggttacaaataaagcaatagcatcacaaatttcacaaataaagcatttttttcactgcattctagttgtggtttgtccaaa
ctcatcaatgtatcttatcatgtctggctctagctatcccgcccctaactccgcccatcccgcccctaactccgcccagttccgcccattctccgccccatggctgact
aattttttttatttatgcagaggccgaggccgcctcggcctctgagctattccagaagtagtgaggaggcttttttggaggcctagggacgtacccaattcgccctata
gtgagtcgtattacgcgcgctcactggccgtcgttttacaacgtcgtgactgggaaaaccctggcgttacccaacttaatcgccttgcagcacatccccctttcgcca
gctggcgtaatagcgaagaggcccgcaccgatcgcccttcccaacagttgcgcagcctgaatggcgaatgggacgcgccctgtagcggcgcattaagcgcgg
cgggtgtggtggttacgcgcagcgtgaccgctacacttgccagcgccctagcgcccgctcctttcgctttcttcccttcctttctcgccacgttcgccggctttcccc
gtcaagctctaaatcgggggctccctttagggttccgatttagtgctttacggcacctcgaccccaaaaaacttgattagggtgatggttcacgtagtgggccatcgc
cctgatagacggtttttcgccctttgacgttggagtccacgttctttaatagtggactcttgttccaaactggaacaacactcaaccctatctcggtctattcttttgatttat
aagggattttgccgatttcggcctattggttaaaaaatgagctgatttaacaaaaatttaacgcgaattttaacaaaatattaacgcttacaatttaggtggcacttttcgg
ggaaatgtgcgcggaacccctatttgtttatttttctaaatacattcaaatatgtatccgctcatgagacaataaccctgataaatgcttcaataatattgaaaaaggaag
agtatgagtattcaacatttccgtgtcgcccttattcccttttttgcggcattttgccttcctgtttttgctcacccagaaacgctggtgaaagtaaaagatgctgaagatc
agttgggtgcacgagtgggttacatcgaactggatctcaacagcggtaagatccttgagagttttcgccccgaagaacgttttccaatgatgagcacttttaaagttct
gctatgtggcgcggtattatcccgtattgacgccgggcaagagcaactcggtcgccgcatacactattctcagaatgacttggttgagtactcaccagtcacagaa
aagcatcttacggatggcatgacagtaagagaattatgcagtgctgccataaccatgagtgataacactgcggccaacttacttctgacaacgatcggaggaccg
aaggagctaaccgcttttttgcacaacatgggggatcatgtaactcgccttgatcgttgggaaccggagctgaatgaagccataccaaacgacgagcgtgacacc
acgatgcctgtagcaatggcaacaacgttgcgcaaactattaactggcgaactacttactctagcttcccggcaacaattaatagactggatggaggcggataaag
ttgcaggaccacttctgcgctcggcccttccggctggctggtttattgctgataaatctggagccggtgagcgtgggtctcgcggtatcattgcagcactggggcca
gatggtaagccctcccgtatcgtagttatctacacgacggggagtcaggcaactatggatgaacgaaatagacagatcgctgagataggtgcctcactgattaag
cattggtaactgtcagaccaagtttactcatatatactttagattgatttaaaacttcatttttaatttaaaaggatctaggtgaagatcctttttgataatctcatgaccaaaa
tcccttaacgtgagttttcgttccactgagcgtcagaccccgtagaaaagatcaaaggatcttcttgagatcctttttttctgcgcgtaatctgctgcttgcaaacaaaaa
aaccaccgctaccagcggtggtttgtttgccggatcaagagctaccaactctttttccgaaggtaactggcttcagcagagcgcagataccaaatactgttcttctag
tgtagccgtagttaggccaccacttcaagaactctgtagcaccgcctacatacctcgctctgctaatcctgttaccagtggctgctgccagtggcgataagtcgtgtc
ttaccgggttggactcaagacgatagttaccggataaggcgcagcggtcgggctgaacggggggttcgtgcacacagcccagcttggagcgaacgacctaca
ccgaactgagatacctacagcgtgagctatgagaaagcgccacgcttcccgaagggagaaaggcggacaggtatccggtaagcggcagggtcggaacagga
gagcgcacgagggagcttccagggggaaacgcctggtatctttatagtcctgtcgggtttcgccacctctgacttgagcgtcgatttttgtgatgctcgtcaggggg
gcggagcctatggaaaaacgccagcaacgcggcctttttacggttcctggccttttgctggccttttgctcacatgttctttcctgcgttatcccctgattctgtggataa
ccgtattaccgcctttgagtgagctgataccgctcgccgcagccgaacgaccgagcgcagcgagtcagtgagcgaggaagcggaagagcgcccaatacgca
aaccgcctctccccgcgcgttggccgattcattaatgcagctggcacgacaggtttcccgactggaaagcgggcagtgagcgcaacgcaattaatgtgagttagc
tcactcattaggcaccccaggctttacactttatgcttccggctcgtatgttgtgtggaattgtgagcggataacaatttcacacaggaaacagctatgaccatgattac
gccaagcgcgcaattaaccctcactaaagggaacaaaagctggagctgcaagctt

A schematized map corresponding to the vector sequence above is shown in Figure 1.

The vector may be constructed fully through de novo gene synthesis, or in part through the cloning of the Numb, Sox and OCT3/4 cDNA sequences into the position occupied by LacZ in the Invitrogen pcDNA4tolacZ vector. Similarly, the tetR gene is found in the Invitrogen pcDNA6/TR vector. Coding sequences of genes referenced are also appropriate for cloning into the pcDNA4lacZ vector.

Alternatively, the tetR gene may be transfected into target cells separately utilizing the pcDNA6/TR vector in combination with a vector comprising the sequence here minus the tetR gene and its PCMV promoter.

Likewise, multiple vectors may be employed so long as elements similar to the elements included in the above sequence are present. This may reduce the likelihood of promoter competition. It is to be understood that other conditional promoter elements may be substituted for the tetracycline sensitive promoter elements.

Example 14. It is expected that intravenous and other administration of pluripotent stem cells produced according to the methods described herein (or other published methods) one or more times can provide replacement cells to the body and that such administration may serve to extend the life or improve the health of the patient suffering age-related senescence.

Example 15. Production of germ cells.

The current invention covers the derivation of germ cells from multipotent, pluripotent, and/or self-renewing stem cells produced according to the methods described herein (or according to other published methods). The production of such germ cells may be suitable for treating infertility and producing embryos in vitro (e.g. Hubner et al., 2003; Kehler et al., 2005; Nayernia et al., 2006a; Nayernia et al., 2006b; Drusenheimer et al., 2007; Moore et al., 2007; etc.)

Example 16: Generation of transgenic animals.

The present invention covers the generation of transgenic animals. As with other pluripotent cells, the pluripotent cells produced by the methods described herein (or other published methods) may be utilized to produce transgenic animals by any method known to the art.

Example 17: Therapeutic vector construction

Examples of retroviral vectors which may be employed include, but are not limited to, those derived from Moloney Murine Leukemia Virus, Moloney Murine Sarcoma Virus, and Rous Sarcoma Virus, FIV, and HIV. Appropriate expression vectors are that may be employed for transfecting DNA or RNA into eukaryotic cells. Such vectors include, but are not limited to, prokaryotic vectors such as, for example, bacterial vectors; eukaryotic vectors, such as, for example, yeast vectors and fungal vectors; and viral vectors, such as, but not limited to, lentiviral vectors, adenoviral vectors, adeno-associated viral vectors, and retroviral vectors.

The replication incompetent pcDNA 6.2 GW/miR and pcDNA 6.2/EmGFP-Bsd/V5-DEST vectors are examples of an appropriate expression vectors (Invitrogen) and allow expression of synthetic oligonucleotides (e.g. miRNAs) that have the capacity to cleave targeted sequences. These vectors include flanking and loop sequences from endogenous miRNA to direct the excision of the engineered miRNA from a longer Pol II transcript (pre-miRNA).

Alternatively, inclusion of the HIV psi sequence allows the therapeutic vector to compete with native HIV genome for packaging into viral particles, also inhibiting HIV transmission.

Combining multiple miRNA sequences directed against a single target increases the likelihood of success in reducing target sequence expression. miRNA sequences may be operably linked to tissue specific promoters such as the EF-1 alpha promoter, any T cell specific promoter, or macrophage specific promoter to ensure expression in the desired cell types.

Utilizing Invitrogen's lentiviral destination (DEST) vectors for gene expression, the resulting therapeutic vector(s) becomes capable of stably transducing both dividing and non-dividing cell types.

In a preferred embodiment, the therapeutic vector(s) contains multiple synthetic oligonucleotide sequences driven by one or more promoters so as to reduce expression of CXCR4, CCR5, and/or any other cellular protein known to act as a co-receptor for HIV infection in target cells.

In one therapeutic vector (constructed in 2006), four miRNA sequences targeting CXCR4 and CCR5 co-receptors were cloned into the pcDNA 6.2 GW/miR vector along with decoy RNA sequences targeting HIV-2 TAR and RRE.

Genetic constructs may include a vector backbone, and a transactivator which regulates a promoter operably linked to heterologous nucleic acid sequences.

Another example of a suitable vector is a retroviral vector. Retroviruses are RNA viruses which contain an RNA genome. The gag, pol, and env genes are flanked by long terminal repeat (LTR) sequences. The 5' and 3' LTR sequences promote transcription and polyadenylation of mRNA's.

The retroviral vector may provide a regulable transactivating element, an internal ribosome reentry site (IRES), a selection marker, and a target heterologous gene operated by a regulable promoter.

Alternatively, multiple sequences may be expressed under the control of multiple promoters. Finally, the retroviral vector may contain cis-acting sequences necessary for reverse transcription and integration. Upon infection, the RNA is reverse transcribed to DNA which integrates efficiently into the host genome. The recombinant retrovirus of this invention is genetically modified in such a way that some of the retroviral, infectious genes of the native virus are removed and in embodiments replaced instead with a target nucleic acid sequence for genetic modification of the cell. The sequences may be exogenous DNA or RNA, in its natural or altered form.

Example 18: Examples methods for generation of the therapeutic vector.

The methods for generation of the therapeutic vector(s) include those taught in Invitrogen's Viral Power Lentiviral Expression Systems Manual (incorporated by reference herein). Briefly, the EmGFP-bsd cassette is cloned as a PmlI-BlpI fragment into the pLenti6/R4R2/V5-DEST vector, while the miR-decoy cassette is simultaneously transferred by BP reaction into pDONR221. Then the EF1a promoter and miR-decoy are Multisite LR crossed into the modified pLenti6/EmGFP-bsd/R4R2-DESTvector.

pLenti6/R4R2/V5-DEST vector sequence:

aatgtagtcttatgcaatactcttgtagtcttgcaacatggtaacgatgagttagcaacatgccttacaaggagagaaaaagcaccgtgcatgccgattggtggaagt
aaggtggtacgatcgtgccttattaggaaggcaacagacgggtctgacatggattggacgaaccactgaattgccgcattgcagagatattgtatttaagtgcctag
ctcgatacataaacgggtctctctggttagaccagatctgagcctgggagctctctggctaactagggaacccactgcttaagcctcaataaagcttgccttgagtg
cttcaagtagtgtgtgcccgtctgttgtgtgactctggtaactagagatccctcagacccttttagtcagtgtggaaaatctctagcagtggcgcccgaacagggact
tgaaagcgaaagggaaaccagaggagctctctcgacgcaggactcggcttgctgaagcgcgcacggcaagaggcgaggggcggcgactggtgagtacgcc
aaaaattttgactagcggaggctagaaggagagagatgggtgcgagagcgtcagtattaagcgggggagaattagatcgcgatgggaaaaaattcggttaagg
ccagggggaaagaaaaaatataaattaaaacatatagtatgggcaagcagggagctagaacgattcgcagttaatcctggcctgttagaaacatcagaaggctgt
agacaaatactgggacagctacaaccatcccttcagacaggatcagaagaacttagatcattatataatacagtagcaaccctctattgtgtgcatcaaaggataga

gataaaagacaccaaggaagctttagacaagatagaggaagagcaaaacaaaagtaagaccaccgcacagcaagcggccgctgatcttcagacctggagga
ggagatatgagggacaattggagaagtgaattatataaatataaagtagtaaaaattgaaccattaggagtagcacccaccaaggcaaagagaagagtggtgca
gagagaaaaaagagcagtgggaataggagctttgttccttgggttcttgggagcagcaggaagcactatgggcgcagcgtcaatgacgctgacggtacaggcc
agacaattattgtctggtatagtgcagcagcagaacaatttgctgagggctattgaggcgcaacagcatctgttgcaactcacagtctggggcatcaagcagctcc
aggcaagaatcctggctgtggaaagatacctaaaggatcaacagctcctggggatttggggttgctctggaaaactcatttgcaccactgctgtgccttggaatgct
agttggagtaataaatctctggaacagatttggaatcacacgacctggatggagtgggacagagaaattaacaattacacaagcttaatacactccttaattgaaga
atcgcaaaaccagcaagaaaagaatgaacaagaattattggaattagataaatgggcaagtttgtggaattggtttaacataacaaattggctgtggtatataaaatt
attcataatgatagtaggaggcttggtaggtttaagaatagtttttgctgtactttctatagtgaatagagttaggcagggatattcaccattatcgtttcagacccacctc
ccaaccccgaggggacccgacaggcccgaaggaatagaagaagaaggtggagagagagacagagacagatccattcgattagtgaacggatctcgacggt
atcgatgtcgacgttaacgctagtgatatcaactttgtatagaaaagttgaacgagaaacgtaaaatgatataaatatcaatatattaaattagattttgcataaaaaaca
gactacataatactgtaaaacacaacatatccagtcactatggcggccgcattaggcaccccaggctttacactttatgcttccggctcgtataatgtgtggatttga
gttaggatccgtcgagattttcaggagctaaggaagctaaaatggagaaaaaaatcactggatataccaccgttgatatatcccaatggcatcgtaaagaacattttg
aggcatttcagtcagttgctcaatgtacctataaccagaccgttcagctggatattacggcctttttaaagaccgtaaagaaaaataagcacaagttttatccggccttt
attcacattcttgcccgcctgatgaatgctcatccggaattccgtatggcaatgaaagacggtgagctggtgatatgggatagtgttcacccttgttacaccgttttcca
tgagcaaactgaaacgttttcatcgctctggagtgaataccacgacgatttccggcagtttctacacatatattcgcaagatgtggcgtgttacggtgaaaacctggc
ctatttccctaaagggtttattgagaatatgtttttcgtctcagccaatccctgggtgagtttcaccagttttgatttaaacgtggccaatatggacaacttcttcgccccc
gttttcaccatgggcaaatattatacgcaaggcgacaaggtgctgatgccgctggcgattcaggttcatcatgccgtttgtgatggcttccatgtcggcagaatgctt
aatgaattacaacagtactgcgatgagtggcagggcggggcgtaaagatctggatccggcttactaaaagccagataacagtatgcgtatttgcgcgctgattttg
cggtataagaatatatactgatatgtatacccgaagtatgtcaaaaagaggtatgctatgaagcagcgtattacagtgacagttgacagcgacagctatcagttgctc
aaggcatatatgatgtcaatatctccggtctggtaagcacaaccatgcagaatgaagcccgtcgtctgcgtgccgaacgctggaaagcggaaaatcaggaaggg
atggctgaggtcgcccggtttattgaaatgaacggctcttttgctgacgagaacagggactggtgaaatgcagtttaaggtttacacctataaaagagagagccgtt
atcgtctgtttgtggatgtacagagtgatattattgacacgcccgggcgacggatggtgatccccctggccagtgcacgtctgctgtcagataaagtctcccgtgaa
ctttacccggtggtgcatatcggggatgaaagctggcgcatgatgaccaccgatatggccagtgtgccggtctccgttatcggggaagaagtggctgatctcagc
caccgcgaaaatgacatcaaaaacgccattaacctgatgttctggggaatataaatgtcaggctccgttatacacagccagtctgcaggtcgaccatagtgactgg
atatgttgtgttttacagtattatgtagtctgtttttttatgcaaaatctaatttaatatattgatatttatatcattttacgtttctcgttcagctttcttgtacaaagtggttgatatc
cagcacagtggcggccgctcgagtctagagggcccgcggttcgaaggtaagcctatccctaaccctctcctcggtctcgattctacgcgtaccggttagtaatga
gtttggaattaattctgtggaatgtgtgtcagttagggtgtggaaagtccccaggctccccagcaggcagaagtatgcaaagcatgcatctcaattagtcagcaacc
aggtgtggaaagtccccaggctccccagcaggcagaagtatgcaaagcatgcatctcaattagtcagcaaccatagtcccgcccctaactccgcccatcccgcc
cctaactccgcccagttccgcccattctccgccccatggctgactaattttttttatttatgcagaggccgaggccgcctctgcctctgagctattccagaagtagtga
ggaggcttttttggaggcctaggcttttgcaaaaagctcccgggagcttgtatatccattttcggatctgatcagcacgtgttgacaattaatcatcggcatagtatatc
ggcatagtataatacgacaaggtgaggaactaaaccatggccaagcctttgtctcaagaagaatccaccctcattgaaagagcaacggctacaatcaacagcatc
cccatctctgaagactacagcgtcgccagcgcagctctctctagcgacggccgcatcttcactggtgtcaatgtatatcattttactgggggaccttgtgcagaactc
gtggtgctgggcactgctgctgctgcggcagctggcaacctgacttgtatcgtcgcgatcggaaatgagaacaggggcatcttgagcccctgcggacggtgccg
acaggtgcttctcgatctgcatcctgggatcaaagccatagtgaaggacagtgatggacagccgacggcagttgggattcgtgaattgctgccctctggttatgtgt
gggagggctaagcacaattcgagctcggtacctttaagaccaatgacttacaaggcagctgtagatcttagccactttttaaaagaaaagggggggactggaaggg
ctaattcactcccaacgaagacaagatctgctttttgcttgtactgggtctctctggttagaccagatctgagcctgggagctctctggctaactagggaacccactgc
ttaagcctcaataaagcttgccttgagtgcttcaagtagtgtgtgcccgtctgttgtgtgactctggtaactagagatccctcagacccttttagtcagtgtggaaaatct
ctagcagtagtagttcatgtcatcttattattcagtatttataacttgcaaagaaatgaatatcagagagtgagaggaacttgtttattgcagcttataatggttacaaata
aagcaatagcatcacaaatttcacaaataaagcatttttttcactgcattctagttgtggtttgtccaaactcatcaatgtatcttatcatgtctggctctagctatcccgcc

cctaactccgcccatcccgcccctaactccgcccagttccgcccattctccgccccatggctgactaattttttttatttatgcagaggccgaggccgcctcggcctct
gagctattccagaagtagtgaggaggcttttttggaggcctagggacgtacccaattcgccctatagtgagtcgtattacgcgcgctcactggccgtcgttttacaac
gtcgtgactgggaaaaccctggcgttacccaacttaatcgccttgcagcacatccccctttcgccagctggcgtaatagcgaagaggcccgcaccgatcgccctt
cccaacagttgcgcagcctgaatggcgaatgggacgcgccctgtagcggcgcattaagcgcggcgggtgtggtggttacgcgcagcgtgaccgctacacttgc
cagcgccctagcgcccgctcctttcgctttcttcccttcctttctcgccacgttcgccggctttccccgtcaagctctaaatcgggggctccctttagggttccgattta
gtgctttacggcacctcgaccccaaaaaacttgattagggtgatggttcacgtagtgggccatcgccctgatagacggtttttcgccctttgacgttggagtccacgt
tctttaatagtggactcttgttccaaactggaacaacactcaaccctatctcggtctattcttttgatttataagggattttgccgatttcggcctattggttaaaaaatgag
ctgatttaacaaaaatttaacgcgaattttaacaaaatattaacgcttacaatttaggtggcacttttcggggaaatgtgcgcggaacccctatttgtttatttttctaaata
cattcaaatatgtatccgctcatgagacaataaccctgataaatgcttcaataatattgaaaaaggaagagtatgagtattcaacatttccgtgtcgcccttattcccttt
ttgcggcattttgccttcctgtttttgctcacccagaaacgctggtgaaagtaaaagatgctgaagatcagttgggtgcacgagtgggttacatcgaactggatctca
acagcggtaagatccttgagagttttcgccccgaagaacgttttccaatgatgagcacttttaaagttctgctatgtggcgcggtattatcccgtattgacgccgggca
agagcaactcggtcgccgcatacactattctcagaatgacttggttgagtactcaccagtcacagaaaagcatcttacggatggcatgacagtaagagaattatgca
gtgctgccataaccatgagtgataacactgcggccaacttacttctgacaacgatcggaggaccgaaggagctaaccgcttttttgcacaacatgggggatcatgt
aactcgccttgatcgttgggaaccggagctgaatgaagccataccaaacgacgagcgtgacaccacgatgcctgtagcaatggcaacaacgttgcgcaaactat
taactggcgaactacttactctagcttcccggcaacaattaatagactggatggaggcggataaagttgcaggaccacttctgcgctcggcccttccggctggctg
gtttattgctgataaatctggagccggtgagcgtgggtctcgcggtatcattgcagcactggggccagatggtaagccctcccgtatcgtagttatctacacgacgg
ggagtcaggcaactatggatgaacgaaatagacagatcgctgagataggtgcctcactgattaagcattggtaactgtcagaccaagtttactcatatatactttaga
ttgatttaaaacttcatttttaatttaaaaggatctaggtgaagatcctttttgataatctcatgaccaaaatcccttaacgtgagttttcgttccactgagcgtcagacccc
gtagaaaagatcaaaggatcttcttgagatcctttttttctgcgcgtaatctgctgcttgcaaacaaaaaaaccaccgctaccagcggtggtttgtttgccggatcaag
agctaccaactctttttccgaaggtaactggcttcagcagagcgcagataccaaatactgttcttctagtgtagccgtagttaggccaccacttcaagaactctgtagc
accgcctacatacctcgctctgctaatcctgttaccagtggctgctgccagtggcgataagtcgtgtcttaccgggttggactcaagacgatagttaccggataagg
cgcagcggtcgggctgaacggggggttcgtgcacacagcccagcttggagcgaacgacctacaccgaactgagatacctacagcgtgagctatgagaaagc
gccacgcttcccgaagggagaaaggcggacaggtatccggtaagcggcagggtcggaacaggagagcgcacgagggagcttccagggggaaacgcctgg
tatctttatagtcctgtcgggtttcgccacctctgacttgagcgtcgatttttgtgatgctcgtcaggggggcggagcctatggaaaaacgccagcaacgcggcctttt
tacggttcctggccttttgctggccttttgctcacatgttctttcctgcgttatcccctgattctgtggataaccgtattaccgcctttgagtgagctgataccgctcgccg
cagccgaacgaccgagcgcagcgagtcagtgagcgaggaagcggaagagcgcccaatacgcaaaccgcctctccccgcgcgttggccgattcattaatgca
gctggcacgacaggtttcccgactggaaagcgggcagtgagcgcaacgcaattaatgtgagttagctcactcattaggcaccccaggctttacactttatgcttcc
ggctcgtatgttgtgtggaattgtgagcggataacaatttcacacaggaaacagctatgaccatgattacgccaagcgcgcaattaaccctcactaaagggaacaa
aagctggagctgcaagctt

Example miR-decoy cassette sequence:

gtcgaccagtggatcctggaggcttgctgaaggctgtatgctgatcgggtgtaaactgagcttggttttggccactgactgaccaagctcattacacccgatcagga
cacaaggcctgttactagcactcacatggaacaaatggcccagatcctggaggcttgctgaaggctgtatgctgataccaggcaggataaggccagttttggcca
ctgactgactggccttactgcctggtatcaggacacaaggcctgttactagcactcacatggaacaaatggcccagatcctggaggcttgctgaaggctgtatgct
gtgaccaggatgaccaatccatgttttggccactgactgacatggattgcatcctggtcacaggacacaaggcctgttactagcactcacatggaacaaatggccc
agatcctggaggcttgctgaaggctgtatgctgatagcttggtccaacctgttagttttggccactgactgactaacaggtgaccaagctatcaggacacaaggcct
gttactagcactcacatggaacaaatggcccagatctccccagtggaaagacgcgcaggcaaaacgcaccacgtgacggagcgtgaccgcgcgccgagcgc
gcgccaaggtcgggcaggaagagggcctatttcccatgattccttcatatttgcatatacgatacaaggctgttagagagataattagaattaatttgactgtaaacac
aaagatattagtacaaaatacgtgacgtagaaagtaataatttcttgggtagtttgcagttttaaaattatgttttaaaatggactatcatatgcttaccgtaacttgaaagt

atttcgatttcttgggtttatatatcttgtggaaaggacggtgctcgcttcggcagcacgtcgtgctagggttcttgggttttctcgcaacagcaggttctgcaatgggc
gcggcgtccctgaccgtgtcggctcagtcccggactttactggccgggatagtgcagcaacagcaacagctgttggacgtggtcaagagacaacaagaactgtt
gcgactgaccgtctggggaacgaaaaacctccaggcaagagtcactgctatagagaagtacctacaggaccaggcgcggctaaattcatggggatgtctagac
ctagagcggacttcggtccgctttttccccagtggaaagacgcgcaggcaaaacgcaccacgtgacggagcgtgaccgcgcgccgagcgcgcgccaaggtc
gggcaggaagagggcctatttcccatgattccttcatatttgcatatacgatacaaggctgttagagagataattagaattaatttgactgtaaacacaaagatattagt
acaaaatacgtgacgtagaaagtaataatttcttgggtagtttgcagttttaaaattatgttttaaaatggactatcatatgcttaccgtaacttgaaagtatttcgatttctt
gggtttatatatcttgtggaaaggacggtgctcgcttcggcagcacgtcggtcgctctgcggagaggctggcagattgagccctgggaggttctctccagcactag
caggtagagcctgggtgttccctgctagactctcaccagtgcttggccggcactgggcagacggctccacgcttgcttgcttaaagacctcttaataaagctgctct
agacctagagcggacttcggtccgctttttacgtactcgag

Example 19: Methods for propagating/proliferating stem/progenitor cells in vivo.

In order to obtain large numbers of target cells that are relatively resistant to 1) HIV infection and/or 2) HIV replication and/or 3) HIV transcription, progenitor/stem cells can be grown in Dulbecco's modified Minimal Essential Medium (DMEM) supplemented with glutamine, beta.-mercaptoethanol, 10% (by volume) horse serum, and human recombinant Leukemia Inhibitory Factor (LIF). The LIF replaces the need for maintaining progenitor/stem cells on feeder layers of cells, (which may also be employed) and is essential for maintaining progenitor/stem cells in an undifferentiated state.

Example 20: Stem cells are collected from individuals, the cells are transfected with the therapeutic vectors, then prepared for transplantation by standard methods, with or without HLA typing and matching.

Example 21: Umbilical cord blood samples are obtained from umbilical blood cord bank. The cells are then transfected with the therapeutic vector of beneficial sequences, then prepared for transplantation by standard methods, with or without HLA typing and matching.

Example 22: Examples of Synthetic oligonucleotide sequences suitable for inclusion in the therapeutic vector.

Any synthetic oligonucleotide sequences that successfully reduce the protein expression of targeted sequences > 70% is covered by the present invention.

Any synthetic oligonucleotide sequences that successfully reduce the ability of target cells to sustain HIV replication by > 70% or to a lesser but therapeutic degree or HIV viral activity by > 70% or to a lesser but therapeutic degree are also covered by this invention.

Examples of miRNA sequences include miRNA sequences derived by IVGN algorithm(Invitrogen). miRNA sequences targeting the CXCR4 gene include top strand: 5'-

TGCTGATACCAGGCAGGATAAGGCCAGTTTTGGCCACTGACTGACTGGCCTTACTGCCTGGTAT -3' and bottom strand : 5'-CCTGATACCAGGCAGTAAGGCCAGTCAGTCAGTGGCCAAAACTGGCCTTATCCTGCCTGGTATC -3'; as well as top strand: 5'-TGCTGTGACCAGGATGACCAATCCATGTTTTGGCCACTGACTGACATGGATTGCATCCTGGTCA -3'and bottom strand: 5'-CCTGTGACCAGGATGCAATCCATGTCAGTCAGTGGCCAAAACATGGATTGGTCATCCTGGTCAC -3'.

Similarly, miRNA sequences targeting the CCR5 gene include top strand: 5'-

TGCTGATCGGGTGTAAACTGAGCTTGGTTTTGGCCACTGACTGACCAAGCTCATTACACCCGAT -3'and bottom strand: 5'-CCTGATCGGGTGTAATGAGCTTGGTCAGTCAGTGGCCAAAACCAAGCTCAGTTTACACCCGATC -3'; as well as top strand5'-TGCTGATAGCTTGGTCCAACCTGTTAGTTTTGGCCACTGACTGACTAACAGGTGACCAAGCTAT -3'and bottom strand: 5'-CCTGATAGCTTGGTCACCTGTTAGTCAGTCAGTGGCCAAAACTAACAGGTTGGACCAAGCTATC -3'.

Example 23: Examples of Decoy RNA suitable for inclusion in the therapeutic vector. Any decoy sequences that successfully reduce the ability of target cells to sustain HIV replication by > 70% or to a lesser but therapeutic degree or HIV viral activity by > 70% or to a lesser but therapeutic degree are covered by this invention.

An example TAR decoy sequence is

gtcgctctgcggagaggctggcagattgagccctgggaggttctctccagcactagcaggtagagcctgggtgttccctgctagactctcaccagtgcttggccggcactgggcagacggctccacgcttgcttgcttaaagacctcttaataaagctgc (Browning et al., 1999)

An example RRE decoy sequence is

tgctagggttcttgggttttctcgcaacagcaggttctgcaatgggcgcggcgtccctgaccgtgtcggctcagtcccggactttactggccgggatagtgcagcaacagcaacagctgttggacgtggtcaagagacaacaagaactgttgcgactgaccgtctggggaacgaaaaacctccaggcaagagtcactgctatagagaagtacctacaggaccaggcgcggctaaattcatggggatg (Dillon et al., 1990).

Example 24: Flanking sequences providing stability for RNA decoys.

Examples of appropriate flanking sequences for RNA decoys are as follows:

GUGCUCGCUUCGGCAGCACGTCGAC ---TAR DECOY SEQ---
UCUAGAGCGGACUUCGGUCCGCUUUU

GUGCUCGCUUCGGCAGCACGTCGAC ---RRE DECOY SEQ---
UCUAGAGCGGACUUCGGUCCGCUUUU

Previously, it was demonstrated that decoy sequences flanked by hairpins on either side, 19 nucleotides (ntds) of the U6 RNA on the 5' side as well as a 3' stem immediately preceding a poly U terminator for POLIII, showed greater stability. This arrangement is expected to protect against 3'-5' exonuclease attack, and to reduce the chances of the 3' trailer interfering with the insert RNA folding. Since only the first 3/4 of the tRNA sequence is present, the 5' end of the insert should be protected and export from the nucleus should be prevented (Good et al., 1997).

Example 25: Introduction of Therapeutic Vector to the Host

In a preferred embodiment, blood stem/progenitor cells, and target cells are transfected with the therapeutic vector(s) (or associated therapeutic virus) in vivo by introduction of the therapeutic vector(s) into the host blood, tissues, or bone marrow, etc. The greatest benefit may be achieved by modifying a large number of endogenous target and stem/progenitor cells. This may be accomplished by using an appropriately-sized, catheter-like device, or needle to inject the therapeutic vector(s) into the venous or arterial circulation. In a preferred embodiment, the virus is pseudotyped with VSV-G envelope glycoprotein and native HIV-1 env proteins.

Example 26: Introduction of genetically-modified cells into the Host

Blood cells, such as mature peripheral blood T lymphocytes, monocytes, macrophages, T cell progenitors, macrophage-monocyte progenitor cells, and/or pluripotent hematopoietic stem cells (such as those found in umbilical cord blood and occupying bone marrow spaces) as well as other stem/progenitor cells can be transfected using the therapeutic vector(s) in vitro. Appropriate concentrations of the therapeutic vector(s) may be those consistent with Browning et al., 1999. Subsequently, cells are expanded (propagated) in vitro, and are then transferred to the host via introduction of the cells to the venous or arterial circulation using a intravenous needle or catheter. Subsequently, cells transfected with the therapeutic vectors are able to "home" to the bone marrow and other tissues.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

Example 27: Examples of expressed or targeted transgenes utilized in the present invention.

Any transgene sequences effective in fulfilling the present invention is suitable for use in the present invention. Suitable nucleotide sequences may be drawn from any species so long as the desired cells or behavior is achieved. Likewise the method of naming such sequences, either in lower case or upper case letters herein, does not imply a particular species. The following sequences stored in the NCBI database (listed by accession number) represent examples of sequences referenced above in the present application. They are also examples of specific transgene encoding sequences (cds) suitable for use in the present invention, but do not in any way limit the practice of the invention:

cardiotrophin1:U43030:atgagccggagggagggaagtctggaagacccccagactgattcctcagtctcacttcttccccacttggaggccaagatccgtcagacacacagccttgcgcacctcctcaccaaatacgctgagcagctgctccaggaatatgtgcagctccagggagacccccttcgggctgcccagcttctcgccgccgcggctgccggtggccggcctgagcgccccggctccgagccacgcggggctgccagtgcacgagcggctgcggctggacgcggcggcgctggccgcgctgcccccgctgctggacgcagtgtgtcgccgccaggccgagctgaacccgcgcgcgccgcgcctgctgcgccgcctggaggacgcggcgcgccaggcccgggccctgggcgccgccgtggaggccttgctggccgcgctgggcgccgccaaccgcgggccccgggccgagcccccgccgccaccgcctcagccgcctccgccaccggggtcttccccgccaaggtgctggggctccgcgtttgcggcctctaccgcgagtggctgagccgcaccgagggcgacctgggccagctgctgcccgggggctcggcctga; NM_001330; NM_013246; BC064416; BC036787; BC049822.

CNTF:BC074964:atggctttcacagagcattcaccgctgacccctcaccgtcgggacctctgtagccgctctatctggctagcaaggaagattcgttcagacctgactgctcttacggaatcctatgtgaagcatcagggcctgaacaagaacatcaacctggactctgcggatgggatgccagtggcaagcactgatcagtggagtgagctgaccgaggcagagcgactccaagagaaccttcaagcttatcgtaccttccatgttttgttggccaggctcttagaagaccagcaggtgcattttaccccaaccgaaggtgacttccatcaagctatacataccttcttctccaagtcgctgcctttgcataccagatagaggagttaatgatactcctggaatacaagatcccccgcaatgaggctgatgggatgcctattaatgttggagatggtggtctctttgagaagaagctgtggggcctaaaggtgctgcaggagctttcacagtggacagtaaggtccatccatgaccttcgtttcatttcttctcatcagactgggatcccagcacgtgggagccattatattgctaacaacaagaaaatgtag; NM_000614; NM_147164; NM_001842.

GP130:NM_175767:atgttgacgttgcagacttggctagtgcaagccttgtttattttcctcaccactgaatctacaggtgaacttctagatccatgtggttatatcagtcctgaatctccagttgtacaacttcattctaatttcactgcagtttgtgtgctaaaggaaaaatgtatggattattttcatgtaaatgctaattacattgtctggaaaa

caaaccattttactattcctaaggagcaatatactatcataaacagaacagcatccagtgtcacctttacagatatagcttcattaaatattcagctcacttgcaacattcttacattcggacagcttgaacagaatgtttatggaatcacaataatttcaggcttgcctccagaaaaacctaaaaatttgagttgcattgtgaacgaggggaagaaaatgaggtgtgagtgggatggtggaagggaaacacacttggagacaaacttcactttaaaatctgaatgggcaacacacaagtttgctgattgcaaagcaaaacgtgacacccccacctcatgcactgttgattattctactgtgtattttgtcaacattgaagtctgggtagaagcagagaatgcccttgggaaggttacatcagatcatatcaattttgatcctgtatataaagtgaagcccaatccgccacataatttatcagtgatcaactcagaggaactgtctagtatcttaaaattgacatggaccaacccaagtattaagagtgttataatactaaaatataacattcaatataggaccaaagatgcctcaacttggagccagattcctcctgaagacacagcatccacccgatcttcattcactgtccaagaccttaaacctttacagaatatgtgtttaggattcgctgtatgaaggaagatggtaagggatactggagtgactggagtgaagaagcaagtgggatcacctatgaagataacattgcctccttttga; NM_002184;EF442778.

IL6:BC015511:atgaactccttctccacaagcgccttcggtccagttgccttctccctggggctgctcctggtgttgcctgctgccttccctgccccagtacccccaggagaagattccaaagatgtagccgccccacacagacagccactcacctcttcagaacgaattgacaaacaaattcggtacatcctcgacggcatctcagccctgagaaaggagacatgtaacaagagtaacatgtgtgaaagcagcaaagaggcactggcagaaaacaacctgaaccttccaaagatggctgaaaaagatggatgcttccaatctggattcaatgaggagacttgcctggtgaaaatcatcactggtcttttggagtttgaggtatacctagagtacctccagaacagatttgagagtagtgaggaacaagccagagctgtgcagatgagtacaaaagtcctgatccagttcctgcagaaaaaggcaaagaatctagatgcaataaccacccctgacccaaccacaaatgccagcctgctgacgaagctgcaggcacagaaccagtggctgcaggacatgacaactcatctcattctgcgcagctttaaggagttcctgcagtccagcctgagggctcttcggcaaatgtag; AB107656.

HOXB4:NM_024015:atggctatgagttcttttttgatcaactcaaactatgtcgaccccaagttccctccatgcgaggaatattcacagagcgattacctacccagcgaccactcgcccgggtactacgccggcggccagaggcgagagagcagcttccagccggaggcgggcttcgggcggcgcgcggcgtgcaccgtgcagcgctacgcggcctgccgggaccctgggcccccgccgcctccgccaccacccccgccgcccccgccaccgcccggtctgtcccctcgggctcctgcgccgccacccgccggggccctcctcccggagcccggccagcgctgcgaggcggtcagcagcagcccccccgccgcctccctgcgcccagaacccccctgcaccccagcccgtcccactccgcgtgcaaagagcccgtcgtctacccctggatgcgcaaagttcacgtgagcacggtaaaccccaattacgccggcggggagcccaagcgctctcggaccgcctacacgcgccagcaggtcttggagctggagaaggaatttcactacaaccgctacctgacacggcgccggagggtggagatcgcccacgcgctctgcctctccgagcgccagatcaagatctggttccagaaccggcgcatgaagtggaaaaaagaccacaagttgcccaacaccaagatccgctcgggtggtgcggcaggctcagccggagggcccccctggccggcccaatggaggcccccgcgcgctctag; NM_010459.

IL6R:NM_000565:atgctggccgtcggctgcgcgctgctggctgccctgctggccgcgccgggagcggcgctggccccaaggcgctgccctgcgcaggaggtggcgagaggcgtgctgaccagtctgccaggagacagcgtgactctgacctgcccgggggtagagccggaagacaatgccactgttcactgggtgctcaggaagccggctgcaggctcccaccccagcagatgggctggcatgggaaggaggctgctgctgaggtcggtgcagctccacgactctggaaactattcatgctaccgggccggccgcccagctgggactgtgcacttgctggtggatgttccccccgaggagccccagctctcctgcttccggaagagcccccctcagcaatgttgtttgtgagtggggtcctcggagcaccccatccctgacgacaaaggctgtgctcttggtgaggaagtttcagaacagtccggccgaagacttccaggagccgtgccagtattcccaggagtcccagaagttctcctgccagttagcagtcccggagggagacagctctttctacatagtgtccatgtgcgtcgccagtagtgtcgggagcaagttcagcaaaactcaaacctttcagggttgtggaatcttgcagcctgatccgcctgccaacatcacagtcactgccgtggccagaaacccccgctggctcagtgtcacctggcaagacccccactcctggaactcatctttctacagactacggtttgagctcagatatcgggctgaacggtcaaagacattcacaacatggatggtcaaggacctccagcatcactgtgtcatccacgacgcctggagcggcctgaggcacgtggtgcagcttcgtgcccaggaggagttcgggcaaggcgagtggagcgagtggagcccggaggccatgggcacgccttggacagaatccaggagtcctccagctgagaacgaggtgtccacccccatgcaggcacttactactaataaagacgatgataatattctcttcagagattctgcaaatgcgacaagcctcccagtgcaagattcttcttcagtaccactgcccacattcctggttgctggagggagcctggccttcggaacgctcctctgcattgccattgttctgaggttcaagaagacgtggaagctgcgggctctgaaggaaggcaagacaagcatgcatccgccgtactctttggggcagctggtcccggagaggcctcgacccaccccagtgcttgttcctctcatctccccaccggtgtcccccagcagcctggggtctgacaatacctcgagccacaaccgaccagatgccagggacccacggagcccttatgacatcagcaatacagactacttcttccccagatag; NM_181359.

IL11:NM_133519:atgaactgtgtttgtcgcctggtcctggtggtgctgagcctctggccagatagagtcgttgcccctgggccaccagctggctcccctc

gagtgtcttcagaccctcgtgcagatctggatagcgctgtcctcttgaccaggtccctcctggcagacacacggcaactagctgcacagatgagagacaaattcccagctgatggagaccacaatctggactccctacctaccttggccatgagcgctgggacactgggatctttgcagcttcctggagtgctgacaaggcttcgagtagacttaatgtcctacttccgacatgtacagtggttgcgccgggcagctggtccttccctaaagactctggagccagagctgggtgccctgcaagcccgactggaacggctacttcgtcgcttacagctcttgatgtctcgcctagccttgccccaggcagccccggaccaacctgcggtccctctgggccctcctgcctcggcctggggaagcatccgggcagctcatgccatcctaggagggctgcacctgaccttggactgggccgtgcggggcctgctgttgttaaagactcggctgtaa; NM_008350.

LIF:NM_002309:atgaaggtcttggcggcaggagttgtgcccctgctgttggttctgcactggaaacatggggcggggagcccctccccatcacccctgtcaacgccacctgtgccatacgccacccatgtcacaacaacctcatgaaccagatcaggagccaactggcacagctcaatggcagtgccaatgccctctttattctctattacacagcccaggggggagccgttccccaacaacctggacaagctatgtggccccaacgtgacggacttcccgcccttccacgccaacggcacggagaaggccaagctggtggagctgtaccgcatagtcgtgtaccttggcacctccctgggcaacatcacccgggaccagaagatcctcaaccccagtgccctcagcctccacagcaagctcaacgccaccgccgacatcctgcgaggcctccttagcaacgtgctgtgccgcctgtgcagcaagtaccacgtgggccatgtggacgtgacctacggccctgacacctcgggtaaggatgtcttccagaagaagaagctgggctgtcaactcctggggaagtataagcagatcatcgccgtgttggcccaggccttctag; NM_008501; BB235045.

LIFR:NM_002310:atgatggatatttacgtatgtttgaaacgaccatcctggatggtggacaataaaagaatgaggactgcttcaaatttccagtggctgttatcaacatttattcttctatatctaatgaatcaagtaaatagccagaaaaaggggctcctcatgatttgaagtgtgtaactaacaatttgcaagtgtggaactgttcttggaaagcaccctctggaacaggccgtggtactgattatgaagtttgcattgaaaacaggtcccgttcttgttatcagttggagaaaaccagtattaaaattccagctctttcacatggtgattatgaaataacaataaattctctacatgattttggaagttctacaagtaaattcacactaaatgaacaaaacgtttccttaattccagatactccagagatcttgaatttgtctgctgatttctcaacctctacattatacctaaagtggaacgacaggggttcagtttttccacaccgctcaaatgttatctgggaaattaaagttctacgtaaagagagtatggagctcgtaaaattagtgacccacaacacaactctgaatggcaaagatacacttcatcactggagttgggcctcagatatgcccttggaatgtgccattcatttgtggaaattagatgctacattgacaatcttcatttttctggtctcgaagagtggagtgactggagccctgtgaagaacatttcttggatacctgattctcagactaaggtttttcctcaagataaagtgatacttgtaggctcagacataacattttgttgtgtgagtcaagaaaaagtgttatcagcactgattggccatacaaactgccccttgatccatcttgatggggaaaatgttgcaatcaagattcgtaatatttctgtttctgcaagtagtggaacaaatgtagtttttacaaccgaagataacatatttggaaccgttatttttgctggatatccaccagatactcctcaacaactgaattgtgagacacatgatttaaaagaaattatatgtagttggaatccaggaagggtgacagcgttggtgggcccacgtgctacaagctacactttagttgaaagtttttcaggaaaatatgttagacttaaaagagctgaagcacctacaaacgaaagctatcaattattatttcaaatgcttccaaatcaagaaatatataatttactttgaatgctcacaatccgctgggtcgatcacaatcaacaattttagttaatataactgaaaaagtttatccccatactcctacttcattcaaagtgaaggatattaattcaacagctgttaaactttcttggcatttaccaggcaactttgcaaagattaatttttatgtgaaattgaaattaagaaatctaattcagtacaagagcagcggaatgtcacaatcaaaggagtagaaaattcaagttatcttgttgctctggacaagttaaatccatacactctatatacttttcggattcgttgttctactgaaactttctggaaatggagcaaatggagcaataaaaaacaacatttaacaacagaagccagtccttcaaaggggcctgatacttggagagagtggagttctgatggaaaaaatttaataatctattggaagcctttacccattaatgaagctaatggaaaaatactttcctacaatgtatcgtgttcatcagatgaggaaacacagtcccttctgaaatccctgatcctcagcacaaagcagagatacgacttgataagaatgactacatcatcagcgtagtggctaaaaattctgtgggctcatcaccaccttccaaaatagcgagtatggaaattccaaatgatgatctcaaaatagaacaagttgttgggatgggaaaggggattctcctcacctggcattacgaccccaacatgacttgcgactacgtcattaagtggtgtaactcgtctcggtcggaaccatgccttatggactggagaaaagttccctcaaacagcactgaaactgtaatagaatctgatgagtttcgaccaggtataagatataatttttcctgtatggatgcagaaatcaaggatatcaattattacgctccatgattggatatatagaagaattggctcccattgttgcaccaaatttactgttgaggatacttctgcagattcgatattagtaaaatgggaagacattcctgtggaagaacttagaggctttttaagaggatatttgttttactttggaaaaggagaaagagacacatctaagatgagggttttagaatcaggtcgttctgacataaaagttaagaatattactgacatatcccagaagacactgagaattgctgatcttcaaggtaaaacaagttaccacctggtcttgcgagcctatacagatggtggagtgggcccggagaagagtatgtatgtggtgacaaaggaaaattctgtgggattaattattgccattctcatcccagtggcagtggctgtcattgttggagtggtgacaagtatcctttgctatcggaaacgagaatggattaaagaaaccttctaccctgatattccaaatccagaaaactgtaaagcattacagtttcaaaagagtgtctgtgagggaagcagtgctcttaaaacattggaaatgaatccttgtaccccaaataatgttgaggttctggaaactcgatcagcatttcctaaaatagaagatacagaaataatttccccagtagctgagcgtcctgaagatcgctctgatgcagagcctgaaaaccatgtggttgtgtcctattgtccacccatcattgaggaagaaataccaaacccagccgcagatgaagctggagggactgcacaggttatttacattgatgttcagtcgatgt

atcagcctcaagcaaaaccagaagaagaacaagaaaatgaccctgtaggaggggcaggctataagccacagatgcacctccccattaattctactgtggaagatatagctgcagaagaggacttagataaaactgcgggttacagacctcaggccaatgtaaatacatggaatttagtgtctccagactctcctagatccatagacagcaacagtgagattgtctcatttggaagtccatgctccattaattcccgacaattttgattcctcctaaagatgaagactctcctaaatctaatggaggagggtggtcctttacaaactttttcagaacaaaccaaacgattaa; NM_013584; NM_031048.

STAT3:NM_003150:atggcccaatggaatcagctacagcagcttgacacacggtacctggagcagctccatcagctctacagtgacagcttcccaatggagctgcggcagtttctggccccttggattgagagtcaagattgggcatatgcggccagcaaagaatcacatgccactttggtgtttcataatctcctgggagagattgaccagcagtatagccgcttcctgcaagagtcgaatgttctctatcagcacaatctacgaagaatcaagcagtttcttcagagcaggtatcttgagaagccaatggagattgcccggattgtggcccggtgcctgtgggaagaatcacgccttctacagactgcagccactgcggcccagcaaggggccaggccaaccaccccacagcagccgtggtgacggagaagcagcagatgctggagcagcaccttcaggatgtccggaagagagtgcaggatctagaacagaaaatgaaagtggtagagaatctccaggatgactttgatttcaactataaaaccctcaagagtcaaggagacatgcaagatctgaatggaaacaaccagtcagtgaccaggcagaagatgcagcagctggaacagatgctcactgcgctggaccagatgcggagaagcatcgtgagtgagctggcggggcttttgtcagcgatggagtacgtgcagaaaactctcacggacgaggagctggctgactggaagaggcggcaacagattgcctgcattggaggcccgcccaacatctgcctagatcggctagaaaactggataacgtcattagcagaatctcaacttcagacccgtcaacaaattaagaaactggaggagttgcagcaaaaagtttcctacaaaggggaccccattgtacagcaccggccgatgctggaggagagaatcgtggagctgtttagaaacttaatgaaaagtgcctttgtggtggagcggcagccctgcatgcccatgcatcctgaccggcccctcgtcatcaagaccggcgtccagttcactactaaagtcaggttgctggtcaaattccctgagttgaattatcagcttaaaattaaagtgtgcattgacaaagactctggggacgttgcagctctcagaggatcccggaaatttaacattctgggcacaaacacaaaagtgatgaacatggaagaatccaacaacggcagcctctctgcagaattcaaacacttgaccctgagggagcagagatgtgggaatgggggccgagccaattgtgatgcttccctgattgtgactgaggagctgcacctgatcacctttgagaccgaggtgtatcaccaaggcctcaagattgacctagagacccactccttgccagttgtggtgatctccaacatctgtcagatgccaaatgcctgggcgtccatcctgtggtacaacatgctgaccaacaatcccaagaatgtaaactttttaccaagcccccaattggaacctgggatcaagtggccgaggtcctgagctggcagttctcctccaccaccaagcgaggactgagcatcgagcagctgactacactggcagagaaactcttgggacctggtgtgaattattcagggtgtcagatcacatgggctaaattttgcaaagaaaacatggctggcaagggcttctccttctgggtctggctggacaatatcattgaccttgtgaaaaagtacatcctggccctttggaacgaagggtacatcatgggctttatcagtaaggagcgggagcgggccatcttgagcactaagcctccaggcaccttcctgctaagattcagtgaaagcagcaaagaaggaggcgtcactttcacttgggtggagaaggacatcagcggtaagacccagatccagtccgtggaaccatacacaaagcagcagctgaacaacatgtcatttgctgaaatcatcatgggctataagatcatggatgctaccaatatcctggtgtctccactggtctatctctatcctgacattcccaaggaggaggcattcggaaagtattgtcggccagagagccaggagcatcctgaagctgacccaggcgctgccccatacctgaagaccaagtttatctgtgtgacaccaacgacctgcagcaataccattgacctgccgatgtcccccgcactttagattcattgatgcagtttggaaataatggtgaaggtgctgaaccctcagcaggagggcagtttgagtccctcacctttgacatggagttgacctcggagtgcgctacctcccccatgtga; NM_213662; NM_139276.

NUMB:

AF171938:atgaacaaattacggcaaagttttaggagaaagaaggatgtttatgttccagaggccagtcgtccacatcagtggcagacagatgaagaaggcgttcgcaccggaaaatgtagcttcccggttaagtaccttggccatgtagaagttgatgaatcaagaggaatgcacatctgtgaagatgctgtaaaaagattgaaagctgaaaggaagttcttcaaaggcttctttggaaaaactggaaagaaagcagttaaagcagttctgtgggtctcagcagatggactcagagttgtggatgaaaaaactaaggacctcatagttgaccagacgatagagaaagtttctttctgtgccccagacaggaactttgatagagccttttcttacatatgccgtgatggcaccactcgtcgctggatctgtcactgcttcatggctgtcaaggacacaggtgaaaggttgagccatgcagtaggctgtgcttttgcagcctgtttagagcgcaagcagaagcgggagaaggaatgtggagtgactgctacttttgatgctagtcggaccacttttacaagagaaggatcattccgtgtcacaacagccactgaacaagcagaaagagaggagatcatgaaacaaatgcaagatgccaagaaagctgaaacagataagatagtcgttggttcatcagttgcccctggcaacactgccccatccccatcctctcccacctctcctacttctgatgccacgacctctctggagatgaacaatcctcatgccatcccacgccggcatgctccaattgaacagcttgctcgccaaggctctttccgaggttttcctgctcttagccagaagatgtcaccctttaaacgccaactatccctacgcatcaatgagttgccttccactatgcagaggaagactgatttccccattaaaaatgcagtgccagaagtagaaggggaggcagagagcatcagctccctgtgctcacagatcaccaatgccttcagcacacctgaggaccccttctcatctgctccgatgaccaaaccagtgacagtggtggcaccacaatctcctaccttccaagctaatggcactgactcagccttccatgtgcttgctaagccagcccatactgctctagcacccgtag

caatgcctgtgcgtgaaaccaacccttgggcccatgcccctgatgctgctaacaaggaaattgcagccacatgttcggggaccgagtggggtcaatcttctggtgctgcctctccaggtctcttccaggccggtcatagacgtactccctctgaggccgaccgatggttagaagaggtgtctaagagcgtccgggctcagcagccccaggcctcagctgctcctctgcagccagttctccagcctcctccacccactgccatctcccagccagcatcacctttccaagggaatgcattcctcacctctcagcctgtgccagtgggtgtggtcccagccctgcaaccagcctttgtccctgcccagtcctatcctgtggccaatggaatgccctatccagcccctaatgtgcctgtggtgggcatcactccctcccagatggtggccaacgtatttggcactgcaggccaccctcaggctgcccatccccatcagtcacccagcctggtcaggcagcagacattccctcactacgaggcaagcagtgctaccaccagtcccttctttaagcctcctgctcagcacctcaacggttctgcagctttcaatggtgtagatgatggcaggttggcctcagcagacaggcatacagaggttcctacaggcacctgcccagtggatccttttgaagcccagtgggctgcattagaaaataagtccaagcagcgtactaatccctcccctaccaaccctttctccagtgacttacagaagacgtttgaaattgaactttaa;

AF171939:atgaacaaattacggcaaagttttaggagaaagaaggatgtttatgttccagaggccagtcgtccacatcagtggcagacagatgaagaaggcgttcgcaccggaaaatgtagcttcccggttaagtaccttggccatgtagaagttgatgaatcaagaggaatgcacatctgtgaagatgctgtaaaaagattgaaagctgaaaggaagttcttcaaaggcttctttggaaaaactggaaagaaagcagttaaagcagttctgtgggtctcagcagatggactcagagttgtggatgaaaaaactaaggacctcatagttgaccagacgatagagaaagtttctttctgtgccccagacaggaactttgatagagccttttcttacatatgccgtgatggcaccactcgtcgctggatctgtcactgcttcatggctgtcaaggacacaggtgaaaggttgagccatgcagtaggctgtgcttttgcagcctgtttagagcgcaagcagaagcgggagaaggaatgtggagtgactgctacttttgatgctagtcggaccacttttacaagagaaggatcattccgtgtcacaacagccactgaacaagcagaaagagaggagatcatgaaacaaatgcaagatgccaagaaagctgaaacagataagatagtcgttggttcatcagttgcccctggcaacactgccccatccccatcctctcccacctctcctacttctgatgccacgacctctctggagatgaacaatcctcatgccatcccacgccggcatgctccaattgaacagcttgctcgccaaggctctttccgaggttttcctgctcttagccagaagatgtcaccctttaaacgccaactatccctacgcatcaatgagttgccttccactatgcagaggaagactgatttccccattaaaaatgcagtgccagaagtagaaggggaggcagagagcatcagctccctgtgctcacagatcaccaatgccttcagcacacctgaggaccccttctcatctgctccgatgaccaaaccagtgacagtggtggcaccacaatctcctaccttccaagggaccgagtggggtcaatcttctggtgctgcctctccaggtctcttccaggccggtcatagacgtactccctctgaggccgaccgatggttagaagaggtgtctaagagcgtccgggctcagcagccccaggcctcagctgctcctctgcagccagttctccagcctcctccacccactgccatctcccagccagcatcacctttccaagggaatgcattcctcacctctcagcctgtgccagtgggtgtggtcccagccctgcaaccagcctttgtccctgcccagtcctatcctgtggccaatggaatgccctatccagcccctaatgtgcctgtggtgggcatcactccctcccagatggtggccaacgtatttggcactgcaggccaccctcaggctgcccatccccatcagtcacccagcctggtcaggcagcagacattccctcactacgaggcaagcagtgctaccaccagtcccttctttaagcctcctgctcagcacctcaacggttctgcagctttcaatggtgtagatgatggcaggttggcctcagcagacaggcatacagaggttcctacaggcacctgcccagtggatccttttgaagcccagtgggctgcattagaaaataagtccaagcagcgtactaatccctcccctaccaaccctttctccagtgacttacagaagacgtttgaaattgaactttaa

AF171940:atgaacaaattacggcaaagttttaggagaaagaaggatgtttatgttccagaggccagtcgtccacatcagtggcagacagatgaagaaggcgttcgcaccggaaaatgtagcttcccggttaagtaccttggccatgtagaagttgatgaatcaagaggaatgcacatctgtgaagatgctgtaaaaagattgaaagctactggaaagaaagcagttaaagcagttctgtgggtctcagcagatggactcagagttgtggatgaaaaaactaaggacctcatagttgaccagacgatagagaaagtttctttctgtgccccagacaggaactttgatagagccttttcttacatatgccgtgatggcaccactcgtcgctggatctgtcactgcttcatggctgtcaaggacacaggtgaaaggttgagccatgcagtaggctgtgcttttgcagcctgtttagagcgcaagcagaagcgggagaaggaatgtggagtgactgctacttttgatgctagtcggaccacttttacaagagaaggatcattccgtgtcacaacagccactgaacaagcagaaagagaggagatcatgaaacaaatgcaagatgccaagaaagctgaaacagataagatagtcgttggttcatcagttgcccctggcaacactgccccatccccatcctctcccacctctcctacttctgatgccacgacctctctggagatgaacaatcctcatgccatcccacgccggcatgctccaattgaacagcttgctcgccaaggctctttccgaggttttcctgctcttagccagaagatgtcaccctttaaacgccaactatccctacgcatcaatgagttgccttccactatgcagaggaagactgatttccccattaaaaatgcagtgccagaagtagaaggggaggcagagagcatcagctccctgtgctcacagatcaccaatgccttcagcacacctgaggaccccttctcatctgctccgatgaccaaaccagtgacagtggtggcaccacaatctcctaccttccaagctaatggcactgactcagccttccatgtgcttgctaagccagcccatactgctctagcacccgtagcaatgcctgtgcgtgaaaccaacccttgggcccatgcccctgatgctgctaacaaggaaattgcagccacatgttcggggaccgagtggggtcaatcttctggtgctgcctctccaggtctcttccaggccggtcatagacgtactccctctgaggccgaccgatggttagaagaggtgtctaagagcgtccgggctcagcagccccaggcctcagctgctcctctgcagccagttctccag

cctcctccacccactgccatctcccagccagcatcacctttccaagggaatgcattcctcacctctcagcctgtgccagtgggtgtggtcccagccctgcaaccagcctttgtccctgcccagtcctatcctgtggccaatggaatgccctatccagcccctaatgtgcctgtggtgggcatcactccctcccagatggtggccaacgtatttggcactgcaggccaccctcaggctgcccatccccatcagtcacccagcctggtcaggcagcagacattccctcactacgaggcaagcagtgctaccaccagtcccttctttaagcctcctgctcagcacctcaacggttctgcagctttcaatggtgtagatgatggcaggttggcctcagcagacaggcatacagaggttcctacaggcacctgcccagtggatccttttgaagcccagtgggctgcattagaaaataagtccaagcagcgtactaatccctcccctaccaaccctttctccagtgacttacagaagacgtttgaaattgaactttaa;

AF171941:atgaacaaattacggcaaagttttaggagaaagaaggatgtttatgttccagaggccagtcgtccacatcagtggcagacagatgaagaaggcgttcgcaccggaaaatgtagcttcccggttaagtaccttggccatgtagaagttgatgaatcaagaggaatgcacatctgtgaagatgctgtaaaaagattgaaagctactggaaagaaagcagttaaagcagttctgtgggtctcagcagatggactcagagttgtggatgaaaaaactaaggacctcatagttgaccagacgatagagaaagtttctttctgtgccccagacaggaactttgatagagccttttcttacatatgccgtgatggcaccactcgtcgctggatctgtcactgcttcatggctgtcaaggacacaggtgaaaggttgagccatgcagtaggctgtgcttttgcagcctgtttagagcgcaagcagaagcgggagaaggaatgtggagtgactgctacttttgatgctagtcggaccacttttacaagagaaggatcattccgtgtcacaacagccactgaacaagcagaaagagaggagatcatgaaacaaatgcaagatgccaagaaagctgaaacagataagatagtcgttggttcatcagttgcccctggcaacactgccccatccccatcctctcccacctctcctacttctgatgccacgacctctctggagatgaacaatcctcatgccatcccacgccggcatgctccaattgaacagcttgctcgccaaggctctttccgaggttttcctgctcttagccagaagatgtcaccctttaaacgccaactatccctacgcatcaatgagttgccttccactatgcagaggaagactgatttccccattaaaaatgcagtgccagaagtagaaggggaggcagagagcatcagctccctgtgctcacagatcaccaatgccttcagcacacctgaggaccccttctcatctgctccgatgaccaaaccagtgacagtggtggcaccacaatctcctaccttccaagggaccgagtggggtcaatcttctggtgctgcctctccaggtctcttccaggccggtcatagacgtactccctctgaggccgaccgatggttagaagaggtgtctaagagcgtccgggctcagcagccccaggcctcagctgctcctctgcagccagttctccagcctcctccacccactgccatctcccagccagcatcacctttccaagggaatgcattcctcacctctcagcctgtgccagtgggtgtggtcccagccctgcaaccagcctttgtccctgcccagtcctatcctgtggccaatggaatgccctatccagcccctaatgtgcctgtggtgggcatcactccctcccagatggtggccaacgtatttggcactgcaggccaccctcaggctgcccatccccatcagtcacccagcctggtcaggcagcagacattccctcactacgaggcaagcagtgctaccaccagtcccttctttaagcctcctgctcagcacctcaacggttctgcagctttcaatggtgtagatgatggcaggttggcctcagcagacaggcatacagaggttcctacaggcacctgcccagtggatccttttgaagcccagtgggctgcattagaaaataagtccaagcagcgtactaatccctcccctaccaaccctttctccagtgacttacagaagacgtttgaaattgaactttaa; NM_010949; NM_133287; BB483123; NM_010950; NM_010949 ; NM_004756; DQ022744.

Numblike:NM_00475:atgtcccgcagcgcggcggccagcggcggaccccggaggcctgagcggcacctgcccccagcccccctgtggggccccggggcccccagaaacctgcaggacggagccagacggggcgggcaccatgaacaagttacggcagagcctgcggcggaggaagccagcctacgtgcccgaggcgtcgcgcccgcaccagtggcaggcagacgaggacgcggtgcggaagggcacgtgcagcttcccggtcaggtacctgggtcacgtggaggtagaggagtcccggggaatgcacgtgtgtgaagatgcggtgaagaagctgaaggcgatgggccgaaagtccgtgaagtctgtcctgtgggtgtcagccgatgggctccgagtggtggacgacaaaaccaaggatcttctggtcgaccagaccatcgaaaaggtctccttttgtgctcctgaccgcaacctggacaaggctttctcctatatctgtcgtgacgggactacccgccgctggatctgccactgttttctggcactgaaggactccggcgagaggctgagccacgctgtgggctgtgcttttgccgcctgcctggagcgaaaacagcgacgggagaaggaatgtggggtcacggccgccttcgatgccagccgcaccagcttcgcccgcgagggctccttccgcctgtctgggggtgggcggcctgctgagcgagaggccccggacaagaagaaagcagaggcagcagctgcccccactgtggctcctggccctgcccagcctgggcacgtgtccccgacaccagccaccacatcccctggtgagaagggtgaggcaggcacccctgtggctgcaggcaccactgcggccgccatcccccggcgccatgcacccctggagcagctggttcgccagggctccttccgtgggttcccagcactcagccagaagaactcgcctttcaaacggcagctgagcctacggctgaatgagctgccatccacgctgcagcgccgcactgacttccaggtgaagggcacagtgcctgagatggagcctcctggtgccggcgacagtgacagcatcaacgctctgtgcacacagatcagttcatcttttgccagtgctggagcgccagcaccagggccaccacctgccacaacagggacttctgcctggggtgagccctccgtgcccccctgcagctgccttccagcctgggcacaagcggacaccttcagaggctgagcgatggctggaggaggtgtcacaggtggccaaggcccagcagcagcagcagcagcaacagcaacagcagcagcagcagcagcagcaacagcagcaagcagcctcagtggccccagtgcccaccatgcctcctgccctgcagcctttccccgcccccgtggggccctttgacgctgcacctgcccaagtggccgtgttcctgccacccccacacatgcagcccccttttgtgcccgcctacccgggcttgggctacccaccgatgccccg

ggtgcccgtggtgggcatcacaccctcacagatggtggcaaacgccttctgctcagccgcccagctccagcctcagcctgccactctgcttgggaaagctggggccttcccgccccctgccatacccagtgcccctgggagccaggcccgccctcgccccaatggggcccccctggccccctgagccagcgcctgccccagctccagagttggacccctttgaggcccagtgggcggcattagaaggcaaagccactgtagagaaacccctccaacccctttctggcgacctgcaaaagacattcgagattgaactgtag; U96441; NM_010950; DQ022744.

NANOG:NM_024865:atgagtgtggatccagcttgtccccaaagcttgccttgctttgaagcatccgactgtaaagaatcttcacctatgcctgtgatttgtgggcctgaagaaaactatccatccttgcaaatgtcttctgctgagatgcctcacacggagactgtctctcctcttccttcctccatggatctgcttattcaggacagccctgattcttccaccagtcccaaaggcaaacaacccacttctgcagagaagagtgtcgcaaaaaaggaagacaaggtcccggtcaagaaacagaagaccagaactgtgttctcttccacccagctgtgtgtactcaatgatagatttcagagacagaaatacctcagcctccagcagatgcaagaactctccaacatcctgaacctcagctacaaacaggtgaagacctggttccagaaccagagaatgaaatctaagaggtggcagaaaaacaactggccgaagaatagcaatggtgtgacgcagaaggcctcagcacctacctaccccagcctttactcttcctaccaccagggatgcctggtgaacccgactgggaaccttccaatgtggagcaaccagacctggaacaattcaacctggagcaaccagacccagaacatccagtcctggagcaaccactcctggaacactcagacctggtgcacccaatcctggaacaatcaggcctggaacagtcccttctataactgtggagaggaatctctgcagtcctgcatgcagttccagccaaattctcctgccagtgacttggaggctgccttggaagctgctggggaaggccttaatgtaatacagcagaccactaggtattttagtactccacaaaccatggatttattcctaaactactccatgaacatgcaacctgaagacgtgtga; BC137873; NM_028016; NM_013633; BC160187

**OncostatinM(OSM):**NM_020530:atgggggtactgctcacacagaggacgctgctcagtctggtccttgcactcctgtttccaagcatggcgagcatggcggctataggcagctgctcgaaagagtaccgcgtgctccttggccagctccagaagcagacagatctcatgcaggacaccagcagactcctggacccctatatacgtatccaaggcctggatgttcctaaactgagagagcactgcagggagcgccccggggccttccccagtgaggagaccctgagggggctgggcaggcggggcttcctgcagaccctcaatgccacactgggctgcgtcctgcacagactggccgacttagagcagcgcctccccaaggcccaggatttggagaggtctgggctgaacatcgaggacttggagaagctgcagatggcgaggccgaacatcctcgggctcaggaacaacatctactgcatggcccagctgctggacaactcagacacggctgagcccacgaaggctggccgggggcctctcagccgcccacccccacccctgcctcggatgcttttcagcgcaagctggagggctgcaggttcctgcatggctaccatcgcttcatgcactcagtggggcgggtcttcagcaagtgggggagagcccgaaccggagccggagacacagcccccaccaggccctgaggaagggggtgcgcaggaccagaccctccaggaaaggcaagagactcatgaccaggggacagctgccccggtag;NM_001013365 ;NP_065391; NP_001013383.

OSMR:NM_003999:atggctctatttgcagtctttcagacaacattcttcttaacattgctgtccttgaggacttaccagagtgaagtcttggctgaacgtttaccattgactcctgtatcacttaaagtttccaccaattctacgcgtcagagtttgcacttacaatggactgtccacaaccttccttatcatcaggaattgaaaatggtatttcagatccagatcagtaggattgaaacatccaatgtcatctgggtggggaattacagcaccactgtgaagtggaaccaggttctgcattggagctgggaatctgagctccctttggaatgtgccacacactttgtaagaataaagagtttggtggacgatgccaagttccctgagccaaatttctggagcaactggagttcctgggaggaagtcagtgtacaagattctactggacaggatatattgttcgttttccctaaagataagctggtggaagaaggcaccaatgttaccatttgttacgtttctaggaacattcaaaataatgtatcctgttatttggaagggaaacagattcatggagaacaacttgatccacatgtaactgcattcaacttgaatagtgtgcctttcattaggaataaagggacaaatatctattgtgaggcaagtcaaggaaatgtcagtgaaggcatgaaaggcatcgttctttttgtctcaaaagtacttgaggagcccaaggacttttcttgtgaaaccgaggacttcaagactttgcactgtacttgggatcctgggacggacactgccttggggtggtctaaacaaccttcccaaagctacactttatttgaatcattttctggggaaaagaaactttgtacacacaaaaactggtgtaattggcaaataactcaagactcacaagaaacctataacttcacactcatagctgaaaattacttaaggaagagaagtgtcaatatccttttaacctgactcatcgagtttatttaatgaatccttttagtgtcaactttgaaaatgtaaatgccacaaatgccatcatgacctggaaggtgcactccataaggaataatttcacatatttgtgtcagattgaactccatggtgaaggaaaaatgatgcaatacaatgtttccatcaaggtgaacggtgagtacttcttaagtgaactggaacctgccacagagtacatggcgcgagtacggtgtgctgatgccagccacttctggaaatggagtgaatggagtggtcagaacttcaccacacttgaagctgctccctcagaggcccctgatgtctggagaattgtgagcttggagccaggaaatcatactgtgaccttattctggaagccattatcaaaactgcatgccaatggaaagatcctgttctataatgtagttgtagaaaacctagacaaaccatccagttcagagctccattccattccagcaccagccaacagcacaaaactaatccttgacaggtgttcctaccaaatctgcgtcatagccaacaacagtgtgggtgcttctcctgcttctgtaatagtcatctctgcagaccccgaaaacaaagaggttgaggaagaaagaattgca

ggcacagagggtggattctctctgtcttggaaaccccaacctggagatgttataggctatgttgtggactggtgtgaccatacccaggatgtgctcggtgatttccagtggaagaatgtaggtcccaataccacaagcacagtcattagcacagatgcttttaggccaggagttcgatatgacttcagaatttatgggttatctacaaaaaggattgcttgtttattagagaaaaaaacaggatactctcaggaacttgctccttcagacaaccctcacgtgctggtggatacattgacatcccactccttcactctgagttggaaagattactctactgaatctcaacctggttttatacaagggtaccatgtctatctgaaatccaaggcgaggcagtgccacccacgatttgaaaaggcagttctttcagatggttcagaatgttgcaaatacaaaattgacaacccggaagaaaaggcattgattgtggacaacctaaagccagaatccttctatgagtttttcatcactccattcactagtgctggtgaaggccccagtgctacgttcacgaaggtcacgactccggatgaacactcctcgatgctgattcatatcctactgcccatggttttctgcgtcttgctcatcatggtcatgtgctacttgaaaagtcagtggatcaaggagacctgttatcctgacatccctgacccttacaagagcagcatcctgtcattaataaaattcaaggagaaccctcacctaataataatgaatgtcagtgactgtatcccagatgctattgaagttgtaagcaagccagaagggacaaagatacagttcctaggcactaggaagtcactcacagaaaccgagttgactaagcctaactacctttatctccttccaacagaaaagaatcactctggccctggcccctgcatctgttttgagaacttgacctataaccaggcagcttctgactctggctcttgtggccatgttccagtatccccaaaagccccaagtatgctgggactaatgacctcacctgaaaatgtactaaaggcactagaaaaaaactacatgaactccctgggagaaatcccagctggagaaacaagtttgaattatgtgtcccagttggcttcacccatgtttggagacaaggacagtctcccaacaaacccagtagaggcaccacactgttcagagtataaaatgcaaatggcagtctccctgcgtcttgccttgcctcccccgaccgagaatagcagcctctcctcaattaccctttttagatccaggtgaacactactgctaa;NP_003990.1

OCT3/4(POU5F1):NM_203289:atgcacttctacagactattccttggggccacacgtaggttcttgaatcccgaatggaaaggggagattgataactggtgtgtttatgttcttacaagtcttctgccttttaaaatccagtcccaggacatcaaagctctgcagaaagaactcgagcaatttgccaagctcctgaagcagaagaggatcaccctgggatatacacaggccgatgtggggctcaccctgggggttctatttgggaaggtattcagccaaacgaccatctgccgctttgaggctctgcagcttagcttcaagaacatgtgtaagctgcggcccttgctgcagaagtgggtggaggaagctgacaacaatgaaaatcttcaggagatatgcaaagcagaaaccctcgtgcaggcccgaaagagaaagcgaaccagtatcgagaaccgagtgagaggcaacctggagaatttgttcctgcagtgcccgaaacccacactgcagcagatcagccacatcgcccagcagcttgggctcgagaaggatgtggtccgagtgtggttctgtaaccggcgccagaagggcaagcgatcaagcagcgactatgcacaacgagaggattttgaggctgctgggtctcctttctcaggggggaccagtgtcctttcctctggccccagggccccatttggtaccccaggctatgggagccctcacttcactgcactgtactcctcggtccctttccctgagggggaagcctttccccctgtctccgtcaccactctgggctctcccatgcattcaaactga;

NM_002701:atggcgggacacctggcttcggatttcgccttctcgcccccctccaggtggtggaggtgatgggccagggggccggagccgggctgggttgatcctcggacctggctaagcttccaaggccctcctggagggccaggaatcgggccgggggttgggccaggctctgaggtgtgggggattcccccatgccccccgccgtatgagttctgtgggggggatggcgtactgtgggccccaggttggagtggggctagtgccccaaggcggcttggagacctctcagcctgagggcgaagcaggagtcggggtggagagcaactccgatggggcctccccggagccctgcaccgtcacccctggtgccgtgaagctggagaaggagaagctggagcaaaacccggaggagtcccaggacatcaaagctctgcagaaagaactcgagcaatttgccaagctcctgaagcagaagaggatcaccctgggatatacacaggccgatgtggggctcaccctggggggttctatttgggaaggtattcagccaaacgaccatctgccgctttgaggctctgcagcttagcttcaagaacatgtgtaagctgcggcccttgctgcagaagtgggtggaggaagctgacaacaatgaaaatcttcaggagatatgcaaagcagaaaccctcgtgcaggcccgaaagagaaagcgaaccagtatcgagaaccgagtgagaggcaacctggagaatttgttcctgcagtgcccgaaacccacactgcagcagatcagccacatcgcccagcagcttgggctcgagaaggatgtggtccgagtgtggttctgtaaccggcgccagaagggcaagcgatcaagcagcgactatgcacaacgagaggattttgaggctgctgggtctcctttctcaggggggaccagtgtcctttcctctggccccagggccccatttggtaccccaggctatgggagccctcacttcactgcactgtactcctcggtccctttccctgagggggaagcctttccccctgtctccgtcaccactctgggctctcccatgcattcaaactga; NM_013633; EF032593; NM_131112; NM_001114955.

SOX2:NM_003106:atgtacaacatgatggagacggagctgaagccgccgggcccgcagcaaacttcgggggcggcggcggcaactccaccgcggcggcggccggcggcaaccagaaaaacagcccggaccgcgtcaagcggcccatgaatgccttcatggtgtggtcccgcgggcagcggcgcaagatggcccaggagaaccccaagatgcacaactcggagatcagcaagcgcctgggcgccgagtggaaacttttgtcggagacggagaagcggccgttcatcgacgaggctaagcggctgcgagcgctgcacatgaaggagcacccggattataaataccggccccggcggaaaaccaagacgctcatgaagaaggataagtacacgctgcccggcgggctgctggcccccggcggcaatagcatggcgagcggggtcggggtgggcgccggcctgggcgcgggcgtgaaccagcgcatggacagttacgc

gcacatgaacggctggagcaacggcagctacagcatgatgcaggaccagctgggctacccgcagcacccgggcctcaatgcgcacggcgcagcgcagatgcagcccatgcaccgctacgacgtgagcgccctgcagtacaactccatgaccagctcgcagacctacatgaacggctcgcccacctacagcatgtcctactcgcagcagggcacccctggcatggctcttggctccatgggttcggtggtcaagtccgaggccagctccagccccctgtggttacctcttcctcccactccagggcgccctgccaggccggggacctccgggacatgatcagcatgtatctccccggcgccgaggtgccggaacccgccgcccccagcagacttcacatgtcccagcactaccagagcggcccggtgcccggcacggccattaacggcacactgcccctctcacacatgtga; NM_011443; NM_00110918.

FGF4:NM_002007:atgtcggggcccgggacggccgcggtagcgctgctcccggcggtcctgctggccttgctggcgccctgggcgggccgagggggcgccgccgcacccactgcacccaacggcacgctggaggccgagctggagcgccgctgggagagcctggtggcgctctcgttggcgcgcctgccggtggcagcgcagcccaaggaggcggccgtccagagcggcgccggcgactacctgctgggcatcaagcggctgcggcggctctactgcaacgtgggcatcggcttccacctccaggcgctccccgacggccgcatcggcggcgcgcacgcggacacccgcgacagcctgctggagctctcgcccgtggagcggggcgtggtgagcatcttcggcgtggccagccggttcttcgtggccatgagcagcaagggcaagctctatggctcgcccttcttcaccgatgagtgcacgttcaaggagattctccttcccaacaactacaacgcctacgagtcctacaagtaccccggcatgttcatcgccctgagcaagaatgggaagaccaagaaggggaaccgagtgtcgcccaccatgaaggtcacccacttcctccccaggctgtga; NM_010202; NM_004380; NM_001025432; NM_004379.2; NP_004370; NM_134442; NP_604391

Gata2:NM_032638:atggaggtggcgccggagcagccgcgctggatggcgcacccggccgtgctgaatgcgcagcaccccgactcacaccacccgggcctggcgcacaactacatggaacccgcgcagctgctgcctccagacgaggtggacgtcttcttcaatcacctcgactcgcagggcaacccctactatgccaaccccgctcacgcgcgggcgcgcgtctcctacagccccgcgcacgcccgcctgaccggaggccagatgtgccgcccacacttgttgcacagcccgggtttgccctggctggacgggggcaaagcagccctctctgccgctgcggcccaccaccacaacccctggaccgtgagccccttctccaagacgccactgcacccctcagctgctggaggccctggaggcccactctctgtgtacccaggggctgggggtgggagcgggggaggcagcgggagctcagtggcctccctcacccctacagcagcccactctggctcccaccttttcggcttcccacccacgccacccaaagaagtgtctcctgaccctagcaccacgggggctgcgtctccagcctcatcttccgcggggggtagtgcagcccgaggagaggacaaggacggcgtcaagtaccaggtgtcactgacggagagcatgaagatggaaagtggcagtcccctgcgcccaggcctagctactatgggcacccagcctgctacacaccaccccatccccacctacccctcctatgtgccggcggctgcccacgactacagcagcggactcttccaccccggaggcttcctgggggggaccggcctccagcttcaccccctaagcagcgcagcaaggctcgttcctgttcagaaggccgggagtgtgtcaactgtggggccacagccacccctctctggcggcgggacggcaccggccactacctgtgcaatgcctgtggcctctaccacaagatgaatgggcagaaccgaccactcatcaagcccaagcgaagactgtcggccgccagaagagccggcacctgttgtgcaaattgtcagacgacaaccaccaccttatggcgccgaaacgccaacggggaccctgtctgcaacgcctgtggcctctactacaagctgcacaatgttaacaggccactgaccatgaagaaggaagggatccagactcggaaccggaagatgtccaacaagtccaagaagagcaagaaaggggcggagtgcttcgaggagctgtcaaagtgcatgcaggagaagtcatccccccttcagtgcagctgccctggctggacacatggcacctgtgggccacctcccgcccttcagccactccggacacatcctgcccactccgacgcccatccacccctcctccagcctctccttcggccacccccacccgtccagcatggtgaccgccatgggctag; AB102789; AB102790; NM_008090.

Gata3:NM_001002295:atggaggtgacggcggaccagccgcgctgggtgagccaccaccaccccgccgtgctcaacgggcagcacccggacacgcaccacccgggcctcagccactcctacatggacgcggcgcagtacccgctgccggaggaggtggatgtgcttttaacatcgacggtcaaggcaaccacgtcccgccctactacggaaactcggtcagggccacggtgcagaggtaccctccgacccaccacgggagccaggtgtgccgcccgcctctgcttcatggatccctaccctggctggacggcggcaaagccctgggcagccaccacaccgcctccccctggaatctcagccccttctccaagacgtccatccaccacggctccccggggccccctctccgtctacccccccggcctcgtcctcctccttgtcgggggggccacgccagcccgcacctcttcaccttcccgcccaccccgccgaaggacgtctccccggacccatcgctgtccaccccaggctcggccggctcggcccggcaggacgagaaagagtgcctcaagtaccaggtgcccctgcccgacagcatgaagctggagtcgtcccactcccgtggcagcatgaccgccctgggtggagcctcctcgtcgacccaccaccccatcaccacctacccgccctacgtgcccgagtacagctccggactcttcccccccagcagcctgctgggcggctcccccaccggcttcggatgcaagtccaggcccaaggcccggtccagcacagaaggcagggagtgtgtgaactgtggggcaacctcgaccccactgtggcggcgagatggcacgggacactacctgtgcaacgcctgcgggctctatcacaaaatgaacggacagaaccggcccctcattaagcccaagcgaaggctgtctgcagccaggagagcagggacgtcctgtgcgaactgtcagaccaccacaaccacactctggaggaggaatgccaatg

gggaccctgtctgcaatgcctgtgggctctactacaagcttcacaatattaacagacccctgactatgaagaaggaaggcatccagaccagaaaccgaaaaatgtctagcaaatccaaaaagtgcaaaaaagtgcatgactcactggaggacttccccaagaacagctcgtttaacccggccgccctctccagacacatgtcctccctgagccacatctcgcccttcagccactccagccacatgctgaccacgcccacgccgatgcacccgccatccagcctgtcctttggaccacaccacccctccagcatggtcaccgccatgggttag; NM_008091; AM392688; AM392571; NM_001002295; NM_002051

Gata4:BC101580:atgtatcagagcttggccatggccgccaaccacgggccgccccccggtgcctacgaggcgggcggccccggcgccttcatgcacggcgcgggcgccgcgtcctcgccagtctacgtgcccacaccgcgggtgccctcctccgtgctgggcctgtcctacctccagggcggaggcgcgggctctgcgtccggaggcgcctcgggcggcagctccggtggggccgcgtctggtgcggggcccgggacccagcagggcagcccgggatggagccaggcgggagccgacggagccgcttacaccccgccgccggtgtcgccgcgcttctccttcccggggaccaccgggtccctggcggccgccgccgccgctgccgcggcccgggaagctgcggcctacagcagtggcggcggagcggcgggtgcgggcctggcgggccgcgagcagtacgggcgcgccggcttcgcgggctcctactccagccccctacccggcttacatggccgacgtgggcgcgtcctgggccgcagccgccgccgcctccgccggccccttcgacagcccggtcctgcacagcctgcccggccgggccaacccggccgcccgacaccccaatctcgatatgtttgacgacttctcagaaggcagagagtgtgtcaactgtggggctatgtccaccccgctctggaggcgagatgggacgggtcactatctgtgcaacgcctgcggcctctaccacaagatgaacggcatcaaccggccgctcatcaagcctcagcgccggctgtccgcctcccgccgagtgggcctctcctgtgccaactgccagaccaccaccaccacgctgtggcgccgcaatgcggagggcgagcctgtgtgcaatgcctgcggcctctacatgaagctccacggggtccccaggcctcttgcaatgcggaaagaggggatccaaaccagaaaacggaagcccaagaacctgaataaatctaagacaccagcagctccttcaggcagtgagagccttcctcccgccagcggtgcttccagcaactccagcaacgccaccaccagcagcagcgaggagatgcgtcccatcaagacggagcctggcctgtcatctcactacgggcacagcagctccgtgtcccagacgttctcagtcagtgcgatgtctggccatgggccctccatccaccctgtcctctcggccctgaagctctccccacaaggctatgcgtctcccgtcagccagtctccacagaccagctccaagcaggactcttggaacagcctggtcttggccgacagtcacggggacataatcactgcgtaa; AF179424; DQ666280.

Gata5:BC117356:atgtaccagagcctggcgctggccgcgagcccccgccaggccgcctacgccgactcgggctccttcctgcacgctccgggcgccggctctccgatgtttgtgccgccggcgcgcgtcccctcgatgctgtcctacctgtccgggtgtgagccgagcccgcagcccccgagctcgctgcgcgccccggctgggcgcagacagccaccgcggattcgtcggccttcggcccgggcagtccgcacccccagccgcgcacccgcccggggccaccgccttccctttcgcgcacagcccctcgggggcccggcagcggcggcagcgcgggggggccgagacggcagtgcctaccagggcgcgctgttgcctcgagaacagttcgcggccccgcttgggcggccggtggggacctcgtactccgccacctacccggcctacgtgagccccgacgtggcccagtcctggactgccgggcccttcgatggcagcgtcctgcacggcctcccaggccgcaggcccaccttcgtgtccgacttcttggaggagttcccgggtgagggtcgtgagtgtgtcaactgcggggccctgtccacaccgctgtggcgccgagatggcaccggccactacctgtgcaatgcctgcggcctctaccacaagatgaatggcgtcaaccggccgctcgttcggcctcagaagcgcctgtcctcgtcccgccgcgccggcctctgctgcaccaactgccacacgaccaacaccacgctgtggcggcggaactcggagggggagcccgtgtgcaatgcctgcggcctctacatgaagctgcacggggtgccgcggcctctggctatgaagaaagaaagcatccagacacggaagcggaagccaaagaccatcgccaaggccaggggctcctcaggatccacaaggaatgcctcggcctccccatctgctgtcgccagcactgacagctcagcagccacttccaaagccaagcccagcctggcgtccccagtgtgccctgggcccagcatggcccccaggcctctggccaggaggatgactctcttgcccccggccacttggagttcaagttcgagcctgaggactttgccttcccctccacggccccgagcccccaggctggcctcagggggctctgcgccaagaggcctggtgtgcgctggccttggcctag; BC105654.

Gata6:NM_005257:atggccttgactgacggcggctggtgcttgccgaagcgcttcggggccgcgggtgcggacgccagcgactccagagcctttccagcgcgggagccctccacgccgccttcccccatctcttcctcgtcctcctcctgctcccggggcggagagcggggccccggcggcgccagcaactgcgggacgcctcagctcgacacggaggcggcggccggacccccggcccgctcgctgctgctcagttcctacgcttcgcatcccttcggggctccccacggaccttcggcgcctggggtcgcgggccccgggggcaacctgtcgagctgggaggacttgctgctgttcactgacctcgaccaagccgcgaccgccagcaagctgctgtggtccagccgcggcgccaagctgagccccttcgcacccgagcagccggaggagatgtaccagaccctcgccgctctctccagccagggtccggccgcctacgacggcgcgcccggcggcttcgtgcactctgcggccgcggcggcagcagccgcggcggcggccagctccccggtctacgtgcccaccacccgcgtgggttccatgctgcccggcctaccgtaccacctgcaggggtcgggcagtgggccagccaaccacgcgggcggcgcgggcgcgcaccccggctggcctcaggcctcggccgacagccctccatacggcagcggaggcggcgcggctggcggcggggccgcggggcctggcggcgctggctcagccgcggcgcacgtctcggcgcgcttccc

ctactctcccagcccgcccatggccaacggcgccgcgcgggagccgggaggctacgcggcggcgggcagtgggggcgcgggaggcgtgagcggcggcggcagtagcctggcggccatgggcggccgcgagccccagtacagctcgctgtcggccgcgcggccgctgaacgggacgtaccaccaccaccaccaccaccaccaccaccatccgagcccctactcgccctacgtgggggcgccactgacgcctgcctggcccgccggacccttcgagaccccggtgctgcacagcctgcagagccgcgccggagccccgctcccggtgccccggggtcccagtgcagacctgctggaggacctgtccgagagccgcgagtgcgtgaactgcggctccatccagacgccgctgtggcggcgggacggcaccggccactacctgtgcaacgcctgcgggctctacagcaagatgaacggcctcagccggcccctcatcaagccgcagaagcgcgtgccttcatcacggcggcttggattgtcctgtgccaactgtcacaccacaactaccaccttatggcgcagaaacgccgagggtgaacccgtgtgcaatgcttgtggactctacatgaaactccatggggtgcccagaccacttgctatgaaaaagagggaattcaaaccaggaaacgaaaacctaagaacataaataaatcaaagacttgctctggtaatagcaataattccattcccatgactccaacttccacctcttctaactcagatgattgcagcaaaaatacttcccccacaacacaacctacagcctcaggggcgggtgccccggtgatgactggtgcgggagagagcaccaatcccgagaacagcgagctcaagtattcgggtcaagatgggctctacataggcgtcagtctcgcctcgccggccgaagtcacgtcctccgtgcgaccggattcctggtgcgccctggccctggcctga;AF179425; EF444980; NM_005257; NP_005248.

HNF1:NM_000458:atggtgtccaagctcacgtcgctccagcaagaactcctgagcgccctgctgagctccggggtcaccaaggaggtgctggttcaggccttggaggagttgctgccatccccgaacttcggggtgaagctggagacgctgcccctgtcccctggcagcggggccgagcccgacaccaagccggtcttccatactctcaccaacggccacgccaagggccgcttgtccggcgacgagggctccgaggacggcgacgactatgacacacctcccatcctcaaggagctgcaggcgctcaacaccgaggaggcggcggagcagcgggcggaggtggaccggatgctcagtgaggacccttggagggctgctaaaatgatcaagggttacatgcagcaacacaacatcccccagagggaggtggtcgatgtcaccggcctgaaccagtcgcacctctcccagcatctcaacaagggcacccctatgaagacccagaagcgtgccgctctgtacacctggtacgtcagaaagcaacgagagatcctccgacaattcaaccagacagtccagagttctggaaatatgacagacaaaagcagtcaggatcagctgctgtttctctttccagagttcagtcaacagagccatgggcctgggcagtccgatgatgcctgctctgagcccaccaacaagaagatgcgccgcaaccggttcaaatgggggcccgcgtcccagcaaatcttgtaccaggcctacgatcggcaaaagaaccccagcaaggaagagagagaggccttagtggaggaatgcaacagggcagaatgtttgcagcgaggggtgtcccctccaaagcccacggcctgggctccaacttggtcactgaggtccgtgtctacaactggtttgcaaaccgcaggaaggaggaggcattccggcaaaagctggccatggacgcctatagctccaaccagactcacagcctgaaccctctgctctcccacggctcccccaccaccagcccagctcctctcctccaaacaagctgtcaggagtgcgctacagccagcagggaaacaatgagatcacttcctcctcaacaatcagtcaccatggcaacagcgccatggtgaccagccagtcggttttacagcaagtctccccagccagcctggacccaggccacaatctcctctcacctgatggtaaaatgatctcagtctcaggaggaggtttgcccccagtcagcaccttgacgaatatccacagcctctcccaccataatccccagcaatctcaaaacctcatcatgacacccctctctggagtcatggcaattgcacaaagcctcaacacctcccaagcacagagtgtccctgtcatcaacagtgtggccggcagcctggcagccctgcagcccgtccagttctcccagcagctgcacagccctcaccagcagcccctcatgcagcagagcccaggcagccacatggcccagcagcccttcatggcagctgtgactcagctgcagaactcacacatgtacgcacacaagcaggaacccccccagtattcccacacctcccggtttccatctgcaatggtggtcacagataccagcagcatcagtacactcaccaacatgtcttcaagtaaacagtgtcctctacaagcctggtga; NM_013103; NM_000545;NM_009327;

NM_012669:atggtttctaagttgagccagctgcagacggagctcctggctgctctgctcgagtcgggcctgagcaaagaggctctgatccaggctctgggggagcccgggccctacctgatggttggagatggtcccctggacaaggggggagtcctgcggtgggactcgaggggacctgaccgagctgcccaatggcctgggggagacgcgtggctcggaagatgacacggatgacgatggggaagacttcgcgccacccattctgaaagagctggagaacctcagcccagaggaggcagcccaccagaaagccgtggtggagtcacttcttcaggaggacccatggcgcgtggcaaagatggtcaagtcgtacctgcagcaacacaacatcccccagcgggaggtggtggacactacgggtctcaaccagtcccacctgtcccagcacctcaacaagggcacccccatgaagacgcagaagcgggccgcgctgtacacctggtacgtccgcaagcagcgagaggtggctcagcaattcacccacgcggggcagggcggactgattgaagagcccacaggtgatgagctgccaaccaaaaggggcggaggaaccggttcaagtggggccccgcatcccagcagatcctgttccaggcttacgagaggcagaagaaccccagcaaggaagagcgagagaccttggtggaggagtgcaatagggcggagtgcatccagagaggggtgtcaccatcgcaggcccaggggctaggctccaaccttgtcaccgaggtgcgtgtctacaactggtttgccaaccggcgcaaggaagaagcctttcggcataagctggccatggacacgtataacgggcctccacccgggccaggccccggccctgcgctacctgcccacagttccccgggcctgcccacaaccaccctctctcccagtaaggtccacggtgtgcggtatggacagtctgcaaccagcgaggcagctgaggtgccctccagcagcggaggtcccttagtcacagtgtctgcggccttacaccaagtgtcccccacaggcttggagcccagcagcctgctgagcaccgaggccaagctggtctc

agccacggggggtcccctgcctcccgtcagcaccctgacagcactgcacagcttggagcagacgtctccaggtctcaaccagcagccgcagaaccttatcatggcctcgctgcctggggtcatgaccatcggcccaggggagcccgcctccctgggtcccacgttcactaacacgggtgcctctaccctggtcattggtctggcctccacacaggcacagagcgtgccagtcatcaacagcatggggagcagcctgaccaccctgcagccggtccagttttcccagccactgcacccttcctatcagcagcctctcatgccccctgtacagagccacgtggcccagagtcccttcatggcaaccatggcccagctgcagagcccccacgccctgtacagccacaagcctgaggtggcccagtacacgcatacaagcctgcttccgcagaccatgctgatcacagacaccaacctcagcacccttgccagcctcacgcccaccaagcaggtcttcacctcagacacagaggcctccagtgagcctgggcttcatgagccgtcgtctccagccacaaccattcacatccccagccaggacccgtcaaacatccagcacctgcagcctgctcaccggctcagcaccagtcccacagtgtcctccagcagcctggtgttgtaccagagttctgactccaacgggcacagccacctgctgccatccaaccacggtgtcatcgagacttttatctccacccagatggcctcctcctcccagtaa; NM_009330.

HNF3:X74936:atgttagggactgtgaagatggaagggcatgagagcaacgactggaacagctactacgcggacacgcaggaggcctactcctctgtccctgtcagcaacatgaactccggcctgggctctatgaactccatgaacacctacatgaccatgaacaccatgaccacgagcggcaacatgaccccggcttccttcaacatgtcctacgccaacacgggcttaggggccggcctgagtcccggtgctgtggctggcatgccaggggcctctgcaggcgccatgaacagcatgactgcggcgggcgtcacggccatgggtacggcgctgagcccgggaggcatgggctccatgggcgcgcagcccgtcacctccatgaacggcctgggtccctacgccgccgccatgaacccgtgcatgagtcccatggcgtacgcgccgtccaacctgggccgcagccgcgcggggggcggcggcgacgccaagacattcaagcgcagctaccctcacgccaagccgccttactcctacatctcgctcatcacgatggccatccagcaggcgcccagcaagatgctcacgctgagcgagatctaccagtggatcatggacctcttcccctattaccgccagaaccagcagcgctggcagaactccatccgccactcgctgtccttcaacgattgtttcgtcaaggtggcacgatccccagacaagccaggcaagggctcctactggacgctgcacccggactccggcaacatgttcgagaacggctgctacttgcgccgccaaaagcgcttcaagtgtgagaagcagccgggggccggaggtgggagtgggggcggcggctccaaagggggcccagaaagtcgcaaggacccctcaggcccggggaaccccagcgccgagtcacccccttcattggggtgtgcacggaaaggctagccagctagagggcgcgccggcccccgggcccgccgccagcccccagactctggaccacagcggggccacggcgacagggggcgcttcggagttgaagtctccagcgtcttcatctgcgccccccataagctccgggccaggggcgctggcatctgtacccccctctcacccggctcacggcctggcaccccacgaatctcagctgcatctgaaaggggatccccactactcctttaatcacccccttctccatcaacaacctcatgtcctcctccgagcaacagcacaagctggacttcaaggcatacgagcaggcgctgcagtactctccttatggcgctaccttgcccgccagtctgccccttggcagcgcctcagtggccacgaggagccccatcgagccctcagccctggagccagcctactaccaaggtgtgtattccagacccgtgctaaatacttcctag

HNF3gammaX74938M:atgctgggctcagtgaagatggaggctcatgacctggccgagtggagctactacccggaggcgggcgaggtgtattctccagtgaatcctgtgcccaccatggcccctctcaactcctacatgaccttgaacccactcagctctccctaccctcccggagggcttcaggcctccccactgcctacaggacccctggcacccccagcccccactgcgcccttggggcccaccttcccaagcttgggcactggtggcagcaccggaggcagtgcttccgggtatgtagccccagggcccgggcttgtacatggaaaagagatggcaaaggggtaccggcggccactggcccacgccaaaccaccatattcctacatctctctcataaccatggctattcagcaggctccaggcaagatgctgaccctgagtgaaatctaccaatggatcatggacctcttcccgtactaccgggagaaccagcaacgttggcagaactccatccggcattcgctgtccttcaatgactgcttcgtcaaggtggcacgctccccagacaagccaggcaaaggctcctactgggccttgcatcccagctctgggaacatgtttgagaacggatgctatctccgccggcagaagcgcttcaagctggaggagaaggcaaagaaaggaaacagcgccacatcggccagcaggaatggtactgcggggtcagccacctctgccaccactacagctgccactgcagtcacctccccggctcagccccagcctacgccatctgagcccgaggcccagagtggggatgatgtggggggtctggactgcgcctcacctccttcgtccacaccttatttcagcggcctggagctcccgggggaactaaagttggatgcgccctataacttcaaccaccctttctctatcaacaacctgatgtcagaacagacatcgacaccttccaaactggatgtggggtttgggggctacggggctgagagtggggagcctggagtctactaccagagcctctattcccgctctctgcttaatgcatcctag

HNF3betaX74937:atgctgggagccgtgaagatggaagggctcgagccatccgactggagcagctactacgcggagcccgagggctactcttccgtgagcaacatgaacgccggcctggggatgaatggcatgaacacatacatgagcatgtccgcggctgccatgggcggcggttccggcaacatgagcgcgggctccatgaacatgtcatcctatgtgggcgctggaatgagcccgtcgctagctggcatgtccccgggcgccggcgccatggcgggcatgagcggctcagccggggcggccggcgtggcgggcatgggacctcacctgagtccgagtctgagcccgctcgggggacaggcggccggggccatgggtggccttgcccccctacgccaacatg

aactcgatgagccccatgtacgggcaggccggcctgagccgcgctcgggaccccaagacataccgacgcagctacacacacgccaaacctccctactcgtacatctcgctcatcaccatggccatccagcagagccccaacaagatgctgacgctgagcgagatctatcagtggatcatggacctcttccctttctaccggcagaaccagcagcgctggcagaactccatccgccactctctctccttcaacgactgctttctcaaggtgccccgctcgccagacaagcctggcaagggctccttctggaccctgcacccagactcgggcaacatgttcgagaacggctgctacctgcgccgccagaagcgcttcaagtgtgagaagcaactggcactgaaggaagccgcgggtgcggccagtagcggaggcaagaagaccgctcctgggtcccaggcctctcaggctcagctcggggaggccgcgggctcggcctccgagactccggcgggcaccgagtcccccccattccagcgcttctccgtgtcaggagcacaagcgaggtggcctaagcgagctaaagggagcacctgcctctgcgctgagtcctcccgagccggcgccctcgcctgggcagcagcagcaggctgcagcccacctgctgggcccacctcaccacccaggcctgccaccagaggcccacctgaagcccgagcaccattacgccttcaaccaccccttctctatcaacaacctcatgtcgtccgagcagcaacatcaccacagccaccaccaccatcagccccacaaaatggacctcaaggcctacgaacaggtcatgcactacccagggggctatggttcccccatgccaggcagcttggccatgggcccagtcacgaacaaagcgggcctggatgcctcgcccctggctgcagacacttcctactaccaaggagtgtactccaggcctattatgaactcatcctaa

HNF3G:AH008133:atgctgggctcagtgaagatggaggcccatgacctggccgagtggagctactacccggaggcgggcgaggtctactcgccggtgaccccagtgcccaccatggccccctcaactcctacatgaccctgaatcctctaagctctccctatcccccctgggggctccctgcctccccactgccctcaggacccctggcacccccagcacctgcagccccccctggggcccactttcccaggcctgggtgtcagcggtggcagcagcagctccgggtacggggccccgggtcctgggctggtgcacgggaaggagatgccgaaggggtatcggcggcccctggcacacgccaagccaccgtattcctatatctcactcatcaccatggccatccagcaggcgccgggcaagatgctgaccttgagtgaaatctaccagtggatcatggacctcttcccttactaccgggagaatcagcagcgctggcagaactccattcgccactcgctgtctttcaacgactgcttcgtcaaggtggcgcgttccccagacaagcctggcaagggctcctactgggccctacaccccagctcagggaacatgtttgagaatggctgctacctgcgccgccagaaacgcttcaagctggaggagaaggtgaaaaaagggggcagcggggctgccaccaccaccaggaacgggacagggtctgctgcctcgaccaccacccccgcggccacagtcacctccccgccccagcccccgcctccagcccctgagcctgaggcccagggcggggaagatgtgggggctctggactgtggctcacccgcttcctccacaccctatttcactggcctggagctcccaggggagctgaagctggacgcgccctacaacttcaaccaccctttctccatcaacaacctaatgtcagaacagacaccagcacctcccaaactggacgtggggtttgggggctacggggctgaaggtggggagcctggagtctactaccagggcctctattcccgctctttgcttaatgcatcctag

HNF3A:AH008132:atgttaggaactgtgaagatggaagggcatgaaaccagcgactggaacagctactacgcagacacgcaggaggcctactcctcggtcccggtcagcaacatgaactcaggcctgggctccatgaactccatgaacacctacatgaccatgaacaccatgactacgagcggcaacatgaccccggcgtccttcaacatgtcctatgccaacccggccttaggggccggcctgagtcccggcgcagtagccggcatgccgggggctcggcgggcgccatgaacagcatgactgcggccggcgtgacggccatgggtacggcgctgagcccgagcggcatgggcgccatgggtgcgcagcaggcggcctccatgatgaatggcctgggcccctacgcggccgccatgaacccgtgcatgagccccatggcgtacgcgccgtccaacctgggccgcagccgcgcgggcggcggcggcgacgccaagacgttcaagcgcagttacccgcacgccaagccgccctactcgtacatctcgctcatcaccatggccatccagcgggcgcccagcaagatgctcacgctgagcgagatctaccagtggatcatggacctcttcccctattaccggcagaaccagcagcgctggcagaactccatccgccactcgctgtccttcaatgactgcttcgtcaaggtggcacgctccccggacaagccgggcaagggctcctactggacgctgcacccggactccggcaacatgttcgagaacggctgctacttgcgccgccagaagcgcttcaagtgcgagaagcagccgggggccggcggcgggggcgggagcggaagcgggggcagcggcgccaagggcggccctgagagccgcaaggacccctctggcgcctctaaccccagcgccgactcgcccctccatcggggtgtgcacgggaagaccggccagctagagggcgcgccggccccgggcccggccgccagccccagactctggaccacagtggggcgacggcgacagggggcgcctcggagttgaagactccagcctcctcaactgcgccccccataagctccgggcccggggcgctggcctctgtgcccgcctctcacccggcacacggcttggcaccccacgagtcccagctgcacctgaaaggggacccccactactccttcaaccacccgttctccatcaacaacctcatgtcctcctcggagcagcagcataagctggacttcaaggcatacgaacaggcactgcaatactcgccttacggctctacgttgcccgccagcctgcctctaggcagcgcctcggtgaccaccaggagccccatcgagccctcagccctggagccggcgtactaccaaggtgtgtattccagacccgtcctaaacacttcctag

HNF4alpha:NM_008261:atgcgactctctaaaacccttgccggcatggatatggccgactacagcgctgccctggacccagcctacaccaccctggagtttgaaaatgtgcaggtgttgaccatgggcaatgacacgtccccatctgaaggtgccaacctcaattcatccaacagcctgggcgtcagtgccctgtgcgccatctgtggcgaccgggccaccggcaaacactacggagcctcgagctgtgacggctgcaaggggttcttcaggaggagcgtgaggaagaaccacatgtactcctgcaggtttagccgacaatgtgtggtagacaaagataagaggaaccagtgtcgttactgcaggcttaagaagtgcttccgggctggcatgaagaaggaagctgtccaaaatgagcgggaccggatcagcacgcggaggtcaagctacgaggacagcagcctgccctccatcaacgcgctcctgcaggcagaggttctgtcccagcagatcacctctcccatctctgggatcaatggcgacattcgggcaaagaagattgccaacatcacagacgtgtgtgagtctatgaaggagcagctgctggtcctggtcgagtgggccaagtacatcccggccttctgcgaactccttctggatgaccaggtggcgctgctcagggcccacgccggtgagcatctgctgcttggagccaccaagaggtccatggtgtttaaggacgtgctgctcctaggcaatgactacatcgtccctcggcactgtccagagctagcggagatgagccgtgtgtccatccgcatcctcgatgagctggtcctgcccttccaagagctgcagattgatgacaatgaatatgcctgcctcaaagccatcatcttctttgatccagatgccaaggggctgagtgacccgggcaagatcaagcggctgcggtcacaggtgcaagtgagcctggaggattacatcaacgaccggcagtacgactctcggggccgctttggagagctgctgctgctgttgcccacgctgcagagcatcacctggcagatgatcgaacagatccagttcatcaagctcttcggcatggccaagattgacaacctgctgcaggagatgcttctcggagggtctgccagtgatgcaccccacacccaccacccccctgcaccctcacctgatgcaagaacacatgggcaccaatgtcattgttgctaacacgatgccctctcacctcagcaatggacagatgtgtgagtggccccgacccagggggcaggcagccactcccgagactccacagccatcaccaccaagtggctcgggatctgaatcctacaagctcctgccaggagccatcaccaccatcgtcaagcctccctctgccattccccagccaacgatcaccaagcaagaagccatctag

HNF4a:NM_022180:atggacatggctgactacagtgctgccttggacccagcctacaccaccctggagtttgaaaatgtgcaggtgttgaccatgggcaatgacacatccccatctgaaggtgccaacctcaactcatccaacagcctgggtgtcagtgccctgtgtgccatctgtggcgatcgggccactggcaaacactacggagcctcaagctgtgacggctgcaagggattcttcaggaggagcgtgaggaagaaccacatgtactcctgcaggtttagcaggcagtgcgtggtagacaaagataagaggaaccagtgtcgttactgcaggctcaagaagtgcttccgggctggcatgaagaaagaagccgtccaaaatgagcgggatcggatcagcacgcggaggtcaagctacgaggacagcagcctaccctccattaatgcgctcctgcaggcagaggtcctgtctcagcagatcacctcccccatctctgggatcaatggcgacattcgggccaagaagattgccaacatcacggatgtgtgtgagtctatgaaggagcagctgctggttctggtcgaatgggccaagtacatcccggccttctgtgaacttcttctggatgaccaggtggcgctgctcagagcccacgctggtgagcacctgctgcttggagccaccaagaggtccatggtgttcaaggatgtgctgctcctaggcaatgactacatcgtccctcggcactgtccagagctagcagagatgagccgtgtgtccattcgcatcctcgatgagctggtcttgcccttccaagagctgcagatcgatgataatgaatacgcctgcctcaaagccatcatcttctttgacccagatgccaaggggctgagtgacccaggcaagatcaagcggctgcggtcacaggtgcaggtgagcctggaggattacatcaacgaccggcagtatgactctcggggtcgttttggagagctgctgctgctcctgcccactctgcagagcattacctggcagatgatcgagcagatccagttcatcaagctctttggcatggccaagattgacaacctgctgcaggagatgctgcttggagggtctgccagtgacgcgccccacgcccaccaccccctgcaccctcacctgatgcaagaacacatgggcaccaatgtcatagttgccaacacgatgccctctcacctcagcaatggacagatgtgtgagtggccccggcccagggggcaggcagccacccctgagactccacagccatcaccaccaagtggctctggatctgaatcctacaagctcctgccaggagccatcaccaccatcgtcaagcctccctctgccatcccccagccaacgatcaccaagcaggaagccatctag

HNF6:U95945:atgaacgcacagctgaccatggaggcgatcggcgagctgcacggggtgagccatgagccggtgcccgcccctgctgacctgctgggcggcagccctcacgcgcgcagctccgtgggacaccgcggcagccacctgcctcccgcgcacccgcgttccatgggcatggcgtccctgctggacggcggcagcggaggcagcgattaccaccaccaccaccgcgcccctgagcacagcttggctggcccccctgcaccccaccatgaccatggcctgtgaaactcccccaggtatgagcatgcccaccacctacactaccttaacccctctgcagccgctgccgcccatctccaccgtgtccgacaagttccctcaccatcatcaccaccaccatcaccaccaccacccacaccaccaccaccagcgcctggcgggcaacgtgagcggtagtttcacacttatgcgggatgagcgcgggctggcctctatgaataacctctataccccctaccacaaggacgtggctggcatgggccagagcctctcgcccctctctggctccggtctgggcagcattcacaactcccagcaaggacttccccactatgctcatcccggcgcggctatgcccaccgacaagatgctcaccccaaatggctttgaagcccaccaccctgccatgctcggtcgccacggggagcagcacctcacgcccacctcggccggcatggtacccatcaacggccttcctccgcaccatcctcatgcccacctgaatgcccagggccacggacagctcctgggcacagcccgagagcccaacccttcggtgaccggcgcgcaggtcagcaatggaagtaattcagggcagatggaagagatcaataccaaagaggtggcgcagcgtatcaccaccgagc

tcaaacgttacagcatcccacaggccatcttcgcgcagagggtgctctgccgttcccaggggaccctttcggacctgctgcgaaaccccaagccctggagcaaactcaagtcgggtcgggagaccttccggaggatgtggaagtggctgcaggagccggagttccagcgcatgtcggcgctccgcttagcagcctgcaaacggaaagagcaagaacatgggaaggacagaggcaacacccccaaaaagcccaggctggtcttcacagacgtccaacgtcgaactctacatgcaatattcaaggaaaataagcgtccgtccaaagaattacaaatcaccatctcccagcagctggggttggagctgagcactgtcagcaacttcttcatgaatgccagaaggaggagtctggacaagtggcaggacgagggcggctccaactcaggcagttcatcgtcctcatcgagcacttgtaccaaagcatga

HLXB9:NM_001096823:atggagaagtccaagaatttcaggattgacgctctcctggcgatagatcccccaaggctcagacctccccattggctctggtcacctcgctgtcctcctcgtctctctccgggagcccccgtccgagcacactgacagcctcaggactgactcccccctcccctccaaggacttgtggactggtccctaaaccaggtttcctgagcagccaccagcacccccaaacatgatgtcattgcacccccaggctgctccagggatccccctcaggccctgtatggacacccgatgtacagctacttggcagcggggcagcacccagctctgtcctacccctactcccagatgcagagcagccaccacccccaccccatggaccccatcaagatcagcgctggcaccttccaactggaccagtggctcagagcctccactgccggcatgatgctgcccaaaatggcagactttaactcccaggcccaatccaacctgctgggaaagtgcagaagaccaaggacagcgtttaccagtcagcagctgttggaactggagcaccaattcaagctgaacaagtacctctccaggccgaaacgctttgaagtggccacttccctgatgctcactgagacgcaggtgaagatctggttccagaacaggcgcatgaaatggaagaggagtaagaaagccaaggagcaggcggcgcaggactcagcagagaaacagcagagggcaggcaagggcagcagcgaggagaagtgctcggatgagctgcaggaagagaagaaatcctaccatctccatcccagggggagcccatcaaagggaacggccgcctgcagcccagagactatacagacagcgaagaggacgaggaggaggacagggaagaggaggaagaggaagatcacagaggggaggggaagcggttttaccatcattcttctgactgcacatccgaggaagaggagaacagccacaataagcagagcggccactga; NM_019944.

atggaaaaatccaaaaatttccgcatcgacgccctgctggccgtggatcccccgcgagccgcctccacgcagagcgcgcctctggccttggtcacttccctcgcgactacagtatctggtcccggccgcggcggcagcggcggcgggggggaccagtagcggggcgagccgtagctgcagtcccgcatcctcggaggccactgcagcgcccggtgaccggctgagagctgagagcccgtcgcccccacgcttgctggctgcacactgcgcgctgctgcccaagcccggattcctgggcgccggaggaggcggcggcgcggcgggtgggccgggcactccccaccaccacgcgcaccctggtgcagcagccgccgcggctgccgctgccgctgccgcggctgccggtggcctggcactggggctgcacccgggggcgcacagggcggcgcgggcctccctgcacaggcggctctctatggacacccggtctacagttattcggcagcagctgcagcggccgcgctagctggccagcacccggcgctttcctactcataccctcaggtgcagggcgcgcaccctgcgcaccctgccgaccccatcaagctgggtgccagcaccttccaactggaccagtggctgcgcgcgtctactgcgggcatgatcctgcccaagatgccggacttcagctgtcaggcgcagtcgaacctcttggggaagtgccgaaggcctcgcacggccttcaccagccagcagctgttggagctggaacaccagttcaagctcaacaagtacctgtctcgacccaagcgttttgaggtggctacctcgctcatgctcaccgagactcaggtgaagatttggttccagaaccgccgaatgaaatggaaacgcagcaaaaaggccaaagagcaggctgcgcaggaggcggagaagcagaagggcggcggcgggggcaccggcaaaggcggcagtgaggagaagacggaagaggagctgatggggcctccggtttcgggggacaaggcaagcggccgtcgcctgcgggacttgcgggacagtgaccctgatgaggacgaggatgatgaagaagaggacaacttcccgtacagcaatggtgccggtgcccatgctgcctcatccgactgctcatctgaggacgactcgcctcctccaagactaggcgggcctggacaccaacctctgccccagtag

NM_005515:atggaaaaatccaaaaatttccgcatcgacgccctgctggccgtggatcccccgcgagccgcctccacgcagagcgcgcctctggccttggtcacttccctcgcgactacagtatctggtcccggccgcggcggcagcggcggcgggggggaccagtagcggggcgagccgtagctgcagtcccgcatcctcggaggccactgcagcgcccggtgaccggctgagagctgagagcccgtcgcccccacgcttgctggctgcacactgcgcgctgctgcccaagcccggattcctgggcgccggaggaggcggcggcgcggcgggtgggccgggcactccccaccaccacgcgcaccctggtgcagcagccgccgcggctgccgctgccgctgccgcggctgccggtggcctggcactggggctgcacccgggggcgcacagggcggcgcgggcctccctgcacaggcggctctctatggacacccggtctacagttattcggcagcagctgcagcggccgcgctagctggccagcacccggcgctttcctactcataccctcaggtgcagggcgcgcaccctgcgcaccctgccgaccccatcaagctgggtgccagcaccttccaactggaccagtggctgcgcgcgtctactgcgggcatgatcctgcccaagatgccggacttcagctgtcaggcgcagtcgaacctcttggggaagtgccgaaggcctcgcacggccttcaccagccagcagctgttggagctggaacaccagttcaagctcaacaagtacctgtctcgacccaagcgttttgaggtggctacctcgctcatgctcaccgagactcaggtgaagatttggttccagaaccgccgaatgaaatggaaacgcagcaaaaaggcca

aagagcaggctgcgcaggaggcggagaagcagaagggcggcggcgggggcaccggcaaaggcggcagtgaggagaagacggaagaggagctgatggggcctccggtttcgggggacaaggcaagcggccgtcgcctgcgggacttgcgggacagtgaccctgatgaggacgaggatgatgaagaagaggacaacttcccgtacagcaatggtgccggtgcccatgctgcctcatccgactgctcatctgaggacgactcgcctcctccaagactaggcgggcctggacaccaacctctgccccagtag

Lbx1:NM_006562:atgacttccaaggaggacggcaaggcggcgccgggggaggagcggcggcgcagcccgctggaccacctgcctccgcctgccaactccaacaagccactgacgccgttcagcatcgaggacatcctcaacaagccgtctgtgcggagaagttactcgctgtgcggggcggcgcacctgctggccgccgcggacaagcacgcgcagggcggcttgcccctggcgggccgcgcgctgctctcgcagacctcgccgctgtgcgcgctggaggagctcgccagcaagacgtttaaggggctggaggtcagcgttctgcaggcagccgaaggccgcgacggtatgaccatctttgggcagcggcagacccctaagaagcggcgaaagtcgcgcacggccttcaccaaccaccagatctatgaattggaaaagcgctttctataccagaagtacctgtcccccgccgatcgcgaccaaatcgcgcagcagctgggcctcaccaacgcgcaagtcatcacctggttccagaatcggcgcgctaagctcaagcgggacctggaggagatgaaggccgacgtagagtccgccaagaaactgggccccagcgggcagatggacatcgtggcgctggccgaactcgagcagaactcggaggccacagccggcggtggcggcggctgcggcagggccaagtcgaggcccggctctccggtcctcccccccaggcgccccgaaggcccccgggcgctggcgccctgcagctctcgcctgcctctccgctcacggaccagccggccagcagccaggactgctcggaggacgaggaagacgaagagatcgacgtggacgattga; NM_010691.

Lmx1b:atgttggacggcatcaagatggaggagcacgccctgcgccccgggcccgccactctgggggtgctgctgggctccgactgcccgcatcccgccgtctgcgagggctgccagcggcccatctccgaccgcttcctgatgcgagtcaacgagtcgtcctggcacgaggagtgtttgcagtgcgcggcgtgtcagcaagccctcaccaccagctgctacttccgggatcggaaactgtactgcaaacaagactaccaacagctcttcgcggccaagtgcagcggctgcatggagaagatcgccccccaccgagttcgtgatgcgggcgctggagtgcgtgtaccacctgggctgcttctgctgctgcgtgtgtgaacggcagctacgcaagggcgacgaattcgtgctcaaggagggccagctgctgtgcaagggtgactacgagaaggagaaggacctgctcagctccgtgagccccgacgagtccgactccgtgaagagcgaggatgaagatggggacatgaagccggccaaggggcagggcagtcagagcaagggcagcggggatgacgggaaggacccgcggaggcccaagcgaccccggaccatcctcaccacgcagcagcgaagagccttcaaggcctccttcgaggtctcgtcgaagccttgccgaaaggtccgagagacactggcagctgagacgggcctcagtgtgcgcgtggtccaggtctggtttcagaaccaaagagcaaagatgaagaagctggcgcggcggcaccagcagcagcaggagcagcagaactcccagcggctgggccaggaggtcctgtccagccgcatggagggcatgatggcttcctacacgccgctggccccaccacagcagcagatcgtggccatggaacagagcccctacggcagcagcgacccettccagcagggcctcacgccgccccaaatgccagggaacgactccatcttccatgacatcgacagcgatacctccttaaccagcctcagcgactgcttcctcggctcctcagacgtgggctccctgcaggcccgcgtggggaaccccatcgaccggctctactccatgcagagttcctacttcgcctcctga; NM_010725

Neurogenin(NEUROG1):NM_006161:atgccagcccgccttgagacctgcatctccgacctcgactgcgccagcagcagcggcagtgacctatccggcttcctcaccgacgaggaagactgtgccagactccaacaggcagcctccgcttcggggccgcccgcgccggcccgcaggggcgcgcccaatatctcccgggcgtctgaggttccaggggcacaggacgacgagcaggagaggcggcggcgccgcggccggacgcgggtccgctccgaggcgctgctgcactcgctgcgcaggagccggcgcgtcaaggccaacgatcgcgagcgcaaccgcatgcacaacttgaacgcggccctggacgcactgcgcagcgtgctgccctcgttccccgacgacaccaagctcaccaaaatcgagacgctgcgcttcgcctacaactacatctgggctctggccgagacactgcgcctggcggatcaagggctgcccggaggcggtgcccgggagcgcctcctgccgccgcagtgcgtcccctgcctgcccggtcccccaagccccgccagcgacgcggagtcctggggctcaggtgccgccgccgcctccccgctctctgaccccagtagcccagccgcctccgaagacttcacctaccgccccggcgaccctgttttctccttcccaagcctgcccaaagacttgctccacacaacgccctgtttcattccttaccactag; BQ169355.

Neurogenin2(NEUROG2):NM_024019:atgttcgtcaaatccgagaccttggagttgaaggaggaagaggacgtgttagtgctgctcggatcggcctcccccgccttggcggccctgaccccgctgtcatccagcgccgacgaagaagaggaggaggagccgggcgcgtcaggcggggcgcgtcggcagcgcggggctgaggccgggcagggggcgcggggcggcgtggctgcgggtgcggagggctgccggcccgcacggctgctgggtctggtacacgattgcaaacggcgcccttcccgggcgcgggccgtctcccgaggcgccaagacggccgagacggtgcagcgcatcaagaagacccgtagactgaaggccaacaaccgcgagc

gaaaccgcatgcacaacctcaacgcggcactggacgcgctgcgcgaggtgctccccacgttccccgaggacgccaagctcaccaagatcgagaccctgcgcttcgcccacaactacatctgggcactcaccgagaccctgcgcctggcggatcactgcgggggcggcggcgggggcctgccgggggcgctcttctccgaggcagtgttgctgagcccgggaggcgccagcgccgccctgagcagcagcggagacagcccctcgcccgcctccacgtggagttgcaccaacagccccgcgccgtcctcctccgtgtcctccaattccacctcccccta cagctgcactttatcgcccgccagcccggccgggtcagacatggactattggcagcccccacctcccgacaagcaccgctatgcacctcacctccccatagccagggattgtatctag; DR001447.

Neurogenin3(NEUROG3):atgacgcctcaaccctcgggtgcgcccactgtccaagtgacccgtgagacggagcggtccttccccagagcctcggaagacgaagtgacctgccccacgtccgccccgcccagccccactcgcacacgggggaactgcgcagaggcggaagagggaggctgccgaggggccccgaggaagctccgggcacggcgcgggggacgcagccggcctaagagcgagttggcactgagcaagcagcgacggagtcggcgaaagaaggccaacgaccgcgagcgcaatcgaatgcacaacctcaactcggcactggacgccctgcgcggtgtcctgcccaccttcccagacgacgcgaagctcaccaagatcgagacgctgcgcttcgcccacaactacatctgggcgctgactcaaacgctgcgcatagcggaccacagcttgtacgcgctggagccgccggcgccgcactgcggggagctgggcagcccaggcggttcccccggggactgggggtccctctactccccagtctcccaggctggcagcctgagtcccgccgcgtcgctggaggagcgacccgggctgctgggggccacctttccgcctgcttgagcccaggcagtctggctttctcagatttctgtga; NM_009719.

MASH1:NM_004316:atggaaagctctgccaagatggagagcggcggcgccggccagcagccccagccgcagccccagcagcccttcctgccgcccgcagcctgtttctttgccacggccgcagccgcggcggccgcagccgccgcagcggcagcgcagagcgcgcagcagcagcagcagcagcagcagcagcagcagcaggcgccgcagctgagaccggcggccgacggccagccctcagggggcggtcacaagtcagcgcccaagcaagtcaagcgacagcgctcgtcttcgcccgaactgatgcgctgcaaacgccggctcaacttcagcggctttggctacagcctgccgcagcagcagccggccgccgtggcgcgccgcaacgagcgcgagcgcaaccgcgtcaagttggtcaacctgggctttgccacccttcgggagcacgtccccaacggcgcggccaacaagaagatgagtaaggtggagacactgcgctcggcggtcgagtacatccgcgcgctgcagcagctgctggacgagcatgacgcggtgagcgccgccttccaggcaggcgtcctgtcgcccaccatctccccaactactccaacgacttgaactccatggccggctcgccggtctcatcctactcgtcggacgagggctcttacgacccgctcagccccgaggagcaggagcttctcgacttcaccaactggttctga; NM_008553.

MyoD:NM_010866:atggagcttctatcgccgccactccgggacatagacttgacaggccccgacggctctctctgctcctttgagacagcagacgacttctatgatgacccgtgtttcgactcaccagacctgcgctttttgaggacctggacccgcgcctggtgcacatgggagccctcctgaaaccggaggagcacgcacacttccctactgcggtgcacccaggcccaggcgctcgtgaggatgagcatgtgcgcgcgcccagcgggcaccaccaggcgggtcgctgcttgctgtgggcctgcaaggcgtgcaagcgcaagaccaccaacgctgatcgccgcaaggccgccaccatgcgcgagcgccgccgcctgagcaaagtgaatgaggccttcgagacgctcaagcgctgcacgtccagcaacccgaaccagcggctacccaaggtggagatcctgcgcaacgccatccgctacatcgaaggtctgcaggctctgctgcgcgaccaggacgccgcgccccctggcgccgctgccttctacgcacctggaccgctgcccccaggccgtggcagcgagcactacagtggcgactcagatgcatccagcccgcgctccaactgctctgatggcatgatggattacagcggcccccccaagcggcccccggcggcagaatggctacgacaccgcctactacagtgaggcggcgcgcgagtccaggccagggaagagtgcggctgtgtcgagcctcgactgcctgtccagcatagtggagcgcatctccacagacagccccgctgcgcctgcgctgcttttggcagatgcaccaccagagtcgcctccgggtccgccagagggggcatccctaagcgacacagaacagggaacccagaccccgtctcccgacgccgcccctcagtgtcctgcaggctcaaaccccaatgcgatttatcaggtgctttga;

NM_002478:atggagctactgtcgccaccgctccgcgacgtagacctgacggcccccgacggctctctctgctcctttgccacaacggacgacttctatgacgacccgtgtttcgactccccggacctgcgcttcttcgaagacctggacccgcgcctgatgcacgtgggcgcgctcctgaaacccgaagagcactcgcacttcccgcggcggtgcacccggccccgggcgcacgtgaggacgagcatgtgcgcgcgcccagcgggcaccaccaggcgggccgctgcctactgtgggcctgcaaggcgtgcaagcgcaagaccaccaacgccgaccgccgcaaggccgccaccatgcgcgagcggcgccgcctgagcaaagtaaatgaggcctttgagacactcaagcgctgcacgtcgagcaatccaaaccagcggttgcccaaggtggagatcctgcgcaacgccatccgctatatcgagggcctgcaggctctgctgcgcgaccaggacgccgcgccccctggcgccgcagccgccttctatgcgccgggcccgctgcccccgggccgcggcggcgagcactacagcggcgactccgacgcgtccagcccgcgctccaactgctccgacggcatgatggactacagcggcccccgagcggcgcccggcggcggaactgctacgaaggcgcctactacaacgaggcgcccagcgaacccaggcccgggaagagtgcggcggtgtcgagcctagactgcctgtccagcatcgtggagcgcatctccaccgagagccctgcggcg

cccgcccicctgctggcggacgtgccttctgagtcgcctccgcgcaggcaagaggctgccgcccccagcgagggagagagcagcggcgaccccacccagtcaccggacgccgccccgcagtgccctgcgggtgcgaaccccaacccgatataccaggtgctctga.

**Myf5:**NM_005593:atggacgtgatggatggctgccagttctcaccttctgagtacttctacgacggctcctgcataccgtcccccgagggtgaatttggggacgagtttgtgccgcgagtggctgccttcggagcgcacaaagcagagctgcagggctcagatgaggacgagcacgtgcgagcgcctaccggccaccaccaggctggtcactgcctcatgtgggcctgcaaagcctgcaagaggaagtccaccaccatggatcggcggaaggcagccactatgcgcgagcggaggcgcctgaagaaggtcaaccaggctttcgaaaccctcaagaggtgtaccacgaccaaccccaaccagaggctgcccaaggtggagatcctcaggaatgccatccgctacatcgagagcctgcaggagttgctgagagagcaggtggagaactactatagcctgccgggacagagctgctcggagcccaccagccccacctccaactgctctgatggcatgcccgaatgtaacagtcctgtctggtccagaaagagcagtacttttgacagcatctactgtcctgatgtatcaaatgtatatgccacagataaaaactccttatccagcttggattgcttatccaacatagtggaccggatcacctcctcagagcaacctgggttgcctctccaggatctggcttctctctctccagttgccagcaccgattcacagcctgcaactccaggggcttctagttccaggcttatctatcatgtgctatga; NM_131576; NM_008656.

**Myf6:**NM_002469:atgatgatggaccttttgaaactggctcctatttcttctacttggatggggaaaatgttactctgcagccattagaagtggcagaaggctctcctttgtatccagggagtgatggtaccttgtcccccctgccaggaccaaatgcccccggaagcggggagcgacagcagcggagaggaacatgtcctggcgccccgggcctgcagcctccacactgccccggccagtgtctgatctgggcttgcaagacctgcaagagaaaatctgcccccactgaccggcgaaaagccgccaccctgcgcgaaaggaggaggctaaagaaaatcaacgaggccttcgaggcactgaagcggcgaactgtggccaaccccaaccagaggctgcccaaggtggagattctgcggagcgccatcagctatattgagcggctgcaggacctgctgcaccggctggatcagcaggagaagatgcaggagctgggggtggaccccttcagctacagacccaaacaagaaaatcttgagggtgcggatttcctgcgcacctgcagctcccagtggccaagtgtttccgatcattccagggggctcgtgataacggctaaggaaggaggagcaagtattgattcgtcagcctcgagtagccttcgatgcctttcttccatcgtggacagtatttcctcggaggaacgcaaactcccctgcgtggaggaagtggtggagaagtaa NM_008657; NM_008657; NM_013172.

**Ifrd1:**NM_001007245:atgccgaagaacaagaagcggaacactccccaccgcggtagcagtgctggcggcggcgggtcaggagcagccgcagcgacggcggcgacagcaggtggccagcatcgaaatgttcagccttttagtgatgaagatgcatcaattgaaacaatgagccattgcagtggttatagcgatccttccagttttgctgaagatggaccagaagtccttgatgaggaaggaactcaagaagacctagagtacaagttgaagggattaattgacctaaccctggataagagtgcgaagacaaggcaagcagctcttgaaggtattaaaaatgcactggcttcaaaaatgctgtatgaatttattctggaaaggagaatgactttaactgatagcattgaacgctgcctgaaaaaaggtaagagtgatgagcaacgtgcagctgcagcgttagcatctgttctttgtattcagctgggccctggaattgaaagtgaagagattttgaaaactcttggaccaatcctaaagaaaatcatttgtgatgggtcagctagtatgcaggctaggcaaacttgtgcaacttgctttggtgtttgctgttttattgccacagatgacattactgaactatactcaactctggaatgtttggaaaatatcttcactaaatcctatctcaaagagaaagacactactgttatttgcagcactcctaatacagtgcttcatatcagctctcttcttgcatggacactactgctgaccatatgcccaatcaatgaagtgaagaaaaagcttgagatgcatttccataagcttccaagcctcctctcttgtgatgatgtaaacatgagaatagctgctggtgaatctttggcacttctctttgaattggccagaggaatagagagtgactttttttatgaagacatggagtccttgacgcagatgcttagggccttggcaacagatggaaataaacaccgggccaaagtggacaagagaaagcagcggtcagttttcagagatgtcctgagggcagtggaggaacgggattttccaacagaaaccattaaatttggtcctgaacgcatgtatattgattgctgggtaaaaaaacacacctatgacacctttaaggaggttcttggatcagggatgcagtaccacttgcagtcaaatgaattccttcgaaatgtatttgaacttggacccccagtgatgcttgatgctgcaacgcttaaaacgatgaagatttctcgtttcgaaaggcatttatataactctgcagccttcaaagctcgaaccaaagctagaagcaaatgtcgagataagagagcagatgttggagaattcttctag.

Mef2A:NM_013172:atggggcggaagaaaatacaaatcacacgcataatggatgaaaggaaccgacaggtcactttacaaagagaaagtttggattaatgaagaaagcctatgaacttagtgtgctctgtgactgtgaaatagcactcatcattttcaacagctctaacaaactgtttcaatatgctagcactgatatggacaaagttcttctcaagtatacagaatataatgaacctcatgaaagcagaaccaactcggatattgttgaggctctgaacaagaaggaacacagagggtgcgacagcccagaccctgatacttcatatgtgctaactccacatacagaagaaaaatataaaaaaattaatgaggaatttgataatatgatgcggaatcataaaatcgcacctggtctgccacctcagaactttttcaatgtctgtcacagttccagtgaccagccccaatgctttgtcctacactaacccagggagttcactggtgtccccatctttggcagccagctcaacgttaacagattcaagcatgctctctccacctcaaaccacattacatagaaatgtgtctcctggagctcctcagagaccaccaagtactggcaatgcaggtgggatgtt

gagcactacagacctcacagtgccaaatggagctggaagcagtccagtggggaatggatttgtaaactcaagagcttctccaaatttgattggagctactggtgcaaatagcttaggcaaagtcatgcctacaaagtctccccctccaccaggtggtggtaatcttggaatgaacagtaggaaaccagatcttcgagttgtcatcccccccttcaagcaagggcatgatgcctccactatcggaggaagaggaattggagttgaacacccaaaggatcagtagttctcaagccactcaacctcttgctaccccagtcgtgtctgtgacaaccccaagcttgcctccgcaaggacttgtgtactcagcaatgccgactgcctacaacactgattattcactgaccagcgctgacctgtcagcccttcaaggcttcaactcgccaggaatgctgtcgctgggacaggtgtcggcctggcagcagcaccacctaggacaagcagccctcagctctcttgttgctggagggcagttatctcagggttccaatttatccattaataccaaccaaaacatcagcatcaagtccgaaccgatttcacctcctcgggatcgtatgaccccatcgggcttccagcagcagcagcagcagcagcagcagcagccgccgccaccaccgcagccccagccacaacccccgcagccccagccccgacaggaaatggggcgctcccctgtggacagtctgagcagctctagtagctcctatgatggcagtgatcgggaggatccacggggcgacttccattctccaattgtgcttggccgacccccaaacactgaggacagagaaagcccttctgtaaagcgaatgaggatggacgcgtgggtgacctaa.

Myogenin:NM_002479:Atggagctgtatgagacatcccccctacttctaccaggaaccccgcttctatgatggggaaaactacctgcctgtccacctccagggcttcgaaccaccaggctacgagcggacggagctcaccctgagccccgaggccccagggccccttgaggacaaggggctggggacccccgagcactgtccaggccagtgcctgccgtgggcgtgtaaggtgtgtaagaggaagtcggtgtccgtggaccggcggcgggcggccacactgagggagaagcgcaggctcaagaaggtgaatgaggccttcgaggccctgaagagaagcaccctgctcaaccccaaccagcggctgcccaaggtggagatcctgcgcagtgccatccagtacatcgagcgcctccaggccctgctcagctccctcaaccaggaggagcgtgacctccgctaccggggcgggggcgggccccagccaggggtgcccagcgaatgcagctctcacagcgcctcctgcagtccagagtggggcagtgcactggagttcagcgccaacccaggggatcatctgctcacggctgaccctacagatgcccacaacctgcactccctcacctccatcgtggacagcatcacagtggaagatgtgtctgtggccttcccagatgaaaccatgcccaactga; BC053899; BC068019; AB257560.

**Nkx2.2**:NM_002509:atgtcgctgaccaacacaaagacggggttttcggtcaaggacatcttagacctgccggacaccaacgatgaggagggctctgtggccgaaggtccggaggaagagaacgaggggcccgagccagccaagagggccgggccgctggggcagggcgccctggacgcggtgcagagcctgcccctgaagaaccccttctacgacagcagcgacaacccgtacacgcgctggctggccagcaccgagggccttcagtactccctgcacggtctggctgccggggcgccccctcaggactcaagctccaagtccccggagccctcggccgacgagtcaccggacaatgacaaggagaccccgggcggcgggggggacgccggcaagaagcgaaagcggcgagtgcttttctccaaggcgcagacctacgagctggagcggcgctttcggcagcagcggtacctgtcggcgcccgagcgcgaacacctggccagcctcatccgcctcacgcccacgcaggtcaagatctggttccagaaccaccgctacaagatgaagcgcgcccgggccgagaaaggtatggaggtgacgccccctgccctcgccgcgccgggtggccgtgcccgtcttggtcagggacggcaaaccatgtcacgcgctcaaagcccaggacctggcagccgccaccttccaggcgggcattcccttttctgcctacagcgcgcagtcgctgcagcacatgcagtacaacgcccagtacagctcggccagcacccccccagtacccgacagcacacccctggtccaggcccagcagtggacttggtga; NM_001077632; NM_010919.

**Notch**

Notch1:NM_017617:atgccgccgctcctggcgcccctgctctgcctggcgctgctgcccgcgctcgccgcacgaggcccgcgatgctcccagcccggtgagacctgcctgaatggcgggaagtgtgaagcggccaatggcacggaggcctgcgtctgtggcggggccttcgtgggcccgcgatgccaggaccccaacccgtgcctcagcacccccctgcaagaacgccgggacatgccacgtggtggaccgcagaggcgtggcagactatgcctgcagctgtgccctgggcttctctgggcccctctgcctgacacccctggacaatgcctgcctcaccaacccctgccgcaacgggggcacctgcgacctgctcacgctgacggagtacaagtgccgctgcccgcccggctggtcagggaaatcgtgccagcaggctgacccgtgcgcctccaacccctgcgccaacggtggccagtgcctgcccttcgaggcctcctacatctgccactgcccacccagcttccatggccccacctgccggcaggatgtcaacgagtgtggccagaagcccgggctttgccgccacggaggcacctgccacaacgaggtcggctcctaccgctgcgtctgccgcgccacccacactggccccaactgcgagcggccctacgtgccctgcagcccctcgccctgccagaacgggggcacctgccgccccacgggcgacgtcacccacgagtgtgcctgcctgccaggcttcaccggccagaactgtgaggaaaatatcgacgattgtccaggaaacaactgca

agaacgggggtgcctgtgtggacggcgtgaacacctacaactgccgctgcccgccagagtggacaggtcagtactgtaccgaggatgtggacgagtgccagc
tgatgccaaatgcctgccagaacggcgggacctgccacaacacccacggtggctacaactgcgtgtgtgtcaacggctggactggtgaggactgcagcgaga
acattgatgactgtgccagcgccgcctgcttccacggcgccacctgccatgaccgtgtggcctccttctactgcgagtgtccccatggccgcacaggtctgctgtg
ccacctcaacgacgcatgcatcagcaacccctgtaacgagggctccaactgcgacaccaaccctgtcaatggcaaggccatctgcacctgcccctcggggtac
acgggcccggcctgcagccaggacgtggatgagtgctcgctgggtgccaacccctgcgagcatgcgggcaagtgcatcaacacgctgggctccttcgagtgc
cagtgtctgcagggctacacgggcccccgatgcgagatcgacgtcaacgagtgcgtctcgaacccgtgccagaacgacgccacctgcctggaccagattggg
gagttccagtgcatctgcatgcccggctacgagggtgtgcactgcgaggtcaacacagacgagtgtgccagcagcccctgcctgcacaatggccgctgcctgg
acaagatcaatgagttccagtgcgagtgccccacgggcttcactgggcatctgtgccagtacgatgtggacgagtgtgccagcacccccctgcaagaatggtgcc
aagtgcctggacggacccaacacttacacctgtgtgtgcacggaagggtacacggggacgcactgcgaggtggacatcgatgagtgcgaccccgacccctgc
cactacggctcctgcaaggacggcgtcgccaccttcacctgcctctgccgcccaggctacacgggccaccactgcgagaccaacatcaacgagtgctccagcc
agccctgccgccacgggggcacctgccaggaccgcgacaacgcctacctctgcttctgcctgaaggggaccacaggacccaactgcgagatcaacctggatg
actgtgccagcagcccctgcgactcgggcacctgtctggacaagatcgatggctacgagtgtgcctgtgagccgggctacacagggagcatgtgtaacatcaac
atcgatgagtgtgcgggcaacccctgccacaacgggggcacctgcgaggacggcatcaatggcttcacctgccgctgccccgagggctaccacgaccccacc
tgcctgtctgaggtcaatgagtgcaacagcaacccctgcgtccacggggcctgccgggacagcctcaacgggtacaagtgcgactgtgaccctgggtggagtg
ggaccaactgtgacatcaacaacaatgagtgtgaatccaacccttgtgtcaacggcggcacctgcaaagacatgaccagtggctacgtgtgcacctgccgggag
ggcttcagcggtcccaactgccagaccaacatcaacgagtgtgcgtccaacccatgtctgaaccagggcacgtgtattgacgacgttgccgggtacaagtgcaa
ctgcctgctgccctacacaggtgccacgtgtgaggtggtgctggccccgtgtgcccccagcccctgcagaaacggcggggagtgcaggcaatccgaggacta
tgagagcttctcctgtgtctgccccacgggctggcaagggcagacctgtgaggtcgacatcaacgagtgcgttctgagcccgtgccggcacggcgcatcctgcc
agaacacccacggcggctaccgctgccactgccaggccggctacagtgggcgcaactgcgagaccgacatcgacgactgccggcccaacccgtgtcacaac
gggggctcctgcacagacggcatcaacacggccttctgcgactgcctgcccggcttccggggcactttctgtgaggaggacatcaacgagtgtgccagtgacc
cctgccgcaacggggccaactgcacggactgcgtggacagctacacgtgcacctgccccgcaggcttcagcgggatccactgtgagaacaacacgcctgact
gcacagagagctcctgcttcaacggtggcacctgcgtggacggcatcaactcgttcacctgcctgtgtccacccggcttcacgggcagctactgccagcacgat
gtcaatgagtgcgactcacagccctgcctgcatggcggcacctgtcaggacggctgcggctcctacaggtgcacctgcccccagggctacactggccccaact
gccagaaccttgtgcactggtgtgactcctcgccctgcaagaacggcggcaaatgctggcagacccacacccagtaccgctgcgagtgccccagcggctgga
ccggcctttactgcgacgtgcccagcgtgtcctgtgaggtggctgcgcagcgacaaggtgttgacgttgcccgcctgtgccagcatggagggctctgtgtggac
gcgggcaacacgcaccactgccgctgccaggcgggctacacaggcagctactgtgaggacctggtggacgagtgctcacccagcccctgccagaacgggg
ccacctgcacggactacctgggcggctactcctgcaagtgcgtggccggctaccacggggtgaactgctctgaggagatcgacgagtgcctctcccacccctg
ccagaacgggggcacctgcctcgacctccccaacacctacaagtgctcctgcccacggggcactcagggtgtgcactgtgagatcaacgtggacgactgcaat
ccccccgttgaccccgtgtcccggagccccaagtgctttaacaacggcacctgcgtggaccaggtgggcggctacagctgcacctgcccgccgggcttcgtgg
gtgagcgctgtgagggggatgtcaacgagtgcctgtccaatccctgcgacgcccgtggcacccagaactgcgtgcagcgcgtcaatgacttccactgcgagtg
ccgtgctggtcacaccgggcgccgctgcgagtccgtcatcaatggctgcaaaggcaagccctgcaagaatgggggcacctgcgccgtggcctccaacaccgc
ccgcgggttcatctgcaagtgccctgcgggcttcgagggcgccacgtgtgagaatgacgctcgtacctgcggcagcctgcgctgcctcaacggcggcacatgc
atctccggcccgcgcagccccacctgcctgtgcctgggccccttcacgggccccgaatgccagttcccggccagcagcccctgcctgggcggcaacccctgct
acaaccaggggacctgtgagcccacatccgagagccccttctaccgttgcctgtgccccgccaaattcaacgggctcttgtgccacatcctggactacagcttcg
gggtggggccgggcgcgacatccccccgccgctgatcgaggaggcgtgcgagctgcccgagtgccaggaggacgcgggcaacaaggtctgcagcctgc
agtgcaacaaccacgcgtgcggctgggacggcggtgactgctccctcaacttcaatgacccctggaagaactgcacgcagtctctgcagtgctggaagtacttc
agtgacggccactgtgacagccagtgcaactcagccggctgcctcttcgacggctttgactgccagcgtgcggaaggccagtgcaacccccctgtacgaccagt
actgcaaggaccacttcagcgacgggcactgcgaccagggctgcaacagcgcggagtgcgagtgggacgggctggactgtgcggagcatgtacccgagag
gctggcggccggcacgctggtggtggtggtgctgatgccgccggagcagctgcgcaacagctccttccacttcctgcgggagctcagccgcgtgctgcacacc

aacgtggtcttcaagcgtgacgcacacggccagcagatgatcttcccctactacggccgcgaggaggagctgcgcaagcaccccatcaagcgtgccgccgagggctgggccgcacctgacgccctgctgggccaggtgaaggcctcgctgctccctggtggcagcgagggtgggcggcggcggagggagctggaccccatggacgtccgcggctccatcgtctacctggagattgacaaccggcagtgtgtgcaggcctcctcgcagtgcttccagagtgccaccgacgtggccgcattcctgggagcgctcgcctcgctgggcagcctcaacatcccctacaagatcgaggccgtgcagagtgagaccgtggagccgcccccgccggcgcagctgcacttcatgtacgtggcggcggccgcctttgtgcttctgttcttcgtgggctgcggggtgctgctgtcccgcaagcgccggcggcagcatggccagctctggttccctgagggcttcaaagtgtctgaggccagcaagaagaagcggcgggagcccctcggcgaggactccgtgggcctcaagcccctgaagaacgcttcagacggtgccctcatggacgacaaccagaatgagtggggggacgaggacctggagaccaagaagttccggttcgaggagcccgtggttctgcctgacctggacgaccagacagaccaccggcagtggactcagcagcacctggatgccgctgacctgcgcatgtctgccatggcccccacaccgccccagggtgaggttgacgccgactgcatggacgtcaatgtccgcgggcctgatggcttcaccccgctcatgatcgcctcctgcagcgggggcggcctggagacgggcaacagcgaggaagaggaggacgcgccggccgtcatctccgacttcatctaccagggcgccagcctgcacaaccagacagaccgcacgggcgagaccgccttgcacctggccgcccgctactcacgctctgatgccgccaagcgcctgctggaggccagcgcagatgccaacatccaggacaacatgggccgcaccccgctgcatgcggctgtgtctgccgacgcacaaggtgtcttccagatcctgatccggaaccgagccacagacctggatgcccgcatgcatgatggcacgacgccactgatcctggctgcccgcctggccgtggagggcatgctggaggacctcatcaactcacacgccgacgtcaacgccgtagatgacctgggcaagtccgccctgcactgggccgccgccgtgaacaatgtggatgccgcagttgtgctcctgaagaacggggctaacaaagatatgcagaacaacagggaggagacacccctgtttctggccgcccgggagggcagctacgagaccgccaaggtgctgctggaccactttgccaaccgggacatcacggatcatatggaccgcctgccgcgcgacatcgcacaggagcgcatgcatcacgacatcgtgaggctgctggacgagtacaacctggtgcgcagcccgcagctgcacggagccccgctgggggggcacgcccaccctgtcgcccccgctctgctcgcccaacggctacctgggcagcctcaagcccggcgtgcagggcaagaaggtccgcaagcccagcagcaaaggcctggcctgtggaagcaaggaggccaaggacctcaaggcacggaggaagaagtcccaggacggcaagggctgcctgctggacagctccggcatgctctcgcccgtggactccctggagtcaccccatggctacctgtcagacgtggcctcgccgccactgctgccctccccgttccagcagtctccgtccgtgcccctcaaccacctgcctgggatgcccgacacccacctgggcatcgggcacctgaacgtggcggccaagcccgagatggcggcgctgggtgggggcggccggctggcctttgagactggcccacctcgtctctcccacctgcctgtggcctctggcaccagcaccgtcctgggctccagcagcggaggggccctgaatttcactgtgggcgggtccaccagtttgaatggtcaatgcgagtggctgtcccggctgcagagcggcatggtgccgaaccaatacaaccctctgcgggggagtgtggcaccaggccccctgagcacacaggccccctccctgcagcatggcatggtaggcccgctgcacagtagccttgctgccagcgccctgtcccagatgatgagctaccagggcctgcccagcacccggctggccacccagcctcacctggtgcagacccagcaggtgcagccacaaaacttacagatgcagcagcagaacctgcagccagcaaacatccagcagcagcaaagcctgcagccgccaccaccaccaccacagccgcaccttggcgtgagctcagcagccagcggccacctgggccggagcttcctgagtggagagccgagccaggcagacgtgcagccactgggccccagcagcctggcggtgcacactattctgccccaggagagccccgccctgcccacgtcgctgccatcctcgctggtcccacccgtgaccgcagcccagttcctgacgccccccctcgcagcacagctactcctcgcctgtggacaacacccccagccaccagctacaggtgcctgagcaccccttcctcaccccgtcccctgagtcccctgaccagtggtccagctcgtccccgcattccaacgtctccgactggtccgagggcgtctccagccctcccaccagcatgcagtcccagatcgcccgcattccggaggccttcaagtaa; AF159231.

NOTCH2:NM_024408; NM_010928.

NOTCH3:NM_000435:atggggccgggggcccgtggccgccgccgccgccgtcgcccgatgtcgccgccaccgccaccgccacccgtgcgggcgctgcccctgctgctgctgctagcggggccgggggctgcagcccccccttgcctggacggaagcccgtgtgcaaatggaggtcgttgcacccagctgccctcccgggaggctgcctgcctgtgcccgcctggctgggtgggtgagcggtgtcagctggaggacccctgtcactcaggcccctgtgctggccgtggtgtctgccagagttcagtggtggctggcaccgcccgattctcatgccggtgcccccgtggcttccgaggccctgactgctccctgccagatccctgcctcagcagcccttgtgcccacggtgcccgctgctcagtggggcccgatggacgcttcctctgctcctgcccacctggctaccagggccgcagctgccgaagcgacgtggatgagtgccgggtgggtgagccctgccgccatggtggcacctgcctcaacacacctggctccttccgctgccagtgtccagctggctacacagggccactatgtgagaaccccgcggtgccctgtgcaccctcaccatgccgtaacgggggcacctgcaggcagagtggcgacctcacttacgactgtgcctgtcttcctgggtttgagggtcagaattgtgaagtgaacgtggacgactgtccaggacaccgatgtctcaatgggggacatgcgtggatggcgtcaacacctataactgccagtgccctcctgagtggacaggccagttctgcacggaggacgtggatgagtgtcagctgcagcccaacgcctgccacaatgggggtacctgcttcaacacgctgggtggccacagctgcgtgtgtg

tcaatggctggacaggcgagagctgcagtcagaatatcgatgactgtgccacagccgtgtgcttccatggggccacctgccatgaccgcgtggcttctttctactg
tgcctgccccatgggcaagactggcctcctgtgtcacctggatgacgcctgtgtcagcaacccctgccacgaggatgctatctgtgacacaaatccggtgaacgg
ccgggccatttgcacctgtcctcccggcttcacgggtggggcatgtgaccaggatgtggacgagtgctctatcggcgccaacccctgcgagcacttgggcaggt
gcgtgaacacgcagggctccttcctgtgccagtgcggtcgtggctacactggacctcgctgtgagaccgatgtcaacgagtgtctgtcggggccctgccgaaac
caggccacgtgcctcgaccgcataggccagttcacctgtatctgtatggcaggcttcacaggaacctattgcgaggtggacattgacgagtgtcagagtagcccc
tgtgtcaacggtggggtctgcaaggaccgagtcaatggcttcagctgcacctgcccctcgggcttcagcggctccacgtgtcagctggacgtggacgaatgcgc
cagcacgccctgcaggaatggcgccaaatgcgtggaccagcccgatggctacgagtgccgctgtgccgagggctttgagggcacgctgtgtgatcgcaacgt
ggacgactgctcccctgacccatgccaccatggtcgctgcgtggatggcatcgccagcttctcatgtgcctgtgctcctggctacacgggcacacgctgcgaga
gccaggtggacgaatgccgcagccagccctgccgccatggcggcaaatgcctagacctggtggacaagtacctctgccgctgcccttctgggaccacaggtgt
gaactgcgaagtgaacattgacgactgtgccagcaacccctgcacctttggagtctgccgtgatggcatcaaccgctacgactgtgtctgccaacctggcttcaca
gggcccctttgtaacgtggagatcaatgagtgtgcttccagcccatgcggcgagggaggttcctgtgtggatggggaaaatggcttccgctgcctctgcccgcct
ggctccttgcccccactctgcctcccccgagccatccctgtgcccatgagccctgcagtcacggcatctgctatgatgcacctggcgggttccgctgtgtgtgtg
agcctggctggagtggcccccgctgcagccagagcctggcccgagacgcctgtgagtcccagccgtgcagggccggtgggacatgcagcagcgatggaat
gggtttccactgcacctgcccgcctggtgtccagggacgtcagtgtgaactcctctccccctgcaccccgaacccctgtgagcatgggggccgctgcgagtctg
cccctggccagctgcctgtctgctcctgcccccagggctggcaaggcccacgatgccagcaggatgtggacgagtgtgctggccccgcaccctgtggccctca
tggtatctgcaccaacctggcagggagtttcagctgcacctgccatggagggtacactggcccttcctgcgatcaggacatcaatgactgtgaccccaacccatg
cctgaacggtggctcgtgccaagacggcgtgggctccttttcctgctcctgcctccctggtttcgccggcccacgatgcgcccgcgatgtggatgagtgcctgag
caacccctgcggcccgggcacctgtaccgaccacgtggcctccttcacctgcacctgcccgccaggctacggaggcttccactgcgaacaggacctgcccgac
tgcagccccagctcctgcttcaatggcgggacctgtgtggacggcgtgaactcgttcagctgcctgtgccgtcccggctacacaggagcccactgccaacatga
ggcagacccctgcctctcgcggccctgcctacacgggggcgtctgcagcgccgcccaccctggcttccgctgcacctgcctcgagagcttcacgggcccgca
gtgccagacgctggtggattggtgcagccgccagccttgtcaaaacgggggtcgctgcgtccagactggggcctattgcctttgtccccctggatggagcggac
gcctctgtgacatccgaagcttgccctgcagggaggccgcagcccagatcggggtgcggctggagcagctgtgtcaggcgggtgggcagtgtgtggatgaag
acagctcccactactgcgtgtgcccagagggccgtactggtagccactgtgagcaggaggtggacccctgcttggcccagccctgccagcatgggggacct
gccgtggctatatgggggctacatgtgtgagtgtcttcctggctacaatggtgataactgtgaggacgacgtggacgagtgtgcctcccagccctgccagcacg
gggttcatgcattgacctcgtggcccgctatctctgctcctgtcccccaggaacgctgggggtgctctgcgagattaatgaggatgactgcggcccaggcccac
cgctggactcagggccccggtgcctacacaatggcacctgcgtggacctggtgggtggtttccgctgcacctgtcccccaggatacactggtttgcgctgcgag
gcagacatcaatgagtgtcgctcaggtgcctgccacgcggcacacacccgggactgcctgcaggacccaggcggaggtttccgttgcctttgtcatgctggcttc
tcaggtcctcgctgtcagactgtcctgtctccctgcgagtcccagccatgccagcatggaggccagtgccgtcctagcccgggtcctgggggtgggctgaccttc
acctgtcactgtgcccagccgttctggggtccgcgttgcgagcgggtggcgcgctcctgccgggagctgcagtgcccggtgggcgtcccatgccagcagacg
ccccgcgggccgcgctgcgcctgcccccagggttgtcgggaccctcctgccgcagcttcccggggtcgccgccgggggccagcaacgccagctgcgcgg
ccgcccccctgtctccacgggggctcctgccgccccgcgccgctcgcgcccttcttccgctgcgcttgcgcgcagggctggaccgggccgcgctgcgaggcgc
ccgccgcggcacccgaggtctcggaggagccgcggtgcccgcgcgccgcctgccaggccaagcgcggggaccagcgctgcgaccgcgagtgcaacagc
ccaggctgcggctgggacggcggcgactgctcgctgagcgtgggcgacccctggcggcaatgcgaggcgctgcagtgctggcgcctcttcaacaacagccg
ctgcgaccccgcctgcagctcgcccgcctgcctctacgacaacttcgactgccacgccggtggccgcgagcgcacttgcaacccggtgtacgagaagtactgc
gccgaccactttgccgacggccgctgcgaccagggctgcaacacggaggagtgcggctgggatgggctggattgtgccagcgaggtgccggccctgctggc
ccgcggcgtgctggtgctcacagtgctgctgccgccagaggagctactgcgttccagcgccgactttctgcagcggctcagcgccatcctgcgcacctcgctgc
gcttccgcctggacgcgcacggccaggccatggtcttcccttaccaccggcctagtcctggctccgaaccccgggcccgtcgggagctggcccccgaggtgat
cggctcggtagtaatgctggagattgacaaccggctctgcctgcagtcgcctgagaatgatcactgcttccccgatgcccagagcgccgctgactacctgggag
cgttgtcagcggtggagcgcctggacttcccgtacccactgcgggacgtgcgggggggagccgctggagcctccagaacccagcgtcccgctgctgccactgc

tagtggcgggcgctgtcttgctgctggtcattctcgtcctgggtgtcatggtggcccggcgcaagcgcgagcacagcaccctctggttccctgagggcttctcactgcacaaggacgtggcctctggtcacaagggccggcgggaacccgtgggccaggacgcgctgggcatgaagaacatggccaagggtgagagcctgatggggaggtggccacagactggatggacacagagtgcccagaggccaagcggctaaaggtagaggagccaggcatgggggctgaggaggctgtggattgccgtcagtggactcaacaccatctggttgctgctgacatccgcgtggcaccagccatggcactgacaccaccacagggcgacgcagatgctgatggcatggatgtcaatgtgcgtggcccagatggcttcaccccgctaatgctggcttccttctgtgggggggctctggagccaatgccaactgaagaggatgaggcagatgacacatcagctagcatcatctccgacctgatctgccagggggctcagcttggggcacggactgaccgtactggcgagactgctttgcacctggctgcccgttatgcccgtgctgatgcagccaagcggctgctggatgctggggcagacaccaatgcccaggaccactcaggccgcactcccctgcacacagctgtcacagccgatgcccagggtgtcttccagattctcatccgaaaccgctctacagacttggatgcccgcatggcagatggctcaacggcactgatcctggcggcccgcctggcagtagagggcatggtggaagagctcatcgccagccatgctgatgtcaatgctgtggatgagcttgggaaatcagccttacactgggctgcggctgtgaacaacgtggaagccactttggccctgctcaaaaatggagccaataaggacatgcaggatagcaaggaggagacccccctattcctggccgcccgcgagggcagctatgaggctgccaagctgctgttggaccactttgccaaccgtgagatcaccgaccacctggacaggctgccgcgggacgtagcccaggagagactgcaccaggacatcgtgcgcttgctggatcaacccagtgggccccgcagcccccccggtccccacggcctggggcctctgctctgtcctccaggggccttcctccctggcctcaaagcggcacagtcggggtccaagaagagcaggaggcccccgggaaggcggggctggggccgcaggggccccgggggcggggcaagaagctgacgctggcctgcccgggccccctggctgacagctcggtcacgctgtcgcccgtggactcgctggactccccgcggcctttcggtgggccccctgcttcccctggtggcttcccccttgaggggccctatgcagctgccactgccactgcagtgtctctggcacagcttggtggcccaggccgggcgggtctagggcgccagcccccctggaggatgtgtactcagcctgggcctgctgaaccctgtggctgtgcccctcgattgggcccggctgcccccacctgcccctccaggcccctcgttcctgctgccactggcgccgggaccccagctgctcaacccagggacccccgtctccccgcaggagcggcccccgccttacctggcagtcccaggacatggcgaggagtacccggcggctggggcacacagcagcccccccaaaggcccgcttcctgcgggttcccagtgagcacccttacctgaccccatcccccgaatcccctgagcactgggccagcccctcacctccctccctctcagactggtccgaatccacgcctagcccagccactgccactggggccatggccaccaccactggggcactgcctgcccagccacttcccttgtctgttcccagctcccttgctcaggcccagacccagctggggccccagccggaagttacccccaagaggcaagtgttggcctga.

**Nurr1:**NM_006186:atgccttgtgttcaggcgcagtatgggtcctcgcctcaaggagccagccccgcttctcagagctacagttaccactcttcgggagaatacagctccgatttcttaactccagagtttgtcaagtttagcatggacctcaccaacactgaaatcactgccaccacttctctccccagcttcagtacctttatggacaactacagcacaggctacgacgtcaagccaccttgcttgtaccaaatgcccctgtccggacagcagtcctccattaaggtagaagacattcagatgcacaactaccagcaacacagccacctgcccccccagtctgaggagatgatgccgcactccgggtcggtttactacaagccctcctcgcccccgacgcccaccaccccgggcttccaggtgcagcacagccccatgtgggacgacccgggatctctccacaacttccaccagaactacgtggccactacgcacatgatcgagcagaggaaaacgccagtctcccgcctctccctcttctcctttaagcaatcgcccccTggcaccccggtgtctagttgccagatgcgcttcgacgggcccctgcacgtccccatgaacccggagcccgccggcagccaccacgtggtggacgggcagaccttcgctgtgcccaaccccattcgcaagcccgcgtccatgggcttcccgggcctgcagatcggccacgcgtctcagctgctcgacacgcaggtgccctcaccgccgtcgcggggctcccccctccaacgagggctgtgcgctgtgtgtggggacaacgcggcctgccaacactacggcgtgcgcacctgtgagggctgcaaaggcttctttaagcgcacagtgcaaaaaaatgcaaaatacgtgtgtttagcaaataaaaactgcccagtggacaagcgtcgccggaatcgctgtcagtactgccgatttcagaagtgcctggctgttgggatggtcaaagaagtggttcgcacagacagtttaaaaggccggagaggtcgtttgccctcgaaaccgaagagcccacaggagccctctcccccttcgcccccggtgagtctgatcagtgccctcgtcagggcccatgtcgactccaacccggctatgaccagcctggactattccaggttccaggcgaaccctgactatcaaatgagtggagatgacacccagcatatccagcaattctatgatctcctgactggctccatggagatcatccggggctgggcagagaagatccctggcttcgcagacctgcccaaagccgaccaagacctgctttttgaatcagctttcttagaactgtttgtccttcgattagcatacaggtccaacccagtggagggtaaactcatcttttgcaatggggtggtcttgcacaggttgcaatgcgttcgtggctttggggaatggattgattccattgttgaattctcctccaacttgcagaatatgaacatcgacatttctgccttctcctgcattgctgccctggctatggtcacagagagacacgggctcaaggaacccaagagagtggaagaactgcaaaacaagattgtaaattgtctcaaagaccacgtgactttcaacaatgggggttgaaccgccccaattatttgtccaaactgttgggggaagctcccagaacttcgtaccctttgcacacaggggctacagcgcattttctacctgaaattggaagacttggtgccaccgccagcaataattgacaaacttttcctggacactttacctttctaa; NM_019328.

**NOV(CCN3)**:NM_002514:atgcagagtgtgcagagcacgagcttttgtctccgaaagcagtgcctttgcctgaccttcctgcttctccatctcctgggacaggtcgctgcgactcagcgctgccctccccagtgcccgggccggtgccctgcgacgccgccgacctgcgcccccggggtgcgcgcggtgctggacggctgctcatgctgtctggtgtgtgcccgccagcgtggcgagagctgctcagatctggagccatgcgacgagagcagtggcctctactgtgatcgcagcgcggaccccagcaaccagactggcatctgcacggcggtagagggagataactgtgtgttcgatggggtcatctaccgcagtggagagaaatttcagccaagctgcaaattccagtgcacctgcagagatgggcagattggctgtgtgccccgctgtcagctggatgtgctactgcctgagcctaactgcccagctccaagaaaagttgaggtgcctggagagtgctgtgaaaagtggatctgtggcccagatgaggaggattcactgggaggccttacccttgcagcttacaggccagaagccaccctaggagtagaagtctctgactcaagtgtcaactgcattgaacagaccacagagtggacagcatgctccaagagctgtggtatggggttctccacccgggtcaccaataggaaccgtcaatgtgagatgctgaaacagactcggctctgcatggtgcggccctgtgaacaagagccagagcagccaacagataagaaaggaaaaaagtgtctccgcaccaagaagtcactcaaagccatccacctgcagttcaagaactgcaccagcctgcacacctacaagcccaggttctgtggggtctgcagtgatggccgctgctgcactccccacaataccaaaaccatccaggcagagtttcagtgctccccagggcaaatagtcaagaagccagtgatggtcattgggacctgcacctgtcacaccaactgtcctaagaacaatgaggccttcctccaggagctggagctgaagactaccagagggaaaatgtaa; NM_010930; NM_030868; BC015028.

**OLIG1**:NM_138983:atgctgcggccacagcggcccggagacttgcagctcggggcctccctctacgagctggtgggctacaggcagccgccctcctcctcctcctcctccacctcctccacctcctccacttcctcctcctccacgacggcccccctcctccccaaggctgcgcgcgagaagccggaggcgccggccgagcctccaggccccgggcccgggtcaggcgcgcacccgggcggcagcgcccggccggacgccaaggaggagcagcagcagcagctgcggcgcaagatcaacagccgcgagcggaagcgcatgcaggacctgaacctggccatggacgccctgcgcgaggtcatcctgccctactcagcggcgcactgccagggcgcgcccggccgcaagctctccaagatagccacgctgctgctcgcccgcaactacatcctactgctgggcagctcgctgcaggagctgcgccgcgcgctgggcgagggcgccgggcccgccgcgccgcgcctgctgctggccgggctgcccctgctcgccgccgcgcccggctccgtgttgctggcgcccggcgccgtaggacccccgacgcgctgcgccccgccaagtacctgtcgctggcgctggacgagccgccgtgcggccagttcgctctccccggcggcggcgcaggcggccccggcctctgcacctgcgccgtgtgcaagttcccgcacctggtcccggccagcctgggcctggccgccgtgcaggcgcaattctccaagtga; NM_016968; NM_001020796.

**OLIG2**:NM_005806:atggactcggacgccagcctggtgtccagccgcccgtcgtcgccagagcccgatgacctttttctgccggcccggagtaagggcagcagcggcagcgccttcactgggggcaccgtgtcctcgtccaccccgagtgactgcccgccggagctgagcgccgagctgcgcggcgctatgggctctgcgggcgcgcatcctggggacaagctaggaggcagtggcttcaagtcatcctcgtccagcacctcgtcgtctacgtcgtcggcggctgcgtcgtccaccaagaaggacaagaagcaaatgacagagccggagctgcagcagctgcgtctcaagatcaacagccgcgagcgcaagcgcatgcacgacctcaacatcgccatggatggcctccgcgaggtcatgccgtacgcacacggcccttcggtgcgcaagctttccaagatcgccacgctgctgctggcgcgcaactacatcctcatgctcaccaactcgctggaggagatgaagcgactggtgagcgagatctacgggggccaccacgctggcttccacccgtcggcctgcggcggcctggcgcactccgcgcccctgcccgccgccaccgcgcacccggcagcagcagcgcacgccgcacatcaccccgcggtgcaccaccccatcctgccgcccgccgccgcagcggctgctgccgccgctgcagccgcggctgtgtccagcgcctctctgcccggatccgggctgccgtcggtcggctccatccgtccaccgcacggcctactcaagtctccgtctgctgccgcggccgccccgctgggggcgggggcggcggcagtggggcgagcgggggcttccagcactggggcggcatgccctgcccctgcagcatgtgccaggtgccgccgccgcaccaccacgtgtcggctatgggcgccggcagcctgccgcgcctcacctccgacgccaagtga.

**Pdx1**:NM_000209:atgaacggcgaggagcagtactacgcggccacgcagctttacaaggacccatgcgcgttccagcgaggcccggcgccggagttcagcgccagccccccctgcgtgcctgtacatgggccgccagcccccgccgccgccgccgcacccgttccctggcgccctgggcgcgctggagcagggcagcccccccggacatctccccgtacgaggtgccccccctcgccgacgaccccgcggtggcgcaccttcaccaccacctcccggctcagctcgcgctcccccacccgcccgccgggcccttcccggagggagccgagccgggcgtcctggaggagcccaaccgcgtccagctgcctttcccatggatgaagtctaccaaagctcacgcgtggaaaggccagtgggcaggcggcgcctacgctgcggagccggaggagaacaagcggacgcgcacggcctacacgcgcgcacagctgctagagctggagaaggagttcctattcaacaagtacatctcacggccgcgccgggtggagctggctgtcatgttgaacttgaccgagagacacatcaagatctggttccaaaaccgccgcatgaagtggaaaaaggaggaggacaagaagcgcggcggcgggacagctgtcgggggtggcggggtcgcggagcctgagcaggactgcgccgtga

cctccggcgaggagcttctggcgctgccgccgccgccgccccccggaggtgctgtgccgcccgctgcccccgttgccgcccgagagggccgcctgccgcct ggccttagcgcgtcgccacagccctccagcgtcgcgcctcggcggccgcaggaaccacgatga; NM_008814; NM_022852.

**Pet1(FEV):**BC138435;NM_017521:atgagacagagcggcgcctcccagcccctgctgatcaacatgtacctgccagatcccgtcggagacggt ctcttcaaggacgggaagaacccgagctgggggccgctgagccccgcggttcagaaaggcagcggacagatccagctgtggcagtttctgctggagctgctg gctgaccgcgcgaacgccggctgcatcgcgtgggagggcggtcacggcgagttcaagctcacggacccggacgaggtggcgcggcggtggggcgagcgc aagagcaagcccaacatgaactacgacaagctgagccgcgccctgcgctactactacgacaagaacatcatgagcaaggtgcatggcaagcgctacgcctac cgcttcgacttccagggcctggcgcaggcctgccagccgccgccccgcgcacgctcatgccgccgccgcagctgctgccgccgccgcggccgcccaggacg gcgcgctctacaagctgcccgccggcctcgccccgctgcccttccccggcctctccaaactcaacctcatggccgcctcggccggggtcgcgcccgccggctt ctcctactggccgggcccgggccccgccgccaccgctgccgccgccaccgccgcgctctaccccagtcccagcttgcagcccccgcccgggcccttcgggg ccgtggccgcagcctcgcacttgggggggccattaccactag;NG_002690; NP_059991.

**Phox2a:**NM_005169:atggactactcctacctcaattcgtacgactcgtgcgtggcggccatggaggcgtccgcctacggcgactttggcgcctgcagc cagcccggcggcttccaatacagcccccctgcggcccgctttccccgcggcagggccgccctgccccgcgctcggctcctccaactgcgcacttggcgccctac gcgaccaccagcccgcgccctactcggcagtgccctacaagttcttcccagagccatccggcctgcacgagaagcgcaagcagcggcgcatccgcaccacgt tcaccagcgcgcagctcaaggagctggagcgcgttttcgctgagacccactaccccgacatttacacgcgtgaggagctggcgctcaagatcgacctcactgag gctcgcgtgcaggtctggttccagaaccgccgggccaagttccgcaaacaggagcgcgcggccagcgccaagggcgcggcgggcgcggcgggcgccaa aaagggcgaggcgcgctgctcctccgaggacgacgattccaaggagtccacgtgcagccccacgcccgatagcaccgcctcgctgccgccgccgcctgcgc ccggcctggccagcccgcgcctgagccccagcccgctgcccgtcgcactgggctccgggccgggacctgggccggggccacagccgctcaagggcgcac tgtgggccggtgtggcgggcggtgggggcggcgggcctggcgcgggagcggccgaactacttaaggcttggcagccggcggagtccggcccccgggccctt ctccggggttctgtcctcctttcaccggaagcccggccccgccctgaagaccaatctcttctag; AJ320270; AY371497; AY371496.

**Phox2b:**NM_003924:atgtataaaatggaatattcttacctcaattcctctgcctacgagtcctgtatggctgggatggacacctcgagcctggcttcagcct atgctgacttcagttcctgcagccaggccagtggcttccagtataacccgataaggaccacttttggggccacgtccggctgcccttccctcacgccgggatcctg cagcctgggcaccctcagggaccaccagagcagtccgtacgccgcagttccttacaaactcttcacggaccacggcggcctcaacgagaagcgcaagcagcg gcgcatccgcaccactttcaccagtgcccagctcaaagagctggaaagggtcttcgcggagactcactaccccgacatctacactcgggaggagctggccctga agatcgacctcacagaggcgcgagtccaggtgtggttccagaaccgccgcgccaagtttcgcaagcaggagcgcgcagcggcagccgcagcggccgcggc caagaacggctcctcgggcaaaaagtctgactcttccagggacgacgagagcaaagaggccaagagcactgacccggacagcactgggggcccaggtccc aatcccaaccccacccccagctgcggggcgaatggaggcggcggcggcgggcccagcccggctggagctccgggggcggcggggcccgggggcccgg gaggcgaacccggcaagggcggcgcagcagcagcggcggcggccgcggcagcggcggcggcggcagcggcagcggcggcagctggaggcctggct gcggctgggggccctggacaaggctgggctcccggccccggccccatcacctccatcccggattcgcttggggggtcccttcgccagcgtcctatcttcgctcca aagacccaacggtgccaaagccgccttagtgaagagcagtatgttctga; NM_008888; AY371498; Y14493.

**Pit1:**NM_000306:atgagttgccaagcttttacttcggctgatacctttatacctctgaattctgacgcctctgcaactctgcctctgataatgcatcacagtgct gccgagtgtctaccagtctccaaccatgccaccaatgtgatgtctacagcaacaggacttcattattctgttccttcctgtcattatggaaaccagccatcaacctatg gagtgatggcaggtagtttaaccccttgtctttataaatttcctgaccacaccttgagtcatggatttcctcctatacaccagcctcttctggcagaggaccccacagct gctgatttcaagcaggaactcaggcggaaaagtaaattggtggaagagccaatagacatggattctccagaaatcagagaacttgaaaagtttgccaatgaattta aagtgagacgaattaaattaggatacacccagacaaatgttggggaggccctggcagctgtgcatggctctgaattcagtcaaacaacaatctgccgatttgaaaa tctgcagctcagctttaaaaatgcatgcaaactgaaagcaatattatccaaatggctggaggaagctgagcaagtaggagctttgtacaatgaaaaagtgggagca aatgaaaggaaaagaaaacgaagaacaactataagcattgctgctaaagatgctctggagagacactttggagaacagaataaaccttcttctcaagagatcatga ggatggctgaagaactgaatctggagaaagaagtagtaagagtttggttttgcaaccggaggcagagagaaaaacgggtgaaaacaagtctgaatcagagtttat

tttctatttctaaggaacatcttgagtgcagataa; M23253.

**PITX3**:NM_005029:atggagttcggcctgctcagcgaggcagaggcccggagccctgccctgtcgctgtcagacgctggcactccgcacccccagctcccagagcacggctgcaagggccaggagcacagcgactcagaaaaggcctcggcttcgctgcccggcggctccccagaggacggttcgctgaaaaagaagcagcggcggcagcgcacgcacttcaccagccagcagctacaggagctagaggcgaccttccagaggaaccgctaccccgacatgagcacgcgcgaggagatcgccgtgtggaccaacctcaccgaggcccgcgtgcgggtgtggttcaagaaccggcgcgccaaatggcggaagcgcgagcgcagccagcaggccgagctatgcaaaggcagcttcgcggcgccgctcgggggggctggtgccgccctacgaggaggtgtaccccggctactcgtacggcaactggccgcccaaggctcttgccccgccgctcgccgccaagacctttccattcgccttcaactcggtcaacgtggggcctctggcttcgcagcccgtcttctcgccacccagctccatcgccgcctccatggtgccctccgccgcggctgccccgggcaccgtgccagggcctggggccctgcagggcctgggcgggggcccccccgggctggctccggccgccgtgtcctccggggccgtgtcctgcccttatgcctcggccgccgccgccgccgcggctgccgcctcttccccctacgtctatcgggacccgtgtaactcgagcctggccagcctgcggctcaaagccaaacagcacgcctccttcagctaccccgctgtgcacgggccgcccccggcagccaaccttagtccgtgccagtacgccgtggaaaggcccgtatga; NM_008852; NM_008987;

**RUNX1:**NM_001001890:atgcgtatccccgtagatgccagcacgagccgccgcttcacgccgccttccaccgcgctgagcccaggcaagatgagcgaggcgttgccgctgggcgccccggacgccggcgctgccctggccggcaagctgaggagcggcgaccgcagcatggtggaggtgctggccgaccacccgggcgagctggtgcgcaccgacagccccaacttcctctgctccgtgctgcctacgcactggcgctgcaacaagaccctgcccatcgctttcaaggtggtggccctaggggatgttccagatggcactctggtcactgtgatggctggcaatgatgaaaactactcggctgagctgagaaatgctaccgcagccatgaagaaccaggttgcaagatttaatgacctcaggtttgtcggtcgaagtggaagagggaaaagcttcactctgaccatcactgtcttcacaaacccaccgcaagtcgccacctaccacagagccatcaaaatcacagtggatgggccccgagaacctcgaagacatcggcagaaactagatgatcagaccaagcccgggagcttgtccttttccgagcggctcagtgaactggagcagctgcggcgcacagccatgagggtcagcccacaccacccagcccccacgcccaaccctcgtgcctccctgaaccactccactgcctttaaccctcagcctcagagtcagatgcaggatacaaggcagatccaaccatccccaccgtggtcctacgatcagtcctaccaatacctgggatccattgcctctccttctgtgcacccagcaacgcccatttcacctggacgtgccagcggcatgacaaccctctctgcagaactttccagtcgactctcaacggcacccgacctgacagcgttcagcgacccgcgccagttccccgcgctgccctccatctccgacccccgcatgcactatccaggcgccttcacctactccccgacgccggtcacctcgggcatcggcatcggcatgtcggccatgggctcggccacgcgctaccacacctacctgccgccgccctaccccggctcgtcgcaagcgcagggaggcccgttccaagccagctcgccctcctaccacctgtactacggcgcctcggccggctcctaccagttctccatggtgggcggcgagcgctcgccgccgcgcatcctgccgccctgcaccaacgcctccaccggctccgcgctgctcaaccccagcctcccgaaccagagcgacgtggtggaggccgagggcagccacagcaactcccccaccaacatggcgccctccgcgcgcctggaggaggccgtgtggaggccctactga; AY509916 ;AY509915 ;NM_001001890.2; NP_001001890.1; NM_001122607.1; NP_001116079.1; NM_001754.4; NP_001745.2

**Runx2:**NM_001015051:atgcttcattcgcctcacaaacaaccacagaaccacaagtgcggtgcaaactttctccaggaggacagcaagaagtctctggtttttaaatggttaatctccgcaggtcactaccagccaccgagaccaacagagtcatttaaggctgcaagcagtatttacaacagagggtacaagttctatctgaaaaaaaaaggagggactatggcatcaaacagcctcttcagcacagtgacaccatgtcagcaaaacttcttttgggatccgagcaccagccggcgcttcagcccccctccagcagcctgcagcccggcaaaatgagcgacgtgagcccggtggtggctgcgcaacagcagcagcaacagcagcagcagcaacagcagcagcagcagcagcaacagcagcagcagcagcaggaggcggcggcggcggctgcggcggcggcggctgcggcggcggcagctgcagtgccccggttgcggccgccccacgacaaccgcaccatggtggagatcatcgccgaccacccggccgaactcgtccgcaccgacagccccaacttcctgtgctcggtgctgccctcgcactggcgctgcaacaagaccctgcccgtggccttcaaggtggtagccctcggagaggtaccagatgggactgtggttactgtcatggcgggtaacgatgaaaattattctgctgagctccggaatgcctctgctgttatgaaaaaccaagtagcaaggttcaacgatctgagatttgtgggccggagtggacgaggcaagagtttcaccttgaccataaccgtcttcacaaatcctccccaagtagctacctatcacagagcaattaaagttacagtagatggacctcgggaacccagaaggcacagacagaagcttgatgactctaaacctagtttgttctctgaccgcctcagtgatttagggcgcattcctcatcccagtatgagagtaggtgtcccgcctcagaacccacggccctccctgaactctgcaccaagtccttttaatccacaaggacagagtcagattacagaccccaggcaggcacagtcttccccgccgtggtcctatgaccagtcttacccctcctacctgagccagatgacgtccccgtccatccactctaccaccccgctgtcttccacacggggcactgggcttcctgccatcaccgatgtgcctaggcgcatttcaggtgct

tcagaactgggcccttttcagaccccaggcagttcccaagcatttcatccctcactgagagccgcttctccaacccacgaatgcactatccagccacctttacttacaccccgccagtcacctcaggcatgtccctcggtatgtccgccaccactcactaccacacctacctgccaccaccctaccccggctcttcccaaagccagagtggaccccttccagaccagcagcactccatatctctactatggcacttcgtcaggatcctatcagtttcccatggtgccgggggagaccggtctccttccagaatgcttccgccatgcaccaccacctcgaatggcagcacgctattaaatccaaatttgcctaaccagaatgatggtgttgacgctgatggaagccacagcagttccccaactgttttgaattctagtggcagaatggatgaatctgtttggcgaccatattga; NM_001015051.2; NP_001015051.2; NM_001015051; NM_001024630.2; NP_001019801.2; NM_004348.3; NP_004339.3

**Shh:**NM_000193:atgctgctgctggcgagatgtctgctgctagtcctcgtctcctcgctgctggtatgctcgggactggcgtgcggaccgggcaggggttcgggaagaggaggcaccccaaaaagctgacccctttagcctacaagcagtttatccccaatgtggccgagaagaccctaggcgccagcggaaggtatgaagggaagatctccagaaactccgagcgatttaaggaactcacccccaattacaaccccgacatcatatttaaggatgaagaaaacaccggagcggacaggctgatgactcagaggtgtaaggacaagttgaacgctttggccatctcggtgatgaaccagtggccaggagtgaaactgcgggtgaccgagggctgggacgaagatggccaccactcagaggagtctctgcactacgagggccgcgcagtggacatcaccacgtctgaccgcgaccgcagcaagtacggcatgctggcccgcctggcggtggaggccggcttcgactgggtgtactacgagtccaaggcacatatccactgctcggtgaaagcagagaactcggtggcggccaaatcgggaggctgcttcccgggctcggccacggtgcacctggagcagggcggcaccaagctggtgaaggacctgagccccggggaccgcgtgctggcggcggacgaccagggccggctgctctacagcgacttcctcactttcctggaccgcgacgacggcgccaagaaggtcttctacgtgatcgagacgcgggagccgcgcgagcgcctgctgctcaccgccgcgcacctgctctttgtggcgccgcacaacgactcggccaccggggagcccgaggcgtcctcgggctcggggccgccttccgggggcgcactggggcctcgggcgctgttcgccagccgcgtgcgcccgggccagcgcgtgtacgtggtggccgagcgtgacggggaccgccggctcctgcccgccgctgtgcacagcgtgaccctaagcgaggaggccgcgggcgcctacgcgccgctcacggcccagggcaccattctcatcaaccgggtgctggcctcgtgctacgcggtcatcgaggagcacagctgggcgcaccgggccttcgcgcccttccgcctggcgcacgcgctcctggctgcactggcgcccgcgcgcacggaccgcggcggggacagcggcggcggggaccgcgggggcggcggcggcagagtagccctaaccgctccaggtgctgccgacgctccgggtgcgggggccaccgcgggcatccactggtactcgcagctgctctaccaaataggcacctggctcctggacagcgaggccctgcacccgctgggcatggcggtcaagtccagctga; NP_000184; NM_009170; NP_033196; NM_204821; NP_990152.

**Sox9**:NM_000346:atgaatctcctggaccccttcatgaagatgaccgacgagcaggagaagggcctgtccggcgcccccagccccaccatgtccgaggactccgcgggctcgccctgcccgtcgggctccggctcggacaccgagaacacgcggccccaggagaacacgttccccaagggcgagcccgatctgaagaaggagagcgaggaggacaagttccccgtgtgcatccgcgaggcggtcagccaggtgctcaaaggctacgactggacgctggtgcccatgccggtgcgcgtcaacggctccagcaagaacaagccgcacgtcaagcggcccatgaacgccttcatggtgtgggcgcaggcggcgcgcaggaagctcgcggaccagtacccgcacttgcacaacgccgagctcagcaagacgctgggcaagctctggagacttctgaacgagagcgagaagcggcccttcgtggaggaggcggagcggctgcgcgtgcagcacaagaaggaccacccggattacaagtaccagccgcggcggaggaagtcggtgaagaacgggcaggcggaggcagaggaggccacggagcagacgcacatctcccccaacgccatcttcaaggcgctgcaggccgactcgccacactcctcctccggcatgagcgaggtgcactcccccggcgagcactcggggcaatcccagggcccaccgaccccacccaccacccccaaaaccgacgtgcagccgggcaaggctgacctgaagcgagaggggcgccccttgccagagggggcagacagcccccctatcgacttccgcgacgtggacatcggcgagctgagcagcgacgtcatctccaacatcgagaccttcgatgtcaacgagtttgaccagtacctgccgcccaacggccacccgggggtgccggccacgcacggccaggtcacctacacgggcagctacggcatcagcagcaccgcggccaccccggcgagcgcgggccacgtgtggatgtccaagcagcaggcgccgccgccacccccgcagcagcccccacaggccccgccggccccgcaggcgcccccgcagccgcaggcggcgcccccacagcagccggcggcacccccgcagcagccacaggcgcacacgctgaccacgctgagcagcgagccgggccagtcccagcgaacgcacatcaagacggagcagctgagccccagccactacagcgagcagcagcagcactcgccccaacagatcgcctacagccccttcaacctcccacactacagcccctcctacccgcccatcacccgctcacagtacgactacaccgaccaccagaactccagctcctactacagccacgcggcaggccagggcaccggcctctactccaccttcacctacatgaaccccgctcagcgccccatgtacacccccatcgccgacacctctggggtcccttccatcccgcagacccacagcccccagcactgggaacaacccgtctacacacagctcactcgaccttga; NM_000346; NP_000337; NM_011448; NP_035578; XM_343981;XP_343982.

Sox17:NM_022454:atgagcagcccggatgcgggatacgccagtgacgaccagagccagacccagagcgcgctgcccgcggtgatggccgggctgggcccctgcccctgggccgagtcgctgagccccatcggggacatgaaggtgaagggcgaggcgccggcgaacagcggagcaccggccggggccgcgggccgagccaagggcgagtcccgtatccggcggccgatgaacgctttcatggtgtgggctaaggacgagcgcaagcggctggcgcagcagaatccagacctgcacaacgccgagttgagcaagatgctgggcaagtcgtggaaggcgctgacgctggcggagaagcggcccttcgtggaggaggcagagcggctgcgcgtgcagcacatgcaggaccaccccaactacaagtaccggccgcggcggcgcaagcaggtgaagcggctgaagcgggtggagggcggcttcctgcacggcctggctgagccgcaggcggccgcgctgggccccgagggcggccgcgtggccatggacggcctgggcctccagttccccgagcagggcttccccgccggcccgccgctgctgcctccgcacatgggcggccactaccgcgactgccagagtctgggcgcgcctccgctcgacggctacccgttgcccacgcccgacacgtccccgctggacggcgtggaccccgacccggctttcttcgccgccccgatgcccggggactgcccggcggccggcacctacagctacgcgcaggtctcggactacgctggccccccggagcctcccgccggtcccatgcacccccgactcggcccagagcccgcgggtccctcgattccgggcctcctggcgccacccagcgcccttcacgtgtactacggcgcgatgggctcgcccggggcgggcggcgggcgcggcttccagatgcagccgcaacaccagcaccagcaccagcaccagcaccaccccccgggccccggacagccgtcgccccctccggaggcactgccctgccgggacggcacggaccccagtcagcccgccgagctcctcggggaggtggaccgcacggaatttgaacagtatctgcacttcgtgtgcaagcctgagatgggcctcccctaccaggggcatgactccggtgtgaatctccccgacagccacggggccatttcctcggtggtgtccgacgccagctccgcggtatattactgcaactatcctgacgtgtga; BC140307; NM_011441.

DLX2:NM_004405:atgactggagtctttgacagtctagtggctgatatgcactcgacccagatcgccgcctccagcacgtaccaccagcaccagcagcccccgagcggcggcggcgccggcccgggtggcaacagcagcagcagcagcagcctccacaagccccaggagtcgcccacccttccggtgtccaccgccaccgacagcagctactacaccaaccagcagcacccggcgggcggcggcggcggcgggggctcgccctacgcgcacatgggttcctaccagtaccaagccagcggcctcaacaacgtcccttactccgccaagagcagctatgacctgggctacaccgccgcctacacctcctacgctccctatggaaccagttcgtccccagccaacaacgagcctgagaaggaggaccttgagcctgaaattcggatagtgaacgggaagccaaagaaagtccggaaaccccgcaccatctactccagtttccagctggcggctcttcagcggcgtttccaaaagactcaatacttggccttgccggagcgagccgagctggcggcctctctgggcctcacccagactcaggtcaaaatctggttccagaaccgccggtccaagttcaagaagatgtggaaaagtggtgagatcccctcggagcagcaccctggggccagcgcttctccaccttgtgcttcgccgccagtctcagcgccggcctcctgggactttggtgtgccgcagcggatggcgggcggcggtggtccgggcagtggcggcagcggcgccggcagctcgggctccagcccgagcagcgcggcctcggcttttctgggcaactacccctggtaccaccagacctcgggatccgcctcacacctgcaggccacggcgccgctgctgcacccactcagaccccgcagccgcatcaccaccaccaccatcacggcggcgggggcgccccggtgagcgcggggacgattttctaa; NP_004396.1; NM_010054.

DLX5:NM_005221:atgacaggagtgtttgacagaagggtccccagcatccgatccggcgacttccaagctccgttccagacgtccgcagctatgcaccatccgtctcaggaatcgccaactttgcccgagtcttcagctaccgattctgactactacagccctacgggggagccccgcacggctactgctctcctacctcggcttcctatggcaaagctctcaacccctaccagtatcagtatcacggcgtgaacggctccgccgggagctacccagccaaagcttatgccgactatagctacgctagctcctaccaccagtacggcggcgcctacaaccgcgtcccaagcgccaccaaccagccagagaaagaagtgaccgagcccgaggtgagaatggtgaatggcaaaccaaagaaagttcgtaaacccaggactatttattccagctttcagctggccgcattacagagaaggtttcagaagactcagtacctcgccttgccggaacgcgccgagctggccgcctcgctgggattgacacaaacacaggtgaaaatctggtttcagaacaaaagatccaagatcaagaagatcatgaaaaacggggagatgccccggagcacagtcccagctccagcgacccaatggcgtgtaactcgccgcagtctccagcggtgtgggagccccagggctcgtcccgctcgctcagccaccaccctcatgcccaccctccgacctccaaccagtccccagcgtccagctacctggagaactctgcatcctggtacacaagtgcagccagctcaatcaattcccacctgccgccgccgggctccttacagcacccgctggcgctggcctccgggacactctattag; NM_005221; NP_005212.

HES1:NM_005524:atgccagctgatataatggagaaaaattcctcgtccccggtggctgctaccccagccagtgtcaacacgacaccggataaaccaaagacagcatctgagcacagaaagtcatcaaagcctattatggagaaaagacgaagagcaagaataaatgaaagtctgagccagctgaaaacactgattttggatgctctgaagaaagatagctcgcggcattccaagctggagaaggcggacattctggaaatgacagtgaagcacctccggaacctgcagcgggcgcagatgacggc

tgcgctgagcacagacccaagtgtgctggggaagtaccgagccggcttcagcgagtgcatgaacgaggtgacccgcttcctgtccacgtgcgagggcgttaataccgaggtgcgcactcggctgctcggccacctggccaactgcatgacccagatcaatgccatgacctaccccgggcagccgcaccccgccttgcaggcgccgccaccgcccccaccgggacccggcggcccccagcacgcgccgttcgcgccgccgccgccactcgtgcccatccccgggggcgcggcgccccctcccggcggcgccccctgcaagctgggcagccaggctggagaggcggctaaggtgtttggaggcttccaggtggtaccggctcccgatggccagtttgctttcctcattcccaacggggccttcgcgcacagcggccctgtcatccccgtctacaccagcaacagcggcacctccgtgggccccaacgcagtgtcaccttccagcggcccctcgcttacggcggactccatgtggaggccgtggcggaactga; NP_005515.1; NM_008235; NP_032261.

FGF8:NM_006119:atgggcagccccgctccgcgctgagctgcctgctgttgcacttgctggtcctctgcctccaagcccaggtaactgttcagtcctcacctaatttacacagcatgtgagggagcagagcctggtgacggatcagctcagccgccgcctcatccggacctaccaactctacagccgcaccagcgggaagcacgtgcaggtcctggccaacaagcgcatcaacgccatggcagaggacggcgaccccttcgcaaagctcatcgtggagacggacacctttggaagcagagttcgagtccgaggagccgagacgggcctctacatctgcatgaacaagaaggggaagctgatcgccaagagcaacggcaaaggcaaggactgcgtcttcacggagattgtgctggagaacaactacacagcgctgcagaatgccaagtacgagggctggtacatggccttcacccgcaagggccggccccgcaagggctccaagacgcggcagcaccagcgtgaggtccacttcatgaagcggctgccccggggccaccacaccaccgagcagagcctgcgcttcgagttcctcaactacccgcccttcacgcgcagcctgcgcggcagccagaggacttgggcccccgagccccgatag; NM_010205; NP_034335; NM_010205; NP_034335; NP_006110

NM_033163; NP_149353; NM_033164; NP_149354; NM_033165; NP_149355.

PITX2:NM_000325:atgaactgcatgaaaggcccgcttcacttggagcaccgagcagcggggaccaagctgtcggccgtctcctcatcttcctgtcaccatccccagccgttagccatggcttcggttctggctcccggtcagccccggtcgctggactcctccaagcacaggctggaggtgcacaccatctccgacacctccagcccggaggccgcagagaaagataaaagccagcaggggaagaatgaggacgtgggcgccgaggacccgtctaagaagaagcggcaaaggcggcagcggactcactttaccagccagcagctccaggagctggaggccactttccagaggaaccgctacccggacatgtccacacgcgaagaaatcgctgtgtggaccaaccttacggaagcccgagtccgggtttggttcaagaatcgtcgggccaaatggagaaagagggagcgcaaccagcaggccgagctatgcaagaatggcttcgggccgcagttcaatgggctcatgcagccctacgacgacatgtacccaggctattcctacaacaactgggccgccaagggccttacatccgcctccctatccaccaagagcttccccttcttcaactctatgaacgtcaacccccctgtcatcacagagcatgttttccccacccaactctatctcgtccatgagcatgtcgtccagcatggtgccctcagcagtgacaggcgtcccgggctccagtctcaacagcctgaataacttgaacaacctgagtagcccgtcgctgaattccgcggtgccgacgcctgcctgtccttacgcgccgccgactcctccgtatgtttatagggacacgtgtaactcgagcctggccagcctgagactgaaagcaaagcagcactccagcttcggctacgccagcgtgcagaacccggcctccaacctgagtgcttgccagtatgcagtggaccggcccgtgtga; NM_000325; NP_000316; NM_153426; NP_700475; NM_153427; NP_700476; NM_001042502; NP_001035967; NM_001042504; NP_001035969.

REST4:DQ644039:atggccacccaggtgatggggcagtcttctggaggaggcagtctcttcaacaacagtgccaacatgggcatggccttaaccaacgacatgtacgacctgcacgagctctcgaaagctgaactggcagcccctcagctcatcatgttagccaacgtggccctgacgggggaggcaagcggcagctgctgcgattacctggtcggtgaagagaggcagatggccgaattgatgcccgtgggagacaaccacttctcagaaagtgaaggagaaggcctggaagagtcggctgacctcaaagggctggaaaacatggaactgggaagtttggagctaagtgctgtagaaccccagcccgtatttgaagcctcagctgccccagaaatatacagcgccaataaagatcccgctccagaaacacccgtggcggaagacaaatgcaggagttctaaggccaagcccttccggtgtaagccttgccagtacgaagccgaatctgaagagcagtttgtgcatcacatccggattcacagcgctaagaagttctttgtggaggaaagtgcagagaaacaggccaaagcctgggagtcggggtcgtctccggccgaagagggcgagttctccaaaggccccatccgctgtgaccgctgtggctacaataccaaccggtatgaccactacatggcacacctgaagcaccacctgcgagctggcgagaacgagcgcatctacaagtgcatcatctgcacgtacacgacggtcagcgagtaccactggaggaaacacctgagaaaccatttccccaggaaagtctacacctgcagcaagtgcaactacttctcagacagaaaaaataactacgttcagcacgtgcgaactcacacaggagaacgcccgtataaatgtgaactttgtccttactcaagctctcagaagactcatctaacgcgacacatgcggactcattcagagtgtgatctagctgggtga.

CREB_binding_protein:NM_134442:atgaccatggaatctggagccgagaaccagcagagtggagatgcagctgtaacagaagctgaaaaccaacaaatgacagttcaagcccagccacagattgccacattagcccaggtatctatgccagcagctcatgcaacatcatctgctcccaccgtaactctagtacagctgcccaatgggcagacagttcaagtccatggagtcattcaggcggcccagccatcagttattcagtctccacaagtccaaacagttcagtcttcctgtaaggacttaaaaagacttttctccggaacacagatttcaactattgcagaaagtgaagattcacaggagtcagtggatagtgtaactgattcccaaaagcgaagggaaattctttcaaggaggccttcctacaggaaaattttgaatgacttatcttctgatgcaccaggagtgccaaggattgaagaagagaagtctgaagaggagacttcagcacctgccatcaccactgtaacggtgccaactccaatttaccaaactagcagtggacagtatattgccattacccagggaggagcaatacagctggctaacaatggtaccgatggggtacagggcctgcaaacattaaccatgaccaatgcagcagccactcagccgggtactaccattctacagtatgcacagaccactgatggacagcagatcttagtgcccagcaaccaagttgttgttcaagctgcctctggagacgtacaaacataccagattcgcacagcacccactagcactattgcccctggagttgttatggcatcctccccagcacttcctacacagcctgctgaagaagcagcacgaaagagagaggtccgtctaatgaagaacagggaagcagctcgagagtgtcgtagaaagaagaaagaatatgtgaaatgtttagaaaacagagtggcagtgcttgaaaatcaaaacaagacattgattgaggagctaaaagcacttaaggacctttactgccacaaatcagatta;NM_004379; NP_004370; NP_604391.

Zfp488:NM_001013777:
atggctgagggcaaaggggctcctctgaggccttcagttgagaagagatggaagctcatggaacccaagcagacccaggcagggatgttcaagaaaatgagccttgtggactctgacactgctgcaggaaagggtagccaagatgaggcctatactgaactgagcctgccaacagcaccgaacaagcctcgactggacaggcctcgggcctgcaaggcatacacagagcagaggcacaataccttcacagagctatcatgtctccaggagaggccaggggacatccaggcccagacgaggaagctggagaacccagaaggccagctcggccctcagcagctgccctcgagtttcctcagagcctcaggtgatggcacagtgtgttcagcatggccaggtgcccccggagtgagcagaaaagtgctttcagcaagccagccaaacgcccagcagagaaacctaagcgctctcccatgcttctggctggtggaagtgcagagggctcatgggagctctcaggactcatcaccactgtggacatcccatattgggctcatctgtcaactttcaagttcatgggtgatttctggaaattgcacacattgtcacagaacattctcctctgcaatgctttccaggggctcccacaccatggctggagcatacccaggtacaagcccccacatcctcagctccttcctccacagcctcccgggctctcttgccgcccacactctcctccttgggcttgtctactcagaactggtgtgcgaagtgcaacctagcctttcgcctgacagctgacctggtcttccacatgcggtcacatcacaaagggaacacgtgggccctgacccacattctaagaaacgaagagaggaagttctcacttgccccgtttgccacgagtacttccgggagcgccaccatctgtccaggcatatggcttcacatagttag; BC089025; XM_224697; XP_224697.

Foxa2:NM_021784:atgctgggagcggtgaagatggaagggcacgagccgtccgactggagcagctactatgcagagcccgagggctactcctccgtgagcaacatgaacgccggcctggggatgaacggcatgaacacgtacatgagcatgtcggcggccgccatgggcagcggctcgggcaacatgagcgcgggctccatgaacatgtcgtcgtacgtgggcgctggcatgagcccgtccctggcggggatgtcccccggcgcgggcgccatggcgggcatgggcggctcggccggggcggccggcgtggcgggcatggggccgcacttgagtcccagcctgagcccgctcgggggggcaggcggccggggccatgggcggcctggcccccctacgccaacatgaactccatgagccccatgtacgggcaggcgggcctgagccgcgcccgcgaccccaagacctacaggcgcagctacacgcacgcaaagccgcccctactcgtacatctcgctcatcaccatggccatccagcagagccccaacaagatgctgacgctgagcgagatctaccagtggatcatggacctcttcccctctaccggcagaaccagcagcgctggcagaactccatccgccactcgctctccttcaacgactgtttcctgaaggtgccccgctcgcccgacaagcccggcaagggctcctctggaccctgcaccctgactcgggcaacatgttcgagaacggctgctacctgcgccgccagaagcgcttcaagtgcgagaagcagctggcgctgaaggaggccgcaggcgccgccggcagcggcaagaaggcggccgccggagcccaggcctcacaggctcaactcggggaggccgccgggccggcctccgagactccggcgggcaccgagtcgcctcactcgagcgcctccccgtgccaggagcacaagcgagggggcctgggagagctgaaggggacgccggctgcggcgctgagccccccagagccggcgccctctcccgggcagcagcagcaggccgcggcccacctgctgggcccgccccaccacccgggcctgccgcctgaggcccacctgaagccggaacaccactacgccttcaaccacccgttctccatcaacaacctcatgtcctcggagcagcagcaccaccacagccaccaccaccaccaaccccacaaaatggacctcaaggcctacgaacaggtgatgcactaccccggctacggttcccccatgcctggcagcttggccatgggcccggtcacgaacaaaacgggcctggacgcctcgcccctggccgcagatacctcctactaccaggggggtgtactcccggcccattatgaactcctcttaa; NP_068556; NM_012743; NP_036875; NM_010446; NP_034576.

Rnx

REN:NM_000537:atggatggatggagaaggatgcctcgctggggactgctgctgctgctctggggctcctgtacctttggtctcccgacagacaccaccacctttaaacggatcttcctcaagagaatgccctcaatccgagaaagcctgaaggaacgaggtgtggacatggccaggcttggtcccgagtggagccaacccatgaagaggctgacacttggcaacaccacctcctccgtgatcctcaccaactacatggacacccagtactatggcgagattgggatcgggaccccaccccaaaccttcaaagtcgtctttgacactggttcgtccaatgtttgggtgccctcctccaagtgcagccgtctctacactgcctgtgtgtatcacaagctcttcgatgcttcggattcctccagctacaagcacaatggaacagaactcaccctccgctattcaacagggacagtcagtggctttctcagccaggacatcatcaccgtgggtggaatcacggtgacacagatgtttggagaggtcacggagatgcccgccttacccttcatgctggccgagtttgatggggttgtgggcatgggcttcattgaacaggccattggcagggtcacccctatcttcgacaacatcatctcccaaggggtgctaaaagaggacgtcttctctttctactacaacagagattccgagaattcccaatcgctgggaggacagatt

gtgctgggaggcagcgacccccagcattacgaagggaatttccactatatcaacctcatcaagactggtgtctggcagattcaaatgaagggggtgtctgtggggtcatccaccttgctctgtgaagacggctgcctggcattggtagacaccggtgcatcctacatctcaggttctaccagctccatagagaagctcatggaggccttgggagccaagaagaggctgtttgattatgtcgtgaagtgtaacgagggccctacactccccgacatctctttccacctgggaggcaaagaatacacgctcaccagcgcggactatgtatttcaggaatcctacagtagtaaaaagctgtgcacactggccatccacgccatggatatcccgccacccactggacccacctgggccctgggggccaccttcatccgaaagttctacacagagtttgatcggcgtaacaaccgcattggcttcgccttggcccgctga;

dHAND(HAND2):NM_021973:atgagtctggtaggtggttttccccaccacccggtggtgcaccacgagggctacccgtttgccgccgccgccgccgcagctgccgccgccgccgccagccgctgcagccatgaggagaacccctacttccatggctggctcatcggccaccccgagatgtcgccccccgactacagcatggccctgtcctacagccccgagtatgccagcggcgccgccggcctggaccactcccattacgggggggtgccgccgggcgccgggcccccgggcctggggggggccgcgcccggtgaagcgccgaggcaccgccaaccgcaaggagcggcgcaggactcagagcatcaacagcgccttcgccgaactgcgcgagtgcatccccaacgtacccgccgacaccaaactctccaaaatcaagaccctgcgcctggccaccagctacatcgcctacctcatggacctgctggccaaggacgaccagaatggcgaggcggaggccttcaaggcagagatcaagaagaccgacgtgaaagaggagaagaggaagaaggagctgaacgaaatcttgaaaagcacagtgagcagcaacgacaagaaaaccaaaggccggacgggctggccgcagcacgtctgggccctggagctcaagcagtga ; NM_010402;

aspartoacylase (Canavan disease) (ASPA):NM_000049; atgacttcttgtcacattgctgaagaacatatacaaaaggttgctatctttggaggaacccatgggaatgagctaaccggagtatttctggttaagcattggctagagaatggcgctgagattcagagaacagggctggaggtaaaaccatttattactaaccccagagcagtgaagaagtgtaccagatatattgactgtgacctgaatcgcattttgaccttgaaaatcttggcaaaaaaatgtcagaagatttgccatatgaagtgagaagggctcaagaaataaatcatttatttggtccaaaagacagtgaagattcctatgacattatttttgaccttcacaacaccacctctaacatggggtgcactcttattcttgaggattccaggaataactttttaattcagatgtttcattacattaagacttctctggctccactaccctgctacgtttatctgattgagcatccttccctcaaatatgcgaccactcgttccatagccaagtatcctgtgggtatagaagttggtcctcagcctcaaggggttctgagagctgatatcttggatcaaatgagaaaaatgattaaacatgctcttgattttatacatcatttcaatgaaggaaaagaatttcctccctgcgccattgaggtctataaaattatagagaaagttgattacccccgggatgaaaatggagaaattgctgctatcatccatcctaatctgcaggatcaagactggaaaccactgcatcctggggatcccatgtttttaactcttgatgggaagacgatcccactgggcggagactgtaccgtgtaccccgtgtttgtgaatgaggccgcatattacgaaaagaaagaagcttttgcaaagacaactaaactaacgctcaatgcaaaaagtattcgctgctgtttacattag; NM_023113.

hexosaminidaseA(HEXA):NM_000520:atgacaagctccaggctttggtttcgctgctgctggcggcagcgttcgcaggacgggcgacggccctctggccctggcctcagaacttccaaacctccgaccagcgctacgtcctttacccgaacaactttcaattccagtacgatgtcagctcggccgcgcagcccggctgctcagtcctcgacgaggccttccagcgctatcgtgacctgcttttcggttccgggtcttggccccgtccttacctcacagggaaacggcatacactggagaagaatgtgttggttgtctctgtagtcacacctggatgtaaccagcttcctactttggagtcagtggagaattataccctgaccataaatgatgaccagtgtttactcctctctgagactgtctggggagctctccgaggtctggagacttttagccagcttgtttggaaatctgctgagggcacattctttatcaacaagactgagattgaggactttccccgctttcctcaccggggcttgctgttggatacatctcgccattacctgccactctctagcatcctggacactctggatgtcatggcgtacaataaattgaacgtgttccactggcatctggtagatgatccttccttcccatatgagagcttcacttttccagagctcatgagaaaggggtcctacaaccctgtcacccacatctacacagcacaggatgtgaaggaggtcattgaatacgcacggctccggggtatccgtgtgcttgcagagtttgacactcctggccacactttgtcctggggaccaggtatccctggattactgactccttgctactctgggtctgagccctctggcacctttggaccagtgaatcccagtctcaataatacctatgagttcatgagcacattcttcttagaagtcagctctgtcttcccagattttatcttcatcttggaggagatgaggttgatttcacctgctggaagtccaacccagagatccaggactttatgaggaagaaaggcttcggtgaggacttcaagcagctggagtccttctacatccagacgctgctggacatcgtctcttcttatggcaagggctatgtggtgtggcaggaggtgtttgataataaagtaaagattcagccagacacaatcatacaggtgtggcgagaggatattccagtgaactatatgaaggagctggaactggtcaccaaggccggcttccgggcccttctctctgccccctggtacctgaaccgtatatcctatggccctgactggaaggatttctacgtagtggaacccctggcatttgaaggtacccctgagcagaaggctctggtgattggtggagaggcttgtatgtggggagaatatgtggacaacacaaacctggtccccaggctctggcccagagcaggggctgttgccgaaaggctgtggagcaacaagttgacatctgacctgacatttgcctatgaacgtttgtcacacttccgctgtgagttgctgaggcgaggtgtccaggcccaacccctcaatgtaggcttctgtgagcagg

agtttgaacagacctga.

Lesch_Nyhan_syndrome(HRPT):NM_000194:atggcgacccgcagccctggcgtcgtgattagtgatgatgaaccaggttatgaccttgatttattttgcatacctaatcattatgctgaggatttggaaagggtgtttattcctcatggactaattatggacaggactgaacgtcttgctcgagatgtgatgaaggagatgggaggccatcacattgtagccctctgtgtgctcaagggggctataaattctttgctgacctgctggattacatcaaagcactgaatagaaatagtgatagatccattcctatgactgtagattttatcagactgaagagctattgtaatgaccagtcaacaggggacataaaagtaattggtggagatgatctctcaactttaactggaaagaatgtcttgattgtggaagatataattgacactggcaaaacaatgcagactttgctttccttggtcaggcagtataatccaaagatggtcaaggtcgcaagcttgctggtgaaaaggaccccacgaagtgttggatataagccagactttgttggatttgaaattccagacaagtttgttgtaggatatgcccttgactataatgaatacttcagggatttgaatcatgtttgtgtcattagtgaaactggaaaagcaaaatacaaagcctaa; NM_204848.

Huntingtin; NM_010414;

GUSB;NM_000181:atggcccgggggtcggcggttgcctgggcggcgctcgggccgttgttgtggggctgcgcgctggggctgcagggcgggatgctgtacccccaggagagcccgtcgcgggagtgcaaggagctggacggcctctggagcttccgcgccgacttctctgacaaccgacgccggggcttcgaggagcagtggtaccggcggccgctgtgggagtcaggccccaccgtggacatgccagttccctccagcttcaatgacatcagccaggactggcgtctgcggcattttgtcggctgggtgtggtacgaacgggaggtgatcctgccggagcgatggacccaggacctgcgcacaagagtggtgctgaggattggcagtgcccattcctatgccatcgtgtgggtgaatggggtcgacacgctagagcatgagggggggctacctcccccttcgaggccgacatcagcaacctggtccaggtggggcccctgccctcccggctccgaatcactatcgccatcaacaacacactcacccccaccaccctgccaccagggaccatccaatacctgactgacacctccaagtatcccaagggttactttgtccagaacacatattttgacttttcaactacgctggactgcagcggtctgtacttctgtacacgacacccaccacctacatcgatgacatcaccgtcaccaccagcgtggagcaagacagtgggctggtgaattaccagatctctgtcaagggcagtaacctgttcaagttggaagtgcgtcttttggatgcagaaaacaaagtcgtggcgaatgggactgggacccagggccaacttaaggtgccaggtgtcagcctctggtggccgtacctgatgcacgaacgccctgcctatctgtattcattggaggtgcagctgactgcacagacgtcactggggcctgtgtctgacttctacacactccctgtggggatccgcactgtggctgtcaccaagagccagttcctcatcaatgggaaacctttctatttccacggtgtcaacaagcatgaggatgcggacatccgagggaagggcttcgactggccgctgctggtgaaggacttcaacctgcttcgctggcttggtgccaacgctttccgtaccagccactacccctatgcagaggaagtgatgcagatgtgtgaccgctatgggattgtggtcatcgatgagtgtcccggcgtgggcctggcgctgccgcagttcttcaacaacgtttctctgcatcaccacatgcaggtgatggaagaagtggtgcgtagggacaagaaccaccccgcggtcgtgatgtggtctgtggccaacgagcctgcgtcccacctagaatctgctggctactacttgaagatggtgatcgctcacaccaaatccttggacccctcccggcctgtgacctttgtgagcaactctaactatgcagcagacaagggggctccgtatgtggatgtgatctgtttgaacagctactactcttggtatcacgactacgggcacctggagttgattcagctgcagctggccacccagtttgagaactggtataagaagtatcagaagcccattattcagagcgagtatggagcagaaacgattgcagggtttcaccaggatccacctctgatgttcactgaagagtaccagaaaagtctgctagagcagtaccatctgggtctggatcaaaaacgcagaaaatacgtggttggagagctcatttggaattttgccgatttcatgactgaacagtcaccgacgagagtgctggggaataaaaaggggatcttcactcggcagagacaaccaaaaagtgcagcgttcctttgcgagagagatactggaagattgccaatgaaaccaggtatccccactcagtagccaagtcacaatgtttggaaaacagcccgtttacttga; NM_010368.

NPC1:NM_000271;NM_006432.

hexosaminidaseB:NM_000521;atggagctgtgcgggctggggctgccccggccgcccatgctgctggcgctgctgttggcgacactgctggcggcgatgttggcgctgctgactcaggtggcgctggtggtgcaggtggcggaggcggctcgggccccgagcgtctcggccaagccggggccggcgctgtggccccctgccgctctcggtgaagatgaccccgaacctgctgcatctcgccccggagaacttctacatcagccacagccccaattccacggcgggcccctcctgcaccctgctggaggaagcgtttcgacgatatcatggctatatttttggtttctacaagtggcatcatgaacctgctgaattccaggctaaaacccaggttcagcaacttcttgtctcaatcacccttcagtcagagtgtgatgctttccccaacatatcttcagatgagtcttatactttacttgtgaaagaaccagtggctgtccttaaggccaacagagtttggggagcattacgaggtttagagacctttagccagttagtttatcaagattcttatggaactttcaccatcaatgaatccaccattattgattctccaaggttttctcacagaggaattttgattgatacatccagacattatctgccagttaagattattcttaaaactctggatgccatggcttttaataagtttaatgttcttcactggcacatagttgatgaccagtctttcccatatcagagcatcactttttcctgagttaagcaataaaggaagctattctttgtctcatgtttatacaccaaatgatgtccgtatggtgattgaatatgccagattacgaggaattcgagtcctgccagaatttgataccccctgggcatacactatcttggggaaaaggtcagaaagacctcctgactccatgttacagtagacaaaaca

agttggactcttttggacctataaaccctactctgaatacaacatacagcttccttactacattttcaaagaaattagtgaggtgtttccagatcaattcattcatttgggaggagatgaagtggaatttaaatgttgggaatcaaatccaaaaattcaagatttcatgaggcaaaaaggctttggcacagattttaagaaactagaatctttctacattcaaaaggttttggatattattgcaaccataaacaagggatccattgtctggcaggaggtttttgatgataaagcaaagcttgcgccgggcacaatagttgaagtatggaaagacagcgcatatcctgaggaactcagtagagtcacagcatctggcttccctgtaatcctttctgctccttggtacttagatttgattagctatggacaagattggaggaaatactataaagtggaacctcttgattttggcggtactcagaaacagaaacaacttttcattggtggagaagcttgtctatggggagaatatgtggatgcaactaacctcactccaagattatggcctcgggcaagtgctgttggtgagagactctggagttccaaagatgtcagagatatggatgacgcctatgacagactgacaaggcaccgctgcaggatggtcgaacgtggaatagctgcacaacctctttatgctggatattgtaaccatgagaacatgtaa.

galactosidase,alpha(GLA):NM_000169:atgcagctgaggaacccagaactacatctgggctgcgcgcttgcgcttcgcttcctggccctcgtttcctgggacatccctggggctagagcactggacaatggattggcaaggacgcctaccatgggctggctgcactgggagcgcttcatgtgcaaccttgactgccaggaagagccagattcctgcatcagtgagaagctcttcatggagatggcagagctcatggtctcagaaggctggaaggatgcaggttatgagtacctctgcattgatgactgttggatggctccccaaagagattcagaaggcagacttcaggcagaccctcagcgctttcctcatgggattcgccagctagctaattatgttcacagcaaaggactgaagctagggatttatgcagatgttggaaataaaacctgcgcaggcttccctgggagttttggatactacgacattgatgcccagacctttgctgactggggagtagatctgctaaaatttgatggttgttactgtgacagtttggaaaatttggcagatggttataagcacatgtccttggccctgaataggactggcagaagcattgtgtactcctgtgagtggcctctttatatgtggccctttcaaaagcccaattatacagaaatccgacagtactgcaatcactggcgaaattttgctgacattgatgattcctggaaaagtataaagagtatcttggactggacatcttttaaccaggagagaattgttgatgttgctggaccaggggggttggaatgacccagatatgttagtgattggcaactttggcctcagctggaatcagcaagtaactcagatggccctctgggctatcatggctgctcctttattcatgtctaatgacctccgacacatcagccctcaagccaaagctctccttcaggataaggacgtaattgccatcaatcaggaccccttgggcaagcaagggtaccagcttagacagggagacaactttgaagtgtgggaacgacctctctcaggcttagcctgggctgtagctatgataaaccggcaggagattggtggacctcgctcttataccatcgcagttgcttccctgggtaaaggagtggcctgtaatcctgcctgcttcatcacacagctcctccctgtgaaaaggaagctagggttctatgaatggacttcaaggttaagaagtcacataaatcccacaggcactgttttgcttcagctagaaaatacaatgcagatgtcattaaaagacttactttaa

glucosidase_beta_acid(GBA):NM_000157:atggagttttcaagtccttccagagaggaatgtcccaagcctttgagtagggtaagcatcatggctggcagcctcacaggattgcttctacttcaggcagtgtcgtgggcatcaggtgcccgcccctgcatccctaaaagcttcggctacagctcggtggtgtgtgtctgcaatgccacatactgtgactcctttgaccccccgacctttcctgcccttggtaccttcagccgctatgagagtacacgcagtgggcgacggatggagctgagtatgggcccatccaggctaatcacacgggcacaggcctgctactgaccctgcagccagaacagaagttccagaaagtgaagggatttggaggggccatgacagatgctgctgctctcaacatccttgccctgtcacccccctgcccaaaatttgctacttaaatcgtacttctctgaagaaggaatcggatataacatcatccgggtacccatggccagctgtgacttctccatccgcacctacacctatgcagacacccctgatgatttccagttgcacaacttcagcctcccagaggaagataccaagctcaagatacccctgattcaccgagccctgcagttggcccagcgtcccgtttcactccttgccagcccctggacatcacccacttggctcaagaccaatggagcggtgaatgggaagggtcactcaagggacagcccggagacatctaccaccagacctgggccagatactttgtgaagttcctggatgcctatgctgagcacaagttacagttctgggcagtgacagctgaaaatgagccttctgctgggctgttgagtggatacccctccagtgcctgggcttcacccctgaacatcagcgagacttcattgcccgtgacctaggtcctaccctcgccaacagtactcaccacaatgtccgcctactcatgctggatgaccaacgcttgctgctgccccactgggcaaaggtggtactgacagacccagaagcagctaaatatgttcatggcattgctgtacattggtacctggactttctggctccagccaaagccaccctaggggagacacaccgcctgttccccaacaccatgctctttgcctcagaggcctgtgtgggctccaagttctgggagcagagtgtgcggctaggctcctgggatcgagggatgcagtacagccacagcatcatcacgaacctcctgtaccatgtggtcggctggaccgactggaaccttgccctgaaccccgaaggaggacccaattgggtgcgtaactttgtcgacagtcccatcattgtagacatcaccaaggacacgttttacaaacagcccatgttctaccaccttggccacttcagcaagttcattcctgagggctcccagagagtggggctggttgccagtcagaagaacgacctggacgcagtggcactgatgcatcccgatggctctgctgttgtggtcgtgctaaaccgctcctctaaggatgtgcctcttaccatcaaggatcctgctgtgggcttcctggagacaatctcacctggctactccattcacacctacctgtggcgtcgccagtga; NM_008094.

von_Hippel_Lindau_tumor_suppressor(VHL):NM_000551:atgccccggagggcggagaactgggacgaggccgaggtaggcgcggaggaggcaggcgtcgaagagtacggccctgaagaagacggcggggaggagtcgggcgccgaggagtccggcccggaagagtccggcccggaggaactgggcgccgaggaggagatggaggccgggcggccgcggcccgtgctgcgctcggtgaactcgcgcgagccctcccaggtcatcttctgcaatcgcagtccg

cgcgtcgtgctgcccgtatggctcaacttcgacggcgagccgcagccctacccaacgctgccgcctggcacgggccgccgcatccacagctaccgaggtcacctttggctcttcagagatgcagggacacacgatgggcttctggttaaccaaactgaattatttgtgccatctctcaatgttgacggacagcctatttttgccaatatcacactgccagtgtatactctgaaagagcgatgcctccaggttgtccggagcctagtcaagcctgagaattacaggagactggacatcgtcaggtcgctctacgaagatctggaagaccacccaaatgtgcagaaagacctggagcggctgacacaggagcgcattgcacatcaacggatgggagattga.

Beta_globin(HBB):NM_000518:atggtgcatctgactcctgaggagaagtctgccgttactgccctgtggggcaaggtgaacgtggatgaagttggtggtgaggccctgggcaggctgctggtggtctacccttggacccagaggttctttgagtcctttggggatctgtccactcctgatgctgttatgggcaaccctaaggtgaaggctcatggcaagaaagtgctcggtgcctttagtgatggcctggctcacctggacaacctcaagggcacctttgccacactgagtgagctgcactgtgacaagctgcacgtggatcctgagaacttcaggctcctgggcaacgtgctggtctgtgtgctggcccatcactttggcaaagaattcaccccaccagtgcaggctgcctatcagaaagtggtggctggtgtggctaatgccctggcccacaagtatcactaa.

PARK2:NM_013988:atgatagtgtttgtcaggttcaactccagccatggtttcccagtggaggtcgattctgacaccagcatcttccagctcaaggaggtggttgctaagcgacaggggggttccggctgaccagttgcgtgtgattttcgcagggaaggagctgaggaatgactggactgtgcaggaatttttctttaaatgtggagcacaccccacctctgacaaggaaacatcagtagctttgcacctgatcgcaacaaatagtcggaacatcacttgcattacgtgcacagacgtcaggagccccgtcctggtttccagtgcaactcccgccacgtgatttgcttagactgtttccacttatactgtgtgacaagactcaatgatcggcagtttgttcacgaccctcaacttggctactccctgccttgtgtggctggctgtcccaactccttgattaaagagctccatcacttcaggattctgggagaagagcagtacaaccggtaccagcagtatggtgcagaggagtgtgtcctgcagatgggggggcgtgttatgcccccgccctggctgtggagcggggctgctgccggagcctgaccagaggaaagtcacctgcgaaggggggcaatggcctgggctgtgggtttgccttctgccgggaatgtaaagaagcgtaccatgaaggggagtgcagtgccgtatttgaagcctcaggaacaactactcaggcctacagagtcgatgaaagagccgccgagcaggctcgttgggaagcagcctccaaagaaaccatcaagaaaaccaccaagccctgtccccgctgccatgtaccagtggaaaaaaatggaggctgcatgcacatgaagtgtccgcagccccagtgcaggctcgagtggtgctggaactgtggctgcgagtggaaccgcgtctgcatggggggaccactggttcgacgtgtag; NM_004562; NM_020093.

The contents of all parenthetically cited publications and the following United States Patents, are noted and incorporated by reference in their entireties: 7211247, 5677139, 6432711 and 5453357, U.S. 05,593,875, U.S. 05,783,566, U.S. 5,928,944, U.S. 05,910,488, U.S. 05,824,547,

## Claims

1. A method for providing a desired cell population capable of further differentiation in vivo comprising: selecting cells from somatic cells; maintaining the cells in growth culture medium until the desired cell number is achieved, transfecting the cells with transcription factors promoting the cells to begin differentiating into the desired cells; culturing said selected cells in a
differentiation medium comprising at least one differentiation agent effective to promote differentiation or modification of the selected cells into desired differentiating cells; and wherein the differentiation agent is selected from the group consisting of retinoic acid, nerve growth factor, dimethylsulfoxide, and hexamethylene bis acrylamide.

2. The method of claim 1, where the desired cells are muscle cells and the cells are transfected with transcription factors, including ones selected from MyoD, myogenin, Myocardin, Myf 5, Myf 6, Mef2A, Gata 4, Gata 5, and Gata 6 depending upon whether the desired muscle cells are skeletal muscle cells, smooth muscle cells or cardiomyocytes.

3. The method of claim 1, where dopaminergic neurons are the desired cells, and the cells are transfected with transcription factors, including ones selected from Mash1, Ngn2, Nurr1, Lmx1b, and/or Ptx-3 prior to culture in the differentiation medium.

4. The method of claim 1, where serotonergic neurons are the desired cells, and the cells are transfected with transcription factors prior to culture in the differentiation medium.

5. The method of claim 4, wherein the transcription factors include a transcription factor selected Mash1, Phox2b, Lmx1b, Nkx2.2, Gata2, Gata3 and Petl.

6. The method of claim 1, where cholinergic neurons are the desired cells, and the cells are transfected with transcription factors, including ones selected from MASH1, Phox2a and REST4 prior to culture in the differentiation medium.

7. The method of claim 1, where GABAergic neurons are the desired cells, and the cells are transfected with transcription factors, including ones selected from MASH1, Phox2a and REST4, prior to culture in differentiation media containing differentiation agents drawn from LIF, NT3, and NGF.

8. The method of claim 1, where noradrenergic neurons are the desired cells, and the cells are transfected with transcription factors, including ones selected from Mash1, dHand(HAND2), Phox2a, Phox2b, Gata2 and Gata3.

9. The method of claim 1, where GABAergic neurons are the desired cells, and the cells are transfected with transcription factors, including ones selected from PITX2, Dlx2, Dlx5 and RNA targeting Hes1.

10. The method of claim 1, where neurons are the desired cells, and the cells are transfected with transcription factors, including ones selected from PITX2, Dlx2, Dlx5, Nurr1, REN, Neurogenin1, Neurogenin2, Neurogenin3, Mash 1, Phox2b, Phox2a, dHand, Gata3, Shh, FGF8, Lmx1b, Nkx2.2, Pet1, Lbxl, Rnx, and/or antisense Hesl RNA.

11. The method of claim 1, where pancreatic islet cells or endoderm cells are the desired cells, the selected cells are transfected with transcription factors, including ones selected from Foxa2, Soxl7, HLXB9 and Pdxl; and where additional differentiating agents are selected from sodium butyrate, activin A, retinoic acid, epidermal growth factor, basic fibroblast growth factor, noggin, insulin-like growth factor II and nicotinamide.

**12.** The method of claim 1, where the desired cells are hematopoietic cells, the cells are transfected with nucleotide sequences encoding Runx1/AML1; and where the differentiating agents are selected from VEGF, thrombopoietin, and colony stimulating factors appropriate to the desired cell type.

**13.** The method of claim 1, where the somatic cells are selected from genetically-modified somatic cells.

**14.** The method of claim 1, utilizing nucleic acid or protein electroporation, liposomes, nanocapsules, nanovaults, and/or other approach avoiding viral integration or other random alteration of the cell's genome.

**15.** The method of claim 1, where said transcription factor is overexpressed.

**16.** The method of claim 1, where said somatic cells are further transfected with numblike or the short PRR-/PTB- numb isoform.

Figure 1.

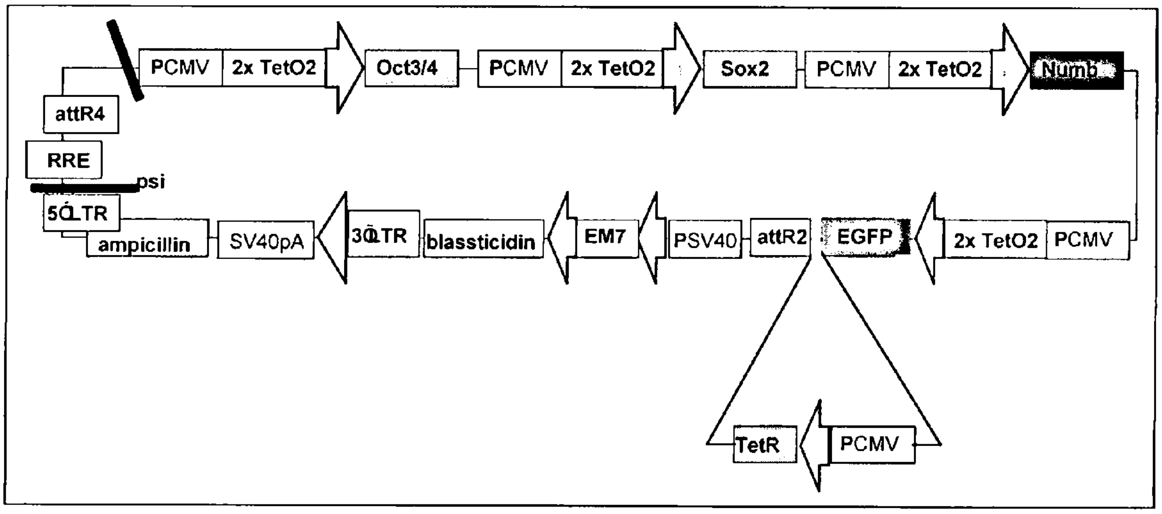


Figure 1. **A schematized map corresponding to the vector sequence provided in Example 13.**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6432711 B
- US 5453357 A
- WO 2003070084 A
- EP 1482871 A
- WO 03070084 A
- US 2395698 A
- US 7297540 B
- US 6995013 B
- US 6800753 B, Isenberg
- US 6734018 B
- US 6962814 B
- US 6479064 B
- US 6376244 B
- US 5032508 A
- US 4902508 A
- US 4956178 A
- US 5281422 A
- US 5554389 A
- US 6099567 A
- US 6206931 B
- US 4361552 A
- US 6576618 B
- US 6753181 B
- US 11162715 B
- WO 2001048153 A
- WO 2002024244 A
- WO 003002165 A
- WO 2001049210 A
- WO 2007025233 A
- EP 1246903 A
- EP 1244396 A
- EP 0987998 A
- EP 1333870 A
- US 6217867 B
- US 5677139 A
- US 20030099621 A
- US 20060099177 A
- US 05593875 A
- US 05783566 A
- US 5928944 A
- US 05910488 A
- US 05824547 A
- US 7211247 B


### Non-patent literature cited in the description


- **SAMBROOK et al.** Molecular Cloning: A Laboratory Manual. Cold Spring Harbor Laboratory press, 1989
- *Invitrogen's Viral Power Lentiviral Expression Systems Manual,* 2007

SEQUENCE LISTING

<110> REID, CHRISTOPHER B.

<120> METHODS FOR PRODUCTION AND USES OF MULTIPOTENT CELL POPULATIONS, PLURIPOTENT CELL POPULATIONS, NEURONAL CELL POPULATIONS, AND MUSCLE CELL POPULATIONS

<130> CBR-002PCT

<140> PCT/US2008/065007
<141> 2008-05-28

<150> 61/064,761
<151> 2008-03-25

<150> 61/006,449
<151> 2008-01-14

<150> 60/933,670
<151> 2007-06-08

<150> 60/933,133
<151> 2007-06-05

<150> 60/932,020
<151> 2007-05-29

<160> 109

<170> PatentIn Ver. 3.3

<210> 1
<211> 8069
<212> DNA
<213> Artificial Sequence

<220>
<223> Description of Artificial Sequence: Synthetic polynucleotide

<400> 1
aatgtagtct tatgcaatac tcttgtagtc ttgcaacatg gtaacgatga gttagcaaca 60
tgccttacaa ggagagaaaa agcaccgtgc atgccgattg gtggaagtaa ggtggtacga 120
tcgtgcctta ttaggaaggc aacagacggg tctgacatgg attggacgaa ccactgaatt 180
gccgcattgc agagatattg tatttaagtg cctagctcga tacataaacg ggtctctctg 240
gttagaccag atctgagcct gggagctctc tggctaacta gggaacccac tgcttaagcc 300
tcaataaagc ttgccttgag tgcttcaagt agtgtgtgcc cgtctgttgt gtgactctgg 360
taactagaga tccctcagac ccttttagtc agtgtggaaa atctctagca gtggcgcccg 420
aacagggact tgaaagcgaa agggaaacca gaggagctct ctcgacgcag gactcggctt 480
gctgaagcgc gcacggcaag aggcgagggg cggcgactgg tgagtacgcc aaaaattttg 540
actagcggag gctagaagga gagagatggg tgcgagagcg tcagtattaa gcgggggaga 600
attagatcgc gatgggaaaa aattcggtta aggccagggg gaaagaaaaa atataaatta 660
aaacatatag tatgggcaag cagggagcta gaacgattcg cagttaatcc tggcctgtta 720
gaaacatcag aaggctgtag acaaatactg ggacagctac aaccatccct tcagacagga 780
tcagaagaac ttagatcatt atataataca gtagcaaccc tctattgtgt gcatcaaagg 840
atagagataa aagacaccaa ggaagcttta gacaagatag aggaagagca aaacaaaagt 900
aagaccaccg cacagcaagc ggccgctgat cttcagacct ggaggaggag atatgaggga 960

caattggaga agtgaattat ataaatataa agtagtaaaa attgaaccat taggagtagc 1020
acccaccaag gcaaagagaa gagtggtgca gagagaaaaa agagcagtgg gaataggagc 1080
tttgttcctt gggttcttgg gagcagcagg aagcactatg ggcgcagcgt caatgacgct 1140
gacggtacag gccagacaat tattgtctgg tatagtgcag cagcagaaca atttgctgag 1200
ggctattgag gcgcaacagc atctgttgca actcacagtc tggggcatca agcagctcca 1260
ggcaagaatc ctggctgtgg aaagatacct aaaggatcaa cagctcctgg ggatttgggg 1320
ttgctctgga aaactcattt gcaccactgc tgtgccttgg aatgctagtt ggagtaataa 1380
atctctggaa cagatttgga atcacacgac ctggatggag tgggacagag aaattaacaa 1440
ttacacaagc ttaatacact ccttaattga agaatcgcaa aaccagcaag aaaagaatga 1500
acaagaatta ttggaattag ataaatgggc aagtttgtgg aattggttta acataacaaa 1560
ttggctgtgg tatataaaat tattcataat gatagtagga ggcttggtag gtttaagaat 1620
agtttttgct gtactttcta tagtgaatag agttaggcag ggatattcac cattatcgtt 1680
tcagacccac ctcccaaccc cgaggggacc cgacaggccc gaaggaatag aagaagaagg 1740
tggagagaga gacagagaca gatccattcg attagtgaac ggatctcgac ggtatcgatg 1800
tcgacgttaa cgctagtgat atcaactttg tatagaaaag ttgaacgaga aacgtaaaat 1860
gatataaata tcaatatatt aaattagatt ttgcataaaa aacagactac ataatactgt 1920
aaaacacaac atatccagtc actatggcgg ccgcattagg caccccaggc tttacacttt 1980
atgcttccgg ctcgtataat gtgtggattt tgagttagga tccgtcgaga ttttcaggag 2040
ctaaggaagc taaaatggag aaaaaaatca ctggatatac caccgttgat atatcccaat 2100
ggcatcgtaa agaacatttt gaggcatttc agtcagttgc tcaatgtacc tataaccaga 2160
ccgttcagct ggatattacg gcctttttaa agaccgtaaa gaaaaataag cacaagtttt 2220
atccggcctt tattcacatt cttgcccgcc tgatgaatgc tcatccggaa ttccgtatgg 2280
caatgaaaga cggtgagctg gtgatatggg atagtgttca cccttgttac accgttttcc 2340
atgagcaaac tgaaacgttt tcatcgctct ggagtgaata ccacgacgat ttccggcagt 2400
ttctacacat atattcgcaa gatgtggcgt gttacggtga aaacctggcc tatttcccta 2460
aagggtttat tgagaatatg tttttcgtct cagccaatcc ctgggtgagt ttcaccagtt 2520
ttgatttaaa cgtggccaat atggacaact tcttcgcccc cgttttcacc atgggcaaat 2580
attatacgca aggcgacaag gtgctgatgc cgctggcgat tcaggttcat catgccgttt 2640
gtgatggctt ccatgtcggc agaatgctta atgaattaca acagtactgc gatgagtggc 2700
agggcggggc gtaaagatct ggatccggct tactaaaagc cagataacag tatgcgtatt 2760
tgcgcgctga tttttgcggt ataagaatat atactgatat gtatacccga agtatgtcaa 2820
aaagaggtat gctatgaagc agcgtattac agtgacagtt gacagcgaca gctatcagtt 2880
gctcaaggca tatatgatgt caatatctcc ggtctggtaa gcacaaccat gcagaatgaa 2940
gcccgtcgtc tgcgtgccga acgctggaaa gcggaaaatc aggaagggat ggctgaggtc 3000
gcccggttta ttgaaatgaa cggctctttt gctgacgaga acagggactg gtgaaatgca 3060
gtttaaggtt tacacctata aaagagagag ccgttatcgt ctgtttgtgg atgtacagag 3120
tgatattatt gacacgcccg ggcgacggat ggtgatcccc ctggccagtg cacgtctgct 3180
gtcagataaa gtctcccgtg aactttaccc ggtggtgcat atcggggatg aaagctggcg 3240
catgatgacc accgatatgg ccagtgtgcc ggtctccgtt atcggggaag aagtggctga 3300
tctcagccac cgcgaaaatg acatcaaaaa cgccattaac ctgatgttct ggggaatata 3360
aatgtcaggc tccgttatac acagccagtc tgcaggtcga ccatagtgac tggatatgtt 3420
gtgttttaca gtattatgta gtctgttttt tatgcaaaat ctaatttaat atattgatat 3480
ttatatcatt ttacgtttct cgttcagctt tcttgtacaa agtggttgat atccagcaca 3540
gtggcggccg ctcgagtcta gagggcccgc ggttcgaagg taagcctatc cctaaccctc 3600
tcctcggtct cgattctacg cgtaccggtt agtaatgagt ttggaattaa ttctgtggaa 3660
tgtgtgtcag ttagggtgtg gaaagtcccc aggctcccca gcaggcagaa gtatgcaaag 3720
catgcatctc aattagtcag caaccaggtg tggaaagtcc ccaggctccc cagcaggcag 3780
aagtatgcaa agcatgcatc tcaattagtc agcaaccata gtcccgcccc taactccgcc 3840
catcccgccc ctaactccgc ccagttccgc ccattctccg ccccatggct gactaatttt 3900
ttttatttat gcagaggccg aggccgcctc tgcctctgag ctattccaga agtagtgagg 3960
aggctttttt ggaggcctag gcttttgcaa aaagctcccg ggagcttgta tatccatttt 4020
cggatctgat cagcacgtgt tgacaattaa tcatcggcat agtatatcgg catagtataa 4080
tacgacaagg tgaggaacta aaccatggcc aagcctttgt ctcaagaaga atccaccctc 4140
attgaaagag caacggctac aatcaacagc atccccatct ctgaagacta cagcgtcgcc 4200
agcgcagctc tctctagcga cggccgcatc ttcactggtg tcaatgtata tcatttttact 4260
gggggacctt gtgcagaact cgtggtgctg ggcactgctg ctgctgcggc agctggcaac 4320
ctgacttgta tcgtcgcgat cggaaatgag aacaggggca tcttgagccc ctgcggacgg 4380
tgccgacagg tgcttctcga tctgcatcct gggatcaaag ccatagtgaa ggacagtgat 4440

```
ggacagccga cggcagttgg gattcgtgaa ttgctgccct ctggttatgt gtgggagggc 4500
taagcacaat tcgagctcgg tacctttaag accaatgact tacaaggcag ctgtagatct 4560
tagccacttt ttaaaagaaa aggggggact ggaagggcta attcactccc aacgaagaca 4620
agatctgctt tttgcttgta ctgggtctct ctggttagac cagatctgag cctgggagct 4680
ctctggctaa ctagggaacc cactgcttaa gcctcaataa agcttgcctt gagtgcttca 4740
agtagtgtgt gcccgtctgt tgtgtgactc tggtaactag agatccctca gaccctttta 4800
gtcagtgtgg aaaatctcta gcagtagtag ttcatgtcat cttattattc agtatttata 4860
acttgcaaag aaatgaatat cagagagtga gaggaacttg tttattgcag cttataatgg 4920
ttacaaataa agcaatagca tcacaaattt cacaaataaa gcattttttt cactgcattc 4980
tagttgtggt ttgtccaaac tcatcaatgt atcttatcat gtctggctct agctatcccg 5040
cccctaactc cgcccatccc gcccctaact ccgcccagtt ccgcccattc tccgccccat 5100
ggctgactaa ttttttttat ttatgcagag gccgaggccg cctcggcctc tgagctattc 5160
cagaagtagt gaggaggctt ttttggaggc ctagggacgt acccaattcg ccctatagtg 5220
agtcgtatta cgcgcgctca ctggccgtcg ttttacaacg tcgtgactgg gaaaaccctg 5280
gcgttaccca acttaatcgc cttgcagcac atcccccttt cgccagctgg cgtaatagcg 5340
aagaggcccg caccgatcgc ccttcccaac agttgcgcag cctgaatggc gaatgggacg 5400
cgccctgtag cggcgcatta agcgcggcgg gtgtggtggt tacgcgcagc gtgaccgcta 5460
cacttgccag cgccctagcg cccgctcctt tcgctttctt cccttccttt ctcgccacgt 5520
tcgccggctt tccccgtcaa gctctaaatc gggggctccc tttagggttc cgatttagtg 5580
ctttacggca cctcgacccc aaaaaacttg attagggtga tggttcacgt agtgggccat 5640
cgccctgata gacggttttt cgccctttga cgttggagtc cacgttcttt aatagtggac 5700
tcttgttcca aactggaaca acactcaacc ctatctcggt ctattctttt gatttataag 5760
ggattttgcc gatttcggcc tattggttaa aaaatgagct gatttaacaa aaatttaacg 5820
cgaattttaa caaaatatta acgcttacaa tttaggtggc acttttcggg gaaatgtgcg 5880
cggaacccct atttgtttat ttttctaaat acattcaaat atgtatccgc tcatgagaca 5940
ataaccctga taaatgcttc aataatattg aaaaaggaag agtatgagta ttcaacattt 6000
ccgtgtcgcc cttattccct tttttgcggc attttgcctt cctgtttttg ctcacccaga 6060
aacgctggtg aaagtaaaag atgctgaaga tcagttgggt gcacgagtgg gttacatcga 6120
actggatctc aacagcggta agatccttga gagttttcgc cccgaagaac gttttccaat 6180
gatgagcact tttaaagttc tgctatgtgg cgcggtatta tcccgtattg acgccgggca 6240
agagcaactc ggtcgccgca tacactattc tcagaatgac ttggttgagt actcaccagt 6300
cacagaaaag catcttacgg atggcatgac agtaagagaa ttatgcagtg ctgccataac 6360
catgagtgat aacactgcgg ccaacttact tctgacaacg atcggaggac cgaaggagct 6420
aaccgctttt ttgcacaaca tgggggatca tgtaactcgc cttgatcgtt gggaaccgga 6480
gctgaatgaa gccataccaa acgacgagcg tgacaccacg atgcctgtag caatggcaac 6540
aacgttgcgc aaactattaa ctggcgaact acttactcta gcttcccggc aacaattaat 6600
agactggatg gaggcggata aagttgcagg accacttctg cgctcggccc ttccggctgg 6660
ctggtttatt gctgataaat ctggagccgg tgagcgtggg tctcgcggta tcattgcagc 6720
actggggcca gatggtaagc cctcccgtat cgtagttatc tacacgacgg ggagtcaggc 6780
aactatggat gaacgaaata gacagatcgc tgagataggt gcctcactga ttaagcattg 6840
gtaactgtca gaccaagttt actcatatat actttagatt gatttaaaac ttcattttta 6900
atttaaaagg atctaggtga agatcctttt tgataatctc atgaccaaaa tcccttaacg 6960
tgagttttcg ttccactgag cgtcagaccc cgtagaaaag atcaaaggat cttcttgaga 7020
tccttttttt ctgcgcgtaa tctgctgctt gcaaacaaaa aaaccaccgc taccagcggt 7080
ggtttgtttg ccggatcaag agctaccaac tctttttccg aaggtaactg gcttcagcag 7140
agcgcagata ccaaatactg ttcttctagt gtagccgtag ttaggccacc acttcaagaa 7200
ctctgtagca ccgcctacat acctcgctct gctaatcctg ttaccagtgg ctgctgccag 7260
tggcgataag tcgtgtctta ccgggttgga ctcaagacga tagttaccgg ataaggcgca 7320
gcggtcgggc tgaacggggg gttcgtgcac acagcccagc ttggagcgaa cgacctacac 7380
cgaactgaga tacctacagc gtgagctatg agaaagcgcc acgcttcccg aagggagaaa 7440
ggcggacagg tatccggtaa gcggcagggt cggaacagga gagcgcacga gggagcttcc 7500
agggggaaac gcctggtatc tttatagtcc tgtcgggttt cgccacctct gacttgagcg 7560
tcgatttttg tgatgctcgt caggggggcg gagcctatgg aaaaacgcca gcaacgcggc 7620
ctttttacgg ttcctggcct tttgctggcc ttttgctcac atgttctttc ctgcgttatc 7680
ccctgattct gtggataacc gtattaccgc ctttgagtga gctgataccg ctcgccgcag 7740
ccgaacgacc gagcgcagcg agtcagtgag cgaggaagcg gaagagcgcc caatacgcaa 7800
accgcctctc cccgcgcgtt ggccgattca ttaatgcagc tggcacgaca ggtttcccga 7860
ctggaaagcg ggcagtgagc gcaacgcaat taatgtgagt tagctcactc attaggcacc 7920
```

ccaggcttta cactttatgc ttccggctcg tatgttgtgt ggaattgtga gcggataaca 7980
atttcacaca ggaaacagct atgaccatga ttacgccaag cgcgcaatta accctcacta 8040
aagggaacaa aagctggagc tgcaagctt 8069

<210> 2
<211> 22460
<212> DNA
<213> Artificial Sequence

<220>
<223> Description of Artificial Sequence: Synthetic polynucleotide

<400> 2
aatgtagtct tatgcaatac tcttgtagtc ttgcaacatg gtaacgatga gttagcaaca 60
tgccttacaa ggagagaaaa agcaccgtgc atgccgattg gtggaagtaa ggtggtacga 120
tcgtgcctta ttaggaaggc aacagacggg tctgacatgg attggacgaa ccactgaatt 180
gccgcattgc agagatattg tatttaagtg cctagctcga tacataaacg ggtctctctg 240
gttagaccag atctgagcct gggagctctc tggctaacta gggaacccac tgcttaagcc 300
tcaataaagc ttgccttgag tgcttcaagt agtgtgtgcc cgtctgttgt gtgactctgg 360
taactagaga tccctcagac ccttttagtc agtgtggaaa atctctagca gtggcgcccg 420
aacagggact tgaaagcgaa agggaaacca gaggagctct ctcgacgcag gactcggctt 480
gctgaagcgc gcacggcaag aggcgagggg cggcgactgg tgagtacgcc aaaaattttg 540
actagcggag gctagaagga gagagatggg tgcgagagcg tcagtattaa gcgggggaga 600
attagatcgc gatgggaaaa aattcggtta aggccagggg gaaagaaaaa atataaatta 660
aaacatatag tatgggcaag cagggagcta gaacgattcg cagttaatcc tggcctgtta 720
gaaacatcag aaggctgtag acaaatactg ggacagctac aaccatccct tcagacagga 780
tcagaagaac ttagatcatt atataataca gtagcaaccc tctattgtgt gcatcaaagg 840
atagagataa aagacaccaa ggaagcttta gacaagatag aggaagagca aaacaaaagt 900
aagaccaccg cacagcaagc ggccgctgat cttcagacct ggaggaggag atatgaggga 960
caattggaga agtgaattat ataaatataa agtagtaaaa attgaaccat taggagtagc 1020
acccaccaag gcaaagagaa gagtggtgca gagagaaaaa agagcagtgg gaataggagc 1080
tttgttcctt gggttcttgg gagcagcagg aagcactatg ggcgcagcgt caatgacgct 1140
gacggtacag gccagacaat tattgtctgg tatagtgcag cagcagaaca atttgctgag 1200
ggctattgag gcgcaacagc atctgttgca actcacagtc tggggcatca agcagctcca 1260
ggcaagaatc ctggctgtgg aaagatacct aaaggatcaa cagctcctgg ggatttgggg 1320
ttgctctgga aaactcattt gcaccactgc tgtgccttgg aatgctagtt ggagtaataa 1380
atctctggaa cagatttgga atcacacgac ctggatggag tgggacagag aaattaacaa 1440
ttacacaagc ttaatacact ccttaattga agaatcgcaa aaccagcaag aaaagaatga 1500
acaagaatta ttggaattag ataaatgggc aagtttgtgg aattggttta acataacaaa 1560
ttggctgtgg tatataaaat tattcataat gatagtagga ggcttggtag gtttaagaat 1620
agtttttgct gtactttcta tagtgaatag agttaggcag ggatattcac cattatcgtt 1680
tcagacccac ctcccaaccc cgaggggacc cgacaggccc gaaggaatag aagaagaagg 1740
tggagagaga gacagagaca gatccattcg attagtgaac ggatctcgac ggtatcgatg 1800
tcgacgttaa cgctagtgat atcaactttg tatagaaaag ttgaacgaga aacgtaaaat 1860
gatataaata tcaatatatt aaattagatt ttgcataaaa aacagactac ataatactgt 1920
aaaacacaac atatccagtc actatgggac ggatcgggag atctcccgat cccctatggt 1980
gcactctcag tacaatctgc tctgatgccg catagttaag ccagtatctg ctccctgctt 2040
gtgtgttgga ggtcgctgag tagtgcgcga gcaaaattta agctacaaca aggcaaggct 2100
tgaccgacaa ttgcatgaag aatctgctta gggttaggcg ttttgcgctg cttcgcgatg 2160
tacgggccag atatacgcgt tgacattgat tattgactag ttattaatag taatcaatta 2220
cggggtcatt agttcatagc ccatatatgg agttccgcgt tacataactt acggtaaatg 2280
gcccgcctgg ctgaccgccc aacgaccccc gcccattgac gtcaataatg acgtatgttc 2340
ccatagtaac gccaataggg actttccatt gacgtcaatg ggtggagtat ttacggtaaa 2400
ctgcccactt ggcagtacat caagtgtatc atatgccaag tacgccccct attgacgtca 2460
atgacggtaa atggcccgcc tggcattatg cccagtacat gaccttatgg gactttccta 2520
cttggcagta catctacgta ttagtcatcg ctattaccat ggtgatgcgg ttttggcagt 2580

acatcaatgg gcgtggatag cggtttgact cacggggatt tccaagtctc caccccattg 2640
acgtcaatgg gagtttgttt tggaaccaaa atcaacggga ctttccaaaa tgtcgtaaca 2700
actccgcccc attgacgcaa atgggcggta ggcgtgtacg gtgggaggtc tatataagca 2760
gagctctccc tatcagtgat agagatctcc ctatcagtga tagagatcgt cgacgagctc 2820
gtttagtgaa ccgtcagatc gcctggagac gccatccacg ctgttttgac ctccatagaa 2880
gacaccggga ccgatccagc ctccggactc tagcgtttaa acttaagctt accatgcctt 2940
cgcaagccct catttcacca ggcccccggc ttggggcgcc ttccttcccc atggcgggac 3000
acctggcttc ggatttcgcc ttctcgcccc ctccaggtgg tggaggtgat gggccagggg 3060
ggccggagcc gggctgggtt gatcctcgga cctggctaag cttccaaggc cctcctggag 3120
ggccaggaat cgggccgggg gttgggccag gctctgaggt gtgggggatt cccccatgcc 3180
ccccgccgta tgagttctgt gggggggatgg cgtactgtgg gccccaggtt ggagtggggc 3240
tagtgcccca aggcggcttg gagacctctc agcctgaggg cgaagcagga gtcggggtgg 3300
agagcaactc cgatggggcc tccccggagc cctgcaccgt caccccctggt gccgtgaagc 3360
tggagaagga gaagctggag caaaacccgg aggagtccca ggacatcaaa gctctgcaga 3420
aagaactcga gcaatttgcc aagctcctga agcagaagag gatcaccctg ggatatacac 3480
aggccgatgt ggggctcacc ctgggggttc tatttgggaa ggtattcagc caaacgacca 3540
tctgccgctt tgaggctctg cagcttagct tcaagaacat gtgtaagctg cggcccttgc 3600
tgcagaagtg ggtggaggaa gctgacaaca atgaaaatct tcaggagata tgcaaagcag 3660
aaaccctcgt gcaggcccga aagagaaagc gaaccagtat cgagaaccga gtgagaggca 3720
acctggagaa tttgttcctg cagtgcccga aacccacact gcagcagatc agccacatcg 3780
cccagcagct tgggctcgag aaggatgtgg tccgagtgtg gttctgtaac cggcgccaga 3840
agggcaagcg atcaagcagc gactatgcac aacgagagga ttttgaggct gctgggtctc 3900
ctttctcagg gggaccagtg tcctttcctc tggccccagg gccccatttt ggtaccccag 3960
gctatgggag ccctcacttc actgcactgt actcctcggt ccctttccct gaggggggaag 4020
cctttccccc tgtctccgtc accactctgg gctctcccat gcattcaaac tgaggtgcct 4080
gcccttctag gaatggggga caggggggagg ggaggagcta gggaaagaaa acctggagtt 4140
tgtgccaggg tttttgggat taagttcttc attcactaag gaaggaattg ggaacacaaa 4200
gggtgggggc aggggagttt ggggcaactg gttggaggga aggtgaagtt caatgatgct 4260
cttgatttta atcccacatc atgtatcact ttttcttaa ataaagaagc ctgggacaca 4320
gtagatagac acacttaaaa aaaaaaacct cgactgtgcc ttctagttgc cagccatctg 4380
ttgtttgccc ctcccccgtg ccttccttga ccctggaagg tgccactccc actgtccttt 4440
cctaataaaa tgaggaaatt gcatcgcatt gtctgagtag gtgtcattct attctggggg 4500
gtggggtggg gcaggacagc aagggggagg attgggaaga caatagcagg catgctgggg 4560
atgcggtggg ctctatggga cggatcggga gatctcccga tcccctatgg tgcactctca 4620
gtacaatctt gctctgatgc cgcatagtta agccagtatc tgctccctgc ttgtgtgttg 4680
gaggtcgctg agtagtgcgc gagcaaaatt taagctacaa caaggcaagg cttgaccgac 4740
aattgcatga agaatctgct tagggttagg cgttttgcgc tgcttcgcga tgtacgggcc 4800
agatatacgc gttgacattg attattgact agttattaat agtaatcaat tacggggtca 4860
ttagttcata gcccatatat ggagttccgc gttacataac ttacggtaaa tggcccgcct 4920
ggctgaccgc ccaacgaccc ccgcccattg acgtcaataa tgacgtatgt tcccatagta 4980
acgccaatag ggactttcca ttgacgtcaa tgggtggagt atttacggta aactgcccac 5040
ttggcagtac atcaagtgta tcatatgcca agtacgcccc ctattgacgt caatgacggt 5100
aaatggcccg cctggcatta tgcccagtac atgaccttat gggactttcc tacttggcag 5160
tacatctacg tattagtcat cgctattacc atggtgatgc ggttttggca gtacatcaat 5220
gggcgtggat agcggtttga ctcacgggga tttccaagtc tccaccccat tgacgtcaat 5280
gggagtttgt tttggaacca aaatcaacgg gactttccaa aatgtcgtaa caactccgcc 5340
ccattgacgc aaatgggcgg taggcgtgta cggtgggagg tctatataag cagagctctc 5400
cctatcagtg atagagatct ccctatcagt gatagagatc gtcgacgagc tcgtttagtg 5460
aaccgtcaga tcgcctggag acgccatcca cgctgttttg acctccatag aagacaccgg 5520
gaccgatcca gcctccggac tctagcgttt aaacttaagc ttaccatgct attaacttgt 5580
tcaaaaaagt atcaggagtt gtcaaggcag agaagagagt gtttgcaaaa gggggaaagt 5640
agtttgctgc ctctttaaga ctaggactga gagaaagaag aggagagaga aagaaaggga 5700
gagaagtttg agccccaggc ttaagccttt ccaaaaaata ataataacaa tcatcggcgg 5760
cggcaggatc ggccagagga ggagggaagc gcttttttttg atcctgattc cagtttgcct 5820
ctctcttttt ttcccccaaa ttattcttcg cctgatttc ctcgcggagc cctgcgctcc 5880
cgacaccccc gcccgcctcc cctcctcctc tcccccccgcc cgcgggcccc ccaaagtccc 5940
ggccgggccg agggtcggcg gccgccggcg ggccgggccc gcgcacagcg cccgcatgta 6000
caacatgatg gagacggagc tgaagccgcc gggcccgcag caaacttcgg ggggcggcgg 6060

cggcaactcc accgcggcgg cggccggcgg caaccagaaa aacagcccgg accgcgtcaa 6120
gcggcccatg aatgccttca tggtgtggtc ccgcgggcag cggcgcaaga tggcccagga 6180
gaaccccaag atgcacaact cggagatcag caagcgcctg ggcgccgagt ggaaactttt 6240
gtcggagacg gagaagcggc cgttcatcga cgaggctaag cggctgcgag cgctgcacat 6300
gaaggagcac ccggattata aataccggcc ccggcggaaa accaagacgc tcatgaagaa 6360
ggataagtac acgctgcccg gcgggctgct ggcccccggc ggcaatagca tggcgagcgg 6420
ggtcggggtg ggcgccggcc tgggcgcggg cgtgaaccag cgcatggaca gttacgcgca 6480
catgaacggc tggagcaacg gcagctacag catgatgcag gaccagctgg gctacccgca 6540
gcacccgggc ctcaatgcgc acggcgcagc gcagatgcag cccatgcacc gctacgacgt 6600
gagcgccctg cagtacaact ccatgaccag ctcgcagacc tacatgaacg gctcgcccac 6660
ctacagcatg tcctactcgc agcagggcac ccctggcatg gctcttggct ccatgggttc 6720
ggtggtcaag tccgaggcca gctccagccc ccctgtggtt acctcttcct cccactccag 6780
ggcgccctgc caggccgggg acctccggga catgatcagc atgtatctcc ccggcgccga 6840
ggtgccggaa cccgccgccc ccagcagact tcacatgtcc cagcactacc agagcggccc 6900
ggtgcccggc acggccatta acggcacact gcccctctca cacatgtgag ggccggacag 6960
cgaactggag gggggagaaa ttttcaaaga aaaacgaggg aaatgggagg ggtgcaaaag 7020
aggagagtaa gaaacagcat ggagaaaacc cggtacgctc aaaaagaaaa aggaaaaaaa 7080
aaaatcccat cacccacagc aaatgacagc tgcaaaagag aacaccaatc ccatccacac 7140
tcacgcaaaa accgcgatgc cgacaagaaa acttttatga gagagatcct ggacttcttt 7200
ttgggggact atttttgtac agagaaaacc tggggagggt ggggagggcg ggggaatgga 7260
ccttgtatag atctggagga aagaaagcta cgaaaaactt tttaaaagtt ctagtggtac 7320
ggtaggagct ttgcaggaag tttgcaaaag tctttaccaa taatatttag agctagtctc 7380
caagcgacga aaaaaatgtt ttaatatttg caagcaactt ttgtacagta tttatcgaga 7440
taaacatggc aatcaaaatg tccattgttt ataagctgag aatttgccaa tatttttcaa 7500
ggagaggctt cttgctgaat tttgattctg cagctgaaat ttaggacagt tgcaaacgtg 7560
aaaagaagaa aattattcaa atttggacat tttaattgtt taaaaattgt acaaaaggaa 7620
aaaattagaa taagtactgg cgaaccatct ctgtggtctt gtttaaaaag ggcaaaagtt 7680
ttagactgta ctaaatttta taacttactg ttaaaagcaa aaatggccat gcaggttgac 7740
accgttggta atttataata gcttttgttc gatcccaact ttccattttg ttcagataaa 7800
aaaaaccatg aaattactgt gtttgaaata ttttcttatg gtttgtaata tttctgtaaa 7860
tttattgtga tattttaagg ttttcccccc tttattttcc gtagttgtat tttaaaagat 7920
tcggctctgt attatttgaa tcagtctgcc gagaatccat gtatatattt gaactaatat 7980
catccttata acaggtacat tttcaactta agtttttact ccattatgca cagtttgaga 8040
taaataaatt tttgaaatat ggacactgaa aaaaaaaaaa aaaaaacctc gactgtgcct 8100
tctagttgcc agccatctgt tgtttgcccc tcccccgtgc cttccttgac cctggaaggt 8160
gccactccca ctgtcctttc ctaataaaat gaggaaattg catcgcattg tctgagtagg 8220
tgtcattcta ttctgggggg tggggtgggg caggacagca agggggagga ttgggaagac 8280
aatagcaggc atgctgggga tgcggtgggc tctatgggac ggatcgggag atctcccgat 8340
cccctatggt gcactctcag tacaatctgc tctgatgccg catagttaag ccagtatctg 8400
ctccctgctt gtgtgttgga ggtcgctgag tagtgcgcga gcaaaattta agctacaaca 8460
aggcaaggct tgaccgacaa ttgcatgaag aatctgctta gggttaggcg ttttgcgctg 8520
cttcgcgatg tacgggccag atatacgcgt tgacattgat tattgactag ttattaatag 8580
taatcaatta cggggtcatt agttcatagc ccatatatgg agttccgcgt tacataactt 8640
acggtaaatg gcccgcctgg ctgaccgccc aacgaccccc gcccattgac gtcaataatg 8700
acgtatgttc ccatagtaac gccaataggg actttccatt gacgtcaatg ggtggagtat 8760
ttacggtaaa ctgcccactt ggcagtacat caagtgtatc atatgccaag tacgccccct 8820
attgacgtca atgacggtaa atggcccgcc tggcattatg cccagtacat gaccttatgg 8880
gactttccta cttggcagta catctacgta ttagtcatcg ctattaccat ggtgatgcgg 8940
ttttggcagt acatcaatgg gcgtggatag cggtttgact cacggggatt tccaagtctc 9000
caccccattg acgtcaatgg gagtttgttt tggaaccaaa atcaacggga ctttccaaaa 9060
tgtcgtaaca actccgcccc attgacgcaa atgggcggta ggcgtgtacg gtgggaggtc 9120
tatataagca gagctctccc tatcagtgat agagatctcc ctatcagtga tagagatcgt 9180
cgacgagctc gtttagtgaa ccgtcagatc gcctggagac gccatccacg ctgttttgac 9240
ctccatagaa gacaccggga ccgatccagc ctccggactc tagcgtttaa acttaagctt 9300
accatggttg tcatggggga ggtggtggcg cttggtggcc actggcggcc gaggtagagg 9360
cagtggcgct tgagttggtc gggggcagcg gcagatttga ggcttaagca acttcttccg 9420
gggaagagtg ccagtgcagc cactgttaca attcaagatc ttgatctata tccatagatt 9480
ggaatattgg tgggccagca atcctcagac gcctcactta ggacaaatga ggaaactgag 9540

```
gcttggtgaa gttacgaaac ttgtccaaaa tcacacaact tgtaaagggc acagccaaga 9600
ttcagagcca ggctgtaaaa attaaaatga acaaattacg gcaaagtttt aggagaaaga 9660
aggatgttta tgttccagag gccagtcgtc cacatcagtg gcagacagat gaagaaggcg 9720
ttcgcaccgg aaaatgtagc ttcccggtta agtaccttgg ccatgtagaa gttgatgaat 9780
caagaggaat gcacatctgt gaagatgctg taaaaagatt gaaagctgaa aggaagttct 9840
tcaaaggctt ctttggaaaa actggaaaga aagcagttaa agcagttctg tgggtctcag 9900
cagatggact cagagttgtg gatgaaaaaa ctaaggacct catagttgac cagacgatag 9960
agaaagtttc tttctgtgcc ccagacagga actttgatag agccttttct tacatatgcc 10020
gtgatggcac cactcgtcgc tggatctgtc actgcttcat ggctgtcaag gacacaggtg 10080
aaaggttgag ccatgcagta ggctgtgctt ttgcagcctg tttagagcgc aagcagaagc 10140
gggagaagga atgtggagtg actgctactt ttgatgctag tcggaccact tttacaagag 10200
aaggatcatt ccgtgtcaca acagccactg aacaagcaga aagagaggag atcatgaaac 10260
aaatgcaaga tgccaagaaa gctgaaacag ataagatagt cgttggttca tcagttgccc 10320
ctggcaacac tgccccatcc ccatcctctc ccacctctcc tacttctgat gccacgacct 10380
ctctggagat gaacaatcct catgccatcc cacgccggca tgctccaatt gaacagcttg 10440
ctcgccaagg ctctttccga ggttttcctg ctcttagcca gaagatgtca ccctttaaac 10500
gccaactatc cctacgcatc aatgagttgc cttccactat gcagaggaag actgatttcc 10560
ccattaaaaa tgcagtgcca gaagtagaag gggaggcaga gagcatcagc tccctgtgct 10620
cacagatcac caatgccttc agcacacctg aggacccctt ctcatctgct ccgatgacca 10680
aaccagtgac agtggtggca ccacaatctc ctaccttcca agctaatggc actgactcag 10740
ccttccatgt gcttgctaag ccagcccata ctgctctagc acccgtagca atgcctgtgc 10800
gtgaaaccaa cccttgggcc catgcccctg atgctgctaa caaggaaatt gcagccacat 10860
gttcggggac cgagtggggt caatcttctg gtgctgcctc tccaggtctc ttccaggccg 10920
gtcatagacg tactccctct gaggccgacc gatggttaga agaggtgtct aagagcgtcc 10980
gggctcagca gccccaggcc tcagctgctc ctctgcagcc agttctccag cctcctccac 11040
ccactgccat ctcccagcca gcatcacctt tccaagggaa tgcattcctc acctctcagc 11100
ctgtgccagt gggtgtggtc ccagccctgc aaccagcctt tgtccctgcc cagtcctatc 11160
ctgtggccaa tggaatgccc tatccagccc ctaatgtgcc tgtggtgggc atcactccct 11220
cccagatggt ggccaacgta tttggcactg caggccaccc tcaggctgcc catccccatc 11280
agtcacccag cctggtcagg cagcagacat tccctcacta cgaggcaagc agtgctacca 11340
ccagtccctt ctttaagcct cctgctcagc acctcaacgg ttctgcagct ttcaatggtg 11400
tagatgatgg caggttggcc tcagcagaca ggcatacaga ggttcctaca ggcacctgcc 11460
cagtggatcc ttttgaagcc cagtgggctg cattagaaaa taagtccaag cagcgtacta 11520
atccctcccc taccaaccct ttctccagtg acttacagaa gacgtttgaa attgaacttt 11580
aagcaatcat tatggctatg tatcttgtcc ataccagaca gggagcaggg ggtagcggtc 11640
aaaggagcaa aacagacttt gtctcctgat tagtactctt ttcactaatc ccaaaggtcc 11700
caaggaacaa gtccaggccc agagtactgt gaggggtgat tttgaaagac atgggaaaaa 11760
gcattcctag agaaaagctg ccttgcaatt aggctaaaga agtcaaggaa atgttgcttt 11820
ctgtactccc tcttccctta cccccttaca aatctctggc aacagagagg caaagtatct 11880
gaacaagaat ctatattcca agcacattta ctgaaatgta aaacacaaca ggaagcaaag 11940
caatctccct ttgtttttca ggccattcac ctgcctcctg tcagtagtgg cctgtattag 12000
agatcaagaa gagtggtttg tgctcaggct ggggaacaga gaggcacgct atgctgccag 12060
aattcccagg agggcatatc agcaactgcc cagcagagct atattttggg ggagaagttg 12120
agcttccatt ttgagtaaca gaataaatat tatatatatc aaaagccaaa atctttattt 12180
ttatgcattt agaatatttt aaatagttct cagatattaa gaagttgtat gagttgtaag 12240
taatcttgcc aaaggtaaag gggctagttg taagaaattg tacataagat tgatttatca 12300
ttgatgccta ctgaaataaa aagaggaaag gctggaagct gcagacagga tccctagctt 12360
gttttctgtc agtcattcat tgtaagtagc acattgcaac aacaatcatg cttatgacca 12420
atacagtcac taggttgtag ttttttttaa ataaaggaaa agcagtattg tcctggtttt 12480
aaacctatga tggaattcta atgtcattat tttaatggaa tcaatcgaaa tatgctctat 12540
agagaatata tcttttatat attgctgcag tttccttatg ttaatccttt aacactaagg 12600
taacatgaca taatcatacc atagaaggga acacaggtta ccatattggt ttgtaatatg 12660
ggtcttggtg ggttttgttt tatcctttaa attttgttcc catgagtttt gtggggatgg 12720
ggattctggt tttattagct ttgtgtgtgt cctcttcccc caaacccccct tttggtgaga 12780
acatcccctt gacagttgca gcctcttgac ctcggataac aataagagag ctcatctcat 12840
ttttactttt gaacgttggc cttacaatca aatgtaagtt atatatattt gtactgatga 12900
aaatttataa tctgctttaa caaaaataaa tgttcatggt agaagctttt aaaaaaaaaa 12960
aaacctcgac tgtgccttct agttgccagc catctgttgt ttgcccctcc cccgtgcctt 13020
```

ccttgaccct ggaaggtgcc actcccactg tcctttccta ataaaatgag gaaattgcat 13080
cgcattgtct gagtaggtgt cattctattc tggggggtgg ggtggggcag gacagcaagg 13140
gggaggattg ggaagacaat agcaggcatg ctggggatgc ggtgggctct atgggacgga 13200
tcgggagatc tcccgatccc ctatggtgca ctctcagtac aatctgctct gatgccgcat 13260
agttaagcca gtatctgctc cctgcttgtg tgttggaggt cgctgagtag tgcgcgagca 13320
aaatttaagc tacaacaagg caaggcttga ccgacaattg catgaagaat ctgcttaggg 13380
ttaggcgttt tgcgctgctt cgcgatgtac gggccagata tacgcgttga cattgattat 13440
tgactagtta ttaatagtaa tcaattacgg ggtcattagt tcatagccca tatatggagt 13500
tccgcgttac ataacttacg gtaaatggcc cgcctggctg accgcccaac gacccccgcc 13560
cattgacgtc aataatgacg tatgttccca tagtaacgcc aatagggact ttccattgac 13620
gtcaatgggt ggagtattta cggtaaactg cccacttggc agtacatcaa gtgtatcata 13680
tgccaagtac gccccctatt gacgtcaatg acggtaaatg gcccgcctgg cattatgccc 13740
agtacatgac cttatgggac tttcctactt ggcagtacat ctacgtatta gtcatcgcta 13800
ttaccatggt gatgcggttt tggcagtaca tcaatgggcg tggatagcgg tttgactcac 13860
ggggatttcc aagtctccac cccattgacg tcaatgggag tttgttttgg aaccaaaatc 13920
aacgggactt tccaaaatgt cgtaacaact ccgccccatt gacgcaaatg ggcggtaggc 13980
gtgtacggtg ggaggtctat ataagcagag ctctccctat cagtgataga gatctcccta 14040
tcagtgatag agatcgtcga cgagctcgtt tagtgaaccg tcagatcgcc tggagacgcc 14100
atccacgctg ttttgacctc catagaagac accgggaccg atccagcctc cggactctag 14160
cgtttaaact taagcttacc atggtgagca agggcgagga gctgttcacc ggggtggtgc 14220
ccatcctggt cgagctggac ggcgacgtaa acggccacaa gttcagcgtg tccggcgagg 14280
gcgagggcga tgccacctac ggcaagctga ccctgaagtt catctgcacc accggcaagc 14340
tgcccgtgcc ctggcccacc ctcgtgacca ccttcaccta cggcgtgcag tgcttcgccc 14400
gctaccccga ccacatgaag cagcacgact tcttcaagtc cgccatgccc gaaggctacg 14460
tccaggagcg caccatcttc ttcaaggacg acggcaacta caagacccgc gccgaggtga 14520
agttcgaggg cgacaccctg gtgaaccgca tcgagctgaa gggcatcgac ttcaaggagg 14580
acggcaacat cctggggcac aagctggagt acaactacaa cagccacaag gtctatatca 14640
ccgccgacaa gcagaagaac ggcatcaagg tgaacttcaa gacccgccac aacatcgagg 14700
acggcagcgt gcagctcgcc gaccactacc agcagaacac ccccatcggc gacggccccg 14760
tgctgctgcc cgacaaccac tacctgagca cccagtccgc cctgagcaaa gaccccaacg 14820
agaagcgcga tcacatggtc ctgctggagt tcgtgaccgc cgccgggatc actctcggca 14880
tggacgagct gtacaagtaa cctcgactgt gccttctagt tgccagccat ctgttgtttg 14940
cccctccccc gtgccttcct tgaccctgga aggtgccact cccactgtcc tttcctaata 15000
aaatgaggaa attgcatcgc attgtctgag taggtgtcat tctattctgg ggggtggggt 15060
ggggcaggac agcaaggggg aggattggga agacaatagc aggcatgctg gggatgcggt 15120
gggctctatg ggacggatcg ggagatctcc cgatccccta tggtgcactc tcagtacaat 15180
ctgctctgat gccgcatagt taagccagta tctgctccct gcttgtgtgt tggaggtcgc 15240
tgagtagtgc gcgagcaaaa tttaagctac aacaaggcaa ggcttgaccg acaattgcat 15300
gaagaatctg cttagggtta ggcgttttgc gctgcttcgc gatgtacggg ccagatatac 15360
gcgttgacat tgattattga ctagttatta atagtaatca attacggggt cattagttca 15420
tagcccatat atggagttcc gcgttacata acttacggta aatggcccgc ctggctgacc 15480
gcccaacgac ccccgcccat tgacgtcaat aatgacgtat gttcccatag taacgccaat 15540
agggactttc cattgacgtc aatgggtgga gtatttacgg taaactgccc acttggcagt 15600
acatcaagtg tatcatatgc caagtacgcc ccctattgac gtcaatgacg gtaaatggcc 15660
cgcctggcat tatgcccagt acatgacctt atgggacttt cctacttggc agtacatcta 15720
cgtattagtc atcgctatta ccatggtgat gcggttttgg cagtacatca atgggcgtgg 15780
atagcggttt gactcacggg gatttccaag tctccacccc attgacgtca atgggagttt 15840
gttttggaac caaaatcaac gggactttcc aaaatgtcgt aacaactccg ccccattgac 15900
gcaaatgggc ggtaggcgtg tacggtggga ggtctatata agcagagctc gtgagtttgg 15960
ggacccttga ttgttctttc tttttcgcta ttgtaaaatt catgttatat ggagggggca 16020
aagttttcag ggtgttgttt agaatgggaa gatgtccctt gtatcaccat ggaccctcat 16080
gataattttg tttctttcac tttctactct gttgacaacc attgtctcct cttattttct 16140
tttcattttc tgtaactttt tcgttaaact ttagcttgca tttgtaacga atttttaaat 16200
tcacttttgt ttatttgtca gattgtaagt actttctcta atcacttttt tttcaaggca 16260
atcagggtat attatattgt acttcagcac agttttagag aacaattgtt ataattaaat 16320
gataaggtag aatatttctg catataaatt ctggctggcg tggaaatatt cttattggta 16380
gaaacaacta catcctggtc atcatcctgc ctttctcttt atggttacaa tgatatacac 16440
tgtttgagat gaggataaaa tactctgagt ccaaaccggg cccctctgct aaccatgttc 16500

atgccttctt ctttttccta cagctcctgg gcaacgtgct ggttattgtg ctgtctcatc 16560
attttggcaa agaattgtaa tacgactcac tatagggcga attgatatgt ctagattaga 16620
taaaagtaaa gtgattaaca gcgcattaga gctgcatgtc tagattagat aaaagtaaag 16680
tgattaacag cgcattagag ctgcttaatg aggtcggaat cgaaggttta acaacccgta 16740
aactcgccca gaagctaggt gtagagcagc ctacattgta ttggcatgta aaaaataagc 16800
gggctttgct cgacgcctta gccattgaga tgttagatag gcaccatact cacttttgcc 16860
ctttagaagg ggaaagctgg caagattttt tacgtaataa cgctaaaagt tttagatgtg 16920
ctttactaag tcatcgcgat ggagcaaaag tacatttagg tacacggcct acagaaaaac 16980
agtatgaaac tctcgaaaat caattagcct ttttatgcca acaaggtttt tcactagaga 17040
atgcattata tgcactcagc gctgtggggc attttacttt aggttgcgta ttggaagatc 17100
aagagcatca agtcgctaaa gaagaaaggg aaacacctac tactgatagt atgccgccat 17160
tattacgaca agctatcgaa ttatttgatc accaaggtgc agagccagcc ttcttattcg 17220
gccttgaatt gatcatatgc ggattagaaa aacaacttaa atgtgaaagt gggtccgcgt 17280
acagcggatc ccgggaattc agatcttatt aaagcagaac ttgtttattg cagcttataa 17340
tggttacaaa taaagcaata gcatcacaaa tttcacaaat aaagcatttt tttcactgca 17400
ttctagttgt ggtttgtcca aactcatcaa tgtatcttat catgtctggt caatgtgtgt 17460
cagttagggt gtggaaagtc cccaggctcc ccagcaggca gaagtatgca aagcatgcat 17520
ctcaattagt cagcaaccag gtgtggaaag tccccaggct ccccagcagg cagaagtatg 17580
caaagcatgc atctcaatta gtcagcaacc atagtcccgc ccctaactcc gcccatcccg 17640
cccctaactc cgcccagttc cgcccattct ccgccccatg gctgactaat tttttttatt 17700
tatgcagagg ccgaggccgc ctctgcctct gagctattcc agaagtagtg aggaggcttt 17760
tttggaggcc taggcttttg caaaaagctc cccatagtga ctggatatgt tgtgttttac 17820
agtattatgt agtctgtttt ttatgcaaaa tctaatttaa tatattgata tttatatcat 17880
tttacgtttc tcgttcagct ttcttgtaca aagtggttga tatccagcac agtggcggcc 17940
gctcgagtct agagggcccg cggttcgaag gtaagcctat ccctaaccct ctcctcggtc 18000
tcgattctac gcgtaccggt tagtaatgag tttggaatta attctgtgga atgtgtgtca 18060
gttagggtgt ggaaagtccc caggctcccc agcaggcaga agtatgcaaa gcatgcatct 18120
caattagtca gcaaccaggt gtggaaagtc cccaggctcc ccagcaggca gaagtatgca 18180
aagcatgcat ctcaattagt cagcaaccat agtcccgccc ctaactccgc ccatcccgcc 18240
cctaactccg cccagttccg cccattctcc gccccatggc tgactaattt tttttattta 18300
tgcagaggcc gaggccgcct ctgcctctga gctattccag aagtagtgag gaggcttttt 18360
tggaggccta ggcttttgca aaaagctccc gggagcttgt atatccattt tcggatctga 18420
tcagcacgtg ttgacaatta atcatcggca tagtatatcg gcatagtata atacgacaag 18480
gtgaggaact aaaccatggc caagcctttg tctcaagaag aatccaccct cattgaaaga 18540
gcaacggcta caatcaacag catccccatc tctgaagact acagcgtcgc cagcgcagct 18600
ctctctagcg acggccgcat cttcactggt gtcaatgtat atcattttac tgggggacct 18660
tgtgcagaac tcgtggtgct gggcactgct gctgctgcgg cagctggcaa cctgacttgt 18720
atcgtcgcga tcggaaatga gaacaggggc atcttgagcc cctgcggacg gtgccgacag 18780
gtgcttctcg atctgcatcc tgggatcaaa gccatagtga aggacagtga tggacagccg 18840
acggcagttg ggattcgtga attgctgccc tctggttatg tgtgggaggg ctaagcacaa 18900
ttcgagctcg gtacctttaa gaccaatgac ttacaaggca gctgtagatc ttagccactt 18960
tttaaaagaa aaggggggac tggaagggct aattcactcc caacgaagac aagatctgct 19020
ttttgcttgt actgggtctc tctggttaga ccagatctga gcctgggagc tctctggcta 19080
actagggaac ccactgctta agcctcaata aagcttgcct tgagtgcttc aagtagtgtg 19140
tgcccgtctg ttgtgtgact ctggtaacta gagatccctc agaccctttt agtcagtgtg 19200
gaaaatctct agcagtagta gttcatgtca tcttattatt cagtatttat aacttgcaaa 19260
gaaatgaata tcagagagtg agaggaactt gtttattgca gcttataatg gttacaaata 19320
aagcaatagc atcacaaatt tcacaaataa agcatttttt tcactgcatt ctagttgtgg 19380
tttgtccaaa ctcatcaatg tatcttatca tgtctggctc tagctatccc gcccctaact 19440
ccgcccatcc cgcccctaac tccgcccagt tccgcccatt ctccgcccca tggctgacta 19500
atttttttta tttatgcaga ggccgaggcc gcctcggcct ctgagctatt ccagaagtag 19560
tgaggaggct tttttggagg cctagggacg tacccaattc gccctatagt gagtcgtatt 19620
acgcgcgctc actggccgtc gttttacaac gtcgtgactg ggaaaaccct ggcgttaccc 19680
aacttaatcg ccttgcagca catccccctt tcgccagctg gcgtaatagc gaagaggccc 19740
gcaccgatcg cccttcccaa cagttgcgca gcctgaatgg cgaatgggac gcgccctgta 19800
gcggcgcatt aagcgcggcg ggtgtggtgg ttacgcgcag cgtgaccgct acacttgcca 19860
gcgccctagc gcccgctcct ttcgctttct tcccttcctt tctcgccacg ttcgccggct 19920
ttccccgtca agctctaaat cgggggctcc ctttagggtt ccgatttagt gctttacggc 19980

acctcgaccc caaaaaactt gattagggtg atggttcacg tagtgggcca tcgccctgat 20040
agacggtttt tcgccctttg acgttggagt ccacgttctt taatagtgga ctcttgttcc 20100
aaactggaac aacactcaac cctatctcgg tctattcttt tgatttataa gggattttgc 20160
cgatttcggc ctattggtta aaaaatgagc tgatttaaca aaaatttaac gcgaatttta 20220
acaaaatatt aacgcttaca atttaggtgg cacttttcgg ggaaatgtgc gcggaacccc 20280
tatttgttta tttttctaaa tacattcaaa tatgtatccg ctcatgagac aataaccctg 20340
ataaatgctt caataatatt gaaaaaggaa gagtatgagt attcaacatt tccgtgtcgc 20400
ccttattccc ttttttgcgg cattttgcct tcctgttttt gctcacccag aaacgctggt 20460
gaaagtaaaa gatgctgaag atcagttggg tgcacgagtg ggttacatcg aactggatct 20520
caacagcggt aagatccttg agagttttcg ccccgaagaa cgttttccaa tgatgagcac 20580
ttttaaagtt ctgctatgtg gcgcggtatt atcccgtatt gacgccgggc aagagcaact 20640
cggtcgccgc atacactatt ctcagaatga cttggttgag tactcaccag tcacagaaaa 20700
gcatcttacg gatggcatga cagtaagaga attatgcagt gctgccataa ccatgagtga 20760
taacactgcg gccaacttac ttctgacaac gatcggagga ccgaaggagc taaccgcttt 20820
tttgcacaac atgggggatc atgtaactcg ccttgatcgt tgggaaccgg agctgaatga 20880
agccatacca aacgacgagc gtgacaccac gatgcctgta gcaatggcaa caacgttgcg 20940
caaactatta actggcgaac tacttactct agcttcccgg caacaattaa tagactggat 21000
ggaggcggat aaagttgcag gaccacttct gcgctcggcc cttccggctg gctggtttat 21060
tgctgataaa tctggagccg gtgagcgtgg gtctcgcggt atcattgcag cactggggcc 21120
agatggtaag ccctcccgta tcgtagttat ctacacgacg gggagtcagg caactatgga 21180
tgaacgaaat agacagatcg ctgagatagg tgcctcactg attaagcatt ggtaactgtc 21240
agaccaagtt tactcatata tactttagat tgatttaaaa cttcattttt aatttaaaag 21300
gatctaggtg aagatccttt ttgataatct catgaccaaa atcccttaac gtgagttttc 21360
gttccactga gcgtcagacc ccgtagaaaa gatcaaagga tcttcttgag atcctttttt 21420
tctgcgcgta atctgctgct tgcaaacaaa aaaaccaccg ctaccagcgg tggtttgttt 21480
gccggatcaa gagctaccaa ctctttttcc gaaggtaact ggcttcagca gagcgcagat 21540
accaaatact gttcttctag tgtagccgta gttaggccac cacttcaaga actctgtagc 21600
accgcctaca tacctcgctc tgctaatcct gttaccagtg gctgctgcca gtggcgataa 21660
gtcgtgtctt accgggttgg actcaagacg atagttaccg gataaggcgc agcggtcggg 21720
ctgaacgggg ggttcgtgca cacagcccag cttggagcga acgacctaca ccgaactgag 21780
atacctacag cgtgagctat gagaaagcgc cacgcttccc gaagggagaa aggcggacag 21840
gtatccggta agcggcaggg tcggaacagg agagcgcacg agggagcttc cagggggaaa 21900
cgcctggtat ctttatagtc ctgtcgggtt tcgccacctc tgacttgagc gtcgattttt 21960
gtgatgctcg tcaggggggc ggagcctatg gaaaaacgcc agcaacgcgg cctttttacg 22020
gttcctggcc ttttgctggc cttttgctca catgttcttt cctgcgttat cccctgattc 22080
tgtggataac cgtattaccg cctttgagtg agctgatacc gctcgccgca gccgaacgac 22140
cgagcgcagc gagtcagtga gcgaggaagc ggaagagcgc ccaatacgca aaccgcctct 22200
ccccgcgcgt tggccgattc attaatgcag ctggcacgac aggtttcccg actggaaagc 22260
gggcagtgag cgcaacgcaa ttaatgtgag ttagctcact cattaggcac cccaggcttt 22320
acactttatg cttccggctc gtatgttgtg tggaattgtg agcggataac aatttcacac 22380
aggaaacagc tatgaccatg attacgccaa gcgcgcaatt aaccctcact aaagggaaca 22440
aaagctggag ctgcaagctt 22460

<210> 3
<211> 1751
<212> DNA
<213> Artificial Sequence

<220>
<223> Description of Artificial Sequence: Synthetic polynucleotide

<400> 3
gtcgaccagt ggatcctgga ggcttgctga aggctgtatg ctgatcgggt gtaaactgag 60
cttggttttg gccactgact gaccaagctc attacacccg atcaggacac aaggcctgtt 120
actagcactc acatggaaca aatggcccag atcctggagg cttgctgaag gctgtatgct 180
gataccaggc aggataaggc cagttttggc cactgactga ctggccttac tgcctggtat 240

caggacacaa ggcctgttac tagcactcac atggaacaaa tggcccagat cctggaggct 300
tgctgaaggc tgtatgctgt gaccaggatg accaatccat gttttggcca ctgactgaca 360
tggattgcat cctggtcaca ggacacaagg cctgttacta gcactcacat ggaacaaatg 420
gcccagatcc tggaggcttg ctgaaggctg tatgctgata gcttggtcca acctgttagt 480
tttggccact gactgactaa caggtgacca agctatcagg acacaaggcc tgttactagc 540
actcacatgg aacaaatggc ccagatctcc ccagtggaaa gacgcgcagg caaaacgcac 600
cacgtgacgg agcgtgaccg cgcgccgagc gcgcgccaag gtcgggcagg aagagggcct 660
atttcccatg attccttcat atttgcatat acgatacaag gctgttagag agataattag 720
aattaatttg actgtaaaca caaagatatt agtacaaaat acgtgacgta gaaagtaata 780
atttcttggg tagtttgcag ttttaaaatt atgttttaaa atggactatc atatgcttac 840
cgtaacttga aagtatttcg atttcttggg tttatatatc ttgtggaaag gacggtgctc 900
gcttcggcag cacgtcgtgc tagggttctt gggtttctc gcaacagcag gttctgcaat 960
gggcgcggcg tccctgaccg tgtcggctca gtcccggact ttactggccg ggatagtgca 1020
gcaacagcaa cagctgttgg acgtggtcaa gagacaacaa gaactgttgc gactgaccgt 1080
ctggggaacg aaaaacctcc aggcaagagt cactgctata gagaagtacc tacaggacca 1140
ggcgcggcta aattcatggg gatgtctaga cctagagcgg acttcggtcc gctttttccc 1200
cagtggaaag acgcgcaggc aaaacgcacc acgtgacgga gcgtgaccgc gcgccgagcg 1260
cgcgccaagg tcgggcagga agagggccta tttcccatga ttccttcata tttgcatata 1320
cgatacaagg ctgttagaga gataattaga attaatttga ctgtaaacac aaagatatta 1380
gtacaaaata cgtgacgtag aaagtaataa tttcttgggt agtttgcagt tttaaaatta 1440
tgttttaaaa tggactatca tatgcttacc gtaacttgaa agtatttcga tttcttgggt 1500
ttatatatct tgtggaaagg acggtgctcg cttcggcagc acgtcggtcg ctctgcggag 1560
aggctggcag attgagccct gggaggttct ctccagcact agcaggtaga gcctgggtgt 1620
tccctgctag actctcacca gtgcttggcc ggcactgggc agacggctcc acgcttgctt 1680
gcttaaagac ctcttaataa agctgctcta gacctagagc ggacttcggt ccgctttttt 1740
acgtactcga g 1751

<210> 4
<211> 64
<212> DNA
<213> Artificial Sequence

<220>
<223> Description of Artificial Sequence: Synthetic oligonucleotide

<400> 4
tgctgatacc aggcaggata aggccagttt tggccactga ctgactggcc ttactgcctg 60
gtat 64

<210> 5
<211> 64
<212> DNA
<213> Artificial Sequence

<220>
<223> Description of Artificial Sequence: Synthetic oligonucleotide

<400> 5
cctgatacca ggcagtaagg ccagtcagtc agtggccaaa actggcctta tcctgcctgg 60
tatc 64

<210> 6
<211> 64
<212> DNA
<213> Artificial Sequence

<220>
<223> Description of Artificial Sequence: Synthetic
oligonucleotide

<400> 6
tgctgtgacc aggatgacca atccatgttt tggccactga ctgacatgga ttgcatcctg 60
gtca 64

<210> 7
<211> 64
<212> DNA
<213> Artificial Sequence

<220>
<223> Description of Artificial Sequence: Synthetic
oligonucleotide

<400> 7
cctgtgacca ggatgcaatc catgtcagtc agtggccaaa acatggattg gtcatcctgg 60
tcac 64

<210> 8
<211> 64
<212> DNA
<213> Artificial Sequence

<220>
<223> Description of Artificial Sequence: Synthetic
oligonucleotide

<400> 8
tgctgatcgg gtgtaaactg agcttggttt tggccactga ctgaccaagc tcattacacc 60
cgat 64

<210> 9
<211> 64
<212> DNA
<213> Artificial Sequence

<220>
<223> Description of Artificial Sequence: Synthetic
oligonucleotide

<400> 9
cctgatcggg tgtaatgagc ttggtcagtc agtggccaaa accaagctca gtttacaccc 60
gatc 64

<210> 10
<211> 64
<212> DNA
<213> Artificial Sequence

<220>
<223> Description of Artificial Sequence: Synthetic
oligonucleotide

<400> 10
tgctgatagc ttggtccaac ctgttagttt tggccactga ctgactaaca ggtgaccaag 60
ctat 64

<210> 11
<211> 64
<212> DNA
<213> Artificial Sequence

<220>
<223> Description of Artificial Sequence: Synthetic
oligonucleotide

<400> 11
cctgatagct tggtcacctg ttagtcagtc agtggccaaa actaacaggt tggaccaagc 60
tatc 64

<210> 12
<211> 160
<212> DNA
<213> Artificial Sequence

<220>
<223> Description of Artificial Sequence: Synthetic
polynucleotide

<400> 12
gtcgctctgc ggagaggctg gcagattgag ccctgggagg ttctctccag cactagcagg 60
tagagcctgg gtgttccctg ctagactctc accagtgctt ggccggcact gggcagacgg 120
ctccacgctt gcttgcttaa agacctctta ataaagctgc 160

<210> 13
<211> 247
<212> DNA
<213> Artificial Sequence

<220>
<223> Description of Artificial Sequence: Synthetic
polynucleotide

<400> 13
tgctagggtt cttgggtttt ctcgcaacag caggttctgc aatgggcgcg gcgtccctga 60
ccgtgtcggc tcagtcccgg actttactgg ccgggatagt gcagcaacag caacagctgt 120
tggacgtggt caagagacaa caagaactgt tgcgactgac cgtctgggga acgaaaaacc 180
tccaggcaag agtcactgct atagagaagt acctacagga ccaggcgcgg ctaaattcat 240
ggggatg 247

<210> 14
<211> 25
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 14
gugcucgcuu cggcagcacg tcgac 25

<210> 15
<211> 26
<212> RNA
<213> Artificial Sequence

<220>
<223> Description of Artificial Sequence: Synthetic olgionucleotide

<400> 15
ucuagagcgg acuucggucc gcuuuu 26

<210> 16
<211> 25
<212> DNA
<213> Artificial Sequence

<220>
<223> Description of Combined DNA/RNA Molecule: Synthetic oligonucleotide

<220>
<223> Description of Artificial Sequence: Synthetic oligonucleotide

<400> 16
gugcucgcuu cggcagcacg tcgac 25

<210> 17
<211> 26
<212> RNA
<213> Artificial Sequence

<220>
<223> Description of Artificial Sequence: Synthetic oligonucleotide

<400> 17
ucuagagcgg acuucggucc gcuuuu 26

<210> 18
<211> 606
<212> DNA
<213> Homo sapiens

<400> 18
atgagccgga gggagggaag tctggaagac ccccagactg attcctcagt ctcacttctt 60
ccccacttgg aggccaagat ccgtcagaca cacagccttg cgcacctcct caccaaatac 120
gctgagcagc tgctccagga atatgtgcag ctccagggag acccccttcgg gctgcccagc 180
ttctcgccgc cgcggctgcc ggtggccggc ctgagcgccc cggctccgag ccacgcgggg 240
ctgccagtgc acgagcggct gcggctggac gcggcggcgc tggccgcgct gcccccgctg 300
ctggacgcag tgtgtcgccg ccaggccgag ctgaacccgc gcgcgccgcg cctgctgcgc 360
cgcctggagg acgcggcgcg ccaggcccgg gccctgggcg ccgccgtgga ggccttgctg 420
gccgcgctgg gcgccgccaa ccgcgggccc cgggccgagc cccccgccgc caccgcctca 480
gccgcctccg ccaccggggt cttccccgcc aaggtgctgg ggctccgcgt ttgcggcctc 540
taccgcgagt ggctgagccg caccgagggc gacctgggcc agctgctgcc cgggggctcg 600
gcctga 606

<210> 19
<211> 603
<212> DNA
<213> Homo sapiens

<400> 19
atggctttca cagagcattc accgctgacc cctcaccgtc gggacctctg tagccgctct 60
atctggctag caaggaagat tcgttcagac ctgactgctc ttacggaatc ctatgtgaag 120
catcagggcc tgaacaagaa catcaacctg gactctgcgg atggatgcc agtggcaagc 180
actgatcagt ggagtgagct gaccgaggca gagcgactcc aagagaacct tcaagcttat 240
cgtaccttcc atgttttgtt ggccaggctc ttagaagacc agcaggtgca ttttacccca 300
accgaaggtg acttccatca agctatacat acccttcttc tccaagtcgc tgcctttgca 360
taccagatag aggagttaat gatactcctg gaatacaaga tcccccgcaa tgaggctgat 420
gggatgccta ttaatgttgg agatggtggt ctctttgaga agaagctgtg ggcctaaag 480
gtgctgcagg agctttcaca gtggacagta aggtccatcc atgaccttcg tttcatttct 540
tctcatcaga ctgggatccc agcacgtggg agccattata ttgctaacaa caagaaaatg 600
tag 603

<210> 20
<211> 990
<212> DNA
<213> Homo sapiens

<400> 20
atgttgacgt tgcagacttg gctagtgcaa gccttgttta ttttcctcac cactgaatct 60
acaggtgaac ttctagatcc atgtggttat atcagtcctg aatctccagt tgtacaactt 120
cattctaatt tcactgcagt ttgtgtgcta aaggaaaaat gtatggatta ttttcatgta 180
aatgctaatt acattgtctg gaaaacaaac cattttacta ttcctaagga gcaatatact 240
atcataaaca gaacagcatc cagtgtcacc tttacagata tagcttcatt aaatattcag 300
ctcacttgca acattcttac attcggacag cttgaacaga atgtttatgg aatcacaata 360
atttcaggct tgcctccaga aaaacctaaa aatttgagtt gcattgtgaa cgaggggaag 420
aaaatgaggt gtgagtggga tggtggaagg gaaacacact tggagacaaa cttcacttta 480
aaatctgaat gggcaacaca caagtttgct gattgcaaag caaaacgtga cacccccacc 540
tcatgcactg ttgattattc tactgtgtat tttgtcaaca ttgaagtctg ggtagaagca 600

```
gagaatgccc ttgggaaggt tacatcagat catatcaatt ttgatcctgt atataaagtg 660
aagcccaatc cgccacataa tttatcagtg atcaactcag aggaactgtc tagtatctta 720
aaattgacat ggaccaaccc aagtattaag agtgttataa tactaaaata taacattcaa 780
tataggacca aagatgcctc aacttggagc cagattcctc ctgaagacac agcatccacc 840
cgatcttcat tcactgtcca agaccttaaa ccttttacag aatatgtgtt taggattcgc 900
tgtatgaagg aagatggtaa gggatactgg agtgactgga gtgaagaagc aagtgggatc 960
acctatgaag ataacattgc ctcctttga                                   990


<210> 21
<211> 639
<212> DNA
<213> Homo sapiens

<400> 21
atgaactcct tctccacaag cgccttcggt ccagttgcct tctccctggg gctgctcctg 60
gtgttgcctg ctgccttccc tgccccagta cccccaggag aagattccaa agatgtagcc 120
gccccacaca gacagccact cacctcttca gaacgaattg acaaacaaat tcggtacatc 180
ctcgacggca tctcagccct gagaaaggag acatgtaaca agagtaacat gtgtgaaagc 240
agcaaagagg cactggcaga aaacaacctg aaccttccaa agatggctga aaaagatgga 300
tgcttccaat ctggattcaa tgaggagact tgcctggtga aaatcatcac tggtcttttg 360
gagtttgagg tatacctaga gtacctccag aacagatttg agagtagtga ggaacaagcc 420
agagctgtgc agatgagtac aaaagtcctg atccagttcc tgcagaaaaa ggcaaagaat 480
ctagatgcaa taaccacccc tgacccaacc acaaatgcca gcctgctgac gaagctgcag 540
gcacagaacc agtggctgca ggacatgaca actcatctca ttctgcgcag ctttaaggag 600
ttcctgcagt ccagcctgag ggctcttcgg caaatgtag                         639


<210> 22
<211> 756
<212> DNA
<213> Homo sapiens

<400> 22
atggctatga gttctttttt gatcaactca aactatgtcg accccaagtt ccctccatgc 60
gaggaatatt cacagagcga ttacctaccc agcgaccact cgcccgggta ctacgccggc 120
ggccagaggc gagagagcag cttccagccg gaggcgggct tcgggcggcg cgcggcgtgc 180
accgtgcagc gctacgcggc ctgccgggac cctggccccc cgccgcctcc gccaccaccc 240
ccgccgcccc cgccaccgcc cggtctgtcc cctcgggctc ctgcgccgcc acccgccggg 300
gccctcctcc cggagcccgg ccagcgctgc gaggcggtca gcagcagccc cccgccgcct 360
ccctgcgccc agaacccccct gcaccccagc ccgtcccact ccgcgtgcaa agagcccgtc 420
gtctacccct ggatgcgcaa agttcacgtg agcacggtaa accccaatta cgccggcggg 480
gagcccaagc gctctcggac cgcctacacg cgccagcagg tcttggagct ggagaaggaa 540
tttcactaca accgctacct gacacggcgc cggagggtgg agatcgccca cgcgctctgc 600
ctctccgagc gccagatcaa gatctggttc cagaaccggc gcatgaagtg gaaaaaagac 660
cacaagttgc ccaacaccaa gatccgctcg ggtggtgcgg caggctcagc cggagggccc 720
cctggccggc ccaatggagg cccccgcgcg ctctag                            756


<210> 23
<211> 1407
<212> DNA
<213> Homo sapiens

<400> 23
atgctggccg tcggctgcgc gctgctggct gccctgctgg ccgcgccggg agcggcgctg 60
gccccaaggc gctgccctgc gcaggaggtg gcgagaggcg tgctgaccag tctgccagga 120
gacagcgtga ctctgacctg cccgggggta gagccggaag acaatgccac tgttcactgg 180
```

```
gtgctcagga agccggctgc aggctcccac cccagcagat gggctggcat gggaaggagg 240
ctgctgctga ggtcggtgca gctccacgac tctggaaact attcatgcta ccgggccggc 300
cgcccagctg ggactgtgca cttgctggtg gatgttcccc ccgaggagcc ccagctctcc 360
tgcttccgga agagcccccct cagcaatgtt gtttgtgagt ggggtcctcg gagcacccca 420
tccctgacga caaaggctgt gctcttggtg aggaagtttc agaacagtcc ggccgaagac 480
ttccaggagc cgtgccagta ttcccaggag tcccagaagt tctcctgcca gttagcagtc 540
ccggagggag acagctcttt ctacatagtg tccatgtgcg tcgccagtag tgtcgggagc 600
aagttcagca aaactcaaac ctttcagggt tgtggaatct tgcagcctga tccgcctgcc 660
aacatcacag tcactgccgt ggccagaaac ccccgctggc tcagtgtcac ctggcaagac 720
ccccactcct ggaactcatc tttctacaga ctacggtttg agctcagata tcgggctgaa 780
cggtcaaaga cattcacaac atggatggtc aaggacctcc agcatcactg tgtcatccac 840
gacgcctgga gcggcctgag gcacgtggtg cagcttcgtg cccaggagga gttcgggcaa 900
ggcgagtgga gcgagtggag cccggaggcc atgggcacgc cttggacaga atccaggagt 960
cctccagctg agaacgaggt gtccaccccc atgcaggcac ttactactaa taaagacgat 1020
gataatattc tcttcagaga ttctgcaaat gcgacaagcc tcccagtgca agattcttct 1080
tcagtaccac tgcccacatt cctggttgct ggagggagcc tggccttcgg aacgctcctc 1140
tgcattgcca ttgttctgag gttcaagaag acgtggaagc tgcgggctct gaaggaaggc 1200
aagacaagca tgcatccgcc gtactctttg ggcagctgg tcccggagag gcctcgaccc 1260
accccagtgc ttgttcctct catctcccca ccggtgtccc ccagcagcct ggggtctgac 1320
aatacctcga gccacaaccg accagatgcc agggacccac ggagccctta tgacatcagc 1380
aatacagact acttcttccc cagatag 1407
```

<210> 24
<211> 600
<212> DNA
<213> Rattus norvegicus

<400> 24

```
atgaactgtg tttgtcgcct ggtcctggtg gtgctgagcc tctggccaga tagagtcgtt 60
gcccctgggc caccagctgg ctcccctcga gtgtcttcag accctcgtgc agatctggat 120
agcgctgtcc tcttgaccag gtccctcctg gcagacacac ggcaactagc tgcacagatg 180
agagacaaat tcccagctga tggagaccac aatctggact ccctacctac cttggccatg 240
agcgctggga cactgggatc tttgcagctt cctggagtgc tgacaaggct tcgagtagac 300
ttaatgtcct acttccgaca tgtacagtgg ttgcgccggg cagctggtcc ttccctaaag 360
actctggagc cagagctggg tgccctgcaa gcccgactgg aacggctact tcgtcgctta 420
cagctcttga tgtctcgcct agccttgccc caggcagccc cggaccaacc tgcggtccct 480
ctgggccctc ctgcctcggc ctggggaagc atccgggcag ctcatgccat cctaggaggg 540
ctgcacctga ccttggactg ggccgtgcgg ggcctgctgt tgttaaagac tcggctgtaa 600
```

<210> 25
<211> 609
<212> DNA
<213> Homo sapiens

<400> 25

```
atgaaggtct tggcggcagg agttgtgccc ctgctgttgg ttctgcactg gaaacatggg 60
gcggggagcc ccctccccat cacccctgtc aacgccacct gtgccatacg ccacccatgt 120
cacaacaacc tcatgaacca gatcaggagc caactggcac agctcaatgg cagtgccaat 180
gccctcttta ttctctatta cacagcccag ggggagccgt tccccaacaa cctggacaag 240
ctatgtggcc ccaacgtgac ggacttcccg cccttccacg ccaacggcac ggagaaggcc 300
aagctggtgg agctgtaccg catagtcgtg taccttggca cctccctggg caacatcacc 360
cgggaccaga agatcctcaa ccccagtgcc ctcagcctcc acagcaagct caacgccacc 420
gccgacatcc tgcgaggcct ccttagcaac gtgctgtgcc gcctgtgcag caagtaccac 480
gtgggccatg tggacgtgac ctacggccct gacacctcgg gtaaggatgt cttccagaag 540
aagaagctgg gctgtcaact cctggggaag tataagcaga tcatcgccgt gttggcccag 600
gccttctag 609
```

<210> 26
<211> 3294
<212> DNA
<213> Homo sapiens

<400> 26
atgatggata tttacgtatg tttgaaacga ccatcctgga tggtggacaa taaaagaatg 60
aggactgctt caaatttcca gtggctgtta tcaacattta ttcttctata tctaatgaat 120
caagtaaata gccagaaaaa gggggctcct catgatttga agtgtgtaac taacaatttg 180
caagtgtgga actgttcttg gaaagcaccc tctggaacag gccgtggtac tgattatgaa 240
gtttgcattg aaaacaggtc ccgttcttgt tatcagttgg agaaaaccag tattaaaatt 300
ccagctcttt cacatggtga ttatgaaata acaataaatt ctctacatga ttttggaagt 360
tctacaagta aattcacact aaatgaacaa aacgtttcct taattccaga tactccagag 420
atcttgaatt tgtctgctga tttctcaacc tctacattat acctaaagtg gaacgacagg 480
ggttcagttt ttccacaccg ctcaaatgtt atctgggaaa ttaaagttct acgtaaagag 540
agtatggagc tcgtaaaatt agtgacccac aacacaactc tgaatggcaa agatacactt 600
catcactgga gttgggcctc agatatgccc ttggaatgtg ccattcattt tgtggaaatt 660
agatgctaca ttgacaatct tcattttct ggtctcgaag agtggagtga ctggagccct 720
gtgaagaaca tttcttggat acctgattct cagactaagg tttttcctca agataaagtg 780
atacttgtag gctcagacat aacattttgt tgtgtgagtc aagaaaaagt gttatcagca 840
ctgattggcc atacaaactg ccccttgatc catcttgatg gggaaaatgt tgcaatcaag 900
attcgtaata tttctgtttc tgcaagtagt ggaacaaatg tagtttttac aaccgaagat 960
aacatatttg gaaccgttat ttttgctgga tatccaccag atactcctca acaactgaat 1020
tgtgagacac atgatttaaa agaaattata tgtagttgga atccaggaag ggtgacagcg 1080
ttggtgggcc cacgtgctac aagctacact ttagttgaaa gtttttcagg aaaatatgtt 1140
agacttaaaa gagctgaagc acctacaaac gaaagctatc aattattatt tcaaatgctt 1200
ccaaatcaag aaatatataa ttttactttg aatgctcaca atccgctggg tcgatcacaa 1260
tcaacaattt tagttaatat aactgaaaaa gtttatcccc atactcctac ttcattcaaa 1320
gtgaaggata ttaattcaac agctgttaaa ctttcttggc atttaccagg caactttgca 1380
aagattaatt ttttatgtga aattgaaatt aagaaatcta attcagtaca agagcagcgg 1440
aatgtcacaa tcaaaggagt agaaaattca agttatcttg ttgctctgga caagttaaat 1500
ccatacactc tatatacttt tcggattcgt tgttctactg aaactttctg gaaatggagc 1560
aaatggagca ataaaaaaca acatttaaca acagaagcca gtccttcaaa ggggcctgat 1620
acttggagag agtggagttc tgatggaaaa aatttaataa tctattggaa gcctttaccc 1680
attaatgaag ctaatggaaa aatactttcc tacaatgtat cgtgttcatc agatgaggaa 1740
acacagtccc tttctgaaat ccctgatcct cagcacaaag cagagatacg acttgataag 1800
aatgactaca tcatcagcgt agtggctaaa aattctgtgg gctcatcacc accttccaaa 1860
atagcgagta tggaaattcc aaatgatgat ctcaaaatag aacaagttgt tgggatggga 1920
aaggggattc tcctcacctg gcattacgac cccaacatga cttgcgacta cgtcattaag 1980
tggtgtaact cgtctcggtc ggaaccatgc cttatggact ggagaaaagt tccctcaaac 2040
agcactgaaa ctgtaataga atctgatgag tttcgaccag gtataagata taatttttc 2100
ctgtatggat gcagaaatca aggatatcaa ttattacgct ccatgattgg atatatagaa 2160
gaattggctc ccattgttgc accaaatttt actgttgagg atacttctgc agattcgata 2220
ttagtaaaat gggaagacat tcctgtggaa gaacttagag gctttttaag aggatatttg 2280
ttttactttg gaaaaggaga aagagacaca tctaagatga gggttttaga atcaggtcgt 2340
tctgacataa aagttaagaa tattactgac atatcccaga agacactgag aattgctgat 2400
cttcaaggta aaacaagtta ccacctggtc ttgcgagcct atacagatgg tggagtgggc 2460
ccggagaaga gtatgtatgt ggtgacaaag gaaaattctg tgggattaat tattgccatt 2520
ctcatcccag tggcagtggc tgtcattgtt ggagtggtga caagtatcct ttgctatcgg 2580
aaacgagaat ggattaaaga aaccttctac cctgatattc caaatccaga aaactgtaaa 2640
gcattacagt ttcaaaagag tgtctgtgag ggaagcagtg ctcttaaaac attggaaatg 2700
aatccttgta ccccaaataa tgttgaggtt ctggaaactc gatcagcatt tcctaaaata 2760
gaagatacag aaataatttc cccagtagct gagcgtcctg aagatcgctc tgatgcagag 2820
cctgaaaacc atgtggttgt gtcctattgt ccacccatca ttgaggaaga aataccaaac 2880
ccagccgcag atgaagctgg agggactgca caggttattt acattgatgt tcagtcgatg 2940
tatcagcctc aagcaaaacc agaagaagaa caagaaaatg accctgtagg aggggcaggc 3000

tataagccac agatgcacct ccccattaat tctactgtgg aagatatagc tgcagaagag 3060
gacttagata aaactgcggg ttacagacct caggccaatg taaatacatg gaatttagtg 3120
tctccagact ctcctagatc catagacagc aacagtgaga ttgtctcatt tggaagtcca 3180
tgctccatta attcccgaca atttttgatt cctcctaaag atgaagactc tcctaaatct 3240
aatggaggag ggtggtcctt tacaaacttt tttcagaaca aaccaaacga ttaa 3294

<210> 27
<211> 2310
<212> DNA
<213> Homo sapiens

<400> 27
atggcccaat ggaatcagct acagcagctt gacacacggt acctggagca gctccatcag 60
ctctacagtg acagcttccc aatggagctg cggcagtttc tggccccttg gattgagagt 120
caagattggg catatgcggc cagcaaagaa tcacatgcca ctttggtgtt tcataatctc 180
ctgggagaga ttgaccagca gtatagccgc ttcctgcaag agtcgaatgt tctctatcag 240
cacaatctac gaagaatcaa gcagtttctt cagagcaggt atcttgagaa gccaatggag 300
attgcccgga ttgtggcccg gtgcctgtgg gaagaatcac gccttctaca gactgcagcc 360
actgcggccc agcaaggggg ccaggccaac caccccacag cagccgtggt gacggagaag 420
cagcagatgc tggagcagca ccttcaggat gtccggaaga gagtgcagga tctagaacag 480
aaaatgaaag tggtagagaa tctccaggat gactttgatt tcaactataa aaccctcaag 540
agtcaaggag acatgcaaga tctgaatgga aacaaccagt cagtgaccag gcagaagatg 600
cagcagctgg aacagatgct cactgcgctg gaccagatgc ggagaagcat cgtgagtgag 660
ctggcggggc ttttgtcagc gatggagtac gtgcagaaaa ctctcacgga cgaggagctg 720
gctgactgga agaggcggca acagattgcc tgcattggag gcccgcccaa catctgccta 780
gatcggctag aaaactggat aacgtcatta gcagaatctc aacttcagac ccgtcaacaa 840
attaagaaac tggaggagtt gcagcaaaaa gtttcctaca aaggggaccc cattgtacag 900
caccggccga tgctggagga gagaatcgtg gagctgttta gaaacttaat gaaaagtgcc 960
tttgtggtgg agcggcagcc ctgcatgccc atgcatcctg accggcccct cgtcatcaag 1020
accggcgtcc agttcactac taaagtcagg ttgctggtca aattccctga gttgaattat 1080
cagcttaaaa ttaaagtgtg cattgacaaa gactctgggg acgttgcagc tctcagagga 1140
tcccggaaat ttaacattct gggcacaaac acaaaagtga tgaacatgga agaatccaac 1200
aacggcagcc tctctgcaga attcaaacac ttgaccctga gggagcagag atgtgggaat 1260
gggggccgag ccaattgtga tgcttccctg attgtgactg aggagctgca cctgatcacc 1320
tttgagaccg aggtgtatca ccaaggcctc aagattgacc tagagaccca ctccttgcca 1380
gttgtggtga tctccaacat ctgtcagatg ccaaatgcct gggcgtccat cctgtggtac 1440
aacatgctga ccaacaatcc caagaatgta aactttttta ccaagccccc aattggaacc 1500
tgggatcaag tggccgaggt cctgagctgg cagttctcct ccaccaccaa gcgaggactg 1560
agcatcgagc agctgactac actggcagag aaactcttgg gacctggtgt gaattattca 1620
gggtgtcaga tcacatgggc taaattttgc aaagaaaaca tggctggcaa gggcttctcc 1680
ttctgggtct ggctggacaa tatcattgac cttgtgaaaa agtacatcct ggccctttgg 1740
aacgaagggt acatcatggg ctttatcagt aaggagcggg agcgggccat cttgagcact 1800
aagcctccag gcaccttcct gctaagattc agtgaaagca gcaaagaagg aggcgtcact 1860
ttcacttggg tggagaagga catcagcggt aagacccaga tccagtccgt ggaaccatac 1920
acaaagcagc agctgaacaa catgtcattt gctgaaatca tcatgggcta taagatcatg 1980
gatgctacca atatcctggt gtctccactg gtctatctct atcctgacat tcccaaggag 2040
gaggcattcg gaaagtattg tcggccagag agccaggagc atcctgaagc tgacccaggc 2100
gctgccccat acctgaagac caagtttatc tgtgtgacac caacgacctg cagcaatacc 2160
attgacctgc cgatgtcccc ccgcacttta gattcattga tgcagtttgg aaataatggt 2220
gaaggtgctg aaccctcagc aggagggcag tttgagtccc tcacctttga catggagttg 2280
acctcggagt gcgctacctc ccccatgtga 2310

<210> 28
<211> 1956
<212> DNA
<213> Homo sapiens

<400> 28
atgaacaaat tacggcaaag ttttaggaga aagaaggatg tttatgttcc agaggccagt 60
cgtccacatc agtggcagac agatgaagaa ggcgttcgca ccggaaaatg tagcttcccg 120
gttaagtacc ttggccatgt agaagttgat gaatcaagag gaatgcacat ctgtgaagat 180
gctgtaaaaa gattgaaagc tgaaaggaag ttcttcaaag gcttctttgg aaaaactgga 240
aagaaagcag ttaaagcagt tctgtgggtc tcagcagatg gactcagagt tgtggatgaa 300
aaaactaagg acctcatagt tgaccagacg atagagaaag tttctttctg tgccccagac 360
aggaactttg atagagcctt ttcttacata tgccgtgatg gcaccactcg tcgctggatc 420
tgtcactgct tcatggctgt caaggacaca ggtgaaaggt tgagccatgc agtaggctgt 480
gcttttgcag cctgtttaga gcgcaagcag aagcgggaga aggaatgtgg agtgactgct 540
acttttgatg ctagtcggac cacttttaca agagaaggat cattccgtgt cacaacagcc 600
actgaacaag cagaaagaga ggagatcatg aaacaaatgc aagatgccaa gaaagctgaa 660
acagataaga tagtcgttgg ttcatcagtt gcccctggca acactgcccc atccccatcc 720
tctcccacct ctcctacttc tgatgccacg acctctctgg agatgaacaa tcctcatgcc 780
atcccacgcc ggcatgctcc aattgaacag cttgctcgcc aaggctcttt ccgaggtttt 840
cctgctctta gccagaagat gtcacccttt aaacgccaac tatccctacg catcaatgag 900
ttgccttcca ctatgcagag gaagactgat ttccccatta aaaatgcagt gccagaagta 960
gaaggggagg cagagagcat cagctccctg tgctcacaga tcaccaatgc cttcagcaca 1020
cctgaggacc ccttctcatc tgctccgatg accaaaccag tgacagtggt ggcaccacaa 1080
tctcctacct tccaagctaa tggcactgac tcagccttcc atgtgcttgc taagccagcc 1140
catactgctc tagcacccgt agcaatgcct gtgcgtgaaa ccaacccttg ggcccatgcc 1200
cctgatgctg ctaacaagga aattgcagcc acatgttcgg ggaccgagtg gggtcaatct 1260
tctggtgctg cctctccagg tctcttccag gccggtcata gacgtactcc ctctgaggcc 1320
gaccgatggt tagaagaggt gtctaagagc gtccgggctc agcagcccca ggcctcagct 1380
gctcctctgc agccagttct ccagcctcct ccacccactg ccatctccca gccagcatca 1440
cctttccaag ggaatgcatt cctcacctct cagcctgtgc cagtgggtgt ggtcccagcc 1500
ctgcaaccag cctttgtccc tgcccagtcc tatcctgtgg ccaatggaat gccctatcca 1560
gcccctaatg tgcctgtggt gggcatcact ccctcccaga tggtggccaa cgtatttggc 1620
actgcaggcc accctcaggc tgcccatccc catcagtcac ccagcctggt caggcagcag 1680
acattccctc actacgaggc aagcagtgct accaccagtc ccttctttaa gcctcctgct 1740
cagcacctca acggttctgc agctttcaat ggtgtagatg atggcaggtt ggcctcagca 1800
gacaggcata cagaggttcc tacaggcacc tgcccagtgg atccttttga agcccagtgg 1860
gctgcattag aaaataagtc caagcagcgt actaatccct cccctaccaa ccctttctcc 1920
agtgacttac agaagacgtt tgaaattgaa ctttaa 1956

<210> 29
<211> 1812
<212> DNA
<213> Homo sapiens

<400> 29
atgaacaaat tacggcaaag ttttaggaga aagaaggatg tttatgttcc agaggccagt 60
cgtccacatc agtggcagac agatgaagaa ggcgttcgca ccggaaaatg tagcttcccg 120
gttaagtacc ttggccatgt agaagttgat gaatcaagag gaatgcacat ctgtgaagat 180
gctgtaaaaa gattgaaagc tgaaaggaag ttcttcaaag gcttctttgg aaaaactgga 240
aagaaagcag ttaaagcagt tctgtgggtc tcagcagatg gactcagagt tgtggatgaa 300
aaaactaagg acctcatagt tgaccagacg atagagaaag tttctttctg tgccccagac 360
aggaactttg atagagcctt ttcttacata tgccgtgatg gcaccactcg tcgctggatc 420
tgtcactgct tcatggctgt caaggacaca ggtgaaaggt tgagccatgc agtaggctgt 480
gcttttgcag cctgtttaga gcgcaagcag aagcgggaga aggaatgtgg agtgactgct 540
acttttgatg ctagtcggac cacttttaca agagaaggat cattccgtgt cacaacagcc 600
actgaacaag cagaaagaga ggagatcatg aaacaaatgc aagatgccaa gaaagctgaa 660
acagataaga tagtcgttgg ttcatcagtt gcccctggca acactgcccc atccccatcc 720
tctcccacct ctcctacttc tgatgccacg acctctctgg agatgaacaa tcctcatgcc 780
atcccacgcc ggcatgctcc aattgaacag cttgctcgcc aaggctcttt ccgaggtttt 840
cctgctctta gccagaagat gtcacccttt aaacgccaac tatccctacg catcaatgag 900

```
ttgccttcca ctatgcagag gaagactgat ttccccatta aaaatgcagt gccagaagta 960
gaaggggagg cagagagcat cagctccctg tgctcacaga tcaccaatgc cttcagcaca 1020
cctgaggacc ccttctcatc tgctccgatg accaaaccag tgacagtggt ggcaccacaa 1080
tctcctacct tccaagggac cgagtggggt caatcttctg gtgctgcctc tccaggtctc 1140
ttccaggccg gtcatagacg tactccctct gaggccgacc gatggttaga agaggtgtct 1200
aagagcgtcc gggctcagca gccccaggcc tcagctgctc ctctgcagcc agttctccag 1260
cctcctccac ccactgccat ctcccagcca gcatcacctt tccaagggaa tgcattcctc 1320
acctctcagc ctgtgccagt gggtgtggtc ccagccctgc aaccagcctt tgtccctgcc 1380
cagtcctatc ctgtggccaa tggaatgccc tatccagccc ctaatgtgcc tgtggtgggc 1440
atcactccct cccagatggt ggccaacgta tttggcactg caggccaccc tcaggctgcc 1500
catccccatc agtcacccag cctggtcagg cagcagacat tccctcacta cgaggcaagc 1560
agtgctacca ccagtccctt ctttaagcct cctgctcagc acctcaacgg ttctgcagct 1620
ttcaatggtg tagatgatgg caggttggcc tcagcagaca ggcatacaga ggttcctaca 1680
ggcacctgcc cagtggatcc ttttgaagcc cagtgggctg cattagaaaa taagtccaag 1740
cagcgtacta atccctcccc taccaaccct ttctccagtg acttacagaa gacgtttgaa 1800
attgaacttt aa                                                     1812
```

<210> 30
<211> 1923
<212> DNA
<213> Homo sapiens

<400> 30

```
atgaacaaat tacggcaaag ttttaggaga aagaaggatg tttatgttcc agaggccagt 60
cgtccacatc agtggcagac agatgaagaa ggcgttcgca ccggaaaatg tagcttcccg 120
gttaagtacc ttggccatgt agaagttgat gaatcaagag gaatgcacat ctgtgaagat 180
gctgtaaaaa gattgaaagc tactggaaag aaagcagtta aagcagttct gtgggtctca 240
gcagatggac tcagagttgt ggatgaaaaa actaaggacc tcatagttga ccagacgata 300
gagaaagttt ctttctgtgc cccagacagg aactttgata gagccttttc ttacatatgc 360
cgtgatggca ccactcgtcg ctggatctgt cactgcttca tggctgtcaa ggacacaggt 420
gaaaggttga gccatgcagt aggctgtgct tttgcagcct gtttagagcg caagcagaag 480
cgggagaagg aatgtggagt gactgctact tttgatgcta gtcggaccac ttttacaaga 540
gaaggatcat tccgtgtcac aacagccact gaacaagcag aaagagagga gatcatgaaa 600
caaatgcaag atgccaagaa agctgaaaca gataagatag tcgttggttc atcagttgcc 660
cctggcaaca ctgccccatc cccatcctct cccacctctc ctacttctga tgccacgacc 720
tctctggaga tgaacaatcc tcatgccatc ccacgccggc atgctccaat tgaacagctt 780
gctcgccaag gctctttccg aggttttcct gctcttagcc agaagatgtc accctttaaa 840
cgccaactat ccctacgcat caatgagttg ccttccacta tgcagaggaa gactgatttc 900
cccattaaaa atgcagtgcc agaagtagaa ggggaggcag agagcatcag ctccctgtgc 960
tcacagatca ccaatgcctt cagcacacct gaggacccct tctcatctgc tccgatgacc 1020
aaaccagtga cagtggtggc accacaatct cctaccttcc aagctaatgg cactgactca 1080
gccttccatg tgcttgctaa gccagcccat actgctctag cacccgtagc aatgcctgtg 1140
cgtgaaacca acccttgggc ccatgcccct gatgctgcta acaaggaaat tgcagccaca 1200
tgttcgggga ccgagtgggg tcaatcttct ggtgctgcct ctccaggtct cttccaggcc 1260
ggtcatagac gtactccctc tgaggccgac cgatggttag aagaggtgtc taagagcgtc 1320
cgggctcagc agccccaggc ctcagctgct cctctgcagc cagttctcca gcctcctcca 1380
cccactgcca tctcccagcc agcatcacct ttccaaggga atgcattcct cacctctcag 1440
cctgtgccag tgggtgtggt cccagccctg caaccagcct ttgtccctgc ccagtcctat 1500
cctgtggcca atggaatgcc ctatccagcc cctaatgtgc ctgtggtggg catcactccc 1560
tcccagatgg tggccaacgt atttggcact gcaggccacc ctcaggctgc ccatccccat 1620
cagtcaccca gcctggtcag gcagcagaca ttccctcact acgaggcaag cagtgctacc 1680
accagtccct tctttaagcc tcctgctcag cacctcaacg gttctgcagc tttcaatggt 1740
gtagatgatg gcaggttggc ctcagcagac aggcatacag aggttcctac aggcacctgc 1800
ccagtggatc cttttgaagc ccagtgggct gcattagaaa ataagtccaa gcagcgtact 1860
aatccctccc ctaccaaccc tttctccagt gacttacaga agacgtttga aattgaactt 1920
taa                                                               1923
```

<210> 31
<211> 1779
<212> DNA
<213> Homo sapiens

<400> 31
atgaacaaat tacggcaaag ttttaggaga aagaaggatg tttatgttcc agaggccagt 60
cgtccacatc agtggcagac agatgaagaa ggcgttcgca ccggaaaatg tagcttcccg 120
gttaagtacc ttggccatgt agaagttgat gaatcaagag gaatgcacat ctgtgaagat 180
gctgtaaaaa gattgaaagc tactggaaag aaagcagtta aagcagttct gtgggtctca 240
gcagatggac tcagagttgt ggatgaaaaa actaaggacc tcatagttga ccagacgata 300
gagaaagttt ctttctgtgc ccagacagg aactttgata gagccttttc ttacatatgc 360
cgtgatggca ccactcgtcg ctggatctgt cactgcttca tggctgtcaa ggacacaggt 420
gaaaggttga gccatgcagt aggctgtgct tttgcagcct gtttagagcg caagcagaag 480
cgggagaagg aatgtggagt gactgctact tttgatgcta gtcggaccac ttttacaaga 540
gaaggatcat tccgtgtcac aacagccact gaacaagcag aaagagagga gatcatgaaa 600
caaatgcaag atgccaagaa agctgaaaca gataagatag tcgttggttc atcagttgcc 660
cctggcaaca ctgccccatc cccatcctct cccacctctc ctacttctga tgccacgacc 720
tctctggaga tgaacaatcc tcatgccatc ccacgccggc atgctccaat tgaacagctt 780
gctcgccaag gctctttccg aggttttcct gctcttagcc agaagatgtc acccttttaaa 840
cgccaactat ccctacgcat caatgagttg ccttccacta tgcagaggaa gactgatttc 900
cccattaaaa atgcagtgcc agaagtagaa ggggaggcag agagcatcag ctccctgtgc 960
tcacagatca ccaatgcctt cagcacacct gaggacccct tctcatctgc tccgatgacc 1020
aaaccagtga cagtggtggc accacaatct cctaccttcc aagggaccga gtggggtcaa 1080
tcttctggtg ctgcctctcc aggtctcttc caggccggtc atagacgtac tccctctgag 1140
gccgaccgat ggttagaaga ggtgtctaag agcgtccggg ctcagcagcc ccaggcctca 1200
gctgctcctc tgcagccagt tctccagcct cctccaccca ctgccatctc ccagccagca 1260
tcacctttcc aagggaatgc attcctcacc tctcagcctg tgccagtggg tgtggtccca 1320
gccctgcaac cagcctttgt ccctgcccag tcctatcctg tggccaatgg aatgccctat 1380
ccagcccta atgtgcctgt ggtgggcatc actccctccc agatggtggc caacgtattt 1440
ggcactgcag gccaccctca ggctgcccat ccccatcagt cacccagcct ggtcaggcag 1500
cagacattcc ctcactacga ggcaagcagt gctaccacca gtcccttctt taagcctcct 1560
gctcagcacc tcaacggttc tgcagctttc aatggtgtag atgatggcag gttggcctca 1620
gcagacaggc atacagaggt tcctacaggc acctgcccag tggatccttt tgaagcccag 1680
tgggctgcat tagaaaataa gtccaagcag cgtactaatc cctcccctac caaccctttc 1740
tccagtgact tacagaagac gtttgaaatt gaactttaa 1779

<210> 32
<211> 1830
<212> DNA
<213> Mus musculus

<400> 32
atgtcccgca gcgcggcggc cagcggcgga ccccggaggc ctgagcggca cctgccccca 60
gccccctgtg gggccccggg gcccccagaa acctgcagga cggagccaga cggggcgggc 120
accatgaaca agttacggca gagcctgcgg cggaggaagc cagcctacgt gcccgaggcg 180
tcgcgcccgc accagtggca ggcagacgag gacgcggtgc ggaagggcac gtgcagcttc 240
ccggtcaggt acctgggtca cgtggaggta gaggagtccc ggggaatgca cgtgtgtgaa 300
gatgcggtga agaagctgaa ggcgatgggc cgaaagtccg tgaagtctgt cctgtgggtg 360
tcagccgatg ggctccgagt ggtggacgac aaaaccaagg atcttctggt cgaccagacc 420
atcgaaaagg tctcctttg tgctcctgac cgcaacctgg acaaggcttt ctcctatatc 480
tgtcgtgacg ggactacccg ccgctggatc tgccactgtt ttctggcact gaaggactcc 540
ggcgagaggc tgagccacgc tgtgggctgt gcttttgccg cctgcctgga gcgaaaacag 600
cgacgggaga aggaatgtgg ggtcacggcc gccttcgatg ccagccgcac cagcttcgcc 660
cgcgagggct ccttccgcct gtctgggggt gggcggcctg ctgagcgaga ggccccggac 720
aagaagaaag cagaggcagc agctgccccc actgtggctc ctggccctgc ccagcctggg 780

cacgtgtccc cgacaccagc caccacatcc cctggtgaga agggtgaggc aggcacccct 840
gtggctgcag gcaccactgc ggccgccatc ccccggcgcc atgcacccct ggagcagctg 900
gttcgccagg gctccttccg tgggttccca gcactcagcc agaagaactc gcctttcaaa 960
cggcagctga gcctacggct gaatgagctg ccatccacgc tgcagcgccg cactgacttc 1020
caggtgaagg gcacagtgcc tgagatggag cctcctggtg ccggcgacag tgacagcatc 1080
aacgctctgt gcacacagat cagttcatct tttgccagtg ctggagcgcc agcaccaggg 1140
ccaccacctg ccacaacagg gacttctgcc tggggtgagc cctccgtgcc ccctgcagct 1200
gccttccagc ctgggcacaa gcggacacct tcagaggctg agcgatggct ggaggaggtg 1260
tcacaggtgg ccaaggccca gcagcagcag cagcagcaac agcaacagca gcagcagcag 1320
cagcagcaac agcagcaagc agcctcagtg gccccagtgc ccaccatgcc tcctgccctg 1380
cagcctttcc ccgcccccgt ggggcccttt gacgctgcac ctgcccaagt ggccgtgttc 1440
ctgccacccc cacacatgca gcccccttt gtgcccgcct acccgggctt gggctaccca 1500
ccgatgcccc gggtgcccgt ggtgggcatc acaccctcac agatggtggc aaacgccttc 1560
tgctcagccg cccagctcca gcctcagcct gccactctgc ttgggaaagc tggggccttc 1620
ccgcccccctg ccatacccag tgcccctggg agccaggccc gccctcgccc caatggggcc 1680
ccctggcccc ctgagccagc gcctgcccca gctccagagt tggacccctt tgaggcccag 1740
tgggcggcat tagaaggcaa agccactgta gagaaaccct ccaacccctt ttctggcgac 1800
ctgcaaaaga cattcgagat tgaactgtag 1830

<210> 33
<211> 918
<212> DNA
<213> Homo sapiens

<400> 33
atgagtgtgg atccagcttg tccccaaagc ttgccttgct ttgaagcatc cgactgtaaa 60
gaatcttcac ctatgcctgt gatttgtggg cctgaagaaa actatccatc cttgcaaatg 120
tcttctgctg agatgcctca cacggagact gtctctcctc ttccttcctc catggatctg 180
cttattcagg acagccctga ttcttccacc agtcccaaag gcaaacaacc cacttctgca 240
gagaagagtg tcgcaaaaaa ggaagacaag gtcccggtca agaaacagaa gaccagaact 300
gtgttctctt ccacccagct gtgtgtactc aatgatagat ttcagagaca gaaatacctc 360
agcctccagc agatgcaaga actctccaac atcctgaacc tcagctacaa acaggtgaag 420
acctggttcc agaaccagag aatgaaatct aagaggtggc agaaaaacaa ctggccgaag 480
aatagcaatg gtgtgacgca gaaggcctca gcacctacct accccagcct ttactcttcc 540
taccaccagg gatgcctggt gaacccgact gggaaccttc caatgtggag caaccagacc 600
tggaacaatt caacctggag caaccagacc cagaacatcc agtcctggag caaccactcc 660
tggaacactc agacctggtg cacccaatcc tggaacaatc aggcctggaa cagtcccttc 720
tataactgtg gagaggaatc tctgcagtcc tgcatgcagt tccagccaaa ttctcctgcc 780
agtgacttgg aggctgcctt ggaagctgct ggggaaggcc ttaatgtaat acagcagacc 840
actaggtatt ttagtactcc acaaaccatg gatttattcc taaactactc catgaacatg 900
caacctgaag acgtgtga 918

<210> 34
<211> 759
<212> DNA
<213> Homo sapiens

<400> 34
atggggggtac tgctcacaca gaggacgctg ctcagtctgg tccttgcact cctgtttcca 60
agcatggcga gcatggcggc tataggcagc tgctcgaaag agtaccgcgt gctccttggc 120
cagctccaga agcagacaga tctcatgcag gacaccagca gactcctgga cccctatata 180
cgtatccaag gcctggatgt tcctaaactg agagagcact gcagggagcg ccccggggcc 240
ttccccagtg aggagaccct gagggggctg ggcaggcggg gcttcctgca gaccctcaat 300
gccacactgg gctgcgtcct gcacagactg gccgacttag agcagcgcct ccccaaggcc 360
caggatttgg agaggtctgg gctgaacatc gaggacttgg agaagctgca gatggcgagg 420
ccgaacatcc tcgggctcag gaacaacatc tactgcatgg cccagctgct ggacaactca 480

```
gacacggctg agcccacgaa ggctggccgg ggggcctctc agccgcccac ccccacccct 540
gcctcggatg cttttcagcg caagctggag ggctgcaggt tcctgcatgg ctaccatcgc 600
ttcatgcact cagtggggcg ggtcttcagc aagtgggggg agagcccgaa ccggagccgg 660
agacacagcc cccaccaggc cctgaggaag gggtgcgca ggaccagacc ctccaggaaa 720
ggcaagagac tcatgaccag gggacagctg ccccggtag                       759
```

<210> 35
<211> 2940
<212> DNA
<213> Homo sapiens

<400> 35

```
atggctctat ttgcagtctt tcagacaaca ttcttcttaa cattgctgtc cttgaggact 60
taccagagtg aagtcttggc tgaacgttta ccattgactc ctgtatcact taaagtttcc 120
accaattcta cgcgtcagag tttgcactta caatggactg tccacaacct tccttatcat 180
caggaattga aaatggtatt tcagatccag atcagtagga ttgaaacatc caatgtcatc 240
tgggtgggga attacagcac cactgtgaag tggaaccagg ttctgcattg gagctgggaa 300
tctgagctcc ctttggaatg tgccacacac tttgtaagaa taaagagttt ggtggacgat 360
gccaagttcc ctgagccaaa tttctggagc aactggagtt cctgggagga agtcagtgta 420
caagattcta ctggacagga tatattgttc gttttcccta aagataagct ggtggaagaa 480
ggcaccaatg ttaccatttg ttacgtttct aggaacattc aaaataatgt atcctgttat 540
ttggaaggga aacagattca tggagaacaa cttgatccac atgtaactgc attcaacttg 600
aatagtgtgc ctttcattag gaataaaggg acaaatatct attgtgaggc aagtcaagga 660
aatgtcagtg aaggcatgaa aggcatcgtt ctttttgtct caaaagtact tgaggagccc 720
aaggactttt cttgtgaaac cgaggacttc aagactttgc actgtacttg ggatcctggg 780
acggacactg ccttggggtg gtctaaacaa ccttcccaaa gctacacttt atttgaatca 840
ttttctgggg aaaagaaact ttgtacacac aaaaactggt gtaattggca aataactcaa 900
gactcacaag aaacctataa cttcacactc atagctgaaa attacttaag gaagagaagt 960
gtcaatatcc tttttaacct gactcatcga gtttatttaa tgaatccttt tagtgtcaac 1020
tttgaaaatg taaatgccac aaatgccatc atgacctgga aggtgcactc cataaggaat 1080
aatttcacat atttgtgtca gattgaactc catggtgaag gaaaaatgat gcaatacaat 1140
gtttccatca aggtgaacgg tgagtacttc ttaagtgaac tggaacctgc cacagagtac 1200
atggcgcgag tacggtgtgc tgatgccagc cacttctgga aatggagtga atggagtggt 1260
cagaacttca ccacacttga agctgctccc tcagaggccc ctgatgtctg gagaattgtg 1320
agcttggagc caggaaatca tactgtgacc ttattctgga agccattatc aaaactgcat 1380
gccaatggaa agatcctgtt ctataatgta gttgtagaaa acctagacaa accatccagt 1440
tcagagctcc attccattcc agcaccagcc aacagcacaa aactaatcct tgacaggtgt 1500
tcctaccaaa tctgcgtcat agccaacaac agtgtgggtg cttctcctgc ttctgtaata 1560
gtcatctctg cagaccccga aaacaaagag gttgaggaag aaagaattgc aggcacagag 1620
ggtggattct ctctgtcttg gaaaccccaa cctggagatg ttataggcta tgttgtggac 1680
tggtgtgacc atacccagga tgtgctcggt gatttccagt ggaagaatgt aggtcccaat 1740
accacaagca cagtcattag cacagatgct tttaggccag gagttcgata tgacttcaga 1800
atttatgggt tatctacaaa aaggattgct tgtttattag agaaaaaaac aggatactct 1860
caggaacttg ctccttcaga caaccctcac gtgctggtgg atacattgac atcccactcc 1920
ttcactctga gttggaaaga ttactctact gaatctcaac ctggttttat acaagggtac 1980
catgtctatc tgaaatccaa ggcgaggcag tgccacccac gatttgaaaa ggcagttctt 2040
tcagatggtt cagaatgttg caaatacaaa attgacaacc cggaagaaaa ggcattgatt 2100
gtggacaacc taaagccaga atccttctat gagtttttca tcactccatt cactagtgct 2160
ggtgaaggcc ccagtgctac gttcacgaag gtcacgactc cggatgaaca ctcctcgatg 2220
ctgattcata tcctactgcc catggttttc tgcgtcttgc tcatcatggt catgtgctac 2280
ttgaaaagtc agtggatcaa ggagacctgt tatcctgaca tccctgaccc ttacaagagc 2340
agcatcctgt cattaataaa attcaaggag aaccctcacc taataataat gaatgtcagt 2400
gactgtatcc cagatgctat tgaagttgta agcaagccag aagggacaaa gatacagttc 2460
ctaggcacta ggaagtcact cacagaaacc gagttgacta agcctaacta cctttatctc 2520
cttccaacag aaaagaatca ctctggccct ggcccctgca tctgttttga gaacttgacc 2580
tataaccagg cagcttctga ctctggctct tgtggccatg ttccagtatc cccaaaagcc 2640
ccaagtatgc tgggactaat gacctcacct gaaaatgtac taaaggcact agaaaaaaac 2700
```

tacatgaact ccctgggaga aatcccagct ggagaaacaa gtttgaatta tgtgtcccag 2760
ttggcttcac ccatgtttgg agacaaggac agtctcccaa caaacccagt agaggcacca 2820
cactgttcag agtataaaat gcaaatggca gtctccctgc gtcttgcctt gcctcccccg 2880
accgagaata gcagcctctc ctcaattacc cttttagatc caggtgaaca ctactgctaa 2940

<210> 36
<211> 798
<212> DNA
<213> Homo sapiens

<400> 36
atgcacttct acagactatt ccttggggcc acacgtaggt tcttgaatcc cgaatggaaa 60
ggggagattg ataactggtg tgtttatgtt cttacaagtc ttctgccttt taaaatccag 120
tcccaggaca tcaaagctct gcagaaagaa ctcgagcaat ttgccaagct cctgaagcag 180
aagaggatca ccctgggata tacacaggcc gatgtggggc tcaccctggg ggttctattt 240
gggaaggtat tcagccaaac gaccatctgc cgctttgagg ctctgcagct tagcttcaag 300
aacatgtgta agctgcggcc cttgctgcag aagtgggtgg aggaagctga caacaatgaa 360
aatcttcagg agatatgcaa agcagaaacc ctcgtgcagg cccgaaagag aaagcgaacc 420
agtatcgaga accgagtgag aggcaacctg gagaatttgt tcctgcagtg cccgaaaccc 480
acactgcagc agatcagcca catcgcccag cagcttgggc tcgagaagga tgtggtccga 540
gtgtggttct gtaaccggcg ccagaagggc aagcgatcaa gcagcgacta tgcacaacga 600
gaggattttg aggctgctgg gtctcctttc tcagggggac cagtgtcctt tcctctggcc 660
ccagggcccc attttggtac cccaggctat gggagccctc acttcactgc actgtactcc 720
tcggtccctt tccctgaggg ggaagccttt ccccctgtct ccgtcaccac tctgggctct 780
cccatgcatt caaactga 798

<210> 37
<211> 1083
<212> DNA
<213> Homo sapiens

<400> 37
atggcgggac acctggcttc ggatttcgcc ttctcgcccc ctccaggtgg tggaggtgat 60
gggccagggg ggccggagcc gggctgggtt gatcctcgga cctggctaag cttccaaggc 120
cctcctggag ggccaggaat cgggccgggg gttgggccag gctctgaggt gtgggggatt 180
cccccatgcc ccccgccgta tgagttctgt gggggggatgg cgtactgtgg gccccaggtt 240
ggagtggggc tagtgcccca aggcggcttg gagacctctc agcctgaggg cgaagcagga 300
gtcggggtgg agagcaactc cgatggggcc tccccggagc cctgcaccgt cacccctggt 360
gccgtgaagc tggagaagga gaagctggag caaaacccgg aggagtccca ggacatcaaa 420
gctctgcaga aagaactcga gcaatttgcc aagctcctga agcagaagag gatcaccctg 480
ggatatacac aggccgatgt ggggctcacc ctgggggttc tatttgggaa ggtattcagc 540
caaacgacca tctgccgctt tgaggctctg cagcttagct tcaagaacat gtgtaagctg 600
cggcccttgc tgcagaagtg ggtggaggaa gctgacaaca atgaaaatct tcaggagata 660
tgcaaagcag aaaccctcgt gcaggcccga aagagaaagc gaaccagtat cgagaaccga 720
gtgagaggca acctggagaa tttgttcctg cagtgcccga aacccacact gcagcagatc 780
agccacatcg cccagcagct tgggctcgag aaggatgtgg tccgagtgtg gttctgtaac 840
cggcgccaga agggcaagcg atcaagcagc gactatgcac aacgagagga ttttgaggct 900
gctgggtctc ctttctcagg gggaccagtg tcctttcctc tggccccagg gccccatttt 960
ggtaccccag gctatgggag ccctcacttc actgcactgt actcctcggt ccctttccct 1020
gagggggaag cctttccccc tgtctccgtc accactctgg gctctcccat gcattcaaac 1080
tga 1083

<210> 38
<211> 953
<212> DNA
<213> Homo sapiens

<400> 38
tgtacaacat gatggagacg gagctgaagc cgccgggccc gcagcaaact tcggggggcg 60
gcggcggcaa ctccaccgcg gcggcggccg gcggcaacca gaaaaacagc ccggaccgcg 120
tcaagcggcc catgaatgcc ttcatggtgt ggtcccgcgg gcagcggcgc aagatggccc 180
aggagaaccc caagatgcac aactcggaga tcagcaagcg cctgggcgcc gagtggaaac 240
ttttgtcgga gacggagaag cggccgttca tcgacgaggc taagcggctg cgagcgctgc 300
acatgaagga gcacccggat tataaatacc ggccccggcg gaaaaccaag acgctcatga 360
agaaggataa gtacacgctg cccggcgggc tgctggcccc cggcggcaat agcatggcga 420
gcggggtcgg ggtgggcgcc ggcctgggcg cgggcgtgaa ccagcgcatg gacagttacg 480
cgcacatgaa cggctggagc aacggcagct acagcatgat gcaggaccag ctgggctacc 540
cgcagcaccc gggcctcaat gcgcacggcg cagcgcagat gcagcccatg caccgctacg 600
acgtgagcgc cctgcagtac aactccatga ccagctcgca gacctacatg aacggctcgc 660
ccacctacag catgtcctac tcgcagcagg gcacccctgg catggctctt ggctccatgg 720
gttcggtggt caagtccgag gccagctcca gcccccctgt ggttacctct tcctcccact 780
ccagggcgcc ctgccaggcc ggggacctcc gggacatgat cagcatgtat ctccccggcg 840
ccgaggtgcc ggaacccgcc gcccccagca gacttcacat gtcccagcac taccagagcg 900
gcccggtgcc cggcacggcc attaacggca cactgcccct ctcacacatg tga 953

<210> 39
<211> 621
<212> DNA
<213> Homo sapiens

<400> 39
atgtcggggc ccgggacggc cgcggtagcg ctgctcccgg cggtcctgct ggccttgctg 60
gcgccctggg cgggccgagg gggcgccgcc gcacccactg cacccaacgg cacgctggag 120
gccgagctgg agcgccgctg ggagagcctg gtggcgctct cgttggcgcg cctgccggtg 180
gcagcgcagc ccaaggaggc ggccgtccag agcggcgccg gcgactacct gctgggcatc 240
aagcggctgc ggcggctcta ctgcaacgtg ggcatcggct tccacctcca ggcgctcccc 300
gacggccgca tcggcggcgc gcacgcggac acccgcgaca gcctgctgga gctctcgccc 360
gtggagcggg gcgtggtgag catcttcggc gtggccagcc ggttcttcgt ggccatgagc 420
agcaagggca agctctatgg ctcgcccttc ttcaccgatg agtgcacgtt caaggagatt 480
ctccttccca acaactacaa cgcctacgag tcctacaagt accccggcat gttcatcgcc 540
ctgagcaaga atgggaagac caagaagggg aaccgagtgt cgcccaccat gaaggtcacc 600
cacttcctcc ccaggctgtg a 621

<210> 40
<211> 1443
<212> DNA
<213> Homo sapiens

<400> 40
atggaggtgg cgccggagca gccgcgctgg atggcgcacc cggccgtgct gaatgcgcag 60
cacccccgact cacaccaccc gggcctggcg cacaactaca tggaacccgc gcagctgctg 120
cctccagacg aggtggacgt cttcttcaat cacctcgact cgcagggcaa cccctactat 180
gccaaccccg ctcacgcgcg ggcgcgcgtc tcctacagcc ccgcgcacgc ccgcctgacc 240
ggaggccaga tgtgccgccc acacttgttg cacagcccgg gtttgccctg gctggacggg 300
ggcaaagcag ccctctctgc cgctgcggcc caccaccaca acccctggac cgtgagcccc 360
ttctccaaga cgccactgca cccctcagct gctggaggcc ctggaggccc actctctgtg 420
tacccagggg ctgggggtgg gagcggggga ggcagcggga gctcagtggc ctccctcacc 480
cctacagcag cccactctgg ctcccacctt ttcggcttcc cacccacgcc acccaaagaa 540

gtgtctcctg accctagcac cacgggggct gcgtctccag cctcatcttc cgcggggggt 600
agtgcagccc gaggagagga caaggacggc gtcaagtacc aggtgtcact gacggagagc 660
atgaagatgg aaagtggcag tcccctgcgc ccaggcctag ctactatggg cacccagcct 720
gctacacacc accccatccc cacctacccc tcctatgtgc cggcggctgc ccacgactac 780
agcagcggac tcttccaccc cggaggcttc ctgggggac cggcctccag cttcacccct 840
aagcagcgca gcaaggctcg ttcctgttca gaaggccggg agtgtgtcaa ctgtggggcc 900
acagccaccc ctctctggcg gcgggacggc accggccact acctgtgcaa tgcctgtggc 960
ctctaccaca agatgaatgg gcagaaccga ccactcatca agcccaagcg aagactgtcg 1020
gccgccagaa gagccggcac ctgttgtgca aattgtcaga cgacaaccac caccttatgg 1080
cgccgaaacg ccaacgggga ccctgtctgc aacgcctgtg gcctctacta caagctgcac 1140
aatgttaaca ggccactgac catgaagaag gaagggatcc agactcggaa ccggaagatg 1200
tccaacaagt ccaagaagag caagaaaggg gcggagtgct tcgaggagct gtcaaagtgc 1260
atgcaggaga agtcatcccc cttcagtgca gctgccctgg ctggacacat ggcacctgtg 1320
ggccacctcc cgcccttcag ccactccgga cacatcctgc ccactccgac gcccatccac 1380
ccctcctcca gcctctcctt cggccacccc cacccgtcca gcatggtgac cgccatgggc 1440
tag 1443

<210> 41
<211> 1335
<212> DNA
<213> Homo sapiens

<400> 41
atggaggtga cggcggacca gccgcgctgg gtgagccacc accaccccgc cgtgctcaac 60
gggcagcacc cggacacgca ccacccgggc ctcagccact cctacatgga cgcggcgcag 120
tacccgctgc cggaggaggt ggatgtgctt tttaacatcg acggtcaagg caaccacgtc 180
ccgccctact acggaaactc ggtcagggcc acggtgcaga ggtaccctcc gacccaccac 240
gggagccagg tgtgccgccc gcctctgctt catggatccc taccctggct ggacggcggc 300
aaagccctgg gcagccacca caccgcctcc ccctggaatc tcagcccctt ctccaagacg 360
tccatccacc acggctcccc ggggcccctc tccgtctacc ccccggcctc gtcctcctcc 420
ttgtcggggg gccacgccag cccgcacctc ttcaccttcc cgcccacccc gccgaaggac 480
gtctccccgg acccatcgct gtccacccca ggctcggccg gctcggcccg gcaggacgag 540
aaagagtgcc tcaagtacca ggtgcccctg cccgacagca tgaagctgga gtcgtcccac 600
tcccgtggca gcatgaccgc cctgggtgga gcctcctcgt cgacccacca ccccatcacc 660
acctacccgc cctacgtgcc cgagtacagc tccggactct tcccccccag cagcctgctg 720
ggcggctccc ccaccggctt cggatgcaag tccaggccca aggcccggtc cagcacagaa 780
ggcagggagt gtgtgaactg tggggcaacc tcgaccccac tgtggcggcg agatggcacg 840
ggacactacc tgtgcaacgc ctgcgggctc tatcacaaaa tgaacggaca gaaccggccc 900
ctcattaagc ccaagcgaag gctgtctgca gccaggagag cagggacgtc ctgtgcgaac 960
tgtcagacca ccacaaccac actctggagg aggaatgcca atggggaccc tgtctgcaat 1020
gcctgtgggc tctactacaa gcttcacaat attaacagac ccctgactat gaagaaggaa 1080
ggcatccaga ccagaaaccg aaaaatgtct agcaaatcca aaaagtgcaa aaaagtgcat 1140
gactcactgg aggacttccc caagaacagc tcgtttaacc cggccgccct ctccagacac 1200
atgtcctccc tgagccacat ctcgcccttc agccactcca gccacatgct gaccacgccc 1260
acgccgatgc acccgccatc cagcctgtcc tttggaccac accacccctc cagcatggtc 1320
accgccatgg gttag 1335

<210> 42
<211> 1329
<212> DNA
<213> Homo sapiens

<400> 42
atgtatcaga gcttggccat ggccgccaac cacgggccgc cccccggtgc ctacgaggcg 60
ggcggccccg gcgccttcat gcacggcgcg ggcgccgcgt cctcgccagt ctacgtgccc 120
acaccgcggg tgccctcctc cgtgctgggc ctgtcctacc tccagggcgg aggcgcgggc 180

tctgcgtccg gaggcgcctc gggcggcagc tccggtgggg ccgcgtctgg tgcggggccc 240
gggacccagc agggcagccc gggatggagc caggcgggag ccgacggagc cgcttacacc 300
ccgccgccgg tgtcgccgcg cttctccttc ccggggacca ccgggtccct ggcggccgcc 360
gccgccgctg ccgcggcccg ggaagctgcg gcctacagca gtggcggcgg agcggcgggt 420
gcgggcctgg cgggccgcga gcagtacggg cgcgccggct tcgcgggctc ctactccagc 480
ccctacccgg cttacatggc cgacgtgggc gcgtcctggg ccgcagccgc cgccgcctcc 540
gccggcccct tcgacagccc ggtcctgcac agcctgcccg gccgggccaa cccggccgcc 600
cgacacccca atctcgatat gtttgacgac ttctcagaag gcagagagtg tgtcaactgt 660
ggggctatgt ccaccccgct ctggaggcga gatgggacgg gtcactatct gtgcaacgcc 720
tgcggcctct accacaagat gaacggcatc aaccggccgc tcatcaagcc tcagcgccgg 780
ctgtccgcct cccgccgagt gggcctctcc tgtgccaact gccagaccac caccaccacg 840
ctgtggcgcc gcaatgcgga gggcgagcct gtgtgcaatg cctgcggcct ctacatgaag 900
ctccacgggg tccccaggcc tcttgcaatg cggaaagagg ggatccaaac cagaaaacgg 960
aagcccaaga acctgaataa atctaagaca ccagcagctc cttcaggcag tgagagcctt 1020
cctcccgcca gcggtgcttc cagcaactcc agcaacgcca ccaccagcag cagcgaggag 1080
atgcgtccca tcaagacgga gcctggcctg tcatctcact acgggcacag cagctccgtg 1140
tcccagacgt tctcagtcag tgcgatgtct ggccatgggc cctccatcca ccctgtcctc 1200
tcggccctga agctctcccc acaaggctat gcgtctcccg tcagccagtc tccacagacc 1260
agctccaagc aggactcttg gaacagcctg gtcttggccg acagtcacgg ggacataatc 1320
actgcgtaa 1329

<210> 43
<211> 1194
<212> DNA
<213> Homo sapiens

<400> 43
atgtaccaga gcctggcgct ggccgcgagc cccgccagg ccgcctacgc cgactcgggc 60
tccttcctgc acgctccggg cgccggctct ccgatgtttg tgccgccggc gcgcgtcccc 120
tcgatgctgt cctacctgtc cgggtgtgag ccgagcccgc agccccccga gctcgctgcg 180
cgccccggct gggcgcagac agccaccgcg gattcgtcgg ccttcggccc gggcagtccg 240
caccccccag ccgcgcaccc gcccggggcc accgccttcc ctttcgcgca cagcccctcg 300
gggcccggca gcggcggcag cgcgggggc cgagacggca gtgcctacca gggcgcgctg 360
ttgcctcgag aacagttcgc ggccccgctt gggcggccgg tggggacctc gtactccgcc 420
acctacccgg cctacgtgag ccccgacgtg gcccagtcct ggactgccgg gcccttcgat 480
ggcagcgtcc tgcacggcct cccaggccgc aggcccacct tcgtgtccga cttcttggag 540
gagttcccgg gtgagggtcg tgagtgtgtc aactgcgggg ccctgtccac accgctgtgg 600
cgccgagatg gcaccggcca ctacctgtgc aatgcctgcg gcctctacca caagatgaat 660
ggcgtcaacc ggccgctcgt tcggcctcag aagcgcctgt cctcgtcccg ccgcgccggc 720
ctctgctgca ccaactgcca cacgaccaac accacgctgt ggcggcggaa ctcggagggg 780
gagcccgtgt gcaatgcctg cggcctctac atgaagctgc acggggtgcc gcggcctctg 840
gctatgaaga aagaaagcat ccagacacgg aagcggaagc caaagaccat cgccaaggcc 900
aggggctcct caggatccac aaggaatgcc tcggcctccc catctgctgt cgccagcact 960
gacagctcag cagccacttc caaagccaag cccagcctgg cgtccccagt gtgccctggg 1020
cccagcatgg ccccccaggc ctctggccag gaggatgact ctcttgcccc cggccacttg 1080
gagttcaagt tcgagcctga ggactttgcc ttcccctcca cggccccgag cccccaggct 1140
ggcctcaggg gggctctgcg ccaagaggcc tggtgtgcgc tggccttggc ctag 1194

<210> 44
<211> 1788
<212> DNA
<213> Homo sapiens

<400> 44
atggccttga ctgacggcgg ctggtgcttg ccgaagcgct tcggggccgc gggtgcggac 60
gccagcgact ccagagcctt tccagcgcgg gagccctcca cgccgccttc ccccatctct 120

tcctcgtcct cctcctgctc ccggggcgga gagcggggcc ccggcggcgc cagcaactgc 180
gggacgcctc agctcgacac ggaggcggcg gccggacccc cggcccgctc gctgctgctc 240
agttcctacg cttcgcatcc cttcggggct ccccacggac cttcggcgcc tggggtcgcg 300
ggccccgggg gcaacctgtc gagctgggag gacttgctgc tgttcactga cctcgaccaa 360
gccgcgaccg ccagcaagct gctgtggtcc agccgcggcg ccaagctgag ccccttcgca 420
cccgagcagc cggaggagat gtaccagacc ctcgccgctc tctccagcca gggtccggcc 480
gcctacgacg gcgcgcccgg cggcttcgtg cactctgcgg ccgcggcggc agcagccgcg 540
gcggcggcca gctccccggt ctacgtgccc accacccgcg tgggttccat gctgcccggc 600
ctaccgtacc acctgcaggg gtcgggcagt gggccagcca accacgcggg cggcgcgggc 660
gcgcaccccg gctggcctca ggcctcggcc gacagccctc catacggcag cggaggcggc 720
gcggctggcg gcggggccgc ggggcctggc ggcgctggct cagccgcggc gcacgtctcg 780
gcgcgcttcc cctactctcc cagcccgccc atggccaacg gcgccgcgcg ggagccggga 840
ggctacgcgg cggcgggcag tgggggcgcg ggaggcgtga gcggcggcgg cagtagcctg 900
gcggccatgg gcggccgcga gccccagtac agctcgctgt cggccgcgcg gccgctgaac 960
gggacgtacc accaccacca ccaccaccac caccaccatc cgagccccta ctcgccctac 1020
gtgggggcgc cactgacgcc tgcctggccc gccggaccct tcgagacccc ggtgctgcac 1080
agcctgcaga gccgcgccgg agccccgctc ccggtgcccc ggggtcccag tgcagacctg 1140
ctggaggacc tgtccgagag ccgcgagtgc gtgaactgcg gctccatcca gacgccgctg 1200
tggcggcggg acggcaccgg ccactacctg tgcaacgcct gcgggctcta cagcaagatg 1260
aacggcctca gccggcccct catcaagccg cagaagcgcg tgccttcatc acggcggctt 1320
ggattgtcct gtgccaactg tcacaccaca actaccacct tatggcgcag aaacgccgag 1380
ggtgaacccg tgtgcaatgc ttgtggactc tacatgaaac tccatggggt gcccagacca 1440
cttgctatga aaaaagaggg aattcaaacc aggaaacgaa aacctaagaa cataaataaa 1500
tcaaagactt gctctggtaa tagcaataat tccattccca tgactccaac ttccacctct 1560
tctaactcag atgattgcag caaaaatact tcccccacaa cacaacctac agcctcaggg 1620
gcgggtgccc cggtgatgac tggtgcggga gagagcacca atcccgagaa cagcgagctc 1680
aagtattcgg gtcaagatgg gctctacata ggcgtcagtc tcgcctcgcc ggccgaagtc 1740
acgtcctccg tgcgaccgga ttcctggtgc gccctggccc tggcctga 1788

<210> 45
<211> 1674
<212> DNA
<213> Homo sapiens

<400> 45
atggtgtcca agctcacgtc gctccagcaa gaactcctga gcgccctgct gagctccggg 60
gtcaccaagg aggtgctggt tcaggccttg gaggagttgc tgccatcccc gaacttcggg 120
gtgaagctgg agacgctgcc cctgtcccct ggcagcgggg ccgagcccga caccaagccg 180
gtcttccata ctctcaccaa cggccacgcc aagggccgct tgtccggcga cgagggctcc 240
gaggacggcg acgactatga cacacctccc atcctcaagg agctgcaggc gctcaacacc 300
gaggaggcgg cggagcagcg ggcggaggtg gaccggatgc tcagtgagga cccttggagg 360
gctgctaaaa tgatcaaggg ttacatgcag caacacaaca tcccccagag ggaggtggtc 420
gatgtcaccg gcctgaacca gtcgcacctc tcccagcatc tcaacaaggg cacccctatg 480
aagacccaga agcgtgccgc tctgtacacc tggtacgtca gaaagcaacg agagatcctc 540
cgacaattca accagacagt ccagagttct ggaaatatga cagacaaaag cagtcaggat 600
cagctgctgt ttctctttcc agagttcagt caacagagcc atgggcctgg gcagtccgat 660
gatgcctgct ctgagcccac caacaagaag atgcgccgca accggttcaa atgggggccc 720
gcgtcccagc aaatcttgta ccaggcctac gatcggcaaa agaaccccag caaggaagag 780
agagaggcct tagtggagga atgcaacagg gcagaatgtt tgcagcgagg ggtgtccccc 840
tccaaagccc acggcctggg ctccaacttg gtcactgagg tccgtgtcta caactggttt 900
gcaaaccgca ggaaggagga ggcattccgg caaaagctgg ccatggacgc ctatagctcc 960
aaccagactc acagcctgaa ccctctgctc tcccacggct cccccacca ccagcccagc 1020
tcctctcctc caaacaagct gtcaggagtg cgctacagcc agcagggaaa caatgagatc 1080
acttcctcct caacaatcag tcaccatggc aacagcgcca tggtgaccag ccagtcggtt 1140
ttacagcaag tctccccagc cagcctggac ccaggccaca atctcctctc acctgatggt 1200
aaaatgatct cagtctcagg aggaggtttg cccccagtca gcaccttgac gaatatccac 1260
agcctctccc accataatcc ccagcaatct caaaacctca tcatgacacc cctctctgga 1320

gtcatggcaa ttgcacaaag cctcaacacc tcccaagcac agagtgtccc tgtcatcaac 1380
agtgtggccg gcagcctggc agccctgcag cccgtccagt tctcccagca gctgcacagc 1440
cctcaccagc agcccctcat gcagcagagc ccaggcagcc acatggccca gcagcccttc 1500
atggcagctg tgactcagct gcagaactca cacatgtacg cacacaagca ggaacccccc 1560
cagtattccc acacctcccg gtttccatct gcaatggtgg tcacagatac cagcagcatc 1620
agtacactca ccaacatgtc ttcaagtaaa cagtgtcctc tacaagcctg gtga 1674

<210> 46
<211> 1887
<212> DNA
<213> Rattus norvegicus

<400> 46
atggtttcta agttgagcca gctgcagacg gagctcctgg ctgctctgct cgagtcgggc 60
ctgagcaaag aggctctgat ccaggctctg gggagcccg ggccctacct gatggttgga 120
gatggtcccc tggacaaggg ggagtcctgc ggtgggactc gaggggacct gaccgagctg 180
cccaatggcc tgggggagac gcgtggctcg gaagatgaca cggatgacga tggggaagac 240
ttcgcgccac ccattctgaa agagctggag aacctcagcc cagaggaggc agcccaccag 300
aaagccgtgg tggagtcact tcttcaggag gacccatggc gcgtggcaaa gatggtcaag 360
tcgtacctgc agcaacacaa catcccccag cgggaggtgg tggacactac gggtctcaac 420
cagtcccacc tgtcccagca cctcaacaag ggcacccca tgaagacgca gaagcgggcc 480
gcgctgtaca cctggtacgt ccgcaagcag cgagaggtgg ctcagcaatt cacccacgcg 540
gggcagggcg gactgattga agagcccaca ggtgatgagc tgccaaccaa aaaggggcgg 600
aggaaccggt tcaagtgggg ccccgcatcc cagcagatcc tgttccaggc ttacgagagg 660
cagaagaacc ccagcaagga agagcgagag accttggtgg aggagtgcaa tagggcggag 720
tgcatccaga gaggggtgtc accatcgcag gcccaggggc taggctccaa ccttgtcacc 780
gaggtgcgtg tctacaactg gtttgccaac cggcgcaagg aagaagcctt tcggcataag 840
ctggccatgg acacgtataa cgggcctcca cccgggccag gccccggccc tgcgctacct 900
gcccacagtt ccccgggcct gcccacaacc accctctctc ccagtaaggt ccacggtgtg 960
cggtatggac agtctgcaac cagcgaggca gctgaggtgc cctccagcag cggaggtccc 1020
ttagtcacag tgtctgcggc cttacaccaa gtgtccccca caggcttgga gcccagcagc 1080
ctgctgagca ccgaggccaa gctggtctca gccacggggg gtcccctgcc tcccgtcagc 1140
accctgacag cactgcacag cttggagcag acgtctccag gtctcaacca gcagccgcag 1200
aaccttatca tggcctcgct gcctggggtc atgaccatcg gcccagggga gcccgcctcc 1260
ctgggtccca cgttcactaa cacgggtgcc tctaccctgg tcattggtct ggcctccaca 1320
caggcacaga gcgtgccagt catcaacagc atggggagca gcctgaccac cctgcagccg 1380
gtccagtttt cccagccact gcacccttcc tatcagcagc ctctcatgcc ccctgtacag 1440
agccacgtgg cccagagtcc cttcatggca accatggccc agctgcagag cccccacgcc 1500
ctgtacagcc acaagcctga ggtggcccag tacacgcata caagcctgct tccgcagacc 1560
atgctgatca cagacaccaa cctcagcacc cttgccagcc tcacgcccac caagcaggtc 1620
ttcacctcag acacagaggc ctccagtgag cctgggcttc atgagccgtc gtctccagcc 1680
acaaccattc acatccccag ccaggacccg tcaaacatcc agcacctgca gcctgctcac 1740
cggctcagca ccagtcccac agtgtcctcc agcagcctgg tgttgtacca gagttctgac 1800
tccaacgggc acagccacct gctgccatcc aaccacggtg tcatcgagac ttttatctcc 1860
acccagatgg cctcctcctc ccagtaa 1887

<210> 47
<211> 1407
<212> DNA
<213> Mus musculus

<400> 47
atgttaggga ctgtgaagat ggaagggcat gagagcaacg actggaacag ctactacgcg 60
gacacgcagg aggcctactc ctctgtccct gtcagcaaca tgaactccgg cctgggctct 120
atgaactcca tgaacaccta catgaccatg aacaccatga ccacgagcgg caacatgacc 180
ccggcttcct tcaacatgtc ctacgccaac acgggcttag ggccggcct gagtcccggt 240

gctgtggctg gcatgccagg ggcctctgca ggcgccatga acagcatgac tgcggcgggc 300
gtcacggcca tgggtacggc gctgagcccg ggaggcatgg gctccatggg cgcgcagccc 360
gtcacctcca tgaacggcct gggtccctac gccgccgcca tgaacccgtg catgagtccc 420
atggcgtacg cgccgtccaa cctgggccgc agccgcgcgg ggggcggcgg cgacgccaag 480
acattcaagc gcagctaccc tcacgccaag ccgccttact cctacatctc gctcatcacg 540
atggccatcc agcaggcgcc cagcaagatg ctcacgctga gcgagatcta ccagtggatc 600
atggacctct tcccctatta ccgccagaac cagcagcgct ggcagaactc catccgccac 660
tcgctgtcct tcaacgattg tttcgtcaag gtggcacgat ccccagacaa gccaggcaag 720
ggctcctact ggacgctgca cccggactcc ggcaacatgt tcgagaacgg ctgctacttg 780
cgccgccaaa agcgcttcaa gtgtgagaag cagccggggg ccggaggtgg gagtgggggc 840
ggcggctcca aggggggccc agaaagtcgc aaggacccct caggcccggg gaaccccagc 900
gccgagtcac cccttcattg ggtgtgcac ggaaaggcta gccagctaga gggcgcgccg 960
gcccccgggc ccgccgccag cccccagact ctggaccaca gcggggccac ggcgacaggg 1020
ggcgcttcgg agttgaagtc tccagcgtct tcatctgcgc cccccataag ctccgggcca 1080
ggggcgctgg catctgtacc cccctctcac ccggctcacg gcctggcacc ccacgaatct 1140
cagctgcatc tgaaagggga tccccactac tcctttaatc acccccttctc catcaacaac 1200
ctcatgtcct cctccgagca acagcacaag ctggacttca aggcatacga gcaggcgctg 1260
cagtactctc cttatggcgc taccttgccc gccagtctgc cccttggcag cgcctcagtg 1320
gccacgagga gccccatcga gccctcagcc ctggagccag cctactacca aggtgtgtat 1380
tccagacccg tgctaaatac ttcctag 1407

<210> 48
<211> 1062
<212> DNA
<213> Mus musculus

<400> 48
atgctgggct cagtgaagat ggaggctcat gacctggccg agtggagcta ctacccggag 60
gcgggcgagg tgtattctcc agtgaatcct gtgcccacca tggcccctct caactcctac 120
atgaccttga acccactcag ctctccctac cctcccggag ggcttcaggc ctccccactg 180
cctacaggac ccctggcacc cccagccccc actgcgccct tggggcccac cttcccaagc 240
ttgggcactg gtggcagcac cggaggcagt gcttccgggt atgtagcccc agggcccggg 300
cttgtacatg gaaaagagat ggcaaagggg taccggcggc cactggccca cgccaaacca 360
ccatattcct acatctctct cataaccatg gctattcagc aggctccagg caagatgctg 420
accctgagtg aaatctacca atggatcatg gacctcttcc cgtactaccg ggagaaccag 480
caacgttggc agaactccat ccggcattcg ctgtccttca atgactgctt cgtcaaggtg 540
gcacgctccc cagacaagcc aggcaaaggc tcctactggg ccttgcatcc cagctctggg 600
aacatgtttg agaacggatg ctatctccgc cggcagaagc gcttcaagct ggaggagaag 660
gcaaagaaag gaaacagcgc cacatcggcc agcaggaatg gtactgcggg gtcagccacc 720
tctgccacca ctacagctgc cactgcagtc acctccccgg ctcagcccca gcctacgcca 780
tctgagcccg aggcccagag tggggatgat gtgggggtc tggactgcgc ctcacctcct 840
tcgtccacac cttatttcag cggcctggag ctcccggggg aactaaagtt ggatgcgccc 900
tataacttca accacccttt ctctatcaac aacctgatgt cagaacagac atcgacacct 960
tccaaactgg atgtggggtt tggggctac ggggctgaga gtggggagcc tggagtctac 1020
taccagagcc tctattcccg ctctctgctt aatgcatcct ag 1062

<210> 49
<211> 1380
<212> DNA
<213> Mus musculus

<400> 49
atgctgggag ccgtgaagat ggaaggctc gagccatccg actggagcag ctactacgcg 60
gagcccgagg gctactcttc cgtgagcaac atgaacgccg gcctggggat gaatggcatg 120
aacacataca tgagcatgtc cgcggctgcc atgggcggcg gttccggcaa catgagcgcg 180
ggctccatga acatgtcatc ctatgtgggc gctggaatga gcccgtcgct agctggcatg 240

tccccgggcg ccggcgccat ggcgggcatg agcggctcag ccggggcggc cggcgtggcg 300
ggcatgggac ctcacctgag tccgagtctg agcccgctcg gggacaggc ggccggggcc 360
atgggtggcc ttgcccccta cgccaacatg aactcgatga gccccatgta cgggcaggcc 420
ggcctgagcc gcgctcggga ccccaagaca taccgacgca gctacacaca cgccaaacct 480
ccctactcgt acatctcgct catcaccatg gccatccagc agagccccaa caagatgctg 540
acgctgagcg agatctatca gtggatcatg gacctcttcc ctttctaccg gcagaaccag 600
cagcgctggc agaactccat ccgccactct ctctccttca acgactgctt tctcaaggtg 660
ccccgctcgc cagacaagcc tggcaagggc tccttctgga ccctgcaccc agactcgggc 720
aacatgttcg agaacggctg ctacctgcgc cgccagaagc gcttcaagtg tgagaagcaa 780
ctggcactga aggaagccgc gggtgcggcc agtagcggag gcaagaagac cgctcctggg 840
tcccaggcct ctcaggctca gctcggggag gccgcgggct cggcctccga gactccggcg 900
ggcaccgagt ccccccattc cagcgcttct ccgtgtcagg agcacaagcg aggtggccta 960
agcgagctaa agggagcacc tgcctctgcg ctgagtcctc ccgagccggc gccctcgcct 1020
gggcagcagc agcaggctgc agcccacctg ctgggcccac ctcaccaccc aggcctgcca 1080
ccagaggccc acctgaagcc cgagcaccat tacgccttca accacccctt ctctatcaac 1140
aacctcatgt cgtccgagca gcaacatcac cacagccacc accaccatca gccccacaaa 1200
atggacctca aggcctacga acaggtcatg cactacccag gggctatgg ttcccccatg 1260
ccaggcagct tggccatggg cccagtcacg aacaaagcgg gcctggatgc ctcgcccctg 1320
gctgcagaca cttcctacta ccaaggagtg tactccaggc ctattatgaa ctcatcctaa 1380

<210> 50
<211> 1053
<212> DNA
<213> Homo sapiens

<400> 50
atgctgggct cagtgaagat ggaggcccat gacctggccg agtggagcta ctacccggag 60
gcgggcgagg tctactcgcc ggtgacccca gtgcccacca tggcccccct caactcctac 120
atgaccctga atcctctaag ctctccctat ccccctgggg ggctccctgc ctccccactg 180
ccctcaggac ccctggcacc cccagcacct gcagcccccc tggggcccac tttcccaggc 240
ctgggtgtca gcggtggcag cagcagctcc gggtacgggg ccccgggtcc tgggctggtg 300
cacgggaagg agatgccgaa ggggtatcgg cggcccctgg cacacgccaa gccaccgtat 360
tcctatatct cactcatcac catggccatc cagcaggcgc cgggcaagat gctgaccttg 420
agtgaaatct accagtggat catggacctc ttcccttact accgggagaa tcagcagcgc 480
tggcagaact ccattcgcca ctcgctgtct ttcaacgact gcttcgtcaa ggtggcgcgt 540
tccccagaca agcctggcaa gggctcctac tgggccctac accccagctc agggaacatg 600
tttgagaatg gctgctacct gcgccgccag aaacgcttca agctggagga gaaggtgaaa 660
aaagggggca gcggggctgc caccaccacc aggaacggga cagggtctgc tgcctcgacc 720
accacccccg cggccacagt cacctccccg ccccagcccc cgcctccagc ccctgagcct 780
gaggcccagg gcggggaaga tgtgggggct ctggactgtg gctcacccgc ttcctccaca 840
ccctatttca ctggcctgga gctcccaggg gagctgaagc tggacgcgcc ctacaacttc 900
aaccacccctt tctccatcaa caacctaatg tcagaacaga caccagcacc tcccaaactg 960
gacgtggggt ttgggggcta cggggctgaa ggtggggagc ctggagtcta ctaccagggc 1020
ctctattccc gctctttgct taatgcatcc tag 1053

<210> 51
<211> 1422
<212> DNA
<213> Homo sapiens

<400> 51
atgttaggaa ctgtgaagat ggaagggcat gaaaccagcg actggaacag ctactacgca 60
gacacgcagg aggcctactc ctcggtcccg gtcagcaaca tgaactcagg cctgggctcc 120
atgaactcca tgaacaccta catgaccatg aacaccatga ctacgagcgg caacatgacc 180
ccggcgtcct tcaacatgtc ctatgccaac ccgggcctag ggccggcct gagtcccggc 240
gcagtagccg gcatgccggg gggctcggcg ggcgccatga acagcatgac tgcggccggc 300

gtgacggcca tgggtacggc gctgagcccg agcggcatgg gcgccatggg tgcgcagcag 360
gcggcctcca tgatgaatgg cctgggcccc tacgcggccg ccatgaaccc gtgcatgagc 420
cccatggcgt acgcgccgtc caacctgggc cgcagccgcg cgggcggcgg cggcgacgcc 480
aagacgttca agcgcagtta cccgcacgcc aagccgccct actcgtacat ctcgctcatc 540
accatggcca tccagcgggc gcccagcaag atgctcacgc tgagcgagat ctaccagtgg 600
atcatggacc tcttccccta ttaccggcag aaccagcagc gctggcagaa ctccatccgc 660
cactcgctgt ccttcaatga ctgcttcgtc aaggtggcac gctccccgga caagccgggc 720
aagggctcct actggacgct gcacccggac tccggcaaca tgttcgagaa cggctgctac 780
ttgcgccgcc agaagcgctt caagtgcgag aagcagccgg gggccggcgg cgggggcggg 840
agcggaagcg gggggcagcgg cgccaagggc ggccctgaga gccgcaagga cccctctggc 900
gcctctaacc ccagcgccga ctcgcccctc catcggggtg tgcacgggaa gaccggccag 960
ctagagggcg cgccggcccc gggcccggcc gccagccccc agactctgga ccacagtggg 1020
gcgacggcga caggggggcgc ctcggagttg aagactccag cctcctcaac tgcgcccccc 1080
ataagctccg ggcccggggc gctggcctct gtgcccgcct ctcacccggc acacggcttg 1140
gcaccccacg agtcccagct gcacctgaaa ggggaccccc actactcctt caaccacccg 1200
ttctccatca acaacctcat gtcctcctcg gagcagcagc ataagctgga cttcaaggca 1260
tacgaacagg cactgcaata ctcgccttac ggctctacgt tgcccgccag cctgcctcta 1320
ggcagcgcct cggtgaccac caggagcccc atcgagccct cagccctgga gccggcgtac 1380
taccaaggtg tgtattccag acccgtccta aacacttcct ag 1422

<210> 52
<211> 1425
<212> DNA
<213> Mus musculus

<400> 52
atgcgactct ctaaaaccct tgccggcatg gatatggccg actacagcgc tgccctggac 60
ccagcctaca ccaccctgga gtttgaaaat gtgcaggtgt tgaccatggg caatgacacg 120
tccccatctg aaggtgccaa cctcaattca tccaacagcc tgggcgtcag tgccctgtgc 180
gccatctgtg gcgaccgggc caccggcaaa cactacggag cctcgagctg tgacggctgc 240
aaggggttct tcaggaggag cgtgaggaag aaccacatgt actcctgcag gtttagccga 300
caatgtgtgg tagacaaaga taagaggaac cagtgtcgtt actgcaggct taagaagtgc 360
ttccgggctg gcatgaagaa ggaagctgtc caaaatgagc gggaccggat cagcacgcgg 420
aggtcaagct acgaggacag cagcctgccc tccatcaacg cgctcctgca ggcagaggtt 480
ctgtcccagc agatcacctc tcccatctct gggatcaatg gcgacattcg ggcaaagaag 540
attgccaaca tcacagacgt gtgtgagtct atgaaggagc agctgctggt cctggtcgag 600
tgggccaagt acatcccggc cttctgcgaa ctccttctgg atgaccaggt ggcgctgctc 660
agggcccacg ccggtgagca tctgctgctt ggagccacca agaggtccat ggtgtttaag 720
gacgtgctgc tcctaggcaa tgactacatc gtccctcggc actgtccaga gctagcggag 780
atgagccgtg tgtccatccg catcctcgat gagctggtcc tgcccttcca agagctgcag 840
attgatgaca atgaatatgc ctgcctcaaa gccatcatct tctttgatcc agatgccaag 900
gggctgagtg acccgggcaa gatcaagcgg ctgcggtcac aggtgcaagt gagcctggag 960
gattacatca acgaccggca gtacgactct cggggccgct ttggagagct gctgctgctg 1020
ttgcccacgc tgcagagcat cacctggcag atgatcgaac agatccagtt catcaagctc 1080
ttcggcatgg ccaagattga caacctgctg caggagatgc ttctcggagg gtctgccagt 1140
gatgcacccc acacccacca ccccctgcac cctcacctga tgcaagaaca catgggcacc 1200
aatgtcattg ttgctaacac gatgccctct cacctcagca atggacagat gtgtgagtgg 1260
ccccgaccca gggggcaggc agccactccc gagactccac agccatcacc accaagtggc 1320
tcgggatctg aatcctacaa gctcctgcca ggagccatca ccaccatcgt caagcctccc 1380
tctgccattc cccagccaac gatcaccaag caagaagcca tctag 1425

<210> 53
<211> 1398
<212> DNA
<213> Rattus norvegicus

<400> 53
atggacatgg ctgactacag tgctgccttg gacccagcct acaccaccct ggagtttgaa 60
aatgtgcagg tgttgaccat gggcaatgac acatccccat ctgaaggtgc caacctcaac 120
tcatccaaca gcctgggtgt cagtgccctg tgtgccatct gtggcgatcg ggccactggc 180
aaacactacg gagcctcaag ctgtgacggc tgcaagggat tcttcaggag gagcgtgagg 240
aagaaccaca tgtactcctg caggtttagc aggcagtgcg tggtagacaa agataagagg 300
aaccagtgtc gttactgcag gctcaagaag tgcttccggg ctggcatgaa gaaagaagcc 360
gtccaaaatg agcgggatcg gatcagcacg cggaggtcaa gctacgagga cagcagccta 420
ccctccatta atgcgctcct gcaggcagag gtcctgtctc agcagatcac ctcccccatc 480
tctgggatca atggcgacat tcgggccaag aagattgcca acatcacgga tgtgtgtgag 540
tctatgaagg agcagctgct ggttctggtc gaatgggcca agtacatccc ggccttctgt 600
gaacttcttc tggatgacca ggtggcgctg ctcagagccc acgctggtga gcacctgctg 660
cttggagcca ccaagaggtc catggtgttc aaggatgtgc tgctcctagg caatgactac 720
atcgtccctc ggcactgtcc agagctagca gagatgagcc gtgtgtccat tcgcatcctc 780
gatgagctgg tcttgccctt ccaagagctg cagatcgatg ataatgaata cgcctgcctc 840
aaagccatca tcttctttga cccagatgcc aaggggctga gtgacccagg caagatcaag 900
cggctgcggt cacaggtgca ggtgagcctg gaggattaca tcaacgaccg gcagtatgac 960
tctcggggtc gttttggaga gctgctgctg ctcctgccca ctctgcagag cattacctgg 1020
cagatgatcg agcagatcca gttcatcaag ctctttggca tggccaagat tgacaacctg 1080
ctgcaggaga tgctgcttgg agggtctgcc agtgacgcgc cccacgccca ccacccccctg 1140
caccctcacc tgatgcaaga acacatgggc accaatgtca tagttgccaa cacgatgccc 1200
tctcacctca gcaatggaca gatgtgtgag tggccccggc ccaggggggca ggcagccacc 1260
cctgagactc cacagccatc accaccaagt ggctctggat ctgaatccta caagctcctg 1320
ccaggagcca tcaccaccat cgtcaagcct ccctctgcca tcccccagcc aacgatcacc 1380
aagcaggaag ccatctag 1398

<210> 54
<211> 1398
<212> DNA
<213> Mus musculus

<400> 54
atgaacgcac agctgaccat ggaggcgatc ggcgagctgc acggggtgag ccatgagccg 60
gtgcccgccc ctgctgacct gctgggcggc agccctcacg cgcgcagctc cgtgggacac 120
cgcggcagcc acctgcctcc cgcgcacccg cgttccatgg gcatggcgtc cctgctggac 180
ggcggcagcg gaggcagcga ttaccaccac caccaccgcg cccctgagca cagcttggct 240
ggcccccctgc accccaccat gaccatggcc tgtgaaactc ccccaggtat gagcatgccc 300
accacctaca ctaccttaac ccctctgcag ccgctgccgc ccatctccac cgtgtccgac 360
aagttccctc accatcatca ccaccaccat caccaccacc acccacacca ccaccagcgc 420
ctggcgggca acgtgagcgg tagtttcaca cttatgcggg atgagcgcgg gctggcctct 480
atgaataacc tctatacccc ctaccacaag gacgtggctg gcatgggcca gagcctctcg 540
cccctctctg gctccggtct gggcagcatt cacaactccc agcaaggact tccccactat 600
gctcatcccg gcgcggctat gcccaccgac aagatgctca ccccaaatgg ctttgaagcc 660
caccaccctg ccatgctcgg tcgccacggg gagcagcacc tcacgcccac ctcggccggc 720
atggtaccca tcaacggcct tcctccgcac catcctcatg cccacctgaa tgcccagggc 780
cacggacagc tcctgggcac agcccgagag cccaaccctt cggtgaccgg cgcgcaggtc 840
agcaatggaa gtaattcagg gcagatggaa gagatcaata ccaaagaggt ggcgcagcgt 900
atcaccaccg agctcaaacg ttacagcatc ccacaggcca tcttcgcgca gagggtgctc 960
tgccgttccc aggggaccct ttcggacctg ctgcgaaacc ccaagccctg gagcaaactc 1020
aagtcgggtc gggagacctt ccggaggatg tggaagtggc tgcaggagcc ggagttccag 1080
cgcatgtcgg cgctccgctt agcagcctgc aaacggaaag agcaagaaca tgggaaggac 1140
agaggcaaca cccccaaaaa gcccaggctg gtcttcacag acgtccaacg tcgaactcta 1200
catgcaatat tcaaggaaaa taagcgtccg tccaaagaat tacaaatcac catctcccag 1260
cagctggggt tggagctgag cactgtcagc aacttcttca tgaatgccag aaggaggagt 1320
ctggacaagt ggcaggacga gggcggctcc aactcaggca gttcatcgtc ctcatcgagc 1380
acttgtacca aagcatga 1398

<210> 55
<211> 1002
<212> DNA
<213> Xenopus laevis

<400> 55
atggagaagt ccaagaattt caggattgac gctctcctgg cgatagatcc ccccaaggct 60
cagacctccc cattggctct ggtcacctcg ctgtcctcct cgtctctctc cgggagcccc 120
ccgtccgagc acactgacag cctcaggact gactccccct cccctccaag gacttgtgga 180
ctggtcccta aaccaggttt cctgagcagc caccagcacc ccccaaacat gatgtcattg 240
cacccccagg ctgctccagg gatccccccct caggccctgt atggacaccc gatgtacagc 300
tacttggcag cggggcagca cccagctctg tcctacccct actcccagat gcagagcagc 360
caccaccccc accccatgga ccccatcaag atcagcgctg gcaccttcca actggaccag 420
tggctcagag cctccactgc cggcatgatg ctgcccaaaa tggcagactt taactcccag 480
gcccaatcca acctgctggg aaagtgcaga agaccaagga cagcgtttac cagtcagcag 540
ctgttggaac tggagcacca attcaagctg aacaagtacc tctccaggcc gaaacgcttt 600
gaagtggcca cttccctgat gctcactgag acgcaggtga agatctggtt ccagaacagg 660
cgcatgaaat ggaagaggag taagaaagcc aaggagcagg cggcgcagga ctcagcagag 720
aaacagcaga gggcaggcaa gggcagcagc gaggagaagt gctcggatga gctgcaggaa 780
gagaagaaat cctaccatct ccatcccagg ggggagccca tcaaagggaa cggccgcctg 840
cagcccagag actatacaga cagcgaagag gacgaggagg aggacaggga agaggaggaa 900
gaggaagatc acagagggga ggggaagcgg ttttaccatc attcttctga ctgcacatcc 960
gaggaagagg agaacagcca caataagcag agcggccact ga 1002

<210> 56
<211> 1215
<212> DNA
<213> Mus musculus

<400> 56
atggaaaaat ccaaaaattt ccgcatcgac gccctgctgg ccgtggatcc cccgcgagcc 60
gcctccacgc agagcgcgcc tctggccttg gtcacttccc tcgcgactac agtatctggt 120
cccggccgcg gcggcagcgg cggcgggggg accagtagcg ggcgagccg tagctgcagt 180
cccgcatcct cggaggccac tgcagcgccc ggtgaccggc tgagagctga gagcccgtcg 240
ccccccacgct tgctggctgc acactgcgcg ctgctgccca agcccggatt cctgggcgcc 300
ggaggaggcg gcggcgcggc gggtgggccg ggcactcccc accaccacgc gcaccctggt 360
gcagcagccg ccgcggctgc cgctgccgct gccgcggctg ccggtggcct ggcactgggg 420
ctgcacccgg ggggcgcaca gggcggcgcg ggcctccctg cacaggcggc tctctatgga 480
cacccggtct acagttattc ggcagcagct gcagcggccg cgctagctgg ccagcacccg 540
gcgctttcct actcataccc tcaggtgcag ggcgcgcacc ctgcgcaccc tgccgacccc 600
atcaagctgg gtgccagcac cttccaactg gaccagtggc tgcgcgcgtc tactgcgggc 660
atgatcctgc ccaagatgcc ggacttcagc tgtcaggcgc agtcgaacct cttggggaag 720
tgccgaaggc ctcgcacggc cttcaccagc cagcagctgt tggagctgga acaccagttc 780
aagctcaaca agtacctgtc tcgacccaag cgttttgagg tggctacctc gctcatgctc 840
accgagactc aggtgaagat ttggttccag aaccgccgaa tgaaatggaa acgcagcaaa 900
aaggccaaag agcaggctgc gcaggaggcg gagaagcaga agggcggcgg cgggggcacc 960
ggcaaaggcg gcagtgagga gaagacggaa gaggagctga tggggcctcc ggtttcgggg 1020
gacaaggcaa gcggccgtcg cctgcgggac ttgcgggaca gtgaccctga tgaggacgag 1080
gatgatgaag aagaggacaa cttcccgtac agcaatggtg ccggtgccca tgctgcctca 1140
tccgactgct catctgagga cgactcgcct cctccaagac taggcgggcc tggacaccaa 1200
cctctgcccc agtag 1215

<210> 57
<211> 1215
<212> DNA
<213> Homo sapiens

<400> 57
atggaaaaat ccaaaaattt ccgcatcgac gccctgctgg ccgtggatcc cccgcgagcc 60
gcctccacgc agagcgcgcc tctggccttg gtcacttccc tcgcgactac agtatctggt 120
cccggccgcg gcggcagcgg cggcgggggg accagtagcg gggcgagccg tagctgcagt 180
cccgcatcct cggaggccac tgcagcgccc ggtgaccggc tgagagctga gagcccgtcg 240
cccccacgct tgctggctgc acactgcgcg ctgctgccca agcccggatt cctgggcgcc 300
ggaggaggcg gcggcgcggc gggtgggccg ggcactcccc accaccacgc gcaccctggt 360
gcagcagccg ccgcggctgc cgctgccgct gccgcggctg ccggtggcct ggcactgggg 420
ctgcacccgg ggggcgcaca gggcggcgcg ggcctccctg cacaggcggc tctctatgga 480
cacccggtct acagttattc ggcagcagct gcagcggccg cgctagctgg ccagcacccg 540
gcgctttcct actcataccc tcaggtgcag ggcgcgcacc ctgcgcaccc tgccgacccc 600
atcaagctgg gtgccagcac cttccaactg gaccagtggc tgcgcgcgtc tactgcgggc 660
atgatcctgc ccaagatgcc ggacttcagc tgtcaggcgc agtcgaacct cttggggaag 720
tgccgaaggc ctcgcacggc cttcaccagc cagcagctgt tggagctgga acaccagttc 780
aagctcaaca agtacctgtc tcgacccaag cgttttgagg tggctacctc gctcatgctc 840
accgagactc aggtgaagat ttggttccag aaccgccgaa tgaaatggaa acgcagcaaa 900
aaggccaaag agcaggctgc gcaggaggcg gagaagcaga agggcggcgg cgggggcacc 960
ggcaaaggcg gcagtgagga gaagacggaa gaggagctga tggggcctcc ggtttcgggg 1020
gacaaggcaa gcggccgtcg cctgcgggac ttgcgggaca gtgaccctga tgaggacgag 1080
gatgatgaag aagaggacaa cttcccgtac agcaatggtg ccggtgccca tgctgcctca 1140
tccgactgct catctgagga cgactcgcct cctccaagac taggcgggcc tggacaccaa 1200
cctctgcccc agtag 1215

<210> 58
<211> 846
<212> DNA
<213> Homo sapiens

<400> 58
atgacttcca aggaggacgg caaggcggcg ccgggggagg agcggcggcg cagcccgctg 60
gaccacctgc ctccgcctgc caactccaac aagccactga cgccgttcag catcgaggac 120
atcctcaaca agccgtctgt gcggagaagt tactcgctgt gcggggcggc gcacctgctg 180
gccgccgcgg acaagcacgc gcagggcggc ttgcccctgg cgggccgcgc gctgctctcg 240
cagacctcgc cgctgtgcgc gctggaggag ctcgccagca agacgtttaa ggggctggag 300
gtcagcgttc tgcaggcagc cgaaggccgc gacggtatga ccatctttgg gcagcggcag 360
acccctaaga agcggcgaaa gtcgcgcacg gccttcacca accaccagat ctatgaattg 420
gaaaagcgct ttctatacca gaagtacctg tcccccgccg atcgcgacca aatcgcgcag 480
cagctgggcc tcaccaacgc gcaagtcatc acctggttcc agaatcggcg cgctaagctc 540
aagcgggacc tggaggagat gaaggccgac gtagagtccg ccaagaaact gggccccagc 600
gggcagatgg acatcgtggc gctggccgaa ctcgagcaga actcggaggc cacagccggc 660
ggtggcggcg gctgcggcag ggccaagtcg aggcccggct ctccggtcct ccccccaggc 720
gccccgaagg ccccgggcgc tggcgccctg cagctctcgc ctgcctctcc gctcacggac 780
cagccggcca gcagccagga ctgctcggag gacgaggaag acgaagagat cgacgtggac 840
gattga 846

<210> 59
<211> 1119
<212> DNA
<213> Mus musculus

<400> 59
atgttggacg gcatcaagat ggaggagcac gccctgcgcc ccgggcccgc cactctgggg 60
gtgctgctgg gctccgactg cccgcatccc gccgtctgcg agggctgcca gcggcccatc 120
tccgaccgct tcctgatgcg agtcaacgag tcgtcctggc acgaggagtg tttgcagtgc 180
gcggcgtgtc agcaagccct caccaccagc tgctacttcc gggatcggaa actgtactgc 240
aaacaagact accaacagct cttcgcggcc aagtgcagcg gctgcatgga gaagatcgcc 300
cccaccgagt tcgtgatgcg ggcgctggag tgcgtgtacc acctgggctg cttctgctgc 360
tgcgtgtgtg aacggcagct acgcaagggc gacgaattcg tgctcaagga gggccagctg 420
ctgtgcaagg gtgactacga gaaggagaag gacctgctca gctccgtgag ccccgacgag 480
tccgactccg tgaagagcga ggatgaagat ggggacatga agccggccaa ggggcagggc 540
agtcagagca agggcagcgg ggatgacggg aaggacccgc ggaggcccaa gcgaccccgg 600
accatcctca ccacgcagca gcgaagagcc ttcaaggcct ccttcgaggt ctcgtcgaag 660
ccttgccgaa aggtccgaga gacactggca gctgagacgg gcctcagtgt gcgcgtggtc 720
caggtctggt ttcagaacca aagagcaaag atgaagaagc tggcgcggcg gcaccagcag 780
cagcaggagc agcagaactc ccagcggctg ggccaggagg tcctgtccag ccgcatggag 840
ggcatgatgg cttcctacac gccgctggcc ccaccacagc agcagatcgt ggccatggaa 900
cagagcccct acggcagcag cgaccccttc cagcagggcc tcacgccgcc ccaaatgcca 960
gggaacgact ccatcttcca tgacatcgac agcgatacct ccttaaccag cctcagcgac 1020
tgcttcctcg gctcctcaga cgtgggctcc ctgcaggccc gcgtggggaa ccccatcgac 1080
cggctctact ccatgcagag ttcctacttc gcctcctga 1119

<210> 60
<211> 714
<212> DNA
<213> Homo sapiens

<400> 60
atgccagccc gccttgagac ctgcatctcc gacctcgact gcgccagcag cagcggcagt 60
gacctatccg gcttcctcac cgacgaggaa gactgtgcca gactccaaca ggcagcctcc 120
gcttcggggc cgcccgcgcc ggcccgcagg ggcgcgccca atatctcccg ggcgtctgag 180
gttccagggg cacaggacga cgagcaggag aggcggcggc gccgcggccg gacgcgggtc 240
cgctccgagg cgctgctgca ctcgctgcgc aggagccggc gcgtcaaggc caacgatcgc 300
gagcgcaacc gcatgcacaa cttgaacgcg gccctggacg cactgcgcag cgtgctgccc 360
tcgttccccg acgacaccaa gctcaccaaa atcgagacgc tgcgcttcgc ctacaactac 420
atctgggctc tggccgagac actgcgcctg gcggatcaag ggctgcccgg aggcggtgcc 480
cgggagcgcc tcctgccgcc gcagtgcgtc cctgcctgc ccggtccccc aagccccgcc 540
agcgacgcgg agtcctgggg ctcaggtgcc gccgccgcct ccccgctctc tgaccccagt 600
agcccagccg cctccgaaga cttcacctac cgccccggcg accctgtttt ctccttccca 660
agcctgccca aagacttgct ccacacaacg ccctgtttca ttccttacca ctag 714

<210> 61
<211> 819
<212> DNA
<213> Homo sapiens

<400> 61
atgttcgtca aatccgagac cttggagttg aaggaggaag aggacgtgtt agtgctgctc 60
ggatcggcct cccccgcctt ggcggccctg accccgctgt catccagcgc cgacgaagaa 120
gaggaggagg agccgggcgc gtcaggcggg gcgcgtcggc agcgcggggc tgaggccggg 180
caggggcgc ggggcggcgt ggctgcgggt gcggagggct gccggcccgc acggctgctg 240
ggtctggtac acgattgcaa acggcgccct tcccgggcgc gggccgtctc ccgaggcgcc 300
aagacggccg agacggtgca gcgcatcaag aagacccgta gactgaaggc caacaaccgc 360
gagcgaaacc gcatgcacaa cctcaacgcg gcactggacg cgctgcgcga ggtgctcccc 420
acgttccccg aggacgccaa gctcaccaag atcgagaccc tgcgcttcgc ccacaactac 480
atctgggcac tcaccgagac cctgcgcctg gcggatcact gcgggggcgg cggcggggc 540
ctgccggggg cgctcttctc cgaggcagtg ttgctgagcc cgggaggcgc cagcgccgcc 600

```
ctgagcagca gcggagacag cccctcgccc gcctccacgt ggagttgcac caacagcccc 660
gcgccgtcct cctccgtgtc ctccaattcc acctccccct acagctgcac tttatcgccc 720
gccagcccgg ccgggtcaga catggactat tggcagcccc cacctcccga caagcaccgc 780
tatgcacctc acctccccat agccagggat tgtatctag                         819
```

<210> 62
<211> 645
<212> DNA
<213> Mus musculus

<400> 62

```
atgacgcctc aaccctcggg tgcgcccact gtccaagtga cccgtgagac ggagcggtcc 60
ttccccagag cctcggaaga cgaagtgacc tgccccacgt ccgccccgcc cagccccact 120
cgcacacggg ggaactgcgc agaggcggaa gagggaggct gccgaggggc cccgaggaag 180
ctccgggcac ggcgcggggg acgcagccgg cctaagagcg agttggcact gagcaagcag 240
cgacggagtc ggcgaaagaa ggccaacgac cgcgagcgca atcgaatgca caacctcaac 300
tcggcactgg acgccctgcg cggtgtcctg cccaccttcc cagacgacgc gaagctcacc 360
aagatcgaga cgctgcgctt cgcccacaac tacatctggg cgctgactca aacgctgcgc 420
atagcggacc acagcttgta cgcgctggag ccgccggcgc cgcactgcgg ggagctgggc 480
agcccaggcg gttcccccgg ggactggggg tccctctact ccccagtctc ccaggctggc 540
agcctgagtc ccgccgcgtc gctggaggag cgacccgggc tgctgggggc caccttttcc 600
gcctgcttga gcccaggcag tctggctttc tcagattttc tgtga                  645
```

<210> 63
<211> 711
<212> DNA
<213> Homo sapiens

<400> 63

```
atggaaagct ctgccaagat ggagagcggc ggcgccggcc agcagcccca gccgcagccc 60
cagcagccct tcctgccgcc cgcagcctgt ttctttgcca cggccgcagc cgcggcggcc 120
gcagccgccg cagcggcagc gcagagcgcg cagcagcagc agcagcagca gcagcagcag 180
cagcaggcgc cgcagctgag accggcggcc gacggccagc cctcaggggg cggtcacaag 240
tcagcgccca agcaagtcaa gcgacagcgc tcgtcttcgc ccgaactgat gcgctgcaaa 300
cgccggctca acttcagcgg ctttggctac agcctgccgc agcagcagcc ggccgccgtg 360
gcgcgccgca acgagcgcga gcgcaaccgc gtcaagttgg tcaacctggg ctttgccacc 420
cttcgggagc acgtccccaa cggcgcggcc aacaagaaga tgagtaaggt ggagacactg 480
cgctcggcgg tcgagtacat ccgcgcgctg cagcagctgc tggacgagca tgacgcggtg 540
agcgccgcct tccaggcagg cgtcctgtcg cccaccatct cccccaacta ctccaacgac 600
ttgaactcca tggccggctc gccggtctca tcctactcgt cggacgaggg ctcttacgac 660
ccgctcagcc ccgaggagca ggagcttctc gacttcacca actggttctg a           711
```

<210> 64
<211> 957
<212> DNA
<213> Mus musculus

<400> 64

```
atggagcttc tatcgccgcc actccgggac atagacttga caggccccga cggctctctc 60
tgctcctttg agacagcaga cgacttctat gatgacccgt gtttcgactc accagacctg 120
cgcttttttg aggacctgga cccgcgcctg gtgcacatgg gagccctcct gaaaccggag 180
gagcacgcac acttccctac tgcggtgcac ccaggcccag gcgctcgtga ggatgagcat 240
gtgcgcgcgc ccagcgggca ccaccaggcg ggtcgctgct tgctgtgggc ctgcaaggcg 300
tgcaagcgca agaccaccaa cgctgatcgc cgcaaggccg ccaccatgcg cgagcgccgc 360
cgcctgagca aagtgaatga ggccttcgag acgctcaagc gctgcacgtc cagcaacccg 420
```

aaccagcggc tacccaaggt ggagatcctg cgcaacgcca tccgctacat cgaaggtctg 480
caggctctgc tgcgcgacca ggacgccgcg ccccctggcg ccgctgcctt ctacgcacct 540
ggaccgctgc ccccaggccg tggcagcgag cactacagtg gcgactcaga tgcatccagc 600
ccgcgctcca actgctctga tggcatgatg gattacagcg gcccccaag cggcccccgg 660
cggcagaatg gctacgacac cgcctactac agtgaggcgg cgcgcgagtc caggccaggg 720
aagagtgcgg ctgtgtcgag cctcgactgc ctgtccagca tagtggagcg catctccaca 780
gacagccccg ctgcgcctgc gctgcttttg gcagatgcac caccagagtc gcctccgggt 840
ccgccagagg gggcatccct aagcgacaca gaacagggaa cccagacccc gtctcccgac 900
gccgcccctc agtgtcctgc aggctcaaac cccaatgcga tttatcaggt gctttga 957

<210> 65
<211> 963
<212> DNA
<213> Homo sapiens

<400> 65
atggagctac tgtcgccacc gctccgcgac gtagacctga cggcccccga cggctctctc 60
tgctcctttg ccacaacgga cgacttctat gacgacccgt gtttcgactc cccggacctg 120
cgcttcttcg aagacctgga cccgcgcctg atgcacgtgg gcgcgctcct gaaacccgaa 180
gagcactcgc acttccccgc ggcggtgcac ccggccccgg gcgcacgtga ggacgagcat 240
gtgcgcgcgc ccagcgggca ccaccaggcg ggccgctgcc tactgtgggc ctgcaaggcg 300
tgcaagcgca agaccaccaa cgccgaccgc cgcaaggccg ccaccatgcg cgagcggcgc 360
cgcctgagca aagtaaatga ggcctttgag acactcaagc gctgcacgtc gagcaatcca 420
aaccagcggt tgcccaaggt ggagatcctg cgcaacgcca tccgctatat cgagggcctg 480
caggctctgc tgcgcgacca ggacgccgcg ccccctggcg ccgcagccgc cttctatgcg 540
ccgggcccgc tgcccccggg ccgcggcggc gagcactaca gcggcgactc cgacgcgtcc 600
agcccgcgct ccaactgctc cgacggcatg atggactaca gcggcccccc gagcggcgcc 660
cggcggcgga actgctacga aggcgcctac tacaacgagg cgcccagcga acccaggccc 720
gggaagagtg cggcggtgtc gagcctagac tgcctgtcca gcatcgtgga gcgcatctcc 780
accgagagcc ctgcggcgcc cgccctcctg ctggcggacg tgccttctga gtcgcctccg 840
cgcaggcaag aggctgccgc ccccagcgag ggagagagca gcggcgaccc cacccagtca 900
ccggacgccg ccccgcagtg ccctgcgggt gcgaacccca acccgatata ccaggtgctc 960
tga 963

<210> 66
<211> 768
<212> DNA
<213> Homo sapiens

<400> 66
atggacgtga tggatggctg ccagttctca ccttctgagt acttctacga cggctcctgc 60
ataccgtccc ccgagggtga atttggggac gagtttgtgc cgcgagtggc tgccttcgga 120
gcgcacaaag cagagctgca gggctcagat gaggacgagc acgtgcgagc gcctaccggc 180
caccaccagg ctggtcactg cctcatgtgg gcctgcaaag cctgcaagag gaagtccacc 240
accatggatc ggcggaaggc agccactatg cgcgagcgga ggcgcctgaa gaaggtcaac 300
caggctttcg aaaccctcaa gaggtgtacc acgaccaacc ccaaccagag gctgcccaag 360
gtggagatcc tcaggaatgc catccgctac atcgagagcc tgcaggagtt gctgagagag 420
caggtggaga actactatag cctgccggga cagagctgct cggagcccac cagccccacc 480
tccaactgct ctgatggcat gcccgaatgt aacagtcctg tctggtccag aaagagcagt 540
acttttgaca gcatctactg tcctgatgta tcaaatgtat atgccacaga taaaaactcc 600
ttatccagct tggattgctt atccaacata gtggaccgga tcacctcctc agagcaacct 660
gggttgcctc tccaggatct ggcttctctc tctccagttg ccagcaccga ttcacagcct 720
gcaactccag ggcttctag ttccaggctt atctatcatg tgctatga 768

<210> 67
<211> 729
<212> DNA
<213> Homo sapiens

<400> 67
atgatgatgg accttttga aactggctcc tatttcttct acttggatgg ggaaaatgtt 60
actctgcagc cattagaagt ggcagaaggc tctcctttgt atccagggag tgatggtacc 120
ttgtccccct gccaggacca aatgcccccg gaagcgggga gcgacagcag cggagaggaa 180
catgtcctgg cgcccccggg cctgcagcct ccacactgcc ccggccagtg tctgatctgg 240
gcttgcaaga cctgcaagag aaaatctgcc cccactgacc ggcgaaaagc cgccaccctg 300
cgcgaaagga ggaggctaaa gaaaatcaac gaggccttcg aggcactgaa gcggcgaact 360
gtggccaacc ccaaccagag gctgcccaag gtggagattc tgcggagcgc catcagctat 420
attgagcggc tgcaggacct gctgcaccgg ctggatcagc aggagaagat gcaggagctg 480
ggggtggacc ccttcagcta cagacccaaa caagaaaatc ttgagggtgc ggatttcctg 540
cgcacctgca gctcccagtg gccaagtgtt tccgatcatt ccagggggct cgtgataacg 600
gctaaggaag gaggagcaag tattgattcg tcagcctcga gtagccttcg atgcctttct 660
tccatcgtgg acagtatttc ctcggaggaa cgcaaactcc cctgcgtgga ggaagtggtg 720
gagaagtaa 729

<210> 68
<211> 1356
<212> DNA
<213> Homo sapiens

<400> 68
atgccgaaga acaagaagcg gaacactccc caccgcggta gcagtgctgg cggcggcggg 60
tcaggagcag ccgcagcgac ggcggcgaca gcaggtggcc agcatcgaaa tgttcagcct 120
tttagtgatg aagatgcatc aattgaaaca atgagccatt gcagtggtta tagcgatcct 180
tccagttttg ctgaagatgg accagaagtc cttgatgagg aaggaactca agaagaccta 240
gagtacaagt tgaagggatt aattgaccta accctggata agagtgcgaa gacaaggcaa 300
gcagctcttg aaggtattaa aaatgcactg gcttcaaaaa tgctgtatga atttattctg 360
gaaaggagaa tgactttaac tgatagcatt gaacgctgcc tgaaaaaagg taagagtgat 420
gagcaacgtg cagctgcagc gttagcatct gttctttgta ttcagctggg ccctggaatt 480
gaaagtgaag agattttgaa aactcttgga ccaatcctaa agaaaatcat ttgtgatggg 540
tcagctagta tgcaggctag gcaaacttgt gcaacttgct ttggtgtttg ctgttttatt 600
gccacagatg acattactga actatactca actctggaat gtttggaaaa tatcttcact 660
aaatcctatc tcaaagagaa agacactact gttatttgca gcactcctaa tacagtgctt 720
catatcagct ctcttcttgc atggacacta ctgctgacca tatgcccaat caatgaagtg 780
aagaaaaagc ttgagatgca tttccataag cttccaagcc tcctctcttg tgatgatgta 840
aacatgagaa tagctgctgg tgaatctttg gcacttctct ttgaattggc cagaggaata 900
gagagtgact ttttttatga agacatggag tccttgacgc agatgcttag ggccttggca 960
acagatggaa ataaacaccg ggccaaagtg gacaagagaa agcagcggtc agttttcaga 1020
gatgtcctga gggcagtgga ggaacgggat tttccaacag aaaccattaa atttggtcct 1080
gaacgcatgt atattgattg ctgggtaaaa aaacacacct atgacacctt taaggaggtt 1140
cttggatcag ggatgcagta ccacttgcag tcaaatgaat tccttcgaaa tgtatttgaa 1200
cttggacccc cagtgatgct tgatgctgca acgcttaaaa cgatgaagat ttctcgtttc 1260
gaaaggcatt tatataactc tgcagccttc aaagctcgaa ccaaagctag aagcaaatgt 1320
cgagataaga gagcagatgt tggagaattc ttctag 1356

<210> 69
<211> 1524
<212> DNA
<213> Rattus norvegicus

<400> 69
atggggcgga agaaaataca aatcacacgc ataatggatg aaaggaaccg acaggtcact 60
tttacaaaga gaaagtttgg attaatgaag aaagcctatg aacttagtgt gctctgtgac 120
tgtgaaatag cactcatcat tttcaacagc tctaacaaac tgtttcaata tgctagcact 180
gatatggaca aagttcttct caagtataca gaatataatg aacctcatga aagcagaacc 240
aactcggata ttgttgaggc tctgaacaag aaggaacaca gagggtgcga cagcccagac 300
cctgatactt catatgtgct aactccacat acagaagaaa aatataaaaa aattaatgag 360
gaatttgata atatgatgcg gaatcataaa atcgcacctg gtctgccacc tcagaacttt 420
tcaatgtctg tcacagttcc agtgaccagc cccaatgctt tgtcctacac taacccaggg 480
agttcactgg tgtccccatc tttggcagcc agctcaacgt taacagattc aagcatgctc 540
tctccacctc aaaccacatt acatagaaat gtgtctcctg gagctcctca gagaccacca 600
agtactggca atgcaggtgg gatgttgagc actacagacc tcacagtgcc aaatggagct 660
ggaagcagtc cagtggggaa tggatttgta aactcaagag cttctccaaa tttgattgga 720
gctactggtg caaatagctt aggcaaagtc atgcctacaa agtctccccc tccaccaggt 780
ggtggtaatc ttggaatgaa cagtaggaaa ccagatcttc gagttgtcat cccccccttca 840
agcaagggca tgatgcctcc actatcggag gaagaggaat tggagttgaa cacccaaagg 900
atcagtagtt ctcaagccac tcaacctctt gctaccccag tcgtgtctgt gacaacccca 960
agcttgcctc cgcaaggact tgtgtactca gcaatgccga ctgcctacaa cactgattat 1020
tcactgacca gcgctgacct gtcagccctt caaggcttca actcgccagg aatgctgtcg 1080
ctgggacagg tgtcggcctg gcagcagcac cacctaggac aagcagccct cagctctctt 1140
gttgctggag ggcagttatc tcagggttcc aatttatcca ttaataccaa ccaaaacatc 1200
agcatcaagt ccgaaccgat ttcacctcct cgggatcgta tgaccccatc gggcttccag 1260
cagcagcagc agcagcagca gcagcagcag ccgccgccac caccgcagcc ccagccacaa 1320
cccccgcagc cccagccccg acaggaaatg ggcgctcccc ctgtggacag tctgagcagc 1380
tctagtagct cctatgatgg cagtgatcgg gaggatccac ggggcgactt ccattctcca 1440
attgtgcttg gccgaccccc aaacactgag gacagagaaa gcccttctgt aaagcgaatg 1500
aggatggacg cgtgggtgac ctaa 1524

<210> 70
<211> 675
<212> DNA
<213> Homo sapiens

<400> 70
atggagctgt atgagacatc cccctacttc taccaggaac cccgcttcta tgatggggaa 60
aactacctgc ctgtccacct ccagggcttc gaaccaccag gctacgagcg gacggagctc 120
accctgagcc ccgaggcccc agggcccctt gaggacaagg ggctggggac ccccgagcac 180
tgtccaggcc agtgcctgcc gtgggcgtgt aaggtgtgta agaggaagtc ggtgtccgtg 240
gaccggcggc gggcggccac actgagggag aagcgcaggc tcaagaaggt gaatgaggcc 300
ttcgaggccc tgaagagaag caccctgctc aaccccaacc agcggctgcc caaggtggag 360
atcctgcgca gtgccatcca gtacatcgag cgcctccagg ccctgctcag ctccctcaac 420
caggaggagc gtgacctccg ctaccggggc gggggcgggc cccagccagg ggtgcccagc 480
gaatgcagct ctcacagcgc ctcctgcagt ccagagtggg gcagtgcact ggagttcagc 540
gccaacccag gggatcatct gctcacggct gaccctacag atgcccacaa cctgcactcc 600
ctcacctcca tcgtggacag catcacagtg gaagatgtgt ctgtggcctt cccagatgaa 660
accatgccca actga 675

<210> 71
<211> 822
<212> DNA
<213> Homo sapiens

<400> 71
atgtcgctga ccaacacaaa gacggggttt tcggtcaagg acatcttaga cctgccggac 60
accaacgatg aggaggctc tgtggccgaa ggtccggagg aagagaacga ggggcccgag 120
ccagccaaga gggccgggcc gctggggcag ggcgccctgg acgcggtgca gagcctgccc 180

```
ctgaagaacc ccttctacga cagcagcgac aacccgtaca cgcgctggct ggccagcacc 240
gagggccttc agtactccct gcacggtctg gctgccgggg cgccccctca ggactcaagc 300
tccaagtccc cggagccctc ggccgacgag tcaccggaca atgacaagga gaccccgggc 360
ggcggggggg acgccggcaa gaagcgaaag cggcgagtgc ttttctccaa ggcgcagacc 420
tacgagctgg agcggcgctt tcggcagcag cggtacctgt cggcgcccga gcgcgaacac 480
ctggccagcc tcatccgcct cacgcccacg caggtcaaga tctggttcca gaaccaccgc 540
tacaagatga agcgcgcccg ggccgagaaa ggtatggagg tgacgcccct gccctcgccg 600
cgccgggtgg ccgtgcccgt cttggtcagg gacggcaaac catgtcacgc gctcaaagcc 660
caggacctgg cagccgccac cttccaggcg ggcattccct tttctgccta cagcgcgcag 720
tcgctgcagc acatgcagta caacgcccag tacagctcgg ccagcacccc ccagtacccg 780
acagcacacc ccctggtcca ggcccagcag tggacttggt ga                    822
```

<210> 72
<211> 7668
<212> DNA
<213> Homo sapiens

<400> 72

```
atgccgccgc tcctggcgcc cctgctctgc ctggcgctgc tgcccgcgct cgccgcacga 60
ggcccgcgat gctcccagcc cggtgagacc tgcctgaatg gcgggaagtg tgaagcggcc 120
aatggcacgg aggcctgcgt ctgtggcggg gccttcgtgg gcccgcgatg ccaggacccc 180
aacccgtgcc tcagcacccc ctgcaagaac gccgggacat gccacgtggt ggaccgcaga 240
ggcgtggcag actatgcctg cagctgtgcc ctgggcttct ctgggcccct ctgcctgaca 300
cccctggaca atgcctgcct caccaacccc tgccgcaacg gggcacctg cgacctgctc 360
acgctgacgg agtacaagtg ccgctgcccg cccggctggt cagggaaatc gtgccagcag 420
gctgacccgt gcgcctccaa cccctgcgcc aacggtggcc agtgcctgcc cttcgaggcc 480
tcctacatct gccactgccc acccagcttc catggcccca cctgccggca ggatgtcaac 540
gagtgtggcc agaagcccgg gctttgccgc cacggaggca cctgccacaa cgaggtcggc 600
tcctaccgct gcgtctgccg cgccacccac actggcccca actgcgagcg gccctacgtg 660
ccctgcagcc cctcgccctg ccagaacggg ggcacctgcc gccccacggg cgacgtcacc 720
cacgagtgtg cctgcctgcc aggcttcacc ggccagaact gtgaggaaaa tatcgacgat 780
tgtccaggaa acaactgcaa gaacgggggt gcctgtgtgg acggcgtgaa cacctacaac 840
tgccgctgcc cgccagagtg gacaggtcag tactgtaccg aggatgtgga cgagtgccag 900
ctgatgccaa atgcctgcca gaacggcggg acctgccaca acacccacgg tggctacaac 960
tgcgtgtgtg tcaacggctg gactggtgag gactgcagcg agaacattga tgactgtgcc 1020
agcgccgcct gcttccacgg cgccacctgc catgaccgtg tggcctcctt ctactgcgag 1080
tgtccccatg gccgcacagg tctgctgtgc cacctcaacg acgcatgcat cagcaacccc 1140
tgtaacgagg gctccaactg cgacaccaac cctgtcaatg gcaaggccat ctgcacctgc 1200
ccctcggggt acacgggccc ggcctgcagc caggacgtgg atgagtgctc gctgggtgcc 1260
aacccctgcg agcatgcggg caagtgcatc aacacgctgg gctccttcga gtgccagtgt 1320
ctgcagggct acacgggccc ccgatgcgag atcgacgtca acgagtgcgt ctcgaacccg 1380
tgccagaacg acgccacctg cctggaccag attggggagt tccagtgcat ctgcatgccc 1440
ggctacgagg gtgtgcactg cgaggtcaac acagacgagt gtgccagcag cccctgcctg 1500
cacaatggcc gctgcctgga caagatcaat gagttccagt gcgagtgccc cacgggcttc 1560
actgggcatc tgtgccagta cgatgtggac gagtgtgcca gcacccccctg caagaatggt 1620
gccaagtgcc tggacggacc caacacttac acctgtgtgt gcacggaagg gtacacgggg 1680
acgcactgcg aggtggacat cgatgagtgc gaccccgacc cctgccacta cggctcctgc 1740
aaggacggcg tcgccacctt cacctgcctc tgccgcccag gctacacggg ccaccactgc 1800
gagaccaaca tcaacgagtg ctccagccag ccctgccgcc acgggggcac ctgccaggac 1860
cgcgacaacg cctacctctg cttctgcctg aaggggacca caggacccaa ctgcgagatc 1920
aacctggatg actgtgccag cagcccctgc gactcgggca cctgtctgga caagatcgat 1980
ggctacgagt gtgcctgtga gccgggctac acagggagca tgtgtaacat caacatcgat 2040
gagtgtgcgg gcaacccctg ccacaacggg ggcacctgcg aggacggcat caatggcttc 2100
acctgccgct gccccgaggg ctaccacgac cccacctgcc tgtctgaggt caatgagtgc 2160
aacagcaacc cctgcgtcca cggggcctgc cgggacagcc tcaacgggta caagtgcgac 2220
tgtgaccctg ggtggagtgg gaccaactgt gacatcaaca acaatgagtg tgaatccaac 2280
ccttgtgtca acggcggcac ctgcaaagac atgaccagtg gctacgtgtg cacctgccgg 2340
```

gagggcttca gcggtcccaa ctgccagacc aacatcaacg agtgtgcgtc caacccatgt 2400
ctgaaccagg gcacgtgtat tgacgacgtt gccgggtaca agtgcaactg cctgctgccc 2460
tacacaggtg ccacgtgtga ggtggtgctg gccccgtgtg cccccagccc ctgcagaaac 2520
ggcggggagt gcaggcaatc cgaggactat gagagcttct cctgtgtctg ccccacgggc 2580
tggcaagggc agacctgtga ggtcgacatc aacgagtgcg ttctgagccc gtgccggcac 2640
ggcgcatcct gccagaacac ccacggcggc taccgctgcc actgccaggc cggctacagt 2700
gggcgcaact gcgagaccga catcgacgac tgccggccca acccgtgtca caacgggggc 2760
tcctgcacag acggcatcaa cacggccttc tgcgactgcc tgcccggctt ccggggcact 2820
ttctgtgagg aggacatcaa cgagtgtgcc agtgacccct gccgcaacgg ggccaactgc 2880
acggactgcg tggacagcta cacgtgcacc tgccccgcag gcttcagcgg gatccactgt 2940
gagaacaaca cgcctgactg cacagagagc tcctgcttca acggtggcac ctgcgtggac 3000
ggcatcaact cgttcacctg cctgtgtcca cccggcttca cgggcagcta ctgccagcac 3060
gatgtcaatg agtgcgactc acagccctgc ctgcatggcg gcacctgtca ggacggctgc 3120
ggctcctaca ggtgcacctg cccccagggc tacactggcc ccaactgcca gaaccttgtg 3180
cactggtgtg actcctcgcc ctgcaagaac ggcggcaaat gctggcagac ccacacccag 3240
taccgctgcg agtgccccag cggctggacc ggcctttact gcgacgtgcc cagcgtgtcc 3300
tgtgaggtgg ctgcgcagcg acaaggtgtt gacgttgccc gcctgtgcca gcatggaggg 3360
ctctgtgtgg acgcgggcaa cacgcaccac tgccgctgcc aggcgggcta cacaggcagc 3420
tactgtgagg acctggtgga cgagtgctca cccagcccct gccagaacgg ggccacctgc 3480
acggactacc tgggcggcta ctcctgcaag tgcgtggccg gctaccacgg ggtgaactgc 3540
tctgaggaga tcgacgagtg cctctcccac ccctgccaga acgggggcac ctgcctcgac 3600
ctccccaaca cctacaagtg ctcctgccca cggggcactc agggtgtgca ctgtgagatc 3660
aacgtggacg actgcaatcc ccccgttgac cccgtgtccc ggagccccaa gtgctttaac 3720
aacggcacct gcgtggacca ggtgggcggc tacagctgca cctgcccgcc gggcttcgtg 3780
ggtgagcgct gtgaggggga tgtcaacgag tgcctgtcca atccctgcga cgcccgtggc 3840
acccagaact gcgtgcagcg cgtcaatgac ttccactgcg agtgccgtgc tggtcacacc 3900
gggcgccgct gcgagtccgt catcaatggc tgcaaaggca agccctgcaa gaatgggggc 3960
acctgcgccg tggcctccaa caccgcccgc gggttcatct gcaagtgccc tgcgggcttc 4020
gagggcgcca cgtgtgagaa tgacgctcgt acctgcggca gcctgcgctg cctcaacggc 4080
ggcacatgca tctccggccc gcgcagcccc acctgcctgt gcctgggccc cttcacgggc 4140
cccgaatgcc agttcccggc cagcagcccc tgcctgggcg gcaacccctg ctacaaccag 4200
gggacctgtg agcccacatc cgagagcccc ttctaccgtt gcctgtgccc cgccaaattc 4260
aacgggctct tgtgccacat cctggactac agcttcgggg gtggggccgg gcgcgacatc 4320
cccccgccgc tgatcgagga ggcgtgcgag ctgcccgagt gccaggagga cgcgggcaac 4380
aaggtctgca gcctgcagtg caacaaccac gcgtgcggct gggacggcgg tgactgctcc 4440
ctcaacttca atgacccctg gaagaactgc acgcagtctc tgcagtgctg gaagtacttc 4500
agtgacggcc actgtgacag ccagtgcaac tcagccggct gcctcttcga cggctttgac 4560
tgccagcgtg cggaaggcca gtgcaacccc ctgtacgacc agtactgcaa ggaccacttc 4620
agcgacgggc actgcgacca gggctgcaac agcgcggagt gcgagtggga cgggctggac 4680
tgtgcggagc atgtacccga gaggctggcg gccggcacgc tggtggtggt ggtgctgatg 4740
ccgccggagc agctgcgcaa cagctccttc cacttcctgc gggagctcag ccgcgtgctg 4800
cacaccaacg tggtcttcaa gcgtgacgca cacggccagc agatgatctt cccctactac 4860
ggccgcgagg aggagctgcg caagcacccc atcaagcgtg ccgccgaggg ctgggccgca 4920
cctgacgccc tgctgggcca ggtgaaggcc tcgctgctcc ctggtggcag cgagggtggg 4980
cggcggcgga gggagctgga ccccatggac gtccgcggct ccatcgtcta cctggagatt 5040
gacaaccggc agtgtgtgca ggcctcctcg cagtgcttcc agagtgccac cgacgtggcc 5100
gcattcctgg gagcgctcgc ctcgctgggc agcctcaaca tcccctacaa gatcgaggcc 5160
gtgcagagtg agaccgtgga gccgcccccg ccggcgcagc tgcacttcat gtacgtggcg 5220
gcggccgcct ttgtgcttct gttcttcgtg ggctgcgggg tgctgctgtc ccgcaagcgc 5280
cggcggcagc atggccagct ctggttccct gagggcttca aagtgtctga ggccagcaag 5340
aagaagcggc gggagcccct cggcgaggac tccgtgggcc tcaagcccct gaagaacgct 5400
tcagacggtg ccctcatgga cgacaaccag aatgagtggg gggacgagga cctggagacc 5460
aagaagttcc ggttcgagga gcccgtggtt ctgcctgacc tggacgacca gacagaccac 5520
cggcagtgga ctcagcagca cctggatgcc gctgacctgc gcatgtctgc catggccccc 5580
acaccgcccc agggtgaggt tgacgccgac tgcatggacg tcaatgtccg cgggcctgat 5640
ggcttcaccc cgctcatgat cgcctcctgc agcgggggcg gcctggagac gggcaacagc 5700
gaggaagagg aggacgcgcc ggccgtcatc tccgacttca tctaccaggg cgccagcctg 5760
cacaaccaga cagaccgcac gggcgagacc gccttgcacc tggccgcccg ctactcacgc 5820

tctgatgccg ccaagcgcct gctggaggcc agcgcagatg ccaacatcca ggacaacatg 5880
ggccgcaccc cgctgcatgc ggctgtgtct gccgacgcac aaggtgtctt ccagatcctg 5940
atccggaacc gagccacaga cctggatgcc cgcatgcatg atggcacgac gccactgatc 6000
ctggctgccc gcctggccgt ggagggcatg ctggaggacc tcatcaactc acacgccgac 6060
gtcaacgccg tagatgacct gggcaagtcc gccctgcact gggccgccgc cgtgaacaat 6120
gtggatgccg cagttgtgct cctgaagaac ggggctaaca aagatatgca gaacaacagg 6180
gaggagacac ccctgtttct ggccgcccgg gagggcagct acgagaccgc caaggtgctg 6240
ctggaccact ttgccaaccg ggacatcacg gatcatatgg accgcctgcc gcgcgacatc 6300
gcacaggagc gcatgcatca cgacatcgtg aggctgctgg acgagtacaa cctggtgcgc 6360
agcccgcagc tgcacggagc cccgctgggg ggcacgccca ccctgtcgcc cccgctctgc 6420
tcgcccaacg gctacctggg cagcctcaag cccggcgtgc agggcaagaa ggtccgcaag 6480
cccagcagca aaggcctggc ctgtggaagc aaggaggcca aggacctcaa ggcacggagg 6540
aagaagtccc aggacggcaa gggctgcctg ctggacagct ccggcatgct ctcgcccgtg 6600
gactccctgg agtcacccca tggctacctg tcagacgtgg cctcgccgcc actgctgccc 6660
tccccgttcc agcagtctcc gtccgtgccc ctcaaccacc tgcctgggat gcccgacacc 6720
cacctgggca tcgggcacct gaacgtggcg gccaagcccg agatggcggc gctgggtggg 6780
ggcggccggc tggcctttga gactggccca cctcgtctct cccacctgcc tgtggcctct 6840
ggcaccagca ccgtcctggg ctccagcagc ggaggggccc tgaatttcac tgtgggcggg 6900
tccaccagtt tgaatggtca atgcgagtgg ctgtcccggc tgcagagcgg catggtgccg 6960
aaccaataca accctctgcg ggggagtgtg gcaccaggcc ccctgagcac acaggccccc 7020
tccctgcagc atggcatggt aggcccgctg cacagtagcc ttgctgccag cgccctgtcc 7080
cagatgatga gctaccaggg cctgcccagc acccggctgg ccacccagcc tcacctggtg 7140
cagacccagc aggtgcagcc acaaaactta cagatgcagc agcagaacct gcagccagca 7200
aacatccagc agcagcaaag cctgcagccg ccaccaccac caccacagcc gcaccttggc 7260
gtgagctcag cagccagcgg ccacctgggc cggagcttcc tgagtggaga gccgagccag 7320
gcagacgtgc agccactggg ccccagcagc ctggcggtgc acactattct gccccaggag 7380
agccccgccc tgcccacgtc gctgccatcc tcgctggtcc cacccgtgac cgcagcccag 7440
ttcctgacgc ccccctcgca gcacagctac tcctcgcctg tggacaacac ccccagccac 7500
cagctacagg tgcctgagca ccccttcctc accccgtccc ctgagtcccc tgaccagtgg 7560
tccagctcgt ccccgcattc caacgtctcc gactggtccg agggcgtctc cagccctccc 7620
accagcatgc agtcccagat cgcccgcatt ccggaggcct tcaagtaa 7668

<210> 73
<211> 6966
<212> DNA
<213> Homo sapiens

<400> 73
atggggccgg gggcccgtgg ccgccgccgc cgccgtcgcc cgatgtcgcc gccaccgcca 60
ccgccacccg tgcgggcgct gcccctgctg ctgctgctag cggggccggg ggctgcagcc 120
ccccccttgcc tggacggaag cccgtgtgca aatggaggtc gttgcaccca gctgccctcc 180
cgggaggctg cctgcctgtg cccgcctggc tgggtgggtg agcggtgtca gctggaggac 240
ccctgtcact caggcccctg tgctggccgt ggtgtctgcc agagttcagt ggtggctggc 300
accgcccgat tctcatgccg gtgcccccgt ggcttccgag gccctgactg ctccctgcca 360
gatccctgcc tcagcagccc ttgtgcccac ggtgcccgct gctcagtggg gcccgatgga 420
cgcttcctct gctcctgccc acctggctac cagggccgca gctgccgaag cgacgtggat 480
gagtgccggg tgggtgagcc ctgccgccat ggtggcacct gcctcaacac acctggctcc 540
ttccgctgcc agtgtccagc tggctacaca gggccactat gtgagaaccc cgcggtgccc 600
tgtgcaccct caccatgccg taacgggggc acctgcaggc agagtggcga cctcacttac 660
gactgtgcct gtcttcctgg gtttgagggt cagaattgtg aagtgaacgt ggacgactgt 720
ccaggacacc gatgtctcaa tgggggggaca tgcgtggatg gcgtcaacac ctataactgc 780
cagtgccctc ctgagtggac aggccagttc tgcacggagg acgtggatga gtgtcagctg 840
cagcccaacg cctgccacaa tgggggtacc tgcttcaaca cgctgggtgg ccacagctgc 900
gtgtgtgtca atggctggac aggcgagagc tgcagtcaga atatcgatga ctgtgccaca 960
gccgtgtgct tccatggggc cacctgccat gaccgcgtgg cttctttcta ctgtgcctgc 1020
cccatgggca agactggcct cctgtgtcac ctggatgacg cctgtgtcag caacccctgc 1080
cacgaggatg ctatctgtga cacaaatccg gtgaacggcc gggccatttg cacctgtcct 1140

cccggcttca cgggtggggc atgtgaccag gatgtggacg agtgctctat cggcgccaac 1200
ccctgcgagc acttgggcag gtgcgtgaac acgcagggct ccttcctgtg ccagtgcggt 1260
cgtggctaca ctggacctcg ctgtgagacc gatgtcaacg agtgtctgtc ggggccctgc 1320
cgaaaccagg ccacgtgcct cgaccgcata ggccagttca cctgtatctg tatggcaggc 1380
ttcacaggaa cctattgcga ggtggacatt gacgagtgtc agagtagccc ctgtgtcaac 1440
ggtggggtct gcaaggaccg agtcaatggc ttcagctgca cctgcccctc gggcttcagc 1500
ggctccacgt gtcagctgga cgtggacgaa tgcgccagca cgccctgcag gaatggcgcc 1560
aaatgcgtgg accagcccga tggctacgag tgccgctgtg ccgagggctt tgagggcacg 1620
ctgtgtgatc gcaacgtgga cgactgctcc cctgacccat gccaccatgg tcgctgcgtg 1680
gatggcatcg ccagcttctc atgtgcctgt gctcctggct acacgggcac acgctgcgag 1740
agccaggtgg acgaatgccg cagccagccc tgccgccatg gcggcaaatg cctagacctg 1800
gtggacaagt acctctgccg ctgcccttct gggaccacag gtgtgaactg cgaagtgaac 1860
attgacgact gtgccagcaa cccctgcacc tttggagtct gccgtgatgg catcaaccgc 1920
tacgactgtg tctgccaacc tggcttcaca gggccccttt gtaacgtgga gatcaatgag 1980
tgtgcttcca gcccatgcgg cgagggaggt tcctgtgtgg atggggaaaa tggcttccgc 2040
tgcctctgcc cgcctggctc cttgccccca ctctgcctcc ccccgagcca tccctgtgcc 2100
catgagccct gcagtcacgg catctgctat gatgcacctg gcgggttccg ctgtgtgtgt 2160
gagcctggct ggagtggccc ccgctgcagc cagagcctgg cccgagacgc ctgtgagtcc 2220
cagccgtgca gggccggtgg gacatgcagc agcgatggaa tgggtttcca ctgcacctgc 2280
ccgcctggtg tccagggacg tcagtgtgaa ctcctctccc cctgcacccc gaacccctgt 2340
gagcatgggg gccgctgcga gtctgcccct ggccagctgc ctgtctgctc ctgcccccag 2400
ggctggcaag gcccacgatg ccagcaggat gtggacgagt gtgctggccc cgcaccctgt 2460
ggccctcatg gtatctgcac caacctggca gggagtttca gctgcacctg ccatggaggg 2520
tacactggcc cttcctgcga tcaggacatc aatgactgtg accccaaccc atgcctgaac 2580
ggtggctcgt gccaagacgg cgtgggctcc ttttcctgct cctgcctccc tggtttcgcc 2640
ggcccacgat gcgcccgcga tgtggatgag tgcctgagca acccctgcgg cccgggcacc 2700
tgtaccgacc acgtggcctc cttcacctgc acctgcccgc caggctacgg aggcttccac 2760
tgcgaacagg acctgcccga ctgcagcccc agctcctgct tcaatggcgg gacctgtgtg 2820
gacggcgtga actcgttcag ctgcctgtgc cgtcccggct acacaggagc ccactgccaa 2880
catgaggcag acccctgcct ctcgcggccc tgcctacacg gggcgtctgc cagcgccgcc 2940
caccctggct tccgctgcac ctgcctcgag agcttcacgg cccgcagtg ccagacgctg 3000
gtggattggt gcagccgcca gccttgtcaa aacgggggtc gctgcgtcca gactggggcc 3060
tattgccttt gtccccctgg atggagcgga cgcctctgtg acatccgaag cttgccctgc 3120
agggaggccg cagcccagat cggggtgcgg ctggagcagc tgtgtcaggc gggtgggcag 3180
tgtgtggatg aagacagctc ccactactgc gtgtgcccag agggccgtac tggtagccac 3240
tgtgagcagg aggtggaccc ctgcttggcc cagccctgcc agcatggggg gacctgccgt 3300
ggctatatgg ggggctacat gtgtgagtgt cttcctggct acaatggtga taactgtgag 3360
gacgacgtgg acgagtgtgc ctcccagccc tgccagcacg gggggttcatg cattgacctc 3420
gtggcccgct atctctgctc ctgtccccca ggaacgctgg gggtgctctg cgagattaat 3480
gaggatgact gcggcccagg cccaccgctg gactcagggc cccggtgcct acacaatggc 3540
acctgcgtgg acctggtggg tggtttccgc tgcacctgtc ccccaggata cactggtttg 3600
cgctgcgagg cagacatcaa tgagtgtcgc tcaggtgcct gccacgcggc acacacccgg 3660
gactgcctgc aggacccagg cggaggtttc cgttgccttt gtcatgctgg cttctcaggt 3720
cctcgctgtc agactgtcct gtctccctgc gagtcccagc catgccagca tggaggccag 3780
tgccgtccta gcccgggtcc tggggggtggg ctgaccttca cctgtcactg tgcccagccg 3840
ttctggggtc cgcgttgcga gcgggtggcg cgctcctgcc gggagctgca gtgcccggtg 3900
ggcgtcccat gccagcagac gccccgcggg ccgcgctgcg cctgcccccc agggttgtcg 3960
ggaccctcct gccgcagctt cccggggtcg ccgccggggg ccagcaacgc cagctgcgcg 4020
gccgccccct gtctccacgg gggctcctgc cgccccgcgc cgctcgcgcc cttcttccgc 4080
tgcgcttgcg cgcagggctg gaccgggccg cgctgcgagg cgcccgccgc ggcacccgag 4140
gtctcggagg agccgcggtg cccgcgcgcc gcctgccagg ccaagcgcgg ggaccagcgc 4200
tgcgaccgcg agtgcaacag cccaggctgc ggctgggacg gcggcgactg ctcgctgagc 4260
gtgggcgacc cctggcggca atgcgaggcg ctgcagtgct ggcgcctctt caacaacagc 4320
cgctgcgacc ccgcctgcag ctcgcccgcc tgcctctacg acaacttcga ctgccacgcc 4380
ggtggccgcg agcgcacttg caacccggtg tacgagaagt actgcgccga ccactttgcc 4440
gacggccgct gcgaccaggg ctgcaacacg gaggagtgcg gctgggatgg gctggattgt 4500
gccagcgagg tgccggccct gctggcccgc ggcgtgctgg tgctcacagt gctgctgccg 4560
ccagaggagc tactgcgttc cagcgccgac tttctgcagc ggctcagcgc catcctgcgc 4620

acctcgctgc gcttccgcct ggacgcgcac ggccaggcca tggtcttccc ttaccaccgg 4680
cctagtcctg gctccgaacc ccgggcccgt cgggagctgg cccccgaggt gatcggctcg 4740
gtagtaatgc tggagattga caaccggctc tgcctgcagt cgcctgagaa tgatcactgc 4800
ttccccgatg cccagagcgc cgctgactac ctgggagcgt tgtcagcggt ggagcgcctg 4860
gacttcccgt acccactgcg ggacgtgcgg ggggagccgc tggagcctcc agaacccagc 4920
gtcccgctgc tgccactgct agtggcgggc gctgtcttgc tgctggtcat tctcgtcctg 4980
ggtgtcatgg tggcccggcg caagcgcgag cacagcaccc tctggttccc tgagggcttc 5040
tcactgcaca aggacgtggc ctctggtcac aagggccggc gggaacccgt gggccaggac 5100
gcgctgggca tgaagaacat ggccaagggt gagagcctga tgggggaggt ggccacagac 5160
tggatggaca cagagtgccc agaggccaag cggctaaagg tagaggagcc aggcatgggg 5220
gctgaggagg ctgtggattg ccgtcagtgg actcaacacc atctggttgc tgctgacatc 5280
cgcgtggcac cagccatggc actgacacca ccacagggcg acgcagatgc tgatggcatg 5340
gatgtcaatg tgcgtggccc agatggcttc accccgctaa tgctggcttc cttctgtggg 5400
ggggctctgg agccaatgcc aactgaagag gatgaggcag atgacacatc agctagcatc 5460
atctccgacc tgatctgcca gggggctcag cttggggcac ggactgaccg tactggcgag 5520
actgctttgc acctggctgc ccgttatgcc cgtgctgatg cagccaagcg gctgctggat 5580
gctggggcag acaccaatgc ccaggaccac tcaggccgca ctcccctgca cacagctgtc 5640
acagccgatg cccagggtgt cttccagatt ctcatccgaa accgctctac agacttggat 5700
gcccgcatgg cagatggctc aacggcactg atcctggcgg cccgcctggc agtagagggc 5760
atggtggaag agctcatcgc cagccatgct gatgtcaatg ctgtggatga gcttgggaaa 5820
tcagccttac actgggctgc ggctgtgaac aacgtggaag ccactttggc cctgctcaaa 5880
aatggagcca ataaggacat gcaggatagc aaggaggaga ccccccctatt cctggccgcc 5940
cgcgagggca gctatgaggc tgccaagctg ctgttggacc actttgccaa ccgtgagatc 6000
accgaccacc tggacaggct gccgcgggac gtagcccagg agagactgca ccaggacatc 6060
gtgcgcttgc tggatcaacc cagtgggccc cgcagccccc ccggtcccca cggcctgggg 6120
cctctgctct gtcctccagg ggccttcctc cctggcctca aagcggcaca gtcggggtcc 6180
aagaagagca ggaggccccc cgggaaggcg gggctggggc cgcaggggcc ccgggggcgg 6240
ggcaagaagc tgacgctggc ctgcccgggc cccctggctg acagctcggt cacgctgtcg 6300
cccgtggact cgctggactc cccgcggcct ttcggtgggc cccctgcttc ccctggtggc 6360
ttccccccttg aggggcccta tgcagctgcc actgccactg cagtgtctct ggcacagctt 6420
ggtggcccag gccgggcggg tctagggcgc cagcccccctg gaggatgtgt actcagcctg 6480
ggcctgctga accctgtggc tgtgcccctc gattgggccc ggctgccccc acctgcccct 6540
ccaggcccct cgttcctgct gccactggcg ccgggacccc agctgctcaa cccagggacc 6600
cccgtctccc cgcaggagcg gcccccgcct tacctggcag tcccaggaca tggcgaggag 6660
tacccggcgg ctggggcaca cagcagcccc ccaaaggccc gcttcctgcg ggttcccagt 6720
gagcaccctt acctgacccc atcccccgaa tcccctgagc actgggccag cccctcacct 6780
ccctccctct cagactggtc cgaatccacg cctagcccag ccactgccac tggggccatg 6840
gccaccacca ctggggcact gcctgcccag ccacttccct tgtctgttcc cagctccctt 6900
gctcaggccc agacccagct ggggccccag ccggaagtta cccccaagag gcaagtgttg 6960
gcctga 6966

<210> 74
<211> 1797
<212> DNA
<213> Homo sapiens

<400> 74
atgccttgtg ttcaggcgca gtatgggtcc tcgcctcaag gagccagccc cgcttctcag 60
agctacagtt accactcttc gggagaatac agctccgatt tcttaactcc agagtttgtc 120
aagtttagca tggacctcac caacactgaa atcactgcca ccacttctct ccccagcttc 180
agtaccttta tggacaacta cagcacaggc tacgacgtca agccaccttg cttgtaccaa 240
atgcccctgt ccggacagca gtcctccatt aaggtagaag acattcagat gcacaactac 300
cagcaacaca gccacctgcc cccccagtct gaggagatga tgccgcactc cgggtcggtt 360
tactacaagc cctcctcgcc cccgacgccc accaccccgg gcttccaggt gcagcacagc 420
cccatgtggg acgacccggg atctctccac aacttccacc agaactacgt ggccactacg 480
cacatgatcg agcagaggaa aacgccagtc tcccgcctct ccctcttctc ctttaagcaa 540
tcgccccctg gcaccccggt gtctagttgc cagatgcgct tcgacgggcc cctgcacgtc 600

cccatgaacc cggagcccgc cggcagccac cacgtggtgg acgggcagac cttcgctgtg 660
cccaacccca ttcgcaagcc cgcgtccatg ggcttcccgg gcctgcagat cggccacgcg 720
tctcagctgc tcgacacgca ggtgccctca ccgccgtcgc ggggctcccc ctccaacgag 780
gggctgtgcg ctgtgtgtgg ggacaacgcg gcctgccaac actacggcgt gcgcacctgt 840
gagggctgca aaggcttctt taagcgcaca gtgcaaaaaa atgcaaaata cgtgtgttta 900
gcaaataaaa actgcccagt ggacaagcgt cgccggaatc gctgtcagta ctgccgattt 960
cagaagtgcc tggctgttgg gatggtcaaa gaagtggttc gcacagacag tttaaaaggc 1020
cggagaggtc gtttgccctc gaaaccgaag agcccacagg agccctctcc cccttcgccc 1080
ccggtgagtc tgatcagtgc cctcgtcagg gcccatgtcg actccaaccc ggctatgacc 1140
agcctggact attccaggtt ccaggcgaac cctgactatc aaatgagtgg agatgacacc 1200
cagcatatcc agcaattcta tgatctcctg actggctcca tggagatcat ccggggctgg 1260
gcagagaaga tccctggctt cgcagacctg cccaaagccg accaagacct gctttttgaa 1320
tcagctttct tagaactgtt tgtccttcga ttagcataca ggtccaaccc agtggagggt 1380
aaactcatct tttgcaatgg ggtggtcttg cacaggttgc aatgcgttcg tggctttggg 1440
gaatggattg attccattgt tgaattctcc tccaacttgc agaatatgaa catcgacatt 1500
tctgccttct cctgcattgc tgccctggct atggtcacag agagacacgg gctcaaggaa 1560
cccaagagag tggaagaact gcaaaacaag attgtaaatt gtctcaaaga ccacgtgact 1620
ttcaacaatg gggggttgaa ccgccccaat tatttgtcca aactgttggg gaagctccca 1680
gaacttcgta ccctttgcac acaggggcta cagcgcattt tctacctgaa attggaagac 1740
ttggtgccac cgccagcaat aattgacaaa cttttcctgg acactttacc tttctaa 1797

<210> 75
<211> 1074
<212> DNA
<213> Homo sapiens

<400> 75
atgcagagtg tgcagagcac gagcttttgt ctccgaaagc agtgcctttg cctgaccttc 60
ctgcttctcc atctcctggg acaggtcgct gcgactcagc gctgccctcc ccagtgcccg 120
ggccggtgcc ctgcgacgcc gccgacctgc gcccccgggg tgcgcgcggt gctggacggc 180
tgctcatgct gtctggtgtg tgcccgccag cgtggcgaga gctgctcaga tctggagcca 240
tgcgacgaga gcagtggcct ctactgtgat cgcagcgcgg accccagcaa ccagactggc 300
atctgcacgg cggtagaggg agataactgt gtgttcgatg gggtcatcta ccgcagtgga 360
gagaaatttc agccaagctg caaattccag tgcacctgca gagatgggca gattggctgt 420
gtgccccgct gtcagctgga tgtgctactg cctgagccta actgcccagc tccaagaaaa 480
gttgaggtgc ctggagagtg ctgtgaaaag tggatctgtg gcccagatga ggaggattca 540
ctgggaggcc ttacccttgc agcttacagg ccagaagcca ccctaggagt agaagtctct 600
gactcaagtg tcaactgcat tgaacagacc acagagtgga cagcatgctc caagagctgt 660
ggtatggggt tctccacccg ggtcaccaat aggaaccgtc aatgtgagat gctgaaacag 720
actcggctct gcatggtgcg gccctgtgaa caagagccag agcagccaac agataagaaa 780
ggaaaaaagt gtctccgcac caagaagtca ctcaaagcca tccacctgca gttcaagaac 840
tgcaccagcc tgcacaccta caagcccagg ttctgtgggg tctgcagtga tggccgctgc 900
tgcactcccc acaataccaa aaccatccag gcagagtttc agtgctcccc agggcaaata 960
gtcaagaagc cagtgatggt cattgggacc tgcacctgtc acaccaactg tcctaagaac 1020
aatgaggcct tcctccagga gctggagctg aagactacca gagggaaaat gtaa 1074

<210> 76
<211> 768
<212> DNA
<213> Homo sapiens

<400> 76
atgctgcggc cacagcggcc cggagacttg cagctcgggg cctccctcta cgagctggtg 60
ggctacaggc agccgccctc ctcctcctcc tcctccacct cctccacctc ctccacttcc 120
tcctcctcca cgacggcccc cctcctcccc aaggctgcgc gcgagaagcc ggaggcgccg 180
gccgagcctc caggccccgg gcccgggtca ggcgcgcacc cgggcggcag cgcccggccg 240

gacgccaagg aggagcagca gcagcagctg cggcgcaaga tcaacagccg cgagcggaag 300
cgcatgcagg acctgaacct ggccatggac gccctgcgcg aggtcatcct gccctactca 360
gcggcgcact gccagggcgc gcccggccgc aagctctcca agatagccac gctgctgctc 420
gcccgcaact acatcctact gctgggcagc tcgctgcagg agctgcgccg cgcgctgggc 480
gagggcgccg ggcccgccgc gccgcgcctg ctgctggccg ggctgcccct gctcgccgcc 540
gcgcccggct ccgtgttgct ggcgcccggc gccgtaggac cccccgacgc gctgcgcccc 600
gccaagtacc tgtcgctggc gctggacgag ccgccgtgcg gccagttcgc tctccccggc 660
ggcggcgcag gcggccccgg cctctgcacc tgcgccgtgt gcaagttccc gcacctggtc 720
ccggccagcc tgggcctggc cgccgtgcag gcgcaattct ccaagtga 768

<210> 77
<211> 972
<212> DNA
<213> Homo sapiens

<400> 77
atggactcgg acgccagcct ggtgtccagc cgcccgtcgt cgccagagcc cgatgacctt 60
tttctgccgg cccggagtaa gggcagcagc ggcagcgcct tcactggggg caccgtgtcc 120
tcgtccaccc cgagtgactg cccgccggag ctgagcgccg agctgcgcgg cgctatgggc 180
tctgcgggcg cgcatcctgg ggacaagcta ggaggcagtg gcttcaagtc atcctcgtcc 240
agcacctcgt cgtctacgtc gtcggcggct gcgtcgtcca ccaagaagga caagaagcaa 300
atgacagagc cggagctgca gcagctgcgt ctcaagatca acagccgcga gcgcaagcgc 360
atgcacgacc tcaacatcgc catggatggc ctccgcgagg tcatgccgta cgcacacggc 420
ccttcggtgc gcaagctttc caagatcgcc acgctgctgc tggcgcgcaa ctacatcctc 480
atgctcacca actcgctgga ggagatgaag cgactggtga gcgagatcta cgggggccac 540
cacgctggct tccacccgtc ggcctgcggc ggcctggcgc actccgcgcc cctgcccgcc 600
gccaccgcgc acccggcagc agcagcgcac gccgcacatc accccgcggt gcaccacccc 660
atcctgccgc ccgccgccgc agcggctgct gccgccgctg cagccgcggc tgtgtccagc 720
gcctctctgc ccggatccgg gctgccgtcg gtcggctcca tccgtccacc gcacggccta 780
ctcaagtctc cgtctgctgc cgcggccgcc ccgctggggg gcgggggcgg cggcagtggg 840
gcgagcgggg gcttccagca ctgggcggc atgccctgcc cctgcagcat gtgccaggtg 900
ccgccgccgc accaccacgt gtcggctatg ggcgccggca gcctgccgcg cctcacctcc 960
gacgccaagt ga 972

<210> 78
<211> 852
<212> DNA
<213> Homo sapiens

<400> 78
atgaacggcg aggagcagta ctacgcggcc acgcagcttt acaaggaccc atgcgcgttc 60
cagcgaggcc cggcgccgga gttcagcgcc agcccccctg cgtgcctgta catgggccgc 120
cagcccccgc cgccgccgcc gcacccgttc cctggcgccc tgggcgcgct ggagcagggc 180
agcccccgg acatctcccc gtacgaggtg cccccccctcg ccgacgaccc cgcggtggcg 240
caccttcacc accacctccc ggctcagctc gcgctccccc acccgcccgc cgggcccttc 300
ccggagggag ccgagccggg cgtcctggag gagcccaacc gcgtccagct gcctttccca 360
tggatgaagt ctaccaaagc tcacgcgtgg aaaggccagt gggcaggcgg cgcctacgct 420
gcggagccgg aggagaacaa gcggacgcgc acggcctaca cgcgcgcaca gctgctagag 480
ctggagaagg agttcctatt caacaagtac atctcacggc cgcgccgggt ggagctggct 540
gtcatgttga acttgaccga gagacacatc aagatctggt tccaaaaccg ccgcatgaag 600
tggaaaaagg aggaggacaa gaagcgcggc ggcgggacag ctgtcggggg tggcggggtc 660
gcggagcctg agcaggactg cgccgtgacc tccggcgagg agcttctggc gctgccgccg 720
ccgccgcccc ccggaggtgc tgtgccgccc gctgcccccg ttgccgcccg agaggccgc 780
ctgccgcctg gccttagcgc gtcgccacag ccctccagcg tcgcgcctcg gcggccgcag 840
gaaccacgat ga 852

<210> 79
<211> 717
<212> DNA
<213> Homo sapiens

<400> 79
atgagacaga gcggcgcctc ccagcccctg ctgatcaaca tgtacctgcc agatcccgtc 60
ggagacggtc tcttcaagga cgggaagaac ccgagctggg ggccgctgag ccccgcggtt 120
cagaaaggca gcggacagat ccagctgtgg cagtttctgc tggagctgct ggctgaccgc 180
gcgaacgccg gctgcatcgc gtgggagggc ggtcacggcg agttcaagct cacggacccg 240
gacgaggtgg cgcggcggtg gggcgagcgc aagagcaagc ccaacatgaa ctacgacaag 300
ctgagccgcg ccctgcgcta ctactacgac aagaacatca tgagcaaggt gcatggcaag 360
cgctacgcct accgcttcga cttccagggc ctggcgcagg cctgccagcc gccgcccgcg 420
cacgctcatg ccgccgccgc agctgctgcc gccgccgcgg ccgcccagga cggcgcgctc 480
tacaagctgc ccgccggcct cgccccgctg cccttccccg gcctctccaa actcaacctc 540
atggccgcct cggccggggt cgcgcccgcc ggcttctcct actggccggg cccgggcccc 600
gccgccaccg ctgccgccgc caccgccgcg ctctacccca gtcccagctt gcagcccccg 660
cccgggccct tcggggccgt ggccgcagcc tcgcacttgg ggggccatta ccactag 717

<210> 80
<211> 855
<212> DNA
<213> Homo sapiens

<400> 80
atggactact cctacctcaa ttcgtacgac tcgtgcgtgg cggccatgga ggcgtccgcc 60
tacggcgact ttggcgcctg cagccagccc ggcggcttcc aatacagccc cctgcggccc 120
gctttccccg cggcagggcc gccctgcccc gcgctcggct cctccaactg cgcacttggc 180
gccctacgcg accaccagcc cgcgccctac tcggcagtgc cctacaagtt cttcccagag 240
ccatccggcc tgcacgagaa gcgcaagcag cggcgcatcc gcaccacgtt caccagcgcg 300
cagctcaagg agctggagcg cgttttcgct gagacccact accccgacat ttacacgcgt 360
gaggagctgg cgctcaagat cgacctcact gaggctcgcg tgcaggtctg gttccagaac 420
cgccgggcca agttccgcaa acaggagcgc gcggccagcg ccaagggcgc ggcgggcgcg 480
gcgggcgcca aaaagggcga ggcgcgctgc tcctccgagg acgacgattc caaggagtcc 540
acgtgcagcc ccacgcccga tagcaccgcc tcgctgccgc cgccgcctgc gcccggcctg 600
gccagcccgc gcctgagccc cagcccgctg cccgtcgcac tgggctccgg gccgggacct 660
gggccggggc cacagccgct caagggcgca ctgtgggccg gtgtggcggg cggtgggggc 720
ggcgggcctg gcgcgggagc ggccgaacta cttaaggctt ggcagccggc ggagtccggc 780
cccgggccct tctccggggt tctgtcctcc tttcaccgga agcccggccc cgccctgaag 840
accaatctct tctag 855

<210> 81
<211> 945
<212> DNA
<213> Homo sapiens

<400> 81
atgtataaaa tggaatattc ttacctcaat tcctctgcct acgagtcctg tatggctggg 60
atggacacct cgagcctggc ttcagcctat gctgacttca gttcctgcag ccaggccagt 120
ggcttccagt ataacccgat aaggaccact tttggggcca cgtccggctg cccttccctc 180
acgccgggat cctgcagcct gggcaccctc agggaccacc agagcagtcc gtacgccgca 240
gttccttaca aactcttcac ggaccacggc ggcctcaacg agaagcgcaa gcagcggcgc 300
atccgcacca ctttcaccag tgcccagctc aaagagctgg aaagggtctt cgcggagact 360
cactaccccg acatctacac tcgggaggag ctggccctga agatcgacct cacagaggcg 420
cgagtccagg tgtggttcca gaaccgccgc gccaagtttc gcaagcagga gcgcgcagcg 480

gcagccgcag cggccgcggc caagaacggc tcctcgggca aaaagtctga ctcttccagg 540
gacgacgaga gcaaagaggc caagagcact gacccggaca gcactggggg cccaggtccc 600
aatcccaacc ccacccccag ctgcggggcg aatggaggcg gcggcggcgg gcccagcccg 660
gctggagctc cgggggcggc ggggcccggg ggcccgggag gcgaacccgg caagggcggc 720
gcagcagcag cggcggcggc cgcggcagcg gcggcggcgg cagcggcagc ggcggcagct 780
ggaggcctgg ctgcggctgg gggccctgga caaggctggg ctcccggccc cggccccatc 840
acctccatcc cggattcgct tgggggtccc ttcgccagcg tcctatcttc gctccaaaga 900
cccaacggtg ccaaagccgc cttagtgaag agcagtatgt tctga 945

<210> 82
<211> 876
<212> DNA
<213> Homo sapiens

<400> 82
atgagttgcc aagcttttac ttcggctgat acctttatac ctctgaattc tgacgcctct 60
gcaactctgc ctctgataat gcatcacagt gctgccgagt gtctaccagt ctccaaccat 120
gccaccaatg tgatgtctac agcaacagga cttcattatt ctgttccttc ctgtcattat 180
ggaaaccagc catcaaccta tggagtgatg gcaggtagtt taaccccttg tctttataaa 240
tttcctgacc acaccttgag tcatggattt cctcctatac accagcctct tctggcagag 300
gaccccacag ctgctgattt caagcaggaa ctcaggcgga aaagtaaatt ggtggaagag 360
ccaatagaca tggattctcc agaaatcaga gaacttgaaa agtttgccaa tgaatttaaa 420
gtgagacgaa ttaaattagg atacacccag acaaatgttg gggaggccct ggcagctgtg 480
catggctctg aattcagtca aacaacaatc tgccgatttg aaaatctgca gctcagcttt 540
aaaaatgcat gcaaactgaa agcaatatta tccaaatggc tggaggaagc tgagcaagta 600
ggagctttgt acaatgaaaa agtgggagca aatgaaagga aaagaaaacg aagaacaact 660
ataagcattg ctgctaaaga tgctctggag agacactttg gagaacagaa taaaccttct 720
tctcaagaga tcatgaggat ggctgaagaa ctgaatctgg agaaagaagt agtaagagtt 780
tggttttgca accggaggca gagagaaaaa cgggtgaaaa caagtctgaa tcagagttta 840
ttttctattt ctaaggaaca tcttgagtgc agataa 876

<210> 83
<211> 909
<212> DNA
<213> Homo sapiens

<400> 83
atggagttcg gcctgctcag cgaggcagag gcccggagcc ctgccctgtc gctgtcagac 60
gctggcactc cgcaccccca gctcccagag cacggctgca agggccagga gcacagcgac 120
tcagaaaagg cctcggcttc gctgcccggc ggctccccag aggacggttc gctgaaaaag 180
aagcagcggc ggcagcgcac gcacttcacc agccagcagc tacaggagct agaggcgacc 240
ttccagagga accgctaccc cgacatgagc acgcgcgagg agatcgccgt gtggaccaac 300
ctcaccgagg cccgcgtgcg ggtgtggttc aagaaccggc gcgccaaatg gcggaagcgc 360
gagcgcagcc agcaggccga gctatgcaaa ggcagcttcg cggcgccgct cgggggggctg 420
gtgccgccct acgaggaggt gtaccccggc tactcgtacg gcaactggcc gcccaaggct 480
cttgccccgc cgctcgccgc caagaccttt ccattcgcct tcaactcggt caacgtgggg 540
cctctggctt cgcagcccgt cttctcgcca cccagctcca tcgccgcctc catggtgccc 600
tccgccgcgg ctgccccggg caccgtgcca gggcctgggg ccctgcaggg cctgggcggg 660
ggcccccccg ggctggctcc ggccgccgtg tcctccgggg ccgtgtcctg cccttatgcc 720
tcggccgccg ccgccgccgc ggctgccgcc tcttccccct acgtctatcg ggacccgtgt 780
aactcgagcc tggccagcct gcggctcaaa gccaaacagc acgcctcctt cagctacccc 840
gctgtgcacg ggccgccccc ggcagccaac cttagtccgt gccagtacgc cgtggaaagg 900
cccgtatga 909

<210> 84
<211> 1362
<212> DNA
<213> Homo sapiens

<400> 84
atgcgtatcc ccgtagatgc cagcacgagc cgccgcttca cgccgccttc caccgcgctg 60
agcccaggca agatgagcga ggcgttgccg ctgggcgccc cggacgccgg cgctgccctg 120
gccggcaagc tgaggagcgg cgaccgcagc atggtggagg tgctggccga ccacccgggc 180
gagctggtgc gcaccgacag ccccaacttc ctctgctccg tgctgcctac gcactggcgc 240
tgcaacaaga ccctgcccat cgctttcaag gtggtggccc taggggatgt tccagatggc 300
actctggtca ctgtgatggc tggcaatgat gaaaactact cggctgagct gagaaatgct 360
accgcagcca tgaagaacca ggttgcaaga tttaatgacc tcaggtttgt cggtcgaagt 420
ggaagaggga aaagcttcac tctgaccatc actgtcttca caaacccacc gcaagtcgcc 480
acctaccaca gagccatcaa aatcacagtg gatgggcccc gagaacctcg aagacatcgg 540
cagaaactag atgatcagac caagcccggg agcttgtcct tttccgagcg gctcagtgaa 600
ctggagcagc tgcggcgcac agccatgagg gtcagcccac accacccagc ccccacgccc 660
aaccctcgtg cctccctgaa ccactccact gcctttaacc ctcagcctca gagtcagatg 720
caggatacaa ggcagatcca accatcccca ccgtggtcct acgatcagtc ctaccaatac 780
ctgggatcca ttgcctctcc ttctgtgcac ccagcaacgc ccatttcacc tggacgtgcc 840
agcggcatga caaccctctc tgcagaactt tccagtcgac tctcaacggc acccgacctg 900
acagcgttca gcgacccgcg ccagttcccc gcgctgccct ccatctccga cccccgcatg 960
cactatccag gcgccttcac ctactccccg acgccggtca cctcgggcat cggcatcggc 1020
atgtcggcca tgggctcggc cacgcgctac cacacctacc tgccgccgcc ctaccccggc 1080
tcgtcgcaag cgcagggagg cccgttccaa gccagctcgc cctcctacca cctgtactac 1140
ggcgcctcgg ccggctccta ccagttctcc atggtgggcg gcgagcgctc gccgccgcgc 1200
atcctgccgc cctgcaccaa cgcctccacc ggctccgcgc tgctcaaccc cagcctcccg 1260
aaccagagcg acgtggtgga ggccgagggc agccacagca actcccccac caacatggcg 1320
ccctccgcgc gcctggagga ggccgtgtgg aggccctact ga 1362

<210> 85
<211> 1704
<212> DNA
<213> Homo sapiens

<400> 85
atgcttcatt cgcctcacaa acaaccacag aaccacaagt gcggtgcaaa ctttctccag 60
gaggacagca agaagtctct ggtttttaaa tggttaatct ccgcaggtca ctaccagcca 120
ccgagaccaa cagagtcatt taaggctgca agcagtattt acaacagagg gtacaagttc 180
tatctgaaaa aaaaaggagg gactatggca tcaaacagcc tcttcagcac agtgacacca 240
tgtcagcaaa acttcttttg ggatccgagc accagccggc gcttcagccc cccctccagc 300
agcctgcagc ccggcaaaat gagcgacgtg agcccggtgg tggctgcgca acagcagcag 360
caacagcagc agcagcaaca gcagcagcag cagcagcaac agcagcagca gcagcaggag 420
gcggcggcgg cggctgcggc ggcggcggcg gctgcggcgg cggcagctgc agtgccccgg 480
ttgcggccgc cccacgacaa ccgcaccatg gtggagatca tcgccgacca cccggccgaa 540
ctcgtccgca ccgacagccc caacttcctg tgctcggtgc tgccctcgca ctggcgctgc 600
aacaagaccc tgcccgtggc cttcaaggtg gtagccctcg gagaggtacc agatgggact 660
gtggttactg tcatggcggg taacgatgaa aattattctg ctgagctccg gaatgcctct 720
gctgttatga aaaaccaagt agcaaggttc aacgatctga gatttgtggg ccggagtgga 780
cgaggcaaga gtttcacctt gaccataacc gtcttcacaa atcctcccca agtagctacc 840
tatcacagag caattaaagt tacagtagat ggacctcggg aacccagaag gcacagacag 900
aagcttgatg actctaaacc tagtttgttc tctgaccgcc tcagtgattt agggcgcatt 960
cctcatccca gtatgagagt aggtgtcccg cctcagaacc cacggccctc cctgaactct 1020
gcaccaagtc cttttaatcc acaaggacag agtcagatta cagaccccag gcaggcacag 1080
tcttccccgc cgtggtccta tgaccagtct tacccctcct acctgagcca gatgacgtcc 1140
ccgtccatcc actctaccac cccgctgtct tccacacggg gcactgggct tcctgccatc 1200
accgatgtgc ctaggcgcat ttcaggtgct tcagaactgg gccctttttc agaccccagg 1260

cagttcccaa gcatttcatc cctcactgag agccgcttct ccaacccacg aatgcactat 1320
ccagccacct ttacttacac cccgccagtc acctcaggca tgtccctcgg tatgtccgcc 1380
accactcact accacaccta cctgccacca ccctaccccg gctcttccca aagccagagt 1440
ggacccttcc agaccagcag cactccatat ctctactatg gcacttcgtc aggatcctat 1500
cagtttccca tggtgccggg gggagaccgg tctccttcca gaatgcttcc gccatgcacc 1560
accacctcga atggcagcac gctattaaat ccaaatttgc ctaaccagaa tgatggtgtt 1620
gacgctgatg gaagccacag cagttcccca actgttttga attctagtgg cagaatggat 1680
gaatctgttt ggcgaccata ttga 1704

<210> 86
<211> 1389
<212> DNA
<213> Homo sapiens

<400> 86
atgctgctgc tggcgagatg tctgctgcta gtcctcgtct cctcgctgct ggtatgctcg 60
ggactggcgt gcggaccggg caggggggttc gggaagagga ggcaccccaa aaagctgacc 120
cctttagcct acaagcagtt tatccccaat gtggccgaga agaccctagg cgccagcgga 180
aggtatgaag ggaagatctc cagaaactcc gagcgattta aggaactcac ccccaattac 240
aaccccgaca tcatatttaa ggatgaagaa aacaccggag cggacaggct gatgactcag 300
aggtgtaagg acaagttgaa cgctttggcc atctcggtga tgaaccagtg gccaggagtg 360
aaactgcggg tgaccgaggg ctgggacgaa gatggccacc actcagagga gtctctgcac 420
tacgagggcc gcgcagtgga catcaccacg tctgaccgcg accgcagcaa gtacggcatg 480
ctggcccgcc tggcggtgga ggccggcttc gactgggtgt actacgagtc caaggcacat 540
atccactgct cggtgaaagc agagaactcg gtggcggcca aatcgggagg ctgcttcccg 600
ggctcggcca cggtgcacct ggagcagggc ggcaccaagc tggtgaagga cctgagcccc 660
ggggaccgcg tgctggcggc ggacgaccag ggccggctgc tctacagcga cttcctcact 720
ttcctggacc gcgacgacgg cgccaagaag gtcttctacg tgatcgagac gcgggagccg 780
cgcgagcgcc tgctgctcac cgccgcgcac ctgctctttg tggcgccgca caacgactcg 840
gccaccgggg agcccgaggc gtcctcgggc tcggggccgc cttccggggg cgcactgggg 900
cctcgggcgc tgttcgccag ccgcgtgcgc ccgggccagc gcgtgtacgt ggtggccgag 960
cgtgacgggg accgccggct cctgcccgcc gctgtgcaca gcgtgaccct aagcgaggag 1020
gccgcgggcg cctacgcgcc gctcacggcc cagggcacca ttctcatcaa ccgggtgctg 1080
gcctcgtgct acgcggtcat cgaggagcac agctgggcgc accgggcctt cgcgcccttc 1140
cgcctggcgc acgcgctcct ggctgcactg gcgcccgcgc gcacggaccg cggcggggac 1200
agcggcggcg gggaccgcgg gggcggcggc ggcagagtag ccctaaccgc tccaggtgct 1260
gccgacgctc cgggtgcggg ggccaccgcg ggcatccact ggtactcgca gctgctctac 1320
caaataggca cctggctcct ggacagcgag gccctgcacc cgctgggcat ggcggtcaag 1380
tccagctga 1389

<210> 87
<211> 1530
<212> DNA
<213> Homo sapiens

<400> 87
atgaatctcc tggacccctt catgaagatg accgacgagc aggagaaggg cctgtccggc 60
gcccccagcc ccaccatgtc cgaggactcc gcgggctcgc cctgcccgtc gggctccggc 120
tcggacaccg agaacacgcg gccccaggag aacacgttcc ccaagggcga gcccgatctg 180
aagaaggaga gcgaggagga caagttcccc gtgtgcatcc gcgaggcggt cagccaggtg 240
ctcaaaggct acgactggac gctggtgccc atgccggtgc gcgtcaacgg ctccagcaag 300
aacaagccgc acgtcaagcg gcccatgaac gccttcatgg tgtggcgcca ggcggcgcgc 360
aggaagctcg cggaccagta cccgcacttg cacaacgccg agctcagcaa gacgctgggc 420
aagctctgga gacttctgaa cgagagcgag aagcggccct tcgtggagga ggcggagcgg 480
ctgcgcgtgc agcacaagaa ggaccacccg gattacaagt accagccgcg gcggaggaag 540
tcggtgaaga acgggcaggc ggaggcagag gaggccacgg agcagacgca catctccccc 600

aacgccatct tcaaggcgct gcaggccgac tcgccacact cctcctccgg catgagcgag 660
gtgcactccc ccggcgagca ctcggggcaa tcccagggcc caccgacccc acccaccacc 720
cccaaaaccg acgtgcagcc gggcaaggct gacctgaagc gagaggggcg ccccttgcca 780
gaggggggca gacagccccc tatcgacttc cgcgacgtgg acatcggcga gctgagcagc 840
gacgtcatct ccaacatcga gaccttcgat gtcaacgagt ttgaccagta cctgccgccc 900
aacggccacc cgggggtgcc ggccacgcac ggccaggtca cctacacggg cagctacggc 960
atcagcagca ccgcggccac cccggcgagc gcgggccacg tgtggatgtc caagcagcag 1020
gcgccgccgc cacccccgca gcagccccca caggccccgc cggccccgca ggcgcccccg 1080
cagccgcagg cggcgccccc acagcagccg gcggcacccc cgcagcagcc acaggcgcac 1140
acgctgacca cgctgagcag cgagccgggc cagtcccagc gaacgcacat caagacggag 1200
cagctgagcc ccagccacta cagcgagcag cagcagcact cgccccaaca gatcgcctac 1260
agccccttca acctcccaca ctacagcccc tcctacccgc ccatcacccg ctcacagtac 1320
gactacaccg accaccagaa ctccagctcc tactacagcc acgcggcagg ccagggcacc 1380
ggcctctact ccaccttcac ctacatgaac cccgctcagc gccccatgta caccccccatc 1440
gccgacacct ctggggtccc ttccatcccg cagacccaca gcccccagca ctgggaacaa 1500
cccgtctaca cacagctcac tcgaccttga 1530

<210> 88
<211> 1245
<212> DNA
<213> Homo sapiens

<400> 88
atgagcagcc cggatgcggg atacgccagt gacgaccaga gccagaccca gagcgcgctg 60
cccgcggtga tggccgggct gggcccctgc ccctgggccg agtcgctgag ccccatcggg 120
gacatgaagg tgaagggcga ggcgccggcg aacagcggag caccggccgg ggccgcgggc 180
cgagccaagg gcgagtcccg tatccggcgg ccgatgaacg ctttcatggt gtgggctaag 240
gacgagcgca agcggctggc gcagcagaat ccagacctgc acaacgccga gttgagcaag 300
atgctgggca agtcgtggaa ggcgctgacg ctggcggaga agcggccctt cgtggaggag 360
gcagagcggc tgcgcgtgca gcacatgcag gaccacccca actacaagta ccggccgcgg 420
cggcgcaagc aggtgaagcg gctgaagcgg gtggagggcg gcttcctgca cggcctggct 480
gagccgcagg cggccgcgct gggccccgag ggcggccgcg tggccatgga cggcctgggc 540
ctccagttcc ccgagcaggg cttccccgcc ggcccgccgc tgctgcctcc gcacatgggc 600
ggccactacc gcgactgcca gagtctgggc gcgcctccgc tcgacggcta cccgttgccc 660
acgcccgaca cgtccccgct ggacggcgtg gaccccgacc cggctttctt cgccgccccg 720
atgcccgggg actgcccggc ggccggcacc tacagctacg cgcaggtctc ggactacgct 780
ggcccccccgg agcctcccgc cggtcccatg cacccccgac tcggcccaga gcccgcgggt 840
ccctcgattc cgggcctcct ggcgccaccc agcgcccttc acgtgtacta cggcgcgatg 900
ggctcgcccg gggcgggcgg cgggcgcggc ttccagatgc agccgcaaca ccagcaccag 960
caccagcacc agcaccaccc ccgggcccc ggacagccgt cgccccctcc ggaggcactg 1020
ccctgccggg acggcacgga ccccagtcag cccgccgagc tcctcgggga ggtggaccgc 1080
acggaatttg aacagtatct gcacttcgtg tgcaagcctg agatgggcct cccctaccag 1140
gggcatgact ccggtgtgaa tctccccgac agccacgggg ccatttcctc ggtggtgtcc 1200
gacgccagct ccgcggtata ttactgcaac tatcctgacg tgtga 1245

<210> 89
<211> 987
<212> DNA
<213> Homo sapiens

<400> 89
atgactggag tctttgacag tctagtggct gatatgcact cgacccagat cgccgcctcc 60
agcacgtacc accagcacca gcagcccccg agcggcggcg gcgccggccc gggtggcaac 120
agcagcagca gcagcagcct ccacaagccc caggagtcgc ccacccttcc ggtgtccacc 180
gccaccgaca gcagctacta caccaaccag cagcacccgg cgggcggcgg cggcggcggg 240
ggctcgccct acgcgcacat gggttcctac cagtaccaag ccagcggcct caacaacgtc 300

ccttactccg ccaagagcag ctatgacctg ggctacaccg ccgcctacac ctcctacgct 360
ccctatggaa ccagttcgtc cccagccaac aacgagcctg agaaggagga ccttgagcct 420
gaaattcgga tagtgaacgg gaagccaaag aaagtccgga aaccccgcac catctactcc 480
agtttccagc tggcggctct tcagcggcgt ttccaaaaga ctcaatactt ggccttgccg 540
gagcgagccg agctggcggc ctctctgggc ctcacccaga ctcaggtcaa aatctggttc 600
cagaaccgcc ggtccaagtt caagaagatg tggaaaagtg gtgagatccc ctcggagcag 660
caccctgggg ccagcgcttc tccaccttgt gcttcgccgc cagtctcagc gccggcctcc 720
tgggactttg gtgtgccgca gcggatggcg ggcggcggtg gtccgggcag tggcggcagc 780
ggcgccggca gctcgggctc cagcccgagc agcgcggcct cggcttttct gggcaactac 840
ccctggtacc accagacctc gggatccgcc tcacacctgc aggccacggc gccgctgctg 900
caccccactc agaccccgca gccgcatcac caccaccacc atcacggcgg cgggggcgcc 960
ccggtgagcg cggggacgat tttctaa 987

<210> 90
<211> 870
<212> DNA
<213> Homo sapiens

<400> 90
atgacaggag tgtttgacag aagggtcccc agcatccgat ccggcgactt ccaagctccg 60
ttccagacgt ccgcagctat gcaccatccg tctcaggaat cgccaacttt gcccgagtct 120
tcagctaccg attctgacta ctacagccct acgggggggag ccccgcacgg ctactgctct 180
cctacctcgg cttcctatgg caaagctctc aacccctacc agtatcagta tcacggcgtg 240
aacggctccg ccgggagcta cccagccaaa gcttatgccg actatagcta cgctagctcc 300
taccaccagt acggcggcgc ctacaaccgc gtcccaagcg ccaccaacca gccagagaaa 360
gaagtgaccg agcccgaggt gagaatggtg aatggcaaac caaagaaagt tcgtaaaccc 420
aggactattt attccagctt tcagctggcc gcattacaga gaaggtttca gaagactcag 480
tacctcgcct tgccggaacg cgccgagctg gccgcctcgc tgggattgac acaaacacag 540
gtgaaaatct ggtttcagaa caaaagatcc aagatcaaga agatcatgaa aaacggggag 600
atgcccccgg agcacagtcc cagctccagc gacccaatgg cgtgtaactc gccgcagtct 660
ccagcggtgt gggagcccca gggctcgtcc cgctcgctca gccaccaccc tcatgcccac 720
cctccgacct ccaaccagtc cccagcgtcc agctacctgg agaactctgc atcctggtac 780
acaagtgcag ccagctcaat caattcccac ctgccgccgc cgggctcctt acagcacccg 840
ctggcgctgg cctccgggac actctattag 870

<210> 91
<211> 843
<212> DNA
<213> Homo sapiens

<400> 91
atgccagctg atataatgga gaaaaattcc tcgtccccgg tggctgctac cccagccagt 60
gtcaacacga caccggataa accaaagaca gcatctgagc acagaaagtc atcaaagcct 120
attatggaga aaagacgaag agcaagaata aatgaaagtc tgagccagct gaaaacactg 180
attttggatg ctctgaagaa agatagctcg cggcattcca agctggagaa ggcggacatt 240
ctggaaatga cagtgaagca cctccggaac ctgcagcggg cgcagatgac ggctgcgctg 300
agcacagacc caagtgtgct ggggaagtac cgagccggct tcagcgagtg catgaacgag 360
gtgacccgct tcctgtccac gtgcgagggc gttaataccg aggtgcgcac tcggctgctc 420
ggccacctgg ccaactgcat gacccagatc aatgccatga cctaccccgg gcagccgcac 480
cccgccttgc aggcgccgcc accgccccca ccgggacccg gcggccccca gcacgcgccg 540
ttcgcgccgc cgccgccact cgtgcccatc cccgggggcg cggcgccccc tcccggcggc 600
gccccctgca agctgggcag ccaggctgga gaggcggcta aggtgtttgg aggcttccag 660
gtggtaccgg ctcccgatgg ccagtttgct ttcctcattc ccaacggggc cttcgcgcac 720
agcggccctg tcatccccgt ctacaccagc aacagcggca cctccgtggg ccccaacgca 780
gtgtcacctt ccagcggccc ctcgcttacg gcggactcca tgtggaggcc gtggcggaac 840
tga 843

<210> 92
<211> 648
<212> DNA
<213> Homo sapiens

<400> 92
atgggcagcc cccgctccgc gctgagctgc ctgctgttgc acttgctggt cctctgcctc 60
caagcccagg taactgttca gtcctcacct aattttacac agcatgtgag ggagcagagc 120
ctggtgacgg atcagctcag ccgccgcctc atccggacct accaactcta cagccgcacc 180
agcgggaagc acgtgcaggt cctggccaac aagcgcatca acgccatggc agaggacggc 240
gaccccttcg caaagctcat cgtggagacg gacacctttg gaagcagagt tcgagtccga 300
ggagccgaga cgggcctcta catctgcatg aacaagaagg ggaagctgat cgccaagagc 360
aacggcaaag gcaaggactg cgtcttcacg gagattgtgc tggagaacaa ctacacagcg 420
ctgcagaatg ccaagtacga gggctggtac atggccttca cccgcaaggg ccggcccgc 480
aagggctcca agacgcggca gcaccagcgt gaggtccact tcatgaagcg gctgccccgg 540
ggccaccaca ccaccgagca gagcctgcgc ttcgagttcc tcaactaccc gcccttcacg 600
cgcagcctgc gcggcagcca gaggacttgg gcccccgagc cccgatag 648

<210> 93
<211> 975
<212> DNA
<213> Homo sapiens

<400> 93
atgaactgca tgaaaggccc gcttcacttg gagcaccgag cagcggggac caagctgtcg 60
gccgtctcct catcttcctg tcaccatccc cagccgttag ccatggcttc ggttctggct 120
cccggtcagc cccggtcgct ggactcctcc aagcacaggc tggaggtgca caccatctcc 180
gacacctcca gcccggaggc cgcagagaaa gataaaagcc agcaggggaa gaatgaggac 240
gtgggcgccg aggacccgtc taagaagaag cggcaaaggc ggcagcggac tcactttacc 300
agccagcagc tccaggagct ggaggccact ttccagagga accgctaccc ggacatgtcc 360
acacgcgaag aaatcgctgt gtggaccaac cttacggaag cccgagtccg ggtttggttc 420
aagaatcgtc gggccaaatg gagaaagagg gagcgcaacc agcaggccga gctatgcaag 480
aatggcttcg ggccgcagtt caatgggctc atgcagccct acgacgacat gtacccaggc 540
tattcctaca acaactgggc cgccaagggc cttacatccg cctccctatc caccaagagc 600
ttccccttct tcaactctat gaacgtcaac cccctgtcat cacagagcat gttttcccca 660
cccaactcta tctcgtccat gagcatgtcg tccagcatgg tgccctcagc agtgacaggc 720
gtcccgggct ccagtctcaa cagcctgaat aacttgaaca acctgagtag cccgtcgctg 780
aattccgcgg tgccgacgcc tgcctgtcct tacgcgccgc cgactcctcc gtatgtttat 840
agggacacgt gtaactcgag cctggccagc ctgagactga aagcaaagca gcactccagc 900
ttcggctacg ccagcgtgca gaacccggcc tccaacctga gtgcttgcca gtatgcagtg 960
gaccggcccg tgtga 975

<210> 94
<211> 987
<212> DNA
<213> Mus musculus

<400> 94
atggccaccc aggtgatggg gcagtcttct ggaggaggca gtctcttcaa caacagtgcc 60
aacatgggca tggccttaac caacgacatg tacgacctgc acgagctctc gaaagctgaa 120
ctggcagccc ctcagctcat catgttagcc aacgtggccc tgacggggga ggcaagcggc 180
agctgctgcg attacctggt cggtgaagag aggcagatgg ccgaattgat gcccgtggga 240
gacaaccact tctcagaaag tgaaggagaa ggcctggaag agtcggctga cctcaaaggg 300
ctggaaaaca tggaactggg aagtttggag ctaagtgctg tagaacccca gcccgtattt 360

gaagcctcag ctgccccaga aatatacagc gccaataaag atcccgctcc agaaacaccc 420
gtggcggaag acaaatgcag gagttctaag gccaagccct tccggtgtaa gccttgccag 480
tacgaagccg aatctgaaga gcagtttgtg catcacatcc ggattcacag cgctaagaag 540
ttctttgtgg aggaaagtgc agagaaacag gccaaagcct gggagtcggg gtcgtctccg 600
gccgaagagg gcgagttctc caaaggcccc atccgctgtg accgctgtgg ctacaatacc 660
aaccggtatg accactacat ggcacacctg aagcaccacc tgcgagctgg cgagaacgag 720
cgcatctaca agtgcatcat ctgcacgtac acgacggtca gcgagtacca ctggaggaaa 780
cacctgagaa accatttccc caggaaagtc tacacctgca gcaagtgcaa ctacttctca 840
gacagaaaaa ataactacgt tcagcacgtg cgaactcaca caggagaacg cccgtataaa 900
tgtgaacttt gtccttactc aagctctcag aagactcatc taacgcgaca catgcggact 960
cattcagagt gtgatctagc tgggtga 987

<210> 95
<211> 1025
<212> DNA
<213> Homo sapiens

<400> 95
atgaccatgg aatctggagc cgagaaccag cagagtggag atgcagctgt aacagaagct 60
gaaaaccaac aaatgacagt tcaagcccag ccacagattg ccacattagc ccaggtatct 120
atgccagcag ctcatgcaac atcatctgct cccaccgtaa ctctagtaca gctgcccaat 180
gggcagacag ttcaagtcca tggagtcatt caggcggccc agccatcagt tattcagtct 240
ccacaagtcc aaacagttca gtcttcctgt aaggacttaa aaagactttt ctccggaaca 300
cagatttcaa ctattgcaga aagtgaagat tcacaggagt cagtggatag tgtaactgat 360
tcccaaaagc gaagggaaat tctttcaagg aggccttcct acaggaaaat tttgaatgac 420
ttatcttctg atgcaccagg agtgccaagg attgaagaag agaagtctga agaggagact 480
tcagcacctg ccatcaccac tgtaacggtg ccaactccaa tttaccaaac tagcagtgga 540
cagtatattg ccattaccca gggaggagca atacagctgg ctaacaatgg taccgatggg 600
gtacagggcc tgcaaacatt aaccatgacc aatgcagcag ccactcagcc gggtactacc 660
attctacagt atgcacagac cactgatgga cagcagatct tagtgcccag caaccaagtt 720
gttgttcaag ctgcctctgg agacgtacaa acataccaga ttcgcacagc acccactagc 780
actattgccc ctggagttgt tatggcatcc tccccagcac ttcctacaca gcctgctgaa 840
gaagcagcac gaaagagaga ggtccgtcta atgaagaaca gggaagcagc tcgagagtgt 900
cgtagaaaga agaaagaata tgtgaaatgt ttagaaaaca gagtggcagt gcttgaaaat 960
caaaacaaga cattgattga ggagctaaaa gcacttaagg acctttactg ccacaaatca 1020
gatta 1025

<210> 96
<211> 963
<212> DNA
<213> Mus musculus

<400> 96
atggctgagg gcaaaggggc tcctctgagg ccttcagttg agaagagatg gaagctcatg 60
gaacccaagc agacccaggc agggatgttc aagaaaatga gccttgtgga ctctgacact 120
gctgcaggaa agggtagcca agatgaggcc tatactgaac tgagcctgcc aacagcaccg 180
aacaagcctc gactggacag gcctcgggcc tgcaaggcat acacagagca gaggcacaat 240
accttcacag agctatcatg tctccaggag aggccagggg acatccaggc ccagacgagg 300
aagctggaga acccagaagg ccagctcggc cctcagcagc tgccctcgag tttcctcaga 360
gcctcaggtg atggcacagt gtgttcagca tggccaggtg ccccccggag tgagcagaaa 420
agtgctttca gcaagccagc caaacgccca gcagagaaac ctaagcgctc tcccatgctt 480
ctggctggtg gaagtgcaga gggctcatgg gagctctcag gactcatcac cactgtggac 540
atcccatatt gggctcatct gtcaactttc aagttcatgg gtgatttctg gaaattgcac 600
acattgtcac agaacattct cctctgcaat gctttccagg gggctcccac accatggctg 660
gagcataccc aggtacaagc ccccacatcc tcagctcctt cctccacagc ctcccgggct 720
ctcttgccgc ccacactctc ctccttgggc ttgtctactc agaactggtg tgcgaagtgc 780

aacctagcct ttcgcctgac agctgacctg gtcttccaca tgcggtcaca tcacaaaagg 840
gaacacgtgg gccctgaccc acattctaag aaacgaagag aggaagttct cacttgcccc 900
gtttgccacg agtacttccg ggagcgccac catctgtcca ggcatatggc ttcacatagt 960
tag 963

<210> 97
<211> 1374
<212> DNA
<213> Homo sapiens

<400> 97
atgctgggag cggtgaagat ggaagggcac gagccgtccg actggagcag ctactatgca 60
gagcccgagg gctactcctc cgtgagcaac atgaacgccg gcctggggat gaacggcatg 120
aacacgtaca tgagcatgtc ggcggccgcc atgggcagcg gctcgggcaa catgagcgcg 180
ggctccatga acatgtcgtc gtacgtgggc gctggcatga gcccgtccct ggcggggatg 240
tcccccggcg cgggcgccat ggcgggcatg ggcggctcgg ccggggcggc cggcgtggcg 300
ggcatggggc cgcacttgag tcccagcctg agcccgctcg gggggcaggc ggccggggcc 360
atgggcggcc tggcccccta cgccaacatg aactccatga gccccatgta cgggcaggcg 420
ggcctgagcc gcgcccgcga ccccaagacc tacaggcgca gctacacgca cgcaaagccg 480
ccctactcgt acatctcgct catcaccatg gccatccagc agagccccaa caagatgctg 540
acgctgagcg agatctacca gtggatcatg gacctcttcc ccttctaccg gcagaaccag 600
cagcgctggc agaactccat ccgccactcg ctctccttca acgactgttt cctgaaggtg 660
ccccgctcgc ccgacaagcc cggcaagggc tccttctgga ccctgcaccc tgactcgggc 720
aacatgttcg agaacggctg ctacctgcgc cgccagaagc gcttcaagtg cgagaagcag 780
ctggcgctga aggaggccgc aggcgccgcc ggcagcggca agaaggcggc cgccggagcc 840
caggcctcac aggctcaact cggggaggcc gccgggccgg cctccgagac tccggcgggc 900
accgagtcgc ctcactcgag cgcctccccg tgccaggagc acaagcgagg gggcctggga 960
gagctgaagg ggacgccggc tgcggcgctg agccccccag agccggcgcc ctctcccggg 1020
cagcagcagc aggccgcggc ccacctgctg ggcccgcccc accacccggg cctgccgcct 1080
gaggcccacc tgaagccgga acaccactac gccttcaacc acccgttctc catcaacaac 1140
ctcatgtcct cggagcagca gcaccaccac agccaccacc accaccaacc ccacaaaatg 1200
gacctcaagg cctacgaaca ggtgatgcac taccccggct acggttcccc catgcctggc 1260
agcttggcca tgggcccggt cacgaacaaa acgggcctgg acgcctcgcc cctggccgca 1320
gatacctcct actaccaggg ggtgtactcc cggcccatta tgaactcctc ttaa 1374

<210> 98
<211> 1221
<212> DNA
<213> Homo sapiens

<400> 98
atggatggat ggagaaggat gcctcgctgg ggactgctgc tgctgctctg ggctcctgt 60
acctttggtc tcccgacaga caccaccacc tttaaacgga tcttcctcaa gagaatgccc 120
tcaatccgag aaagcctgaa ggaacgaggt gtggacatgg ccaggcttgg tcccgagtgg 180
agccaaccca tgaagaggct gacacttggc aacaccacct cctccgtgat cctcaccaac 240
tacatggaca cccagtacta tggcgagatt gggatcggga ccccacccca aaccttcaaa 300
gtcgtctttg acactggttc gtccaatgtt tgggtgccct cctccaagtg cagccgtctc 360
tacactgcct gtgtgtatca caagctcttc gatgcttcgg attcctccag ctacaagcac 420
aatggaacag aactcaccct ccgctattca acagggacag tcagtggctt tctcagccag 480
gacatcatca ccgtgggtgg aatcacggtg acacagatgt ttggagaggt cacggagatg 540
cccgccttac ccttcatgct ggccgagttt gatggggttg tgggcatggg cttcattgaa 600
caggccattg gcagggtcac ccctatcttc gacaacatca tctcccaagg ggtgctaaaa 660
gaggacgtct tctctttcta ctacaacaga gattccgaga attcccaatc gctgggagga 720
cagattgtgc tgggaggcag cgacccccag cattacgaag ggaatttcca ctatatcaac 780
ctcatcaaga ctggtgtctg gcagattcaa atgaaggggg tgtctgtggg gtcatccacc 840
ttgctctgtg aagacggctg cctggcattg gtagacaccg gtgcatccta catctcaggt 900

tctaccagct ccatagagaa gctcatggag gccttgggag ccaagaagag gctgtttgat 960
tatgtcgtga agtgtaacga gggccctaca ctccccgaca tctctttcca cctgggaggc 1020
aaagaataca cgctcaccag cgcggactat gtatttcagg aatcctacag tagtaaaaag 1080
ctgtgcacac tggccatcca cgccatggat atcccgccac ccactggacc cacctgggcc 1140
ctgggggcca ccttcatccg aaagttctac acagagtttg atcggcgtaa caaccgcatt 1200
ggcttcgcct tggcccgctg a 1221

<210> 99
<211> 654
<212> DNA
<213> Homo sapiens

<400> 99
atgagtctgg taggtggttt tccccaccac ccggtggtgc accacgaggg ctacccgttt 60
gccgccgccg ccgccgcagc tgccgccgcc gccgccagcc gctgcagcca tgaggagaac 120
ccctacttcc atggctggct catcggccac cccgagatgt cgccccccga ctacagcatg 180
gccctgtcct acagccccga gtatgccagc ggcgccgccg gcctggacca ctcccattac 240
gggggggtgc cgccgggcgc cgggcccccg ggcctggggg ggccgcgccc ggtgaagcgc 300
cgaggcaccg ccaaccgcaa ggagcggcgc aggactcaga gcatcaacag cgccttcgcc 360
gaactgcgcg agtgcatccc caacgtaccc gccgacacca aactctccaa aatcaagacc 420
ctgcgcctgg ccaccagcta catcgcctac ctcatggacc tgctggccaa ggacgaccag 480
aatggcgagg cggaggcctt caaggcagag atcaagaaga ccgacgtgaa agaggagaag 540
aggaagaagg agctgaacga aatcttgaaa agcacagtga gcagcaacga caagaaaacc 600
aaaggccgga cgggctggcc gcagcacgtc tgggccctgg agctcaagca gtga 654

<210> 100
<211> 942
<212> DNA
<213> Homo sapiens

<400> 100
atgacttctt gtcacattgc tgaagaacat atacaaaagg ttgctatctt tggaggaacc 60
catgggaatg agctaaccgg agtatttctg gttaagcatt ggctagagaa tggcgctgag 120
attcagagaa cagggctgga ggtaaaacca tttattacta accccagagc agtgaagaag 180
tgtaccagat atattgactg tgacctgaat cgcatttttg accttgaaaa tcttggcaaa 240
aaaatgtcag aagatttgcc atatgaagtg agaagggctc aagaaataaa tcatttattt 300
ggtccaaaag acagtgaaga ttcctatgac attatttttg accttcacaa caccacctct 360
aacatggggt gcactcttat tcttgaggat tccaggaata actttttaat tcagatgttt 420
cattacatta agacttctct ggctccacta ccctgctacg tttatctgat tgagcatcct 480
tccctcaaat atgcgaccac tcgttccata gccaagtatc ctgtgggtat agaagttggt 540
cctcagcctc aaggggttct gagagctgat atcttggatc aaatgagaaa aatgattaaa 600
catgctcttg attttataca tcatttcaat gaaggaaaag aatttcctcc ctgcgccatt 660
gaggtctata aaattataga gaaagttgat taccccccggg atgaaaatgg agaaattgct 720
gctatcatcc atcctaatct gcaggatcaa gactggaaac cactgcatcc tggggatccc 780
atgtttttaa ctcttgatgg gaagacgatc ccactgggcg gagactgtac cgtgtacccc 840
gtgtttgtga atgaggccgc atattacgaa aagaaagaag cttttgcaaa gacaactaaa 900
ctaacgctca atgcaaaaag tattcgctgc tgtttacatt ag 942

<210> 101
<211> 1590
<212> DNA
<213> Homo sapiens

<400> 101
atgacaagct ccaggctttg gttttcgctg ctgctggcgg cagcgttcgc aggacgggcg 60

acggccctct ggccctggcc tcagaacttc caaacctccg accagcgcta cgtcctttac 120
ccgaacaact ttcaattcca gtacgatgtc agctcggccg cgcagcccgg ctgctcagtc 180
ctcgacgagg ccttccagcg ctatcgtgac ctgcttttcg gttccgggtc ttggccccgt 240
ccttacctca cagggaaacg gcatacactg gagaagaatg tgttggttgt ctctgtagtc 300
acacctggat gtaaccagct tcctactttg gagtcagtgg agaattatac cctgaccata 360
aatgatgacc agtgtttact cctctctgag actgtctggg gagctctccg aggtctggag 420
acttttagcc agcttgtttg gaaatctgct gagggcacat tctttatcaa caagactgag 480
attgaggact ttccccgctt tcctcaccgg ggcttgctgt tggatacatc tcgccattac 540
ctgccactct ctagcatcct ggacactctg gatgtcatgg cgtacaataa attgaacgtg 600
ttccactggc atctggtaga tgatccttcc ttcccatatg agagcttcac ttttccagag 660
ctcatgagaa aggggtccta caaccctgtc acccacatct acacagcaca ggatgtgaag 720
gaggtcattg aatacgcacg gctccggggt atccgtgtgc ttgcagagtt tgacactcct 780
ggccacactt tgtcctgggg accaggtatc cctggattac tgactccttg ctactctggg 840
tctgagccct ctggcacctt tggaccagtg aatcccagtc tcaataatac ctatgagttc 900
atgagcacat tcttcttaga agtcagctct gtcttcccag atttttatct tcatcttgga 960
ggagatgagg ttgatttcac ctgctggaag tccaacccag agatccagga ctttatgagg 1020
aagaaaggct tcggtgagga cttcaagcag ctggagtcct tctacatcca gacgctgctg 1080
gacatcgtct cttcttatgg caagggctat gtggtgtggc aggaggtgtt tgataataaa 1140
gtaaagattc agccagacac aatcatacag gtgtggcgag aggatattcc agtgaactat 1200
atgaaggagc tggaactggt caccaaggcc ggcttccggg cccttctctc tgccccctgg 1260
tacctgaacc gtatatccta tggccctgac tggaaggatt tctacgtagt ggaacccctg 1320
gcatttgaag gtacccctga gcagaaggct ctggtgattg gtggagaggc ttgtatgtgg 1380
ggagaatatg tggacaacac aaacctggtc cccaggctct ggcccagagc aggggctgtt 1440
gccgaaaggc tgtggagcaa caagttgaca tctgacctga catttgccta tgaacgtttg 1500
tcacacttcc gctgtgagtt gctgaggcga ggtgtccagg cccaacccct caatgtaggc 1560
ttctgtgagc aggagtttga acagacctga 1590

<210> 102
<211> 657
<212> DNA
<213> Homo sapiens

<400> 102
atggcgaccc gcagccctgg cgtcgtgatt agtgatgatg aaccaggtta tgaccttgat 60
ttattttgca tacctaatca ttatgctgag gatttggaaa gggtgtttat tcctcatgga 120
ctaattatgg acaggactga acgtcttgct cgagatgtga tgaaggagat gggaggccat 180
cacattgtag ccctctgtgt gctcaagggg ggctataaat tctttgctga cctgctggat 240
tacatcaaag cactgaatag aaatagtgat agatccattc ctatgactgt agattttatc 300
agactgaaga gctattgtaa tgaccagtca acaggggaca taaaagtaat tggtggagat 360
gatctctcaa ctttaactgg aaagaatgtc ttgattgtgg aagatataat tgacactggc 420
aaaacaatgc agactttgct ttccttggtc aggcagtata atccaaagat ggtcaaggtc 480
gcaagcttgc tggtgaaaag gaccccacga agtgttggat ataagccaga ctttgttgga 540
tttgaaattc cagacaagtt tgttgtagga tatgcccttg actataatga atacttcagg 600
gatttgaatc atgtttgtgt cattagtgaa actggaaaag caaaatacaa agcctaa 657

<210> 103
<211> 1956
<212> DNA
<213> Homo sapiens

<400> 103
atggcccggg ggtcggcggt tgcctgggcg gcgctcgggc cgttgttgtg ggctgcgcg 60
ctggggctgc agggcgggat gctgtacccc caggagagcc cgtcgcggga gtgcaaggag 120
ctggacggcc tctggagctt ccgcgccgac ttctctgaca accgacgccg ggcttcgag 180
gagcagtggt accggcggcc gctgtgggag tcaggcccca ccgtggacat gccagttccc 240
tccagcttca atgacatcag ccaggactgg cgtctgcggc atttgtcgg ctgggtgtgg 300

tacgaacggg aggtgatcct gccggagcga tggacccagg acctgcgcac aagagtggtg 360
ctgaggattg gcagtgccca ttcctatgcc atcgtgtggg tgaatggggt cgacacgcta 420
gagcatgagg ggggctacct ccccttcgag gccgacatca gcaacctggt ccaggtgggg 480
cccctgccct cccggctccg aatcactatc gccatcaaca acacactcac cccccaccacc 540
ctgccaccag ggaccatcca atacctgact gacacctcca agtatcccaa gggttacttt 600
gtccagaaca catattttga ctttttcaac tacgctggac tgcagcggtc tgtacttctg 660
tacacgacac ccaccaccta catcgatgac atcaccgtca ccaccagcgt ggagcaagac 720
agtgggctgg tgaattacca gatctctgtc aagggcagta acctgttcaa gttggaagtg 780
cgtcttttgg atgcagaaaa caaagtcgtg gcgaatggga ctgggaccca gggccaactt 840
aaggtgccag gtgtcagcct ctggtggccg tacctgatgc acgaacgccc tgcctatctg 900
tattcattgg aggtgcagct gactgcacag acgtcactgg ggcctgtgtc tgacttctac 960
acactccctg tggggatccg cactgtggct gtcaccaaga gccagttcct catcaatggg 1020
aaacctttct atttccacgg tgtcaacaag catgaggatg cggacatccg agggaagggc 1080
ttcgactggc cgctgctggt gaaggacttc aacctgcttc gctggcttgg tgccaacgct 1140
ttccgtacca gccactaccc ctatgcagag gaagtgatgc agatgtgtga ccgctatggg 1200
attgtggtca tcgatgagtg tcccggcgtg ggcctggcgc tgccgcagtt cttcaacaac 1260
gtttctctgc atcaccacat gcaggtgatg gaagaagtgg tgcgtaggga caagaaccac 1320
cccgcggtcg tgatgtggtc tgtggccaac gagcctgcgt cccacctaga atctgctggc 1380
tactacttga agatggtgat cgctcacacc aaatccttgg acccctcccg gcctgtgacc 1440
tttgtgagca actctaacta tgcagcagac aagggggctc cgtatgtgga tgtgatctgt 1500
ttgaacagct actactcttg gtatcacgac tacgggcacc tggagttgat tcagctgcag 1560
ctggccaccc agtttgagaa ctggtataag aagtatcaga agcccattat tcagagcgag 1620
tatggagcag aaacgattgc agggtttcac caggatccac ctctgatgtt cactgaagag 1680
taccagaaaa gtctgctaga gcagtaccat ctgggtctgg atcaaaaacg cagaaaatac 1740
gtggttggag agctcatttg gaattttgcc gatttcatga ctgaacagtc accgacgaga 1800
gtgctgggga ataaaaaggg gatcttcact cggcagagac aaccaaaaag tgcagcgttc 1860
cttttgcgag agagatactg gaagattgcc aatgaaacca ggtatcccca ctcagtagcc 1920
aagtcacaat gtttggaaaa cagcccgttt acttga 1956

<210> 104
<211> 1671
<212> DNA
<213> Homo sapiens

<400> 104
atggagctgt gcgggctggg gctgccccgg ccgcccatgc tgctggcgct gctgttggcg 60
acactgctgg cggcgatgtt ggcgctgctg actcaggtgg cgctggtggt gcaggtggcg 120
gaggcggctc gggccccgag cgtctcggcc aagccggggc cggcgctgtg gcccctgccg 180
ctctcggtga agatgacccc gaacctgctg catctcgccc cggagaactt ctacatcagc 240
cacagcccca attccacggc gggcccctcc tgcaccctgc tggaggaagc gtttcgacga 300
tatcatggct atatttttgg tttctacaag tggcatcatg aacctgctga attccaggct 360
aaaacccagg ttcagcaact tcttgtctca atcacccttc agtcagagtg tgatgctttc 420
cccaacatat cttcagatga gtcttatact ttacttgtga aagaaccagt ggctgtcctt 480
aaggccaaca gagtttgggg agcattacga ggtttagaga cctttagcca gttagtttat 540
caagattctt atggaacttt caccatcaat gaatccacca ttattgattc tccaaggttt 600
tctcacagag gaattttgat tgatacatcc agacattatc tgccagttaa gattattctt 660
aaaactctgg atgccatggc ttttaataag tttaatgttc ttcactggca catagttgat 720
gaccagtctt tcccatatca gagcatcact tttcctgagt taagcaataa aggaagctat 780
tctttgtctc atgtttatac accaaatgat gtccgtatgg tgattgaata tgccagatta 840
cgaggaattc gagtcctgcc agaatttgat accccctgggc atacactatc ttggggaaaa 900
ggtcagaaag acctcctgac tccatgttac agtagacaaa acaagttgga ctcttttgga 960
cctataaacc ctactctgaa tacaacatac agcttcctta ctacattttt caaagaaatt 1020
agtgaggtgt ttccagatca attcattcat ttgggaggag atgaagtgga atttaaatgt 1080
tgggaatcaa atccaaaaat tcaagatttc atgaggcaaa aaggctttgg cacagatttt 1140
aagaaactag aatctttcta cattcaaaag gttttggata ttattgcaac cataaacaag 1200
ggatccattg tctggcagga ggtttttgat gataaagcaa agcttgcgcc gggcacaata 1260
gttgaagtat ggaaagacag cgcatatcct gaggaactca gtagagtcac agcatctggc 1320

ttccctgtaa tcctttctgc tccttggtac ttagatttga ttagctatgg acaagattgg 1380
aggaaatact ataaagtgga acctcttgat tttggcggta ctcagaaaca gaaacaactt 1440
ttcattggtg gagaagcttg tctatgggga gaatatgtgg atgcaactaa cctcactcca 1500
agattatggc ctcgggcaag tgctgttggt gagagactct ggagttccaa agatgtcaga 1560
gatatggatg acgcctatga cagactgaca aggcaccgct gcaggatggt cgaacgtgga 1620
atagctgcac aacctcttta tgctggatat tgtaaccatg agaacatgta a 1671

<210> 105
<211> 1290
<212> DNA
<213> Homo sapiens

<400> 105
atgcagctga ggaacccaga actacatctg ggctgcgcgc ttgcgcttcg cttcctggcc 60
ctcgtttcct gggacatccc tggggctaga gcactggaca atggattggc aaggacgcct 120
accatgggct ggctgcactg ggagcgcttc atgtgcaacc ttgactgcca ggaagagcca 180
gattcctgca tcagtgagaa gctcttcatg gagatggcag agctcatggt ctcagaaggc 240
tggaaggatg caggttatga gtacctctgc attgatgact gttggatggc tccccaaaga 300
gattcagaag gcagacttca ggcagaccct cagcgctttc ctcatgggat tcgccagcta 360
gctaattatg ttcacagcaa aggactgaag ctagggattt atgcagatgt tggaaataaa 420
acctgcgcag gcttccctgg gagttttgga tactacgaca ttgatgccca gacctttgct 480
gactggggag tagatctgct aaaatttgat ggttgttact gtgacagttt ggaaaatttg 540
gcagatggtt ataagcacat gtccttggcc ctgaatagga ctggcagaag cattgtgtac 600
tcctgtgagt ggcctcttta tatgtggccc tttcaaaagc ccaattatac agaaatccga 660
cagtactgca atcactggcg aaattttgct gacattgatg attcctggaa aagtataaag 720
agtatcttgg actggacatc ttttaaccag gagagaattg ttgatgttgc tggaccaggg 780
ggttggaatg acccagatat gttagtgatt ggcaactttg gcctcagctg gaatcagcaa 840
gtaactcaga tggccctctg ggctatcatg gctgctcctt tattcatgtc taatgacctc 900
cgacacatca gccctcaagc caaagctctc cttcaggata aggacgtaat tgccatcaat 960
caggacccct tgggcaagca agggtaccag cttagacagg gagacaactt tgaagtgtgg 1020
gaacgacctc tctcaggctt agcctgggct gtagctatga taaaccggca ggagattggt 1080
ggacctcgct cttataccat cgcagttgct tccctgggta aaggagtggc ctgtaatcct 1140
gcctgcttca tcacacagct cctccctgtg aaaaggaagc tagggttcta tgaatggact 1200
tcaaggttaa gaagtcacat aaatcccaca ggcactgttt tgcttcagct agaaaataca 1260
atgcagatgt cattaaaaga cttactttaa 1290

<210> 106
<211> 1611
<212> DNA
<213> Homo sapiens

<400> 106
atggagtttt caagtccttc cagagaggaa tgtcccaagc ctttgagtag ggtaagcatc 60
atggctggca gcctcacagg attgcttcta cttcaggcag tgtcgtgggc atcaggtgcc 120
cgcccctgca tccctaaaag cttcggctac agctcggtgg tgtgtgtctg caatgccaca 180
tactgtgact cctttgaccc cccgaccttt cctgcccttg gtaccttcag ccgctatgag 240
agtacacgca gtgggcgacg gatggagctg agtatggggc ccatccaggc taatcacacg 300
ggcacaggcc tgctactgac cctgcagcca gaacagaagt tccagaaagt gaagggattt 360
ggaggggcca tgacagatgc tgctgctctc aacatccttg ccctgtcacc ccctgcccaa 420
aatttgctac ttaaatcgta cttctctgaa gaaggaatcg gatataacat catccgggta 480
cccatggcca gctgtgactt ctccatccgc acctacacct atgcagacac ccctgatgat 540
ttccagttgc acaacttcag cctcccagag gaagatacca agctcaagat accccctgatt 600
caccgagccc tgcagttggc ccagcgtccc gtttcactcc ttgccagccc ctggacatca 660
cccacttggc tcaagaccaa tggagcggtg aatgggaagg ggtcactcaa gggacagccc 720
ggagacatct accaccagac ctgggccaga tactttgtga agttcctgga tgcctatgct 780
gagcacaagt tacagttctg ggcagtgaca gctgaaaatg agccttctgc tgggctgttg 840

```
agtggatacc ccttccagtg cctgggcttc acccctgaac atcagcgaga cttcattgcc 900
cgtgacctag gtcctaccct cgccaacagt actcaccaca atgtccgcct actcatgctg 960
gatgaccaac gcttgctgct gccccactgg gcaaaggtgg tactgacaga cccagaagca 1020
gctaaatatg ttcatggcat tgctgtacat tggtacctgg actttctggc tccagccaaa 1080
gccaccctag gggagacaca ccgcctgttc cccaacacca tgctctttgc ctcagaggcc 1140
tgtgtgggct ccaagttctg ggagcagagt gtgcggctag gctcctggga tcgagggatg 1200
cagtacagcc acagcatcat cacgaacctc ctgtaccatg tggtcggctg gaccgactgg 1260
aaccttgccc tgaaccccga aggaggaccc aattgggtgc gtaactttgt cgacagtccc 1320
atcattgtag acatcaccaa ggacacgttt tacaaacagc ccatgttcta ccaccttggc 1380
cacttcagca agttcattcc tgagggctcc cagagagtgg ggctggttgc cagtcagaag 1440
aacgacctgg acgcagtggc actgatgcat cccgatggct ctgctgttgt ggtcgtgcta 1500
aaccgctcct ctaaggatgt gcctcttacc atcaaggatc ctgctgtggg cttcctggag 1560
acaatctcac ctggctactc cattcacacc tacctgtggc gtcgccagtg a          1611
```

<210> 107
<211> 642
<212> DNA
<213> Homo sapiens

<400> 107

```
atgccccgga gggcggagaa ctgggacgag gccgaggtag gcgcggagga ggcaggcgtc 60
gaagagtacg gccctgaaga agacggcggg gaggagtcgg gcgccgagga gtccggcccg 120
gaagagtccg gcccggagga actgggcgcc gaggaggaga tggaggccgg gcggccgcgg 180
cccgtgctgc gctcggtgaa ctcgcgcgag ccctcccagg tcatcttctg caatcgcagt 240
ccgcgcgtcg tgctgcccgt atggctcaac ttcgacggcg agccgcagcc ctacccaacg 300
ctgccgcctg gcacgggccg ccgcatccac agctaccgag gtcacctttg gctcttcaga 360
gatgcaggga cacacgatgg gcttctggtt aaccaaactg aattatttgt gccatctctc 420
aatgttgacg gacagcctat ttttgccaat atcacactgc cagtgtatac tctgaaagag 480
cgatgcctcc aggttgtccg gagcctagtc aagcctgaga attacaggag actggacatc 540
gtcaggtcgc tctacgaaga tctggaagac cacccaaatg tgcagaaaga cctggagcgg 600
ctgacacagg agcgcattgc acatcaacgg atgggagatt ga                    642
```

<210> 108
<211> 444
<212> DNA
<213> Homo sapiens

<400> 108

```
atggtgcatc tgactcctga ggagaagtct gccgttactg ccctgtgggg caaggtgaac 60
gtggatgaag ttggtggtga ggccctgggc aggctgctgg tggtctaccc ttggacccag 120
aggttctttg agtcctttgg ggatctgtcc actcctgatg ctgttatggg caaccctaag 180
gtgaaggctc atggcaagaa agtgctcggt gcctttagtg atggcctggc tcacctggac 240
aacctcaagg gcacctttgc cacactgagt gagctgcact gtgacaagct gcacgtggat 300
cctgagaact tcaggctcct gggcaacgtg ctggtctgtg tgctggccca tcactttggc 360
aaagaattca ccccaccagt gcaggctgcc tatcagaaag tggtggctgg tgtggctaat 420
gccctggccc acaagtatca ctaa                                        444
```

<210> 109
<211> 951
<212> DNA
<213> Homo sapiens

<400> 109

```
atgatagtgt ttgtcaggtt caactccagc catggtttcc cagtggaggt cgattctgac 60
accagcatct tccagctcaa ggaggtggtt gctaagcgac aggggggttcc ggctgaccag 120
```

```
ttgcgtgtga ttttcgcagg gaaggagctg aggaatgact ggactgtgca ggaatttttc 180
tttaaatgtg gagcacaccc cacctctgac aaggaaacat cagtagcttt gcacctgatc 240
gcaacaaata gtcggaacat cacttgcatt acgtgcacag acgtcaggag ccccgtcctg 300
gttttccagt gcaactcccg ccacgtgatt tgcttagact gtttccactt atactgtgtg 360
acaagactca atgatcggca gtttgttcac gaccctcaac ttggctactc cctgccttgt 420
gtggctggct gtcccaactc cttgattaaa gagctccatc acttcaggat tctgggagaa 480
gagcagtaca accggtacca gcagtatggt gcagaggagt gtgtcctgca gatgggggc 540
gtgttatgcc cccgccctgg ctgtggagcg gggctgctgc cggagcctga ccagaggaaa 600
gtcacctgcg aagggggcaa tggcctgggc tgtgggtttg ccttctgccg ggaatgtaaa 660
gaagcgtacc atgaagggga gtgcagtgcc gtatttgaag cctcaggaac aactactcag 720
gcctacagag tcgatgaaag agccgccgag caggctcgtt gggaagcagc ctccaaagaa 780
accatcaaga aaaccaccaa gccctgtccc cgctgccatg taccagtgga aaaaaatgga 840
ggctgcatgc acatgaagtg tccgcagccc cagtgcaggc tcgagtggtg ctggaactgt 900
ggctgcgagt ggaaccgcgt ctgcatgggg gaccactggt tcgacgtgta g          951
```